(19) Europäisches Patentamt
European Patent Office
Office européen des brevets

(11) Veröffentlichungsnummer: **0 537 463 A2**

# (12) EUROPÄISCHE PATENTANMELDUNG

(21) Anmeldenummer: **92114978.7**

(22) Anmeldetag: **02.09.92**

(51) Int. Cl.5: **A01N 25/00**, C07D 471/04, //(C07D471/04,239:00,221:00)

(30) Priorität: **18.09.91 DE 4131029**

(43) Veröffentlichungstag der Anmeldung: **21.04.93 Patentblatt 93/16**

(84) Benannte Vertragsstaaten: **AT BE CH DE DK FR GB IT LI NL**

(71) Anmelder: **BASF Aktiengesellschaft**
**Carl-Bosch-Strasse 38**
**W-6700 Ludwigshafen(DE)**

(72) Erfinder: **Bratz, Matthias, Dr.**
**Schwabsgasse 2**
**W-6720 Speyer(DE)**
Erfinder: **Kober, Reiner, Dr.**
**Im Schlittweg 20**
**W-6701 Fussgoenheim(DE)**
Erfinder: **Seele, Rainer, Dr.**
**Sonnenbergstrasse 1**
**W-6701 Ellerstadt(DE)**
Erfinder: **Saupe, Thomas, Dr.**
**Kressenwiesenweg 13**
**W-6902 Sandhausen(DE)**
Erfinder: **Meyer, Norbert, Dr.**
**Dossenheimer Weg 22**
**W-6802 Ladenburg(DE)**
Erfinder: **Walker, Nigel, Dr.**
**Frauenpfad 20**
**W-6915 Dossenheim(DE)**
Erfinder: **Landes, Andreas, Dr.**
**Untere Hart 12**
**W-6703 Limburgerhof(DE)**
Erfinder: **Walter, Helmut, Dr.**
**Gruenstadter Strasse 82**
**W-6719 Obrigheim(DE)**

## (54) Substituierte Pyrido(2,3-d)pyrimidine als Antidots.

(57) Herbizide Mittel, enthaltend mindestens ein substituiertes Pyrido[2,3-d]pyrimidin I

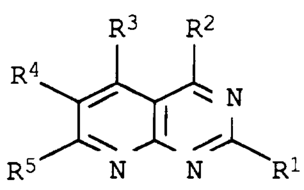

I

$R^1$, $R^2$
Wasserstoff; ggf. subst. Alkyl; Alkoxy; Halogenalkoxy; Alkylamino; Alkenyl; Alkinyl; ggf. subst. Cycloalkyl; ggf. subst. Aryl oder Heteroaryl;
$R^3$
Hydroxy; ggf. subst. Amino; Halogen; Alkylthio; Alkoxycarbonyl; oder ein Rest $R^1$;
$R^4$
ein Rest $R^1$; CN; $NO_2$; COOH; CSOH; $SO_2$-$R^6$; C(=X)-$R^7$; C(=Y)-$R^8$, oder $R^7$-C($YR^9$)-$ZR^{10}$;
$R^5$
ein Rest $R^1$; Hydroxy; ggf. subst. Amino; Halogen; Alkylthio; Pyrrolidin-1-yl; Piperidin-1-yl; Morpholin-1-yl; ggf. subst. Alkylcarbonyloxy; ggf. subst. Alkylsulfonyloxy;
ggf. subst. Aryloxy, Arylamino, Benzyloxy, Benzylamino, Aroyloxy oder Phenylsulfonyloxy; N($R^{12}$)-$SO_2$-$NR^{13}$; N-($R^{12}$)-CO-$R^{14}$; N($R^{12}$)-CS-$R^{14}$;

EP 0 537 463 A2

sowie die pflanzenverträglichen Salze derjenigen Verbindungen I, bei denen mindestens einer der Substituenten $R^1$ bis $R^5$ eine saure oder basische Gruppe bedeutet,
und mindestens einen herbiziden Wirkstoff aus

A) der Gruppe der Cyclohexenon-Derivate II,

oder

B) der Gruppe der 2-(4-Heteroaryloxy)- oder 2-(4-Aryloxy)-phenoxycarbonsäurederivate III.

Die vorliegende Erfindung betrifft herbizide Mittel, enthaltend mindestens ein antagonistisch wirksames substituiertes Pyrido[2,3-d]pyrimidin der allgemeinen Formel I

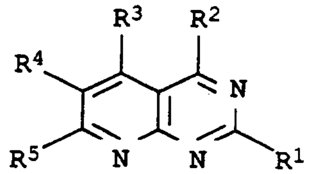

I

in der die Variablen folgende Bedeutung haben:

$R^1$, $R^2$

Wasserstoff; $C_1$-$C_8$-Alkyl; $C_1$-$C_8$-Halogenalkyl; $C_1$-$C_6$-Alkoxy; $C_1$-$C_6$-Halogenalkoxy; $C_1$-$C_4$-Alkoxy-$C_1$-$C_6$-alkyl; $C_1$-$C_8$-Alkylamino; $C_2$-$C_8$-Alkenyl; $C_2$-$C_8$-Alkinyl;

$C_3$-$C_8$-Cycloalkyl, an welches ein Benzolrest anneliert sein kann, wobei diese Gruppe noch ein bis drei der folgenden Reste tragen kann: Hydroxy, Halogen, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

Phenyl, Naphthyl, Phenyl-$C_1$-$C_6$-alkyl, 5-gliedrige aromatische Ringe, welche neben Kohlenstoffatomen ein bis drei Stickstoffatome und ein Sauerstoff- oder ein Schwefelatom als Heteroatome enthalten können, oder welche neben Kohlenstoffatomen ein bis drei Stickstoffatome oder ein Sauerstoff- oder ein Schwefelatom als Heteroatome enthalten können, 6-gliedrige aromatische Ringe, welche neben Kohlenstoffatomen ein bis drei Stickstoffatome als Heteroatome enthalten können, wobei an die vorstehend genannten 5- und 6-gliedrigen Heteroaromaten ein Benzolring anneliert sein kann, und wobei die aromatischen und heteroaromatischen Reste zusätzlich ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_3$-$C_6$-Alkenyl und $C_3$-$C_6$-Alkinyl;

$R^3$

Hydroxy; Amino; Halogen; $C_1$-$C_6$-Alkylthio; Di-($C_1$-$C_8$-alkyl)-amino; $C_3$-$C_8$-Cycloalkylamino; $C_1$-$C_8$-Alkoxycarbonyl; oder eine der für $R^1$ genannten Gruppen;

$R^4$

eine der für $R^1$ genannten Gruppen;

CN; $NO_2$; COOH; CSOH; Di-($C_1$-$C_4$-alkyl)-amino-$C_1$-$C_4$-alkyl;

$SO_2$-$R^6$; C(=X)-$R^7$; C(=Y)-$R^8$, oder $R^7$-C(Y$R^9$)-Z$R^{10}$;

| | |
|---|---|
| $R^6$ | eine der für $R^1$ genannten Gruppen; Hydroxy; Amino; Di-($C_1$-$C_8$-alkyl)-amino; $C_3$-$C_8$-Cycloalkylamino; $C_1$-$C_6$-Alkylthio; |
| $R^7$ | Amino; Oxyamino (-NH-OH); $C_1$-$C_8$-Alkylamino; Di-($C_1$-$C_8$-alkyl)-amino; $C_3$-$C_8$-Cycloalkylamino; $C_1$-$C_8$-Alkoxy; $C_1$-$C_6$-Alkylthio; Phenylamino; |
| $R^8$ | eine der für $R^1$ genannten Gruppen; |
| $R^9$,$R^{10}$ | $C_1$-$C_8$-Alkyl; $C_1$-$C_6$-Halogenalkyl; $C_1$-$C_4$-Alkoxy-$C_2$-$C_6$-alkyl; $C_2$-$C_8$-Alkenyl; oder |
| $R^9$ und $R^{10}$ | gemeinsam $-CH_2CH_2-$, $-CH_2CH_2CH_2-$ oder $-CH_2CH_2CH_2CH_2-$, wobei ein oder zwei Wasserstoffatome in diesen Gruppen durch die folgenden Reste ersetzt sein können: =O, $C_1$-$C_8$-Alkyl, $C_1$-$C_6$-Halogenalkyl oder $C_1$-$C_6$-Alkoxy; |
| X | Sauerstoff, Schwefel oder $NR^{11}$, worin |
| $R^{11}$ | für eine der für $R^1$ genannten Gruppen steht, oder die folgende Bedeutung hat: Wasserstoff; Hydroxy; Amino; Di-($C_1$-$C_8$-alkyl)-amino; $C_3$-$C_8$-Cycloalkylamino; Phenoxy, Naphthyloxy, Phenylamino oder Naphthylamino, wobei die aromatischen Reste ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_3$-$C_6$-Alkenyl und $C_3$-$C_6$-Alkinyl; |
| Y | Sauerstoff oder Schwefel; |

$R^5$

eine der für $R^1$ genannten Gruppen;

Hydroxy; Amino; Halogen; $C_1$-$C_6$-Alkylthio; Di-($C_1$-$C_8$-alkyl)-amino; $C_3$-$C_8$-Cycloalkylamino; Pyrrolidin-1-yl; Piperidin-1-yl; Morpholin-1-yl; $C_1$-$C_8$-Alkylcarbonyloxy; $C_1$-$C_4$-Halogenalkylcarbonyloxy; $C_1$-$C_8$-Alkylsulfonyloxy; $C_1$-$C_8$-Halogenalkylsulfonyloxy;

Phenoxy, Naphthyloxy, Phenylamino, Naphthylamino, Benzyloxy, Benzylamino, Benzoyloxy, 2-Naphthoyloxy oder Phenylsulfonyloxy, wobei die aromatischen Reste ein bis drei der folgenden Gruppen tragen

können: Halogen, $C_1$-$C_4$-Alkyl, $C_1$-$C_4$-Halogenalkyl und $C_1$-$C_4$-Alkoxy;
$N(R^{12})$-$SO_2$-$R^{13}$; $N(R^{12})$-CO-$R^{14}$; $N(R^{12})$-CS-$R^{14}$;

$R^{12}$ Wasserstoff; $C_1$-$C_4$-Alkyl; Phenyl, welches ein bis drei der folgenden Reste tragen kann: Halogen, $C_1$-$C_4$-Alkyl, $C_1$-$C_4$-Halogenalkyl und $C_1$-$C_4$-Alkoxy;

$R^{13}$ eine der für $R^1$ genannten Gruppen; Amino, Di-($C_1$-$C_8$-alkyl)-amino oder $C_3$-$C_8$-Cycloalkylamino;

$R^{14}$ eine der für $R^1$ genannten Gruppen;
Amino; Oxyamino (-NH-OH); Di-($C_1$-$C_6$-alkyl)-amino; $C_3$-$C_8$-Cycloalkylamino;

sowie die pflanzenverträglichen Salze derjenigen Verbindungen I, bei denen mindestens einer der Substituenten $R^1$ bis $R^5$ eine saure oder basische Gruppe bedeutet,
und mindestens einen herbiziden Wirkstoff aus
A) der Gruppe der Cyclohexenon-Derivate der allgemeinen Formel II,

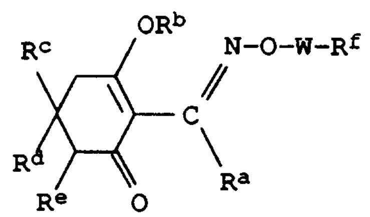

II

in der die Substituenten die folgende Bedeutung haben:
$R^a$
$C_1$-$C_6$-Alkyl;
$R^b$
Wasserstoff;
das Äquivalent eines landwirtschaftlich brauchbaren Kations;
$C_1$-$C_8$-Alkylcarbonyl; $C_1$-$C_{10}$-Alkylsulfonyl; $C_1$-$C_{10}$-Alkylphosphonyl;
Benzoyl, Benzolsulfonyl oder Benzolphosphonyl, wobei die aromatischen Ringe ein bis fünf Halogenatome tragen können;
$R^c$
Wasserstoff; CN; CHO;
$C_1$-$C_6$-Alkyl, welches einen der folgenden Reste tragen kann: $C_1$-$C_4$-Alkoxy, $C_1$-$C_4$-Alkylthio, Phenyloxy, Phenylthio, Pyridyloxy oder Pyridylthio, wobei die aromatischen Reste ihrerseits ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_3$-$C_6$-Alkenyl, $C_3$-$C_6$-Alkenyloxy, $C_3$-$C_6$-Alkinyl, $C_3$-$C_6$-Alkinyloxy oder $NR^gR^h$;

$R^g$ Wasserstoff; $C_1$-$C_4$-Alkyl; $C_3$-$C_6$-Alkenyl; $C_3$-$C_6$-Alkinyl; $C_1$-$C_6$-Alkylcarbonyl; Benzoyl, welches ein bis drei der folgenden Reste tragen kann: Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio;

$R^h$ Wasserstoff; $C_1$-$C_4$-Alkyl; $C_3$-$C_6$-Alkenyl; $C_3$-$C_6$-Alkinyl;

$R^c$ bedeutet desweiteren:

$C_3$-$C_7$-Cycloalkyl oder $C_5$-$C_7$-Cycloalkenyl, wobei diese Ringe ein bis drei der folgenden Reste tragen können: Hydroxy, Halogen, $C_1$-$C_4$-Alkyl, $C_1$-$C_4$-Halogenalkyl, $C_1$-$C_4$-Alkoxy, $C_1$-$C_4$-Alkylthio, Benzylthio, $C_1$-$C_4$-Alkylsulfonyl, $C_1$-$C_4$-Alkylsulfenyl und $C_1$-$C_4$-Alkylsulfinyl;
5-gliedrige gesättigte Ringe, welche neben Kohlenstoffringgliedern ein oder zwei Sauerstoff- oder Schwefelatome oder ein Sauerstoff- und ein Schwefelatom enthalten, wobei diese Ringe ein bis drei der folgenden Reste tragen können: $C_1$-$C_4$-Alkyl, $C_1$-$C_4$-Halogenalkyl, $C_1$-$C_4$-Alkoxy und $C_1$-$C_4$-Alkylthio;
6- oder 7-gliedrige gesättigte oder ein- oder zweifach ungesättigte Ringe, welche neben Kohlenstoffringgliedern ein oder zwei Sauerstoff- oder Schwefelatome oder oder ein Sauerstoff- und ein Schwefelatom enthalten, wobei diese Ringe ein bis drei der folgenden Reste tragen können: Hydroxy, Halogen, $C_1$-$C_4$-Alkyl, $C_1$-$C_4$-Halogenalkyl, $C_1$-$C_4$-Alkoxy und $C_1$-$C_4$-Alkylthio;
5-gliedrige aromatische Ringe, welche neben Kohlenstoffringgliedern ein oder zwei Stickstoffatome und ein Sauerstoff- oder Schwefelatom oder ein bis drei Stickstoffatome oder ein Sauerstoff- oder ein Schwefelatom enthalten, wobei diese Ringe ein bis drei der folgenden Reste tragen können: Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_2$-$C_6$-Alkenyl, $C_2$-$C_6$-Alkenyloxy, $C_2$-$C_6$-Alkinyl, $C_2$-$C_6$-Alkinyloxy und $C_1$-$C_4$-Alkoxy-$C_1$-$C_4$-alkyl;
Phenyl oder Pyridyl, wobei diese Ringe ein bis drei der folgenden Reste tragen können: Nitro, Formyl,

Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$- Alkylthio, $C_2$-$C_6$-Alkenyl, $C_2$-$C_6$-Alkenyloxy, $C_3$-$C_6$-Alkinyl, $C_3$-$C_6$-Alkinyloxy und $NR^kR^l$;

$R^k$ Wasserstoff; $C_1$-$C_4$-Alkyl; $C_3$-$C_6$-Alkenyl; $C_3$-$C_6$-Alkinyl;

$R^l$ Wasserstoff; $C_1$-$C_4$-Alkyl; $C_3$-$C_6$-Alkenyl; $C_3$-$C_6$-Alkinyl; $C_1$-$C_6$-Alkylcarbonyl; Benzoyl, welches ein bis drei der folgenden Reste tragen kann: Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio;

$R^d$

Wasserstoff; Hydroxy;

oder, sofern $R^c$ für $C_1$-$C_6$-Alkyl steht, ebenfalls $C_1$-$C_6$-Alkyl;

$R^e$

Wasserstoff; Cyano; Halogen; $C_1$-$C_4$-Alkoxycarbonyl;

$C_1$-$C_4$-Alkylketoxim;

W

$C_1$-$C_6$-Alkylen, $C_3$-$C_6$-Alkenylen oder Alkinylen, wobei diese Gruppen $X^1$ eine Methylengruppe (= $CH_2$) und/oder ein bis drei der folgenden Reste tragen können: Halogen und $C_1$-$C_3$-Alkyl;

$C_3$-$C_6$-Alkylen oder $C_3$-$C_6$-Alkenylen, wobei in diesen Resten jeweils eine Methylengruppe durch Sauerstoff, Schwefel, SO, $SO_2$ oder $NR^i$ ersetzt ist, und wobei in diesen Gruppen ein bis drei Wasserstoffatome durch $C_1$-$C_3$-Alkylreste ersetzt sein können;

$R^i$ Wasserstoff; $C_1$-$C_4$-Alkyl; $C_3$-$C_6$-Alkenyl; $C_3$-$C_6$-Alkinyl;

$R^f$

Wasserstoff; $CH=CH$-$Z^1$, worin

$Z^1$ Wasserstoff; Cyano; Carboxyl; Halogen; $C_1$-$C_4$-Alkyl; $C_1$-$C_4$-Halogenalkyl; $C_1$-$C_4$-Alkoxy; $C_1$-$C_8$-Alkoxycarbonyl; Benzyloxycarbonyl;

$C_3$-$C_6$-Cycloalkyl, welches seinerseits ein bis drei der folgenden Reste tragen kann: Hydroxy, Halogen, $C_1$-$C_4$-Alkyl, $C_1$-$C_4$-Halogenalkyl und $C_1$-$C_4$-Alkoxy;

Phenyl, Halogenphenyl, Dihalogenphenyl, Thienyl oder Pyridyl, wobei dieser Reste ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_3$-$C_6$-Cycloalkyl, wobei der cyclische Rest seinerseits noch ein bis drei der folgenden Gruppen tragen kann: Halogen, $C_1$-$c_4$-Alkyl, $C_1$-$C_4$-Halogenalkyl und $C_1$-$C_4$-Alkoxy, bedeutet;

$R^f$ bedeutet ferner

Ethinyl, welches einen der folgenden Reste tragen kann: $C_1$-$C_4$-Alkyl oder $C_3$-$C_6$-Cyckloalkyl, wobei diese Gruppen desweiteren ein bis drei der folgenden Reste tragen können: Hydroxy, Halogen, $C_1$-$C_4$-Alkyl, $C_1$-$C_4$-Halogenalkyl und $C_1$-$C_4$-Alkoxy;

Ethinyl, welches einen der folgenden Reste trägt: Phenyl, Thienyl oder Pyridyl, wobei die aromatischen Reste ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

Phenyl, Halogenphenyl, Dihalogenphenyl, 5-gliedrige romatische Ringe, welche neben Kohlenstoffringgliedern ein oder zwei Stickstoffatome und ein Sauerstoff- oder Schwefelatom oder ein bis drei Stickstoffatome oder ein Sauerstoff- oder ein Schwefelatom enthalten, oder 6-gliedrige aromatische Ringe, welche neben Kohlenstoffringgliedern ein bis vier Stickstoffatome enthalten, wobei diese aromatischen und heteroaromatischen Gruppen ein bis drei der folgenden Reste tragen können: Nitro, $C_1$-$C_4$-Alkoxy, $C_1$-$C_4$-Halogenalkoxy, $C_1$-$C_4$-Alkylthio, $C_1$-$C_4$-Halogenalkylthio, die bei $Z^1$ genannten oder

B) der Gruppe der 2-(4-Heteroaryloxy)- oder 2-(4-Aryloxy)-phenoxycarbonsäurederivate der Formel III

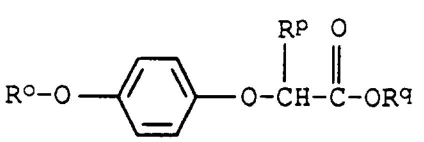

III

in der die Substituenten die folgende Bedeutung haben:

$R^o$

Phenyl, Pyridyl, Benzoxazolyl, Benzthiazolyl oder Benzpyrazinyl, wobei diese aromatischen und heteroaromatischen Ringsysteme ein oder zwei der folgenden Reste tragen können: Nitro, Halogen, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

$R^p$

Wasserstoff oder Methyl;

$R^q$

Wasserstoff; $C_1$-$C_4$-Alkyl; $C_3$-$C_4$-Alkenyl; $C_3$-$C_4$-Alkinyl; $C_1$-$C_4$-Alkoxy-$C_1$-$C_4$-alkyl; $C_3$-$C_4$-Alkylideniminooxy-$C_2$-$C_3$-alkyl; Tetrahydrofuranylmethyl; Isoxazolidinyl;

oder das Äquivalent eines landwirtschaftlich brauchbaren Kations.

Außerdem betrifft die Erfindung Verfahren zur selektiven Bekämpfung von unerwünschtem Pflanzenwuchs auf Anbauflächen von Kulturpflanzen mit diesen herbiziden Mitteln sowie neue Pyrido[2,3-d]-pyrimidine I'.

Substituierte Pyrido[2,3-d]pyrimidine vom Typ der Verbindungen I sind sind bereits aus folgenden Druckschriften bekannt:

- W.J. Irwin et al., J. Chem. Soc. (C), 1745, (1967);
- Shinsaku Minami et al., Chem. Pharm. Bull. 19, 1483 (1971) [R3 = Hydroxyl];
- Sadao Nishigaki et al., Chem. Pharm. Bull. 18, 1385 (1970);
- Rizkalla et al., J. Org. Chem. 37, 3980 (1972) [R3 = Hydroxyl];
- Evans et al., J. Org. Chem. 40, 1438 (1975);
- Söllhuber-Kretzer et al., Arch. Pharm. 316, 346 (1983);
- Nishino et al., Bull chem. Soc. Jpn. 45, 1127 (1972);
- Bredereck et al., Chem. Ber. 96, 1868 (1963);
- Bennett et al., J. Med. Chem. 24, 382 (1981);
- EP-A 329 012;
- EP-A 18 151 [6-Aryl-7-amino-pyrido[2,3-d]pyrimidine als blutdrucksenkende Mittel];

Eine antidotische oder antagonistische Wirkung der bekannten Verbindungen in Kombination mit herbiziden Wirkstoffen ist den genannten Druckschriften jedoch nicht zu entnehmen.

Aufgabe der vorliegenden Erfindung war es, herbizide Mittel bereitzustellen, die eine gute Bekämpfung unerwünschter Pflanzen gewährleisten, ohne jedoch die Nutzpflanzen nennenswert zu schädigen oder deren Ernteertrag wesentlich herabzusetzen.

Gemäß diese Aufgabe wurden die Eingangs definierten herbiziden Mittel gefunden.

Des weiteren wurden Verfahren zur Behandlung von Pflanzenkulturen mit den antagonistisch wirksamen Verbindungen I und den Herbiziden II oder den Herbiziden III gefunden, wobei es unerheblich ist, ob die Verbindungen I und II oder I und III gemeinsam oder getrennt formuliert und ausgebracht werden und in welcher Reihenfolge die Applikation bei getrennter Ausbringung erfolgt.

Die herbiziden Mittel enthalten mindestens eine antagonistisch wirksame Verbindung I und mindestens eine Herbizid II oder ein Herbizid III.

Es können jedoch noch weitere antagonistisch oder herbizid wirksame Verbindungen in den erfindungsgemäßen herbiziden Mitteln enthalten sein.

Substituierte Pyrido[2,3-d]pyrimidine der Formel I'

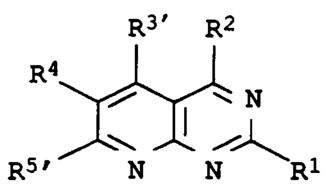

I'

in der die Substituenten folgende Bedeutung haben, sind neu:

in der die Reste $R^1$, $R^2$ und $R^4$ die in vorstehend gegebene Bedeutung haben und $R^{3'}$ und $R^{5'}$ wie folgt definiert sind:

$R^{3'}$

Halogen; $C_1$-$C_6$-Alkylthio; oder eine der für $R^1$ genannten Gruppen;

$R^{5'}$

eine der für $R^1$ genannten Gruppen;

Hydroxy; Halogen; $C_1$-$C_6$-Alkylthio; $C_1$-$C_8$-Alkylcarbonyloxy; $C_1$-$C_8$-Alkylsulfonyloxy; Phenoxy; Benzoyloxy; Phenylsulfonyloxy, wobei der aromatische Rest ein bis drei der folgenden Gruppen tragen kann: Halogen, $C_1$-$C_4$-Alkyl, $C_1$-$C_4$-Halogenalkyl und $C_1$-$C_4$-Alkoxy;

mit der Maßgabe, daß $R^1$ und $R^{3'}$ nicht gleichzeitig Wasserstoff bedeuten, wenn $R^2$ für Wasserstoff oder Phenyl und $R^4$ für Phenyl oder $R^{5'}$ für Phenyl, Halogenphenyl, Naphthyl oder Pyridyl steht, und mit der Maßgabe, daß die Reste $R^2$, $R^{3'}$, $R^4$ und $R^{5'}$ nicht gleichzeitig Wasserstoff bedeuten, wenn $R^1$ für Wasserstoff oder Pyridyl steht,

sowie die pflanzenverträglichen Salze derjenigen Verbindungen I', bei denen mindestens einer der Substituenten $R^1$, $R^2$, $R^{3'}$, $R^4$ und $R^{5'}$ eine saure oder basische Gruppe bedeutet.

Die substituierten Pyrido[2,3-d]pyrimidine I und I' sind auf verschiedene Weise erhältlich, und zwar vorzugsweise nach einem der folgenden Verfahren:

a) Kondensation von 4-Aminopyrimidinen IV mit Methylencarbonyl-Verbindungen V

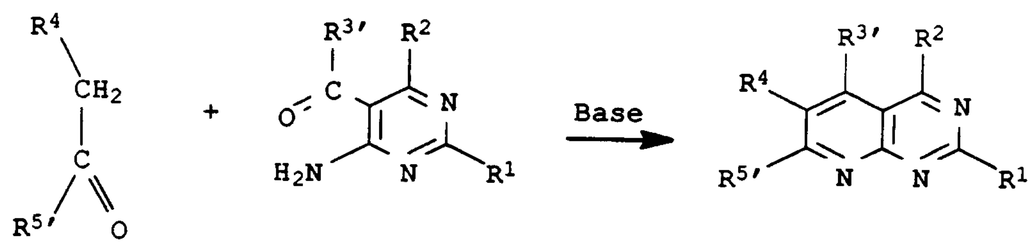

V IV I

Die Umsetzung erfolgt bevorzugt in an sich bekannter Weise (vgl. Caluwe et al. J.Org. Chem. 1981, 40, 1438-1439) in einem inerten Lösungs- oder Verdünnungsmittel, beispielsweise in Wasser, in einem Alkohol wie Methanol, Ethanol, Propanol, Isopropanol und Ethoxyethanol, in flüssigem Ammoniak, in einem Ether wie Tetrahydrofuran oder Dioxan, in einem aromatischen Kohlenwasserstoff wie Benzol, Toluol, Chlorbenzol und Nitrobenzol, in einem polaren aprotischen Lösungsmittel wie Acetonitril, Dimethylformamid, Dimethylsulfoxid und N-Methylpyrrolidon oder in Gemischen der genannten Lösungsmittel.

Vorteilhaft führt man die Umsetzungen in Gegenwart einer organischen oder anorganischen Base durch, wobei z.B. die Hydroxide, Hydride, Alkoxide, Amide, Carbonate und Hydrogencarbonate der Alkali- und Erdalkalimetalle in Betracht kommen. Besonders eignen sich Alkalimetallhydroxide wie Lithiumhydroxid, Natriumhydroxid und Kaliumhydroxid, Erdalkalimetallhydroxdide wie Bariumhydroxid und Calciumhydroxid, Alkalimetallhydride wie Natriumhydrid und Kaliumhydrid, Erdalkalimetallhydride wie Calciumhydrid, Alkalimetallalkoholate wie Natriummethanolat, Natriumethanolat und Kalium-tert.-butylat, Alkalimetallamide wie Natriumamid und Lithium-diisopropylamid, Alkalimetallcarbonate und -hydrogencarbonate wie Natriumbicarbonat, Natriumhydrogencarbonat, Kaliumbicarbonat und Kaliumhydrogencarbonat. Unter den organische Basen sind aliphatische Amine wie Triethylamin, Dimethylamin, Diethylamin, und Diisopropylamin, cycloaliphatische Amine wie Piperidin, Morpholin, Pyrrolidin, DBU und DABCO sowie aromatische Amine wie Pyridin, N,N-Dimethylaminopyridin und Chinolin besonders bevorzugt.

Verwendet man ein Amin als Base, so kann auch lösungsmittelfrei in einem Überschuß der Base gearbeitet werden.

Zweckmäßigerweise setzt man Edukte IV und V in etwa stöchiometrischem Verhältnis ein oder man arbeitet mit einem Überschuß an Methylenverbindung V bis ca. 100 mol-%.

Die Menge an Base ist nicht kritisch. Sie beträgt in der Regel 10-50 mol-%, kann jedoch auch im Überschuß eingesetzt werden.

Bei Verwendung einer organischen Base kann ohne Lösungsmittel in einem Überschuß an Base, bis zur etwa 10fachen molaren Menge, bezogen auf das 4-Aminopyrimidin IV, gearbeitet werden.

Im allgemeinen liegt die Reaktionstemperatur zwischen 0 und 200 °C, bevorzugt zwischen 20 und 150 °C, insbesondere bei etwa 20-30 °C (Raumtemperatur) oder bei der Siedetemperatur des jeweiligen Lösungsmittels.

In der Regel arbeitet man unter Atmosphärendruck oder unter dem Eigendruck des Systems. Höherer oder niedrigerer Druck sind möglich, bringen im allgemeinen aber keine Vorteile.

Die verwendeten 4-Aminopyrimidine IV sind aus der Literatur bekannt oder können analog den dort beschriebenen Verfahren dargestellt werden (vgl. z.B. Benett et al., J. Med. Chem. 24, 381-389 (1981) und die dort zitierte Literatur).

b) Kondensation von 4-Aminopyrimidinen IV mit Acetonitrilen VI und gewünschtenfalls anschließende Derivatisierung der Aminogruppe

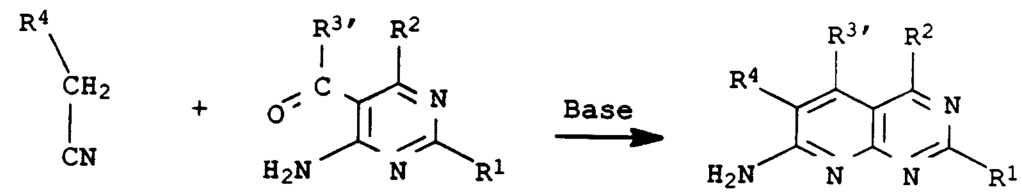


VI IV Ia

Die Umsetzung erfolgt normalerweise nach an sich bekannten Methoden [vgl. z. B. Benett et al., J. Med. Chem. 24, 381-389 (1981)]. Eine nachfolgende Derivatisierung kann z.B. analog den in der EP-A 329 012 beschrieben Methoden erfolgen.

c) Umsetzung von 4-Aminopyridinen IV mit Amiden VII [vgl. Söllhuber-Kretzer in Arch. Pharm. 316, 346-352 (1983)]

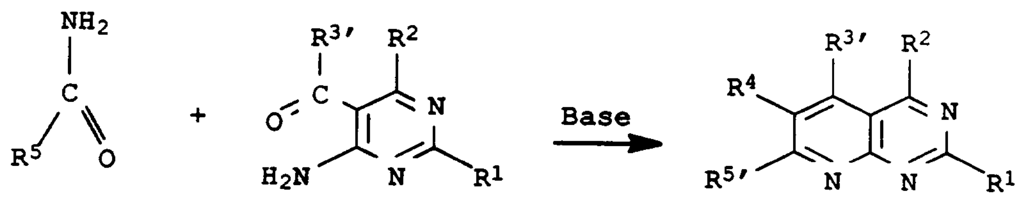


VII IV I

d) Umsetzung von 4-Aminopyrimidinen IV ($R^3$,$R^{3'}$ = $OC_2H_5$) mit CH-aciden Verbindungen VIIIa oder VIIIb nach Art einer Claisen-Kondensation

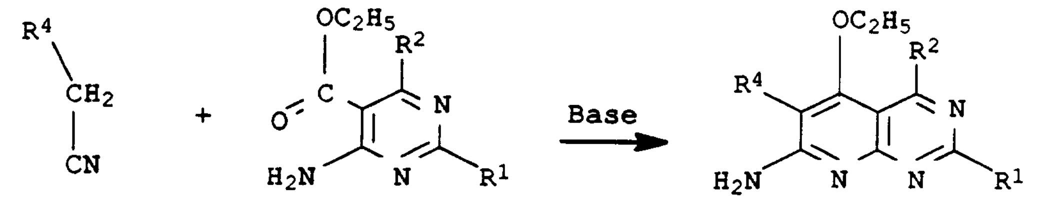


VIIIa IV ($R^3$=$OC_2H_5$) I ($R^3$=$OC_2H_5$; $R^5$=$NH_2$)

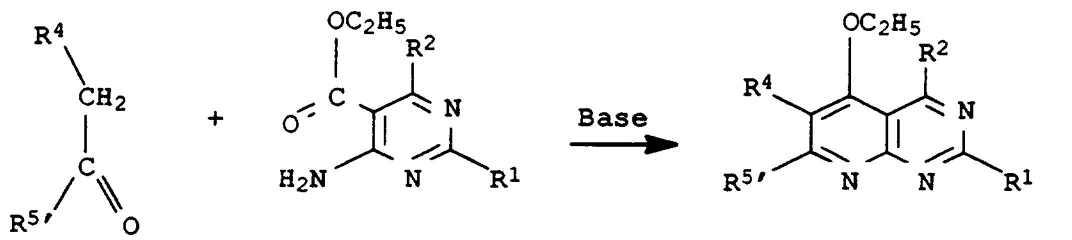


VIIIb IV ($R^3$=$OC_2H_5$) I' ($R^{3'}$=$OC_2H_5$; $R^{5'}$=OH)

$R^5$ bedeutet vorzugsweise Halogen, besonders bevorzugt Chlor oder $C_1$-$C_4$-Alkoxy, insbesondere Ethoxy.

Die Umsetzung erfolgt nach an sich bekannten Methoden [vgl. Bredereck et al., Chem. Ber. 96, 1868-1872 (1963)] in Gegenwart von Natrium oder einem Alkalimetallalkoholat wie Natriummethanolat, Natriumethanolat und Kalium-tert.-butylat.

Bei der Reaktionsführung in Gegenwart von Natrium arbeitet man zweckmäßigerweise ohne Lösungsmittel in einem Überschuß der CH-aciden Verbindung VIIIa oder VIIIb, bis etwa zur 10fachen molaren Menge. Bei der Reaktionsführung in Gegenwart von Alkoholaten empfiehlt es sich als Lösungsmittel den entsprechenden Alkohol zu verwenden, wobei die Edukte IV und VIIIa bzw. VIIIb bevorzugt in etwa stöchiometrischen Mengen eingesetzt werden.

Eine Übersicht über weitere Herstellungsmethoden ist einem Artikel von E. Lunt und C.G. Newton in "Comprehensive Heterocyclic Chemistry" (editors: A. Katritzky und C.W.Rees) Vol. 3, S. 215ff. zu entnehmen. Ferner sei diesbezüglich auf die folgenden Druckschriften verwiesen:

- C.J. Blankley et al., J. Med. Chem. 24, 382-389 (1990),
- M. Söllhuber-Kretzer et al., Arch. d. Pharm. 316, 346-352 (1983),
- P. Caluwe et al., J. Org. Chem. 40, 1438-1439 (1975).

Im Hinblick auf die biologische Wirksamkeit der Verbindungen I als Antidots sind solche Derivate bevorzugt, in denen die Substituenten die folgende Bedeutung haben:

$R^1$, $R^2$

Wasserstoff;

$C_1$-$C_8$-Alkyl, besonders $C_1$-$C_6$-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, vorzugsweise Methyl, Ethyl, Propyl, 1-Methylethyl, Butyl und 1-Methyl-propyl;

$C_1$-$C_8$-Halogenalkyl, besonders $C_1$-$C_4$-Halogenalkyl, insbesondere $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;

$C_1$-$C_6$-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy, insbesondere Chlormethyl, Trichlormethyl und Trifluormethyl;

$C_1$-$C_6$-Halogenalkoxy, besonders $C_1$-$C_4$-Halogenalkoxy, insbesondere $C_1$-$C_2$-Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy, insbesondere Trifluormethoxy;

$C_1$-$C_4$-Alkoxy-$C_1$-$C_6$-alkyl steht für durch $C_1$-$C_4$-Alkoxy wie vorstehend genannt substituiertes $C_1$-$C_6$-Alkyl wie vorstehend genannt, insbesondere Methoxymethyl;

$C_1$-$C_8$-Alkylamino, besonders $C_1$-$C_6$-Alkylamino wie Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methylpentylamino, 4-Methylpentylamino, 1,1-Dimethylbutylamino, 1,2-Dimethylbutylamino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethylpropylamino, 1,2,2-Trimethylpropylamino, 1-Ethyl-1-methylpropylamino und 1-Ethyl-2-methylpropylamino, insbesondere Methylamino und Ethylamino;

$C_2$-$C_8$-Alkenyl, besonders $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl,3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, insbesondere Ethenyl und 2-Propenyl;

$C_2$-$C_8$-Alkinyl, besonders $C_2$-$C_6$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl, insbesondere 2-Propinyl;

$C_3$-$C_8$-Cycloalkyl wie Cyclopropyl, Cyclobutyl, Cyclopentyl, Cyclohexyl, Cycloheptyl und Cyclooctyl, insbesondere Cyclopropyl, Cyclopentyl und Cyclohexyl, an welches ein Benzolrest anneliert sein kann, wobei diese Gruppe noch ein bis drei der folgenden Reste tragen kann: Hydroxy,

- Halogen wie Fluor, Chlor, Brom und Jod, vorzugsweise Fluor und Chlor;
- $C_1$-$C_4$-Alkyl wie Methyl, Ethyl, Propyl, 1-Methylethyl, Butyl, 1-Methylpropyl, 2-Methylpropyl und 1,1-Dimethylethyl, insbesondere Methyl, Ethyl und 1-Methylethyl;
- $C_1$-$C_4$-Halogenalkyl, besonders $C_1$-$C_2$-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl, insbesondere Trifluormethyl;
- $C_1$-$C_4$-Alkoxy wie Methoxy, Ethoxy, Propyloxy, 1-Methylethoxy, Butyloxy, 1-Methyl-propyloxy, 2-Methylpropyloxy und 1,1-Dimethylethoxy, vorzugsweise Methoxy und Ethoxy;

- $C_1$-$C_4$-Halogenalkoxy, besonders $C_1$-$C_2$-Halogenalkoxy wie vorstehend genannt, insbesondere Difluormethoxy;
- $C_1$-$C_4$-Alkylthio wie Methylthio, Ethylthio, Propylthio, 1-Methylethylthio, Butylthio, 1-Methyl-propylthio, 2-Methylpropylthio und 1,1-Dimethylethylthio, vorzugsweise Methylthio und Ethylthio;

Phenyl, Naphthyl, Phenyl-$C_1$-$C_6$-alkyl (welches für durch Phenyl substituiertes $C_1$-$C_6$-Alkyl wie vorstehend genannt steht),

5-gliedrige aromatische Ringe, welche neben Kohlenstoffatomen ein bis drei Stickstoffatome und ein Sauerstoff- oder ein Schwefelatom als Heteroatome enthalten können, oder welche neben Kohlenstoffatomen ein bis drei Stickstoffatome oder ein Sauerstoff- oder ein Schwefelatom als Heteroatome enthalten können, 6-gliedrige aromatische Ringe, welche neben Kohlenstoffatomen ein bis drei Stickstoffatome als Heteroatome enthalten können, wobei an die vorstehend genannten 5- und 6-gliedrigen Heteroaromaten ein Benzolring anneliert sein kann, und wobei die aromatischen und heteroaromatischen Reste zusätzlich ein bis drei der folgenden Gruppen tragen können:

- Nitro, Cyano,
- Halogen wie vorstehend genannt, vorzugsweise Fluor und Chlor;
- $C_1$-$C_4$-Alkyl wie vorstehend genannt, vorzugsweise Methyl, Ethyl und 1-Methylethyl;
- $C_1$-$C_4$-Halogenalkyl, besonders $C_1$-$C_2$-Halogenalkyl wie vorstehend genannt, vorzugsweise Trifluormethyl und Difluormethyl;
- $C_1$-$C_4$-Alkoxy wie vorstehend genannt, vorzugsweise Methoxy, Ethoxy und 1-Methylethoxy;
- $C_1$-$C_4$-Halogenalkoxy, besonders $C_1$-$C_2$-Halogenalkoxy wie vorstehend genannt, vorzugsweise Difluormethoxy und Trifluormethoxy;
- $C_1$-$C_4$-Alkoxycarbonyl wie Methoxycarbonyl, Ethoxycarbonyl, Propyloxycarbonyl, 1-Methylethoxycarbonyl, Butyloxycarbonyl, 1-Methyl-propyloxycarbonyl, 2-Methyl-propyloxycarbonyl und 1,1-Dimethylethoxycarbonyl, vorzugsweise Methoxycarbonyl und Ethoxycarbonyl;
- $C_1$-$C_4$-Alkylthio wie vorstehend genannt, vorzugsweise Methylthio und Ethylthio;
- $C_3$-$C_6$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 3,3-Dimethyl-2-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl und 1-Ethyl-2-methyl-2-propenyl, vorzugsweise 2-Propenyl;
- $C_3$-$C_6$-Alkinyl wie 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl,1-Ethyl-3-butinyl, 2-Ethyl-3-butinyl und 1-Ethyl-1-methyl-2-propinyl, vorzugsweise 2-Propinyl;

$R^3$

Hydroxy; Amino;

Halogen wie vorstehend genannt, vorzugsweise Fluor und Chlor;

$C_1$-$C_6$-Alkylthio wie Methylthio, Ethylthio, Propylthio, 1-Methylethylthio, Butylthio, 1-Methylpropylthio, 2-Methylpropylthio,1,1-Dimethylethylthio, Pentylthio, 1-Methylbutylthio, 2-Methylbutylthio, 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio,3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio und 1-Ethyl-2-methylpropylthio, vorzugsweise $C_1$-$C_4$-Alkylthio, insbesondere Methylthio und Ethylthio;

Di-($C_1$-$C_8$-alkyl)-amino, besonders Di-($C_1$-$C_6$-alkyl)-amino, insbesondere Di-($C_1$-$C_4$-alkyl)-amino wie N,N-Dimethylamino, N,N-Diethylamino, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)- -N-methylamino, N-Ethyl-N-propylamino, N-Ethyl-N-(1-methyl- ethyl)amino, N-Butyl-N-ethylamino, N-Ethyl-N-(1-methyl-

propyl)amino, N-Ethyl-N-(2-methylpropyl)amino, N-Ethyl-N-(1,1-dimethylethyl)amino, N-(1-Methylethyl)- -N-propylamino, N-Butyl-N-propylamino, N-(1-Methylpropyl)- -N-propylamino, N-(2-Methylpropyl)-N-propylamino, N-(1,1-Dimethylethyl)-N-propylamino, N-Butyl-N-(1-methyl- ethyl)amino, N-(1-Methylethyl)-N-(1-methylpropyl)amino, N-(1-Methylethyl)-N-(2-methylpropyl)amino, N-(1,1-Di- methylethyl)-N-(1-methylethyl)-amino, N-Butyl-N-(1-methyl- propyl)amino, N-Butyl-N-(2-methylpropyl)amino, N-Butyl-N- (1,1-dimethylethyl)-amino, N-(1-Methylpropyl)-N-(2-methyl- propyl)amino, N-(1,1-Dimethylethyl)-N-(1-methylpropyl)amino und N-(1,1-Dimethylethyl)-N-(2-methylpropyl)amino;

$C_3$-$C_8$-Cycloalkylamino wie Cyclopropylamino, Cyclobutylamino, Cyclopentylamino, Cyclohexylamino, Cycloheptylamino und Cyclooctylamino, vorzugsweise Cyclopropylamino, Cyclopentylamino und Cyclohexylamino;

$C_1$-$C_8$-Alkoxycarbonyl, besonders $C_1$-$C_6$-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl, 1,1-Dimethylethoxycarbonyl, Pentyloxycarbonyl, 1-Methylbutyloxycarbonyl, 2-Methylbutyloxycarbonyl, 3-Methylbutyloxycarbonyl, 2,2-Dimethylpropyloxycarbonyl, 1-Ethylpropyloxycarbonyl, Hexyloxycarbonyl, 1,1-Dimethyl-propoxycarbonyl, 1,2-Dimethylpropyloxycarbonyl, 1-Methylpentyloxycarbonyl, 2-Methylpentyloxycarbonyl, 3-Methylpentyloxycarbonyl, 4-Methylpentyloxycarbonyl, 1,1-Dimethylbutyloxycarbonyl, 1,2-Dimethylbutyloxycarbonyl, 1,3-Dimethylbutyloxycarbonyl, 2,2-Dimethylbutyloxycarbonyl, 2,3-Dimethylbutyloxycarbonyl, 3,3-Dimethylbutyloxycarbonyl, 1-Ethylbutyloxycarbonyl, 2-Ethylbutyloxycarbonyl, 1,1,2-Trimethylpropyloxycarbonyl, 1,2,2-Trimethylpropyloxycarbonyl, 1-Ethyl-1-methylpropyloxycarbonyl und 1-Ethyl-2-methylpropyloxycarbonyl, vorzugsweise $C_1$-$C_4$-Alkoxycarbonyl;

oder eine der für $R^1$ genannten Gruppen;

$R^4$

eine der für $R^1$ genannten Gruppen;

CN; $NO_2$; COOH; CSOH;

Di-($C_1$-$C_4$-alkyl)-amino-$C_1$-$C_4$-alkyl steht für durch Di-($C_1$-$C_4$-alkyl)-amino wie vorstehend genannt substituiertes $C_1$-$C_4$-Alkyl wie vorstehend genannt;

$SO_2$-$R^6$; C(=X)-$R^7$; C(=Y)-$R^8$, oder $R^7$-C($YR^9$)-$ZR^{10}$;

| | |
|---|---|
| $R^6$ | eine der für $R^1$ genannten Gruppen;<br>Hydroxy; Amino;<br>Di-($C_1$-$C_8$-alkyl)-amino, besonders Di-($C_1$-$C_6$-alkyl)-amino, insbesondere Di-($C_1$-$C_4$-alkyl)-amino wie vorstehend genannt,<br>$C_3$-$C_8$-Cycloalkylamino wie vorstehend genannt, insbesondere Cyclopropylamino, Cyclopentylamino und Cyclohexylamino;<br>$C_1$-$C_6$-Alkylthio wie vorstehend genannt, vorzugsweise $C_1$-$C_4$-Alkylthio, insbesondere $C_1$-$C_2$-Alkylthio; |
| $R^7$ | Amino; Oxyamino (-NH-OH);<br>$C_1$-$C_8$-Alkylamino, besonders $C_1$-$C_6$-Alkylamino wie vorstehend genannt, vorzugsweise $C_1$-$C_4$-Alkylamino, insbesondere $C_1$-$C_2$-Alkylamino;<br>Di-($C_1$-$C_8$-alkyl)-amino, besonders Di-($C_1$-$C_6$-alkyl)-amino, insbesondere Di-($C_1$-$C_4$-alkyl)-amino wie vorstehend genannt;<br>$C_3$-$C_8$-Cycloalkylamino wie vorstehend genannt, vorzugsweise Cyclopropylamino, Cyclopentylamino und Cyclohexylamino;<br>$C_1$-$C_8$-Alkoxy wie vorstehend genannt, vorzugsweise $C_1$-$C_4$-Alkoxy, insbesondere $C_1$-$C_2$-Alkoxy;<br>$C_1$-$C_6$-Alkylthio wie vorstehend genannt, vorzugsweise $C_1$-$C_4$-Alkylthio, insbesondere $C_1$-$C_2$-Alkylthio;<br>Phenylamino; |
| $R^8$ | eine der für $R^1$ genannten Gruppen; |
| $R^9$,$R^{10}$ | $C_1$-$C_8$-Alkyl wie vorstehend genannt;<br>$C_1$-$C_8$-Halogenalkyl wie vorstehend genannt;<br>$C_1$-$C_4$-Alkoxy-$C_2$-$C_6$-alkyl steht für durch $C_1$-$C_4$-Alkoxy wie vorstehend genannt substituiertes $C_2$-$C_6$-Alkyl wie vorstehend genannt, vorzugsweise durch Methoxy oder Ethoxy substituiertes Ethyl, Propyl oder 1-Methylethyl;<br>$C_2$-$C_8$-Alkenyl wie vorstehend genannt; oder |
| $R^9$ und $R^{10}$ | gemeinsam $-CH_2CH_2-$, $-CH_2CH_2CH_2-$ oder $-CH_2CH_2CH_2CH_2-$, wobei ein oder zwei Wasserstoffatome in diesen Gruppen durch die folgenden Reste ersetzt sein können: =O (vicinale H-Atome),<br>- $C_1$-$C_8$-Alkyl wie vorstehend genannt, vorzugsweise Methyl oder Ethyl; |

- $C_1$-$C_6$-Halogenalkyl, besonders $C_1$-$C_4$-Halogenalkyl, insbesondere $C_1$-$C_2$-Halogenalkyl wie vorstehend genannt;
- $C_1$-$C_6$-Alkoxy wie vorstehend genannt, vorzugsweise Methoxy oder Ethoxy;

X Sauerstoff, Schwefel oder $NR^{11}$, worin

$R^{11}$ für eine der für $R^1$ genannten Gruppen steht, oder die folgende Bedeutung hat:
Wasserstoff; Hydroxy; Amino;
Di-($C_1$-$C_8$-alkyl)-amino, besonders Di-($C_1$-$C_6$-alkyl)-amino, insbesondere Di-($C_1$-$C_4$-alkyl)-amino wie vorstehend genannt,
$C_3$-$C_8$-Cycloalkylamino wie vorstehend genannt, vorzugsweise Cyclopropylamino, Cyclopentylamino und Cyclohexylamino;
Phenoxy, Naphthyloxy, Phenylamino oder Naphthylamino, wobei die aromatischen Reste ein bis drei der folgenden Gruppen tragen können: Nitro, Cyano,
- Halogen wie vorstehend genannt, vorzugsweise Fluor und Chlor;
- $C_1$-$C_4$-Alkyl wie vorstehend genannt, vorzugsweise Methyl oder Ethyl;
- $C_1$-$C_4$-Halogenalkyl, besonders $C_1$-$C_2$-Halogenalkyl wie vorstehend genannt, vorzugsweise Trifluormethyl;
- $C_1$-$C_4$-Alkoxy wie vorstehend genannt, vorzugsweise Methoxy;
- $C_1$-$C_4$-Halogenalkoxy, besonders $C_1$-$C_2$-Halogenalkoxy wie vorstehend genannt, vorzugsweise Difluormethoxy;
- $C_1$-$C_4$-Alkoxycarbonyl wie vorstehend genannt, vorzugsweise $C_1$-$C_2$-Alkoxycarbonyl;
- $C_1$-$C_4$-Alkylthio wie vorstehend genannt, vorzugsweise insbesondere Methylthio;
- $C_3$-$C_6$-Alkenyl wie vorstehend genannt, vorzugsweise 2-Propenyl;
- $C_3$-$C_6$-Alkinyl wie vorstehend genannt, vorzugsweise 2-Propinyl;

Y Sauerstoff oder Schwefel;

$R^5$

eine der für $R^1$ genannten Gruppen;
Hydroxy; Amino;
Halogen wie vorstehend genannt, vorzugsweise Fluor und Chlor;
$C_1$-$C_6$-Alkylthio wie vorstehend genannt, vorzugsweise $C_1$-$C_4$-Alkylthio, insbesondere $C_1$-$C_2$-Alkylthio;
Di-($C_1$-$C_8$-alkyl)-amino, besonders Di-($C_1$-$C_6$-alkyl)-amino, insbesondere Di-($C_1$-$C_4$-alkyl)-amino wie vorstehend genannt, vorzugsweise Di-($C_1$-$C_2$-alkyl)-amino;
$C_3$-$C_8$-Cycloalkylamino wie vorstehend genannt, insbesondere Cyclopropylamino, Cyclopentylamino und Cyclohexylamino;
Pyrrolidin-1-yl; Piperidin-1-yl; Morpholin-1-yl;
$C_1$-$C_8$-Alkylcarbonyloxy, besonders $C_1$-$C_6$-Alkylcarbonyloxy wie Methylcarbonyloxy, Ethylcarbonyloxy, Propylcarbonyloxy, 1-Methylethylcarbonyloxy, Butylcarbonyloxy, 1-Methyl-propylcarbonyloxy, 2-Methylpropylcarbonyloxy, 1,1-Dimethylethylcarbonyloxy, Pentylcarbonyloxy, 1-Methylbutylcarbonyloxy, 2-Methylbutylcarbonyloxy, 3-Methylbutylcarbonyloxy, 2,2-Dimethylpropylcarbonyloxy, 1-Ethylpropylcarbonyloxy, Hexylcarbonyloxy, 1,1-Dimethylpropylcarbonyloxy, 1,2-Dimethylpropylcarbonyloxy, 1-Methylpentylcarbonyloxy, 2-Methylpentylcarbonyloxy, 3-Methylpentylcarbonyloxy, 4-Methylpentylcarbonyloxy, 1,1-Dimethylbutylcarbonyloxy, 1,2-Dimethylbutylcarbonyloxy, 1,3-Dimethylbutylcarbonyloxy, 2,2-Dimethylbutylcarbonyloxy, 2,3-Dimethylbutylcarbonyloxy, 3,3-Dimethylbutylcarbonyloxy, 1-Ethylbutylcarbonyloxy, 2-Ethylbutylcarbonyloxy, 1,1,2-Trimethylpropylcarbonyloxy, 1,2,2-Trimethylpropylcarbonyloxy, 1-Ethyl-1-methylpropylcarbonyloxy und 1-Ethyl-2-methylpropylcarbonyloxy, vorzugsweise $C_1$-$C_4$-Alkylcarbonyloxy, insbesondere $C_1$-$C_2$-Alkylcarbonyloxy;
$C_1$-$C_4$-Halogenalkylcarbonyloxy, besonders $C_1$-$C_2$-Halogenalkylcarbonyloxy wie Chlormethylcarbonyloxy, Dichlormethylcarbonyloxy, Trichlormethylcarbonyloxy, Fluormethylcarbonyloxy, Difluormethylcarbonyloxy, Trifluormethylcarbonyloxy, Chlorfluormethylcarbonyloxy, Dichlorfluormethylcarbonyloxy, Chlordifluormethylcarbonyloxy, 1-Fluorethylcarbonyloxy, 2-Fluorethylcarbonyloxy, 2,2-Difluorethylcarbonyloxy, 2,2,2-Trifluorethylcarbonyloxy, 2-Chlor-2-fluorethylcarbonyloxy, 2-Chlor-2,2-difluorethylcarbonyloxy, 2,2-Dichlor-2-fluorethylcarbonyloxy,2,2,2-Trichlorethylcarbonyloxy und Pentafluorethylcarbonyloxy, vorzugsweise Trifluormethylcarbonyloxy;
$C_1$-$C_8$-Alkylsulfonyloxy, besonders $C_1$-$C_6$-Alkylsulfonyloxy wie Methylsulfonyloxy, Ethylsulfonyloxy, Propylsulfonyloxy, 1-Methylethylsulfonyloxy, Butylsulfonyloxy, 1-Methyl-propylsulfonyloxy, 2-Methylpropylsulfonyloxy, 1,1-Dimethylethylsulfonyloxy, Pentylsulfonyloxy, 1-Methylbutylsulfonyloxy, 2-Methylbutylsulfonyloxy, 3-Methylbutylsulfonyloxy, 2,2-Dimethylpropylsulfonyloxy, 1-Ethylpropylsulfonyloxy, Hexylsulfonyloxy, 1,1-Dimethylpropylsulfonyloxy, 1,2-Dimethylpropylsulfonyloxy, 1-Methylpentylsulfonyloxy, 2-Methylpentylsulfonyloxy, 3-Methylpentylsulfonyloxy, 4-Methylpentylsulfonyloxy, 1,1-Dimethylbutylsulfonyloxy, 1,2-Dimethylbutylsulfonyloxy, 1,3-Dimethylbutylsulfonyloxy, 2,2-Dimethylbutylsulfonyloxy, 2,3-Dimethylbutylsulfonyloxy, 3,3-Dimethylbutylsulfonyloxy, 1-Ethylbutylsulfonyloxy, 2-Ethylbutylsulfonyloxy, 1,1,2-Trimethylpropylsulfonyloxy, 1,2,2-Trimethylpropylsulfonyloxy, 1-Ethyl-1-methylpropylsulfonyloxy und 1-Ethyl-2-methylpropylsulfonyloxy, vorzugsweise $C_1$-$C_4$-Alkylsulfonyloxy, insbesondere $C_1$-$C_2$-Alkylsulfonyloxy;
$C_1$-$C_8$-Halogenalkylsulfonyloxy, besonders $C_1$-$C_4$-Halogenalkylsulfonyloxy, insbesondere $C_1$-$C_2$-Halogenalkylsulfonyloxy wie Chlormethylsulfonyloxy, Dichlormethylsulfonyloxy, Trichlormethylsulfonyloxy, Fluormethylsulfonyloxy, Difluormethylsulfonyloxy, Trifluormethylsulfonyloxy, Chlorfluormethylsulfonyloxy, Dichlorfluormethylsulfonyloxy, Chlordifluormethylsulfonyloxy, 1-Fluorethylsulfonyloxy, 2-Fluorethylsulfonyloxy, 2,2-Difluorethylsulfonyloxy, 2,2,2-Trifluorethylsulfonyloxy, 2-Chlor-2-fluorethylsulfonyloxy, 2-Chlor-2,2-difluorethylsulfonyloxy, 2,2-Dichlor-2-fluorethylsulfonyloxy,2,2,2-Trichlorethylsulfonyloxy und Pentafluorethylsulfonyloxy;
Phenoxy, Naphthyloxy, Phenylamino, Naphthylamino, Benzyloxy, Benzylamino, Benzoyloxy, 2-Naphthoyloxy oder Phenylsulfonyloxy, wobei die aromatischen Reste ein bis drei der folgenden Gruppen tragen können:

- Halogen wie vorstehend genannt, vorzugsweise Fluor und Chlor;
- $C_1$-$C_4$-Alkyl wie vorstehend genannt, vorzugsweise Methyl;
- $C_1$-$C_4$-Halogenalkyl, besonders $C_1$-$C_2$-Halogenalkyl wie vorstehend genannt, vorzugsweise Trifluormethyl;
- $C_1$-$C_4$-Alkoxy wie vorstehend genannt, vorzugsweise Methoxy;

$N(R^{12})$-$SO_2$-$R^{13}$; $N(R^{12})$-CO-$R^{14}$; $N(R^{12})$-CS-$R^{14}$;

$R^{12}$ Wasserstoff;
$C_1$-$C_4$-Alkyl wie vorstehend genannt, vorzugsweise $C_1$-$C_3$-Alkyl;
Phenyl, welches ein bis drei der folgenden Reste tragen kann:

- Halogen wie vorstehend genannt, vorzugsweise Fluor und Chlor;
- $C_1$-$C_4$-Alkyl wie vorstehend genannt, vorzugsweise Methyl;
- $C_1$-$C_4$-Halogenalkyl, besonders $C_1$-$C_2$-Halogenalkyl wie vorstehend genannt, vorzugsweise Trifluormethyl;
- $C_1$-$C_4$-Alkoxy wie vorstehend genannt, vorzugsweise insbesondere Methoxy;

$R^{13}$ eine der für $R^1$ genannten Gruppen;
Amino,
Di-($C_1$-$C_8$-alkyl)-amino, besonders Di-($C_1$-$C_6$-alkyl)-amino, insbesondere Di-($C_1$-$C_4$-alkyl)-amino wie vorstehend genannt;
$C_3$-$C_8$-Cycloalkylamino wie vorstehend genannt, vorzugsweise Cyclopropylamino, Cyclopentylamino oder Cyclohexylamino;

$R^{14}$ eine der für $R^1$ genannten Gruppen;
Amino; Oxyamino (-NH-OH);
Di-($C_1$-$C_8$-alkyl)-amino, besonders Di-($C_1$-$C_6$-alkyl)-amino, insbesondere Di-($C_1$-$C_4$-alkyl)-amino wie vorstehend genannt, vorzugsweise Di-($C_1$-$C_2$-alkyl)-amino;
$C_3$-$C_8$-Cycloalkylamino wie vorstehend genannt, insbesondere Cyclopropylamino, Cyclopentylamino oder Cyclohexylamino;

sowie die pflanzenverträglichen Salze derjenigen Verbindungen I, bei denen mindestens einer der Substituenten $R^1$ bis $R^5$ eine saure oder basische Gruppe bedeutet.

Unter 5-gliedrigen aromatischen Ringen, welche neben Kohlenstoffatomen ein bis drei Stickstoffatome und ein Sauerstoff- oder ein Schwefelatom als Heteroatome enthalten können, oder welche neben Kohlenstoffatomen ein bis drei Stickstoffatome oder ein Sauerstoff- oder ein Schwefelatom als Heteroatome enthalten können, sind die folgenden Gruppen zu verstehen: 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, vorzugsweise 2-Thienyl und 3-Thienyl, wobei an die vorstehend genannten 5-gliedrigen Heteroaromaten ein Benzolring anneliert sein kann, sofern sie für einen Rest $R^1$ oder $R^2$ stehen.

Unter 6-gliedrigen aromatischen Ringen, welche neben Kohlenstoffatonen ein bis drei Stickstoffatome als Heteroatome enthalten können, sind die folgenden Gruppen zu verstehen: 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, vorzugsweise 2-Pyridinyl, 3-Pyridinyl und 4-Pyridinyl, wobei an die vorstehend genannten 6-gliedrigen Heteroaromaten ein Benzolring anneliert sein kann, sofern sie für einen Rest $R^1$ oder $R^2$ stehen.

Derivate I und I' mit sauren Endgruppen oder mit basischen Stickstoffatomen können in Form ihrer landwirtschaftlich brauchbaren Salze vorliegen.

Als landwirtschaftlich brauchbare Salze kommen im allgemeinen die Salze von solchen Säuren oder Basen in Betracht, welche die antagonistische Wirkung von I und I' nicht beeinträchtigen.

Als Säureadditionssalze eignen sich beispielsweise die Hydrochloride und -bromide, Sulfate, Nitrate, Phosphate, Oxalate oder die Dodecylbenzolsulfonate.

Als basische Salze eignen sich beispielsweise diejenigen der Alkalimetalle, insbesondere die Natrium- und Kaliumsalze, die der Erdalkalimetalle, insbesondere Calcium-, Magnesium-, und Bariumsalze und die der Übergangsmetalle, insbesondere Mangan-, Kupfer, Zink- und Eisensalze sowie die Ammoniumsalze, die ein bis drei $C_1$-$C_4$-Alkyl-, Hydroxy-$C_1$-$C_4$-alkylsubstituenten und/oder einen Phenyl- oder Benzylsubstituenten tragen können, insbesondere Diisopropylammonium-, Tetramethylammonium-, Tetrabutylammonium-, Trimethylbenzylammonium-, und Trimethyl-(2-hydroxyethyl)-ammoniumsalze, die Phosphoniumsalze, die Sulfoniumsalze, insbesondere Tri-($C_1$-$C_4$-)alkylsufoniumsalze und die Sulfoxoniumsalze, insbesondere Tri-($C_1$-$C_4$-)alkylsulfoxoniumsalze. Besonders bevorzugte Verbindungen der Formel I sind in den folgenden Tabellen A und B zusammengestellt.

Tabelle A

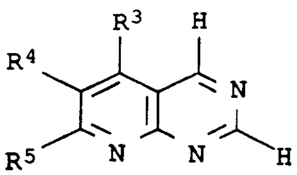

I.1

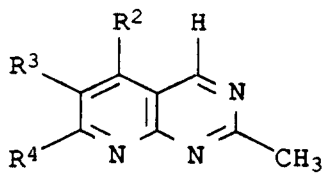

I.2

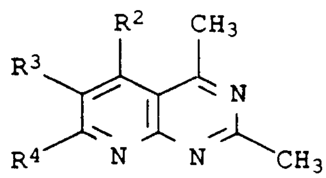

I.3

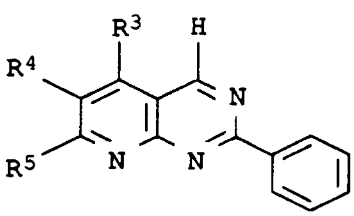

I.4

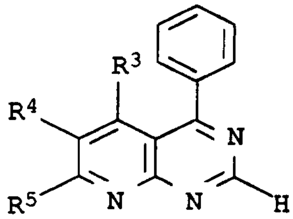

I.5

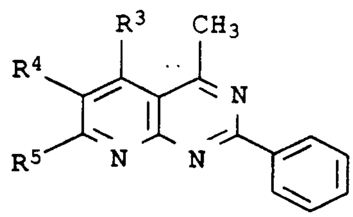

I.6

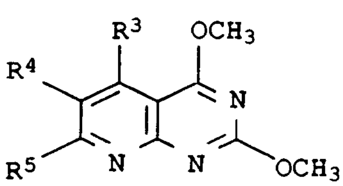

I.7

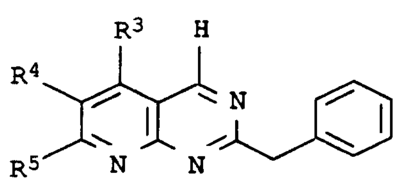

I.8

| $R^3$ | $R^4$ | $R^5$ |
|---|---|---|
| H | H | $C_6H_5$ |
| H | H | $2-CH_3-C_6H_4$ |
| H | H | $3-CH_3-C_6H_4$ |
| H | H | $4-CH_3-C_6H_4$ |
| H | H | $2-F-C_6H_4$ |
| H | H | $3-F-C_6H_4$ |
| H | H | $4-F-C_6H_4$ |
| H | H | $2-Cl-C_6H_4$ |
| H | H | $3-Cl-C_6H_4$ |
| H | H | $4-Cl-C_6H_4$ |
| H | H | $2-Br-C_6H_4$ |
| H | H | $3-Br-C_6H_4$ |
| H | H | $4-Br-C_6H_4$ |
| H | H | $2-OH-C_6H_4$ |
| H | H | $3-OH-C_6H_4$ |
| H | H | $4-OH-C_6H_4$ |
| H | H | $2-OCH_3-C_6H_4$ |
| H | H | $3-OCH_3-C_6H_4$ |
| H | H | $4-OCH_3-C_6H_4$ |
| H | H | $4-C_6H_5-C_6H_4$ |
| H | H | $3-C(CH_3)_3-C_6H_4$ |
| H | H | $4-C(CH_3)_3-C_6H_4$ |
| H | H | $2-CF_3-C_6H_4$ |
| H | H | $3-CF_3-C_6H_4$ |
| H | H | $4-CF_3-C_6H_4$ |
| H | H | $2-NO_2-C_6H_4$ |
| H | H | $3-NO_2-C_6H_4$ |
| H | H | $4-NO_2-C_6H_4$ |
| H | H | $2-CN-C_6H_4$ |
| H | H | $3-CN-C_6H_4$ |
| H | H | $4-CN-C_6H_4$ |
| H | H | $2-(CO_2C_2H_5)-C_6H_4$ |
| H | H | $3-(CO_2C_2H_5)-C_6H_4$ |
| H | H | $4-(CO_2C_2H_5)-C_6H_4$ |
| H | H | 2-Carbamoyl-$C_6H_4$ |
| H | H | 3-Carbamoyl-$C_6H_4$ |

| $R^3$ | $R^4$ | $R^5$ |
|---|---|---|
| H | H | 4-Carbamoyl-$C_6H_4$ |
| H | H | 2-$NH_2$-$C_6H_4$ |
| H | H | 3-$NH_2$-$C_6H_4$ |
| H | H | 4-$NH_2$-$C_6H_4$ |
| H | H | 4-Pyrrolidino-$C_6H_4$ |
| H | H | 2-$SCH_3$-$C_6H_4$ |
| H | H | 3-$SCH_3$-$C_6H_4$ |
| H | H | 4-$SCH_3$-$C_6H_4$ |
| H | H | 2-Sulfo-$C_6H_4$ |
| H | H | 3-Sulfo-$C_6H_4$ |
| H | H | 4-Sulfo-$C_6H_4$ |
| H | H | 3-$OC(CH_3)_3$-$C_6H_4$ |
| H | H | 4-$OC(CH_3)_3$-$C_6H_4$ |
| H | H | 2,4-$(CH_3)_2$-$C_6H_3$ |
| H | H | 3,4-$(CH_3)_2$-$C_6H_3$ |
| H | H | 2,6-$(CH_3)_2$-$C_6H_3$ |
| H | H | 2,4-$(OCH_3)_2$-$C_6H_3$ |
| H | H | 3,4-$(OCH_3)_2$-$C_6H_3$ |
| H | H | 2,6-$(OCH_3)_2$-$C_6H_3$ |
| H | H | 2,4-$F_2$-$C_6H_3$ |
| H | H | 3,4-$F_2$-$C_6H_3$ |
| H | H | 2,6-$F_2$-$C_6H_3$ |
| H | H | 2,4-$Cl_2$-$C_6H_3$ |
| H | H | 3,4-$Cl_2$-$C_6H_3$ |
| H | H | 2,6-$Cl_2$-$C_6H_3$ |
| H | H | 2,4-$(OH)_2$-$C_6H_3$ |
| H | H | 3,4-$(OH)_2$-$C_6H_3$ |
| H | H | 2,6-$(OH)_2$-$C_6H_3$ |
| H | H | 3-$NO_2$-4-$CH_3$-$C_6H_3$ |
| H | H | 3-$NO_2$-4-F-$C_6H_3$ |
| H | H | 3-$NO_2$-4-Cl-$C_6H_3$ |
| H | H | 3-$NO_2$-4-$OCH_3$-$C_6H_3$ |
| H | H | 1-Naphthyl |
| H | H | 2-Naphthyl |
| H | H | Tetralin-2-yl |
| H | H | Thien-2-yl |

| $R^3$ | $R^4$ | $R^5$ |
|---|---|---|
| H | H | Thien-3-yl |
| H | H | 5-$CH_3$-thien-2-yl |
| H | H | 5-Cl-thien-2-yl |
| H | H | 5-Br-thien-2-yl |
| H | H | 2,5-$(CH_3)_2$-thien-3-yl |
| H | H | 4,5-benzothien-2-yl |
| H | H | Thiazol-2-yl |
| H | H | Thiazol-4-yl |
| H | H | Thiazol-5-yl |
| H | H | 5-$CH_3$-thiazol-2-yl |
| H | H | 5-Cl-thiazol-2-yl |
| H | H | 5-Br-thiazol-2-yl |
| H | H | 2,4-$(CH_3)_2$-thiazol-5-yl |
| H | H | 4,5-benzothiazol-2-yl |
| H | H | Furan-2-yl |
| H | H | Furan-3-yl |
| H | H | 5-$CH_3$-furan-2-yl |
| H | H | 5-Cl-furan-2-yl |
| H | H | 5-Br-furan-2-yl |
| H | H | 2,5-$(CH_3)_2$-furan-3-yl |
| H | H | 4,5-benzofuran-2-yl |
| H | H | Pyrrol-2-yl |
| H | H | Pyrrol-3-yl |
| H | H | 1-$CH_3$-pyrrol-2-yl |
| H | H | 1-$CH_3$-pyrrol-3-yl |
| H | H | 2,5-$(CH_3)_2$-pyrrol-3-yl |
| H | H | 1,5-$(CH_3)_2$-pyrrol-2-yl |
| H | H | 1,5-$(CH_3)_2$-pyrrol-3-yl |
| H | H | Indol-2-yl |
| H | H | Indol-3-yl |
| H | H | Oxazol-2-yl |
| H | H | Oxazol-4-yl |
| H | H | 5-$CH_3$-oxazol-2-yl |
| H | H | 5-Cl-oxazol-2-yl |
| H | H | 5-Br-oxazol-2-yl |
| H | H | 2,5-$(CH_3)_2$-oxazol-4-yl |

| $R^3$ | $R^4$ | $R^5$ |
|---|---|---|
| H | H | 4,5-benzoxazol-2-yl |
| H | H | Imidazol-2-yl |
| H | H | Imidazol-4-yl |
| H | H | Imidazol-5-yl |
| H | H | 5-$CH_3$-imidazol-2-yl |
| H | H | 5-Cl-imidazol-2-yl |
| H | H | 5-Br-imidazol-2-yl |
| H | H | 2,5-$(CH_3)_2$-imidazol-4-yl |
| H | H | 4,5-benzimidazol-2-yl |
| H | H | Pyridin-2-yl |
| H | H | Pyridin-3-yl |
| H | H | Pyridin-4-yl |
| H | H | 5-$CH_3$-pyridin-2-yl |
| H | H | 5-Cl-pyridin-2-yl |
| H | H | 5-Br-pyridin-2-yl |
| H | H | 5-$CH_3$-pyridin-3-yl |
| H | H | 5-Cl-pyridin-3-yl |
| H | H | 5-Br-pyridin-3-yl |
| H | H | 2-$CH_3$-pyridin-3-yl |
| H | H | 2-Cl-pyridin-3-yl |
| H | H | 2-Br-pyridin-3-yl |
| H | H | 2,5-$(CH_3)_2$-pyridin-3-yl |
| H | H | 4,5-benzopyridin-2-yl |
| H | H | Pyrazin-2-yl |
| H | H | 5-$CH_3$-pyrazin-2-yl |
| H | H | 5-Cl-pyrazin-2-yl |
| H | H | 5-Br-pyrazin-2-yl |
| H | H | Pyrimidin-2-yl |
| H | H | Pyrimidin-4-yl |
| H | H | Pyrimidin-5-yl |
| H | H | 4,5-benzopyrimidin-2-yl |
| H | $CH_3$ | $C_6H_5$ |
| H | $CH_3$ | 4-$CH_3$-$C_6H_4$ |
| H | $CH_3$ | 4-F-$C_6H_4$ |
| H | $CH_3$ | 4-Cl-$C_6H_4$ |
| H | $CH_3$ | 4-Br-$C_6H_4$ |

| $R^3$ | $R^4$ | $R^5$ |
|---|---|---|
| H | $CH_3$ | $4-CH(CH_3)_2-C_6H_4$ |
| H | $CH_3$ | $4-OCH_3-C_6H_4$ |
| H | $CH_3$ | $4-C(CH_3)_3-C_6H_4$ |
| H | $CH_3$ | $4-CF_3-C_6H_4$ |
| H | $CH_3$ | $4-NO_2-C_6H_4$ |
| H | $CH_3$ | $4-CN-C_6H_4$ |
| H | $CH_3$ | $4-(NHCOCH_3)-C_6H_4$ |
| H | $CH_3$ | $3,4-(OCH_3)_2-C_6H_3$ |
| H | $CH_3$ | $2,4-Cl_2-C_6H_3$ |
| H | $CH_3$ | $3-NO_2-4-CH_3-C_6H_3$ |
| H | $CH_3$ | 2-Naphthyl |
| H | $CH_3$ | Thien-2-yl |
| H | $CH_2CH_3$ | $C_6H_5$ |
| H | $CH_2CH_3$ | $4-CH_3-C_6H_4$ |
| H | $CH_2CH_3$ | $4-F-C_6H_4$ |
| H | $CH_2CH_3$ | $4-Cl-C_6H_4$ |
| H | $CH_2CH_3$ | $4-Br-C_6H_4$ |
| H | $CH_2CH_3$ | $4-CH(CH_3)_2-C_6H_4$ |
| H | $CH_2CH_3$ | $4-OCH_3-C_6H_4$ |
| H | $CH_2CH_3$ | $4-C(CH_3)_3-C_6H_4$ |
| H | $CH_2CH_3$ | $4-CF_3-C_6H_4$ |
| H | $CH_2CH_3$ | $4-NO_2-C_6H_4$ |
| H | $CH_2CH_3$ | $4-CN-C_6H_4$ |
| H | $CH_2CH_3$ | $4-(NHCOCH_3)-C_6H_4$ |
| H | $CH_2CH_3$ | $3,4-(OCH_3)_2-C_6H_3$ |
| H | $CH_2CH_3$ | $2,4-Cl_2-C_6H_3$ |
| H | $CH_2CH_3$ | $3-NO_2-4-CH_3-C_6H_3$ |
| H | $CH_2CH_3$ | 2-Naphthyl |
| H | $CH_2CH_3$ | Thien-2-yl |
| H | $CH_2CH_2CH_3$ | $C_6H_5$ |
| H | $CH_2CH_2CH_3$ | $4-CH_3-C_6H_4$ |
| H | $CH_2CH_2CH_3$ | $4-F-C_6H_4$ |
| H | $CH_2CH_2CH_3$ | $4-Cl-C_6H_4$ |
| H | $CH_2CH_2CH_3$ | $4-Br-C_6H_4$ |
| H | $CH_2CH_2CH_3$ | $4-CH(CH_3)_2-C_6H_4$ |
| H | $CH_2CH_2CH_3$ | $4-OCH_3-C_6H_4$ |

| $R^3$ | $R^4$ | $R^5$ |
|---|---|---|
| H | $CH_2CH_2CH_3$ | 4-$C(CH_3)_3$-$C_6H_4$ |
| H | $CH_2CH_2CH_3$ | 4-$CF_3$-$C_6H_4$ |
| H | $CH_2CH_2CH_3$ | 4-$NO_2$-$C_6H_4$ |
| H | $CH_2CH_2CH_3$ | 4-CN-$C_6H_4$ |
| H | $CH_2CH_2CH_3$ | 4-($NHCOCH_3$)-$C_6H_4$ |
| H | $CH_2CH_2CH_3$ | 3,4-$(OCH_3)_2$-$C_6H_3$ |
| H | $CH_2CH_2CH_3$ | 2,4-$Cl_2$-$C_6H_3$ |
| H | $CH_2CH_2CH_3$ | 3-$NO_2$-4-$CH_3$-$C_6H_3$ |
| H | $CH_2CH_2CH_3$ | 2-Naphthyl |
| H | $CH_2CH_2CH_3$ | Thien-2-yl |
| H | $CH(CH_3)_2$ | $C_6H_5$ |
| H | $CH(CH_3)_2$ | 4-$CH_3$-$C_6H_4$ |
| H | $CH(CH_3)_2$ | 4-F-$C_6H_4$ |
| H | $CH(CH_3)_2$ | 4-Cl-$C_6H_4$ |
| H | $CH(CH_3)_2$ | 4-Br-$C_6H_4$ |
| H | $CH(CH_3)_2$ | 4-$CH(CH_3)_2$-$C_6H_4$ |
| H | $CH(CH_3)_2$ | 4-$OCH_3$-$C_6H_4$ |
| H | $CH(CH_3)_2$ | 4-$C(CH_3)_3$-$C_6H_4$ |
| H | $CH(CH_3)_2$ | 4-$CF_3$-$C_6H_4$ |
| H | $CH(CH_3)_2$ | 4-$NO_2$-$C_6H_4$ |
| H | $CH(CH_3)_2$ | 4-CN-$C_6H_4$ |
| H | $CH(CH_3)_2$ | 4-($NHCOCH_3$)-$C_6H_4$ |
| H | $CH(CH_3)_2$ | 3,4-$(OCH_3)_2$-$C_6H_3$ |
| H | $CH(CH_3)_2$ | 2,4-$Cl_2$-$C_6H_3$ |
| H | $CH(CH_3)_2$ | 3-$NO_2$-4-$CH_3$-$C_6H_3$ |
| H | $CH(CH_3)_2$ | 2-Naphthyl |
| H | $CH(CH_3)_2$ | Thien-2-yl |
| H | $CH_2CH(CH_3)_2$ | $C_6H_5$ |
| H | $CH_2CH(CH_3)_2$ | 4-$CH_3$-$C_6H_4$ |
| H | $CH_2CH(CH_3)_2$ | 4-F-$C_6H_4$ |
| H | $CH_2CH(CH_3)_2$ | 4-Cl-$C_6H_4$ |
| H | $CH_2CH(CH_3)_2$ | 4-Br-$C_6H_4$ |
| H | $CH_2CH(CH_3)_2$ | 4-$CH(CH_3)_2$-$C_6H_4$ |
| H | $CH_2CH(CH_3)_2$ | 4-$OCH_3$-$C_6H_4$ |
| H | $CH_2CH(CH_3)_2$ | 4-$C(CH_3)_3$-$C_6H_4$ |
| H | $CH_2CH(CH_3)_2$ | 4-$CF_3$-$C_6H_4$ |

| $R^3$ | $R^4$ | $R^5$ |
|---|---|---|
| H | $CH_2CH(CH_3)_2$ | $4-NO_2-C_6H_4$ |
| H | $CH_2CH(CH_3)_2$ | $4-CN-C_6H_4$ |
| H | $CH_2CH(CH_3)_2$ | $4-(NHCOCH_3)-C_6H_4$ |
| H | $CH_2CH(CH_3)_2$ | $3,4-(OCH_3)_2-C_6H_3$ |
| H | $CH_2CH(CH_3)_2$ | $2,4-Cl_2-C_6H_3$ |
| H | $CH_2CH(CH_3)_2$ | $3-NO_2-4-CH_3-C_6H_3$ |
| H | $CH_2CH(CH_3)_2$ | 2-Naphthyl |
| H | $CH_2CH(CH_3)_2$ | Thien-2-yl |
| H | $(CH_2)_3CH_3$ | $C_6H_5$ |
| H | $(CH_2)_3CH_3$ | $4-CH_3-C_6H_4$ |
| H | $(CH_2)_3CH_3$ | $4-F-C_6H_4$ |
| H | $(CH_2)_3CH_3$ | $4-Cl-C_6H_4$ |
| H | $(CH_2)_3CH_3$ | $4-Br-C_6H_4$ |
| H | $(CH_2)_3CH_3$ | $4-CH(CH_3)_2-C_6H_4$ |
| H | $(CH_2)_3CH_3$ | $4-OCH_3-C_6H_4$ |
| H | $(CH_2)_3CH_3$ | $4-C(CH_3)_3-C_6H_4$ |
| H | $(CH_2)_3CH_3$ | $4-CF_3-C_6H_4$ |
| H | $(CH_2)_3CH_3$ | $4-NO_2-C_6H_4$ |
| H | $(CH_2)_3CH_3$ | $4-CN-C_6H_4$ |
| H | $(CH_2)_3CH_3$ | $4-(NHCOCH_3)-C_6H_4$ |
| H | $(CH_2)_3CH_3$ | $3,4-(OCH_3)_2-C_6H_3$ |
| H | $(CH_2)_3CH_3$ | $2,4-Cl_2-C_6H_3$ |
| H | $(CH_2)_3CH_3$ | $3-NO_2-4-CH_3-C_6H_3$ |
| H | $(CH_2)_3CH_3$ | 2-Naphthyl |
| H | $(CH_2)_3CH_3$ | Thien-2-yl |
| H | $C(CH_3)_3$ | $C_6H_5$ |
| H | $C(CH_3)_3$ | $4-CH_3-C_6H_4$ |
| H | $C(CH_3)_3$ | $4-F-C_6H_4$ |
| H | $C(CH_3)_3$ | $4-Cl-C_6H_4$ |
| H | $C(CH_3)_3$ | $4-Br-C_6H_4$ |
| H | $C(CH_3)_3$ | $4-CH(CH_3)_2-C_6H_4$ |
| H | $C(CH_3)_3$ | $4-OCH_3-C_6H_4$ |
| H | $C(CH_3)_3$ | $4-C(CH_3)_3-C_6H_4$ |
| H | $C(CH_3)_3$ | $4-CF_3-C_6H_4$ |
| H | $C(CH_3)_3$ | $4-NO_2-C_6H_4$ |
| H | $C(CH_3)_3$ | $4-CN-C_6H_4$ |

| $R^3$ | $R^4$ | $R^5$ |
|---|---|---|
| H | $C(CH_3)_3$ | $4-(NHCOCH_3)-C_6H_4$ |
| H | $C(CH_3)_3$ | $3,4-(OCH_3)_2-C_6H_3$ |
| H | $C(CH_3)_3$ | $2,4-Cl_2-C_6H_3$ |
| H | $C(CH_3)_3$ | $3-NO_2-4-CH_3-C_6H_3$ |
| H | $C(CH_3)_3$ | 2-Naphthyl |
| H | $C(CH_3)_3$ | Thien-2-yl |
| H | $C_6H_5$ | $CH_3$ |
| H | $C_6H_5$ | $CH_2CH_3$ |
| H | $C_6H_5$ | $CH_2CH_2CH_3$ |
| H | $C_6H_5$ | $CH(CH_3)_2$ |
| H | $C_6H_5$ | $CH_2CH_2CH_2CH_3$ |
| H | $C_6H_5$ | $CH_2CH(CH_3)_2$ |
| H | $C_6H_5$ | $C_6H_5$ |
| H | $C_6H_5$ | $CO_2H$ |
| H | $C_6H_5$ | $CO_2CH_2CH_3$ |
| H | $C_6H_5$ | $CONH_2$ |
| H | $C_6H_5$ | $COCH_3$ |
| H | $C_6H_5$ | $COCF_3$ |
| H | $C_6H_5$ | $COC_6H_5$ |
| H | $C_6H_5$ | $CO-(4-CH_3-C_6H_4)$ |
| H | $C_6H_5$ | $CO_2C_6H_5$ |
| H | $C_6H_5$ | $CO_2CH_2C_6H_5$ |
| H | $C_6H_5$ | F |
| H | $C_6H_5$ | Cl |
| H | $C_6H_5$ | Br |
| H | $C_6H_5$ | $OCH_3$ |
| H | $C_6H_5$ | $CF_3$ |
| H | $C_6H_5$ | $NO_2$ |
| H | $C_6H_5$ | CN |
| H | $C_6H_5$ | $SO_2CH_3$ |
| H | $C_6H_5$ | $SO_2C_6H_5$ |
| H | $C_6H_5$ | $SO_2-(4-CH_3-C_6H_4)$ |
| H | $C_6H_5$ | $C_6H_5$ |
| H | $C_6H_5$ | $4-CH_3-C_6H_4$ |
| H | $C_6H_5$ | $4-F-C_6H_4$ |
| H | $C_6H_5$ | $4-Cl-C_6H_4$ |

| $R^3$ | $R^4$ | $R^5$ |
|---|---|---|
| H | $C_6H_5$ | 4-Br-$C_6H_4$ |
| H | $C_6H_5$ | 4-CH$(CH_3)_2$-$C_6H_4$ |
| H | $C_6H_5$ | 4-$OCH_3$-$C_6H_4$ |
| H | $C_6H_5$ | 4-C$(CH_3)_3$-$C_6H_4$ |
| H | $C_6H_5$ | 4-$CF_3$-$C_6H_4$ |
| H | $C_6H_5$ | 4-$NO_2$-$C_6H_4$ |
| H | $C_6H_5$ | 4-CN-$C_6H_4$ |
| H | $C_6H_5$ | 4-($NHCOCH_3$)-$C_6H_4$ |
| H | $C_6H_5$ | 3,4-$(OCH_3)_2$-$C_6H_3$ |
| H | $C_6H_5$ | 2,4-$Cl_2$-$C_6H_3$ |
| H | $C_6H_5$ | 3-$NO_2$-4-$CH_3$-$C_6H_3$ |
| H | $C_6H_5$ | 2-Naphthyl |
| H | $C_6H_5$ | Thien-2-yl |
| H | $CH_2C_6H_5$ | $C_6H_5$ |
| H | $CH_2C_6H_5$ | 4-$CH_3$-$C_6H_4$ |
| H | $CH_2C_6H_5$ | 4-F-$C_6H_4$ |
| H | $CH_2C_6H_5$ | 4-Cl-$C_6H_4$ |
| H | $CH_2C_6H_5$ | 4-Br-$C_6H_4$ |
| H | $CH_2C_6H_5$ | 4-CH$(CH_3)_2$-$C_6H_4$ |
| H | $CH_2C_6H_5$ | 4-$OCH_3$-$C_6H_4$ |
| H | $CH_2C_6H_5$ | 4-C$(CH_3)_3$-$C_6H_4$ |
| H | $CH_2C_6H_5$ | 4-$CF_3$-$C_6H_4$ |
| H | $CH_2C_6H_5$ | 4-$NO_2$-$C_6H_4$ |
| H | $CH_2C_6H_5$ | 4-CN-$C_6H_4$ |
| H | $CH_2C_6H_5$ | 4-($NHCOCH_3$)-$C_6H_4$ |
| H | $CH_2C_6H_5$ | 3,4-$(OCH_3)_2$-$C_6H_3$ |
| H | $CH_2C_6H_5$ | 2,4-$Cl_2$-$C_6H_3$ |
| H | $CH_2C_6H_5$ | 3-$NO_2$-4-$CH_3$-$C_6H_3$ |
| H | $CH_2C_6H_5$ | 2-Naphthyl |
| H | $CH_2C_6H_5$ | Thien-2-yl |
| H | $CO_2C_2H_5$ | $C_6H_5$ |
| H | $CO_2C_2H_5$ | 4-$CH_3$-$C_6H_4$ |
| H | $CO_2C_2H_5$ | 4-F-$C_6H_4$ |
| H | $CO_2C_2H_5$ | 4-Cl-$C_6H_4$ |
| H | $CO_2C_2H_5$ | 4-Br-$C_6H_4$ |
| H | $CO_2C_2H_5$ | 4-CH$(CH_3)_2$-$C_6H_4$ |

| $R^3$ | $R^4$ | $R^5$ |
|---|---|---|
| H | $CO_2C_2H_5$ | $4-OCH_3-C_6H_4$ |
| H | $CO_2C_2H_5$ | $4-C(CH_3)_3-C_6H_4$ |
| H | $CO_2C_2H_5$ | $4-CF_3-C_6H_4$ |
| H | $CO_2C_2H_5$ | $4-NO_2-C_6H_4$ |
| H | $CO_2C_2H_5$ | $4-CN-C_6H_4$ |
| H | $CO_2C_2H_5$ | $4-(NHCOCH_3)-C_6H_4$ |
| H | $CO_2C_2H_5$ | $3,4-(OCH_3)_2-C_6H_3$ |
| H | $CO_2C_2H_5$ | $2,4-Cl_2-C_6H_3$ |
| H | $CO_2C_2H_5$ | $3-NO_2-4-CH_3-C_6H_3$ |
| H | $CO_2C_2H_5$ | 2-Naphthyl |
| H | $CO_2C_2H_5$ | Thien-2-yl |
| H | $2-CH_3-C_6H_4$ | $CH_3$ |
| H | $3-CH_3-C_6H_4$ | $CH_3$ |
| H | $4-CH_3-C_6H_4$ | $CH_3$ |
| H | $2-F-C_6H_4$ | $CH_3$ |
| H | $3-F-C_6H_4$ | $CH_3$ |
| H | $4-F-C_6H_4$ | $CH_3$ |
| H | $2-Cl-C_6H_4$ | $CH_3$ |
| H | $3-Cl-C_6H_4$ | $CH_3$ |
| H | $4-Cl-C_6H_4$ | $CH_3$ |
| H | $2-Br-C_6H_4$ | $CH_3$ |
| H | $3-Br-C_6H_4$ | $CH_3$ |
| H | $4-Br-C_6H_4$ | $CH_3$ |
| H | $2-OCH_3-C_6H_4$ | $CH_3$ |
| H | $3-OCH_3-C_6H_4$ | $CH_3$ |
| H | $4-OCH_3-C_6H_4$ | $CH_3$ |
| H | $3-C(CH_3)_3-C_6H_4$ | $CH_3$ |
| H | $4-C(CH_3)_3-C_6H_4$ | $CH_3$ |
| H | $2-CF_3-C_6H_4$ | $CH_3$ |
| H | $3-CF_3-C_6H_4$ | $CH_3$ |
| H | $4-CF_3-C_6H_4$ | $CH_3$ |
| H | $2-NO_2-C_6H_4$ | $CH_3$ |
| H | $3-NO_2-C_6H_4$ | $CH_3$ |
| H | $4-NO_2-C_6H_4$ | $CH_3$ |
| H | $2-CN-C_6H_4$ | $CH_3$ |
| H | $3-CN-C_6H_4$ | $CH_3$ |

| $R^3$ | $R^4$ | $R^5$ |
|---|---|---|
| H | 4-CN-$C_6H_4$ | $CH_3$ |
| H | 3,4-$(OCH_3)_2$-$C_6H_3$ | $CH_3$ |
| H | 2,4-$Cl_2$-$C_6H_3$ | $CH_3$ |
| H | 3-$NO_2$-4-$CH_3$-$C_6H_3$ | $CH_3$ |
| H | 2-Naphthyl | $CH_3$ |
| H | Thien-2-yl | $CH_3$ |
| H | Furan-2-yl | $CH_3$ |
| H | Isoxazol-2-yl | $CH_3$ |
| H | $CH_3$ | $CH_3$ |
| H | $CH_2CH_3$ | $CH_3$ |
| H | $CH(CH_3)_2$ | $CH_3$ |
| H | $CH_2CH(CH_3)_2$ | $CH_3$ |
| H | $C(CH_3)_3$ | $CH_3$ |
| H | Cyclopropyl | $CH_3$ |
| H | $CH_2CH=CH_2$ | $CH_3$ |
| H | $CH_2C$*$CH$ | $CH_3$ *=Dreifachbindung |
| H | $CH_2CH=CHCH_3$ | $CH_3$ |
| H | $CH_2C$*$CCH_3$ | $CH_3$ *=Dreifachbindung |
| H | $CH_2Cl$ | $CH_3$ |
| H | $CH_2CH_2Cl$ | $CH_3$ |
| H | $CH_2CH_2CH_2Cl$ | $CH_3$ |
| H | $CH_2CH=CHCl$ | $CH_3$ |
| H | $CH_2OH$ | $CH_3$ |
| H | $CH_2CH_2OH$ | $CH_3$ |
| H | $CH_2CH_2CH_2OH$ | $CH_3$ |
| H | $CH_2OCH_3$ | $CH_3$ |
| H | $CH_2CH_2OCH_3$ | $CH_3$ |
| H | $CH_2CH_2CH_2OCH_3$ | $CH_3$ |
| H | $CH_2CO_2CH_2CH_3$ | $CH_3$ |
| H | $CH_2CH_2CO_2CH_2CH_3$ | $CH_3$ |
| H | $CH_2CH_2CH_2CO_2CH_2CH_3$ | $CH_3$ |
| H | $CH_2NH_2$ | $CH_3$ |
| H | $CH_2CH_2NH_2$ | $CH_3$ |
| H | $CH_2CH_2CH_2NH_2$ | $CH_3$ |
| H | $CH_2N(CH_3)_2$ | $CH_3$ |
| H | $CH_2CH_2N(CH_3)_2$ | $CH_3$ |

| $R^3$ | $R^4$ | $R^5$ |
|---|---|---|
| H | $CH_2CH_2CH_2N(CH_3)_2$ | $CH_3$ |
| H | $CH_2NHCOCH_3$ | $CH_3$ |
| H | $CH_2CH_2NHCOCH_3$ | $CH_3$ |
| H | $CH_2CH_2CH_2NHCOCH_3$ | $CH_3$ |
| H | $COCH_3$ | $CH_3$ |
| H | $COCF_3$ | $CH_3$ |
| H | $COC_6H_5$ | $CH_3$ |
| H | $CO-(4-CH_3-C_6H_4)$ | $CH_3$ |
| H | $CO_2C_6H_5$ | $CH_3$ |
| H | $CO_2CH_2C_6H_5$ | $CH_3$ |
| H | F | $CH_3$ |
| H | Cl | $CH_3$ |
| H | Br | $CH_3$ |
| H | $OCH_3$ | $CH_3$ |
| H | $CF_3$ | $CH_3$ |
| H | $NO_2$ | $CH_3$ |
| H | CN | $CH_3$ |
| H | $SO_2CH_3$ | $CH_3$ |
| H | $SO_2C_6H_5$ | $CH_3$ |
| H | $SO_2-(4-CH_3-C_6H_4)$ | $CH_3$ |
| H | $CH_2OCH_2C_6H_5$ | $CH_3$ |
| H | $COCH_2CH_2CH_3$ | $CH_3$ |
| H | $CO_2H$ | $CH_3$ |
| H | $CO_2CH_3$ | $CH_3$ |
| H | $CO_2CH_2CH_3$ | $CH_3$ |
| H | $CONH_2$ | $CH_3$ |
| H | $CONHCH_3$ | $CH_3$ |
| H | $CON(CH_3)_2$ | $CH_3$ |
| H | $CONHC_6H_5$ | $CH_3$ |
| H | 3-Pyridyl | $CH_3$ |
| H | 2-Pyridyl | $CH_3$ |
| H | $4-SCH_3-C_6H_4$ | $CH_3$ |
| H | $2-CH_3-C_6H_4$ | $C_6H_5$ |
| H | $3-CH_3-C_6H_4$ | $C_6H_5$ |
| H | $4-CH_3-C_6H_4$ | $C_6H_5$ |
| H | $2-F-C_6H_4$ | $C_6H_5$ |

| $R^3$ | $R^4$ | $R^5$ |
|---|---|---|
| H | $3-F-C_6H_4$ | $C_6H_5$ |
| H | $4-F-C_6H_4$ | $C_6H_5$ |
| H | $2-Cl-C_6H_4$ | $C_6H_5$ |
| H | $3-Cl-C_6H_4$ | $C_6H_5$ |
| H | $4-Cl-C_6H_4$ | $C_6H_5$ |
| H | $2-Br-C_6H_4$ | $C_6H_5$ |
| H | $3-Br-C_6H_4$ | $C_6H_5$ |
| H | $4-Br-C_6H_4$ | $C_6H_5$ |
| H | $2-OCH_3-C_6H_4$ | $C_6H_5$ |
| H | $3-OCH_3-C_6H_4$ | $C_6H_5$ |
| H | $4-OCH_3-C_6H_4$ | $C_6H_5$ |
| H | $3-C(CH_3)_3-C_6H_4$ | $C_6H_5$ |
| H | $4-C(CH_3)_3-C_6H_4$ | $C_6H_5$ |
| H | $2-CF_3-C_6H_4$ | $C_6H_5$ |
| H | $3-CF_3-C_6H_4$ | $C_6H_5$ |
| H | $4-CF_3-C_6H_4$ | $C_6H_5$ |
| H | $2-NO_2-C_6H_4$ | $C_6H_5$ |
| H | $3-NO_2-C_6H_4$ | $C_6H_5$ |
| H | $4-NO_2-C_6H_4$ | $C_6H_5$ |
| H | $2-CN-C_6H_4$ | $C_6H_5$ |
| H | $3-CN-C_6H_4$ | $C_6H_5$ |
| H | $4-CN-C_6H_4$ | $C_6H_5$ |
| H | $3,4-(OCH_3)_2-C_6H_3$ | $C_6H_5$ |
| H | $2,4-Cl_2-C_6H_3$ | $C_6H_5$ |
| H | $3-NO_2-4-CH_3-C_6H_3$ | $C_6H_5$ |
| H | 2-Naphthyl | $C_6H_5$ |
| H | Thien-2-yl | $C_6H_5$ |
| H | Furan-2-yl | $C_6H_5$ |
| H | Isoxazol-2-yl | $C_6H_5$ |
| H | $CH_3$ | $C_6H_5$ |
| H | $CH_2CH_3$ | $C_6H_5$ |
| H | $CH(CH_3)_2$ | $C_6H_5$ |
| H | $CH_2CH(CH_3)_2$ | $C_6H_5$ |
| H | $C(CH_3)_3$ | $C_6H_5$ |
| H | Cyclopropyl | $C_6H_5$ |
| H | $CH_2CH=CH_2$ | $C_6H_5$ |

| $R^3$ | $R^4$ | $R^5$ |
|---|---|---|
| H | $CH_2C{*}CH$ | $C_6H_5$ *=Dreifachbindung |
| H | $CH_2CH{=}CHCH_3$ | $C_6H_5$ |
| H | $CH_2C{*}CCH_3$ | $C_6H_5$ *=Dreifachbindung |
| H | $CH_2Cl$ | $C_6H_5$ |
| H | $CH_2CH_2Cl$ | $C_6H_5$ |
| H | $CH_2CH_2CH_2Cl$ | $C_6H_5$ |
| H | $CH_2CH{=}CHCl$ | $C_6H_5$ |
| H | $CH_2OH$ | $C_6H_5$ |
| H | $CH_2CH_2OH$ | $C_6H_5$ |
| H | $CH_2CH_2CH_2OH$ | $C_6H_5$ |
| H | $CH_2OCH_3$ | $C_6H_5$ |
| H | $CH_2CH_2OCH_3$ | $C_6H_5$ |
| H | $CH_2CH_2CH_2OCH_3$ | $C_6H_5$ |
| H | $CH_2CO_2CH_2CH_3$ | $C_6H_5$ |
| H | $CH_2CH_2CO_2CH_2CH_3$ | $C_6H_5$ |
| H | $CH_2CH_2CH_2CO_2CH_2CH_3$ | $C_6H_5$ |
| H | $CH_2NH_2$ | $C_6H_5$ |
| H | $CH_2CH_2NH_2$ | $C_6H_5$ |
| H | $CH_2CH_2CH_2NH_2$ | $C_6H_5$ |
| H | $CH_2N(CH_3)_2$ | $C_6H_5$ |
| H | $CH_2CH_2N(CH_3)_2$ | $C_6H_5$ |
| H | $CH_2CH_2CH_2N(CH_3)_2$ | $C_6H_5$ |
| H | $CH_2NHCOCH_3$ | $C_6H_5$ |
| H | $CH_2CH_2NHCOCH_3$ | $C_6H_5$ |
| H | $CH_2CH_2CH_2NHCOCH_3$ | $C_6H_5$ |
| H | $COCH_3$ | $C_6H_5$ |
| H | $COCF_3$ | $C_6H_5$ |
| H | $COC_6H_5$ | $C_6H_5$ |
| H | $CO-(4-CH_3-C_6H_4)$ | $C_6H_5$ |
| H | $CO_2C_6H_5$ | $C_6H_5$ |
| H | $CO_2CH_2C_6H_5$ | $C_6H_5$ |
| H | F | $C_6H_5$ |
| H | Cl | $C_6H_5$ |
| H | Br | $C_6H_5$ |
| H | $OCH_3$ | $C_6H_5$ |
| H | $CF_3$ | $C_6H_5$ |

| $R^3$ | $R^4$ | $R^5$ |
|---|---|---|
| H | $NO_2$ | $C_6H_5$ |
| H | CN | $C_6H_5$ |
| H | $SO_2CH_3$ | $C_6H_5$ |
| H | $SO_2C_6H_5$ | $C_6H_5$ |
| H | $SO_2-(4-CH_3-C_6H_4)$ | $C_6H_5$ |
| H | $CH_2OCH_2C_6H_5$ | $C_6H_5$ |
| H | $COCH_2CH_2CH_3$ | $C_6H_5$ |
| H | $CO_2H$ | $C_6H_5$ |
| H | $CO_2CH_3$ | $C_6H_5$ |
| H | $CO_2CH_2CH_3$ | $C_6H_5$ |
| H | $CONH_2$ | $C_6H_5$ |
| H | $CONHCH_3$ | $C_6H_5$ |
| H | $CON(CH_3)_2$ | $C_6H_5$ |
| H | $CONHC_6H_5$ | $C_6H_5$ |
| H | 3-Pyridyl | $C_6H_5$ |
| H | 2-Pyridyl | $C_6H_5$ |
| H | $4-SCH_3-C_6H_4$ | $C_6H_5$ |
| H | $C_6H_5$ | OH |
| H | $2-CH_3-C_6H_4$ | OH |
| H | $3-CH_3-C_6H_4$ | OH |
| H | $4-CH_3-C_6H_4$ | OH |
| H | $2-F-C_6H_4$ | OH |
| H | $3-F-C_6H_4$ | OH |
| H | $4-F-C_6H_4$ | OH |
| H | $2-Cl-C_6H_4$ | OH |
| H | $3-Cl-C_6H_4$ | OH |
| H | $4-Cl-C_6H_4$ | OH |
| H | $2-Br-C_6H_4$ | OH |
| H | $3-Br-C_6H_4$ | OH |
| H | $4-Br-C_6H_4$ | OH |
| H | $2-OCH_3-C_6H_4$ | OH |
| H | $3-OCH_3-C_6H_4$ | OH |
| H | $4-OCH_3-C_6H_4$ | OH |
| H | $3-C(CH_3)_3-C_6H_4$ | OH |
| H | $4-C(CH_3)_3-C_6H_4$ | OH |
| H | $2-CF_3-C_6H_4$ | OH |

| $R^3$ | $R^4$ | $R^5$ |
|---|---|---|
| H | $3-CF_3-C_6H_4$ | OH |
| H | $4-CF_3-C_6H_4$ | OH |
| H | $2-NO_2-C_6H_4$ | OH |
| H | $3-NO_2-C_6H_4$ | OH |
| H | $4-NO_2-C_6H_4$ | OH |
| H | $2-CN-C_6H_4$ | OH |
| H | $3-CN-C_6H_4$ | OH |
| H | $4-CN-C_6H_4$ | OH |
| H | $3,4-(OCH_3)_2-C_6H_3$ | OH |
| H | $2,4-Cl_2-C_6H_3$ | OH |
| H | $3-NO_2-4-CH_3-C_6H_3$ | OH |
| H | 2-Naphthyl | OH |
| H | Thien-2-yl | OH |
| H | Furan-2-yl | OH |
| H | Isoxazol-2-yl | OH |
| H | $CH_3$ | OH |
| H | $CH_2CH_3$ | OH |
| H | $CH(CH_3)_2$ | OH |
| H | $CH_2CH(CH_3)_2$ | OH |
| H | $C(CH_3)_3$ | OH |
| H | Cyclopropyl | OH |
| H | $CH_2CH=CH_2$ | OH |
| H | $CH_2C*CH$ | OH *=Dreifachbindung |
| H | $CH_2CH=CHCH_3$ | OH |
| H | $CH_2C*CCH_3$ | OH *=Dreifachbindung |
| H | $CH_2Cl$ | OH |
| H | $CH_2CH_2Cl$ | OH |
| H | $CH_2CH_2CH_2Cl$ | OH |
| H | $CH_2CH=CHCl$ | OH |
| H | $CH_2OH$ | OH |
| H | $CH_2CH_2OH$ | OH |
| H | $CH_2CH_2CH_2OH$ | OH |
| H | $CH_2OCH_3$ | OH |
| H | $CH_2CH_2OCH_3$ | OH |
| H | $CH_2CH_2CH_2OCH_3$ | OH |
| H | $CH_2CO_2CH_2CH_3$ | OH |

| $R^3$ | $R^4$ | $R^5$ |
|---|---|---|
| H | $CH_2CH_2CO_2CH_2CH_3$ | OH |
| H | $CH_2CH_2CH_2CO_2CH_2CH_3$ | OH |
| H | $CH_2NH_2$ | OH |
| H | $CH_2CH_2NH_2$ | OH |
| H | $CH_2CH_2CH_2NH_2$ | OH |
| H | $CH_2N(CH_3)_2$ | OH |
| H | $CH_2CH_2N(CH_3)_2$ | OH |
| H | $CH_2CH_2CH_2N(CH_3)_2$ | OH |
| H | $CH_2NHCOCH_3$ | OH |
| H | $CH_2CH_2NHCOCH_3$ | OH |
| H | $CH_2CH_2CH_2NHCOCH_3$ | OH |
| H | $COCH_3$ | OH |
| H | $COCF_3$ | OH |
| H | $COC_6H_5$ | OH |
| H | $CO-(4-CH_3-C_6H_4)$ | OH |
| H | $CO_2C_6H_5$ | OH |
| H | $CO_2CH_2C_6H_5$ | OH |
| H | F | OH |
| H | Cl | OH |
| H | Br | OH |
| H | $OCH_3$ | OH |
| H | $CF_3$ | OH |
| H | $NO_2$ | OH |
| H | CN | OH |
| H | $SO_2CH_3$ | OH |
| H | $SO_2C_6H_5$ | OH |
| H | $SO_2-(4-CH_3-C_6H_4)$ | OH |
| H | $CH_2OCH_2C_6H_5$ | OH |
| H | $COCH_2CH_2CH_3$ | OH |
| H | $CO_2H$ | OH |
| H | $CO_2CH_3$ | OH |
| H | $CO_2CH_2CH_3$ | OH |
| H | $CONH_2$ | OH |
| H | $CONHCH_3$ | OH |
| H | $CON(CH_3)_2$ | OH |
| H | $CONHC_6H_5$ | OH |

| $R^3$ | $R^4$ | $R^5$ |
|---|---|---|
| H | 3-Pyridyl | OH |
| H | 2-Pyridyl | OH |
| H | $4-SCH_3-C_6H_4$ | OH |
| H | $C_6H_5$ | $NH_2$ |
| H | $2-CH_3-C_6H_4$ | $NH_2$ |
| H | $3-CH_3-C_6H_4$ | $NH_2$ |
| H | $4-CH_3-C_6H_4$ | $NH_2$ |
| H | $2-F-C_6H_4$ | $NH_2$ |
| H | $3-F-C_6H_4$ | $NH_2$ |
| H | $4-F-C_6H_4$ | $NH_2$ |
| H | $2-Cl-C_6H_4$ | $NH_2$ |
| H | $3-Cl-C_6H_4$ | $NH_2$ |
| H | $4-Cl-C_6H_4$ | $NH_2$ |
| H | $2-Br-C_6H_4$ | $NH_2$ |
| H | $3-Br-C_6H_4$ | $NH_2$ |
| H | $4-Br-C_6H_4$ | $NH_2$ |
| H | $2-OCH_3-C_6H_4$ | $NH_2$ |
| H | $3-OCH_3-C_6H_4$ | $NH_2$ |
| H | $4-OCH_3-C_6H_4$ | $NH_2$ |
| H | $3-C(CH_3)_3-C_6H_4$ | $NH_2$ |
| H | $4-C(CH_3)_3-C_6H_4$ | $NH_2$ |
| H | $2-CF_3-C_6H_4$ | $NH_2$ |
| H | $3-CF_3-C_6H_4$ | $NH_2$ |
| H | $4-CF_3-C_6H_4$ | $NH_2$ |
| H | $2-NO_2-C_6H_4$ | $NH_2$ |
| H | $3-NO_2-C_6H_4$ | $NH_2$ |
| H | $4-NO_2-C_6H_4$ | $NH_2$ |
| H | $2-CN-C_6H_4$ | $NH_2$ |
| H | $3-CN-C_6H_4$ | $NH_2$ |
| H | $4-CN-C_6H_4$ | $NH_2$ |
| H | $2,4-(OCH_3)_2-C_6H_3$ | $NH_2$ |
| H | $2,4-Cl_2-C_6H_3$ | $NH_2$ |
| H | $3-NO_2-4-CH_3-C_6H_3$ | $NH_2$ |
| H | 2-Naphthyl | $NH_2$ |
| H | Thien-2-yl | $NH_2$ |
| H | Furan-2-yl | $NH_2$ |

| $R^3$ | $R^4$ | $R^5$ |
|---|---|---|
| H | Isoxazol-2-yl | $NH_2$ |
| H | $CH_3$ | $NH_2$ |
| H | $CH_2CH_3$ | $NH_2$ |
| H | $CH(CH_3)_2$ | $NH_2$ |
| H | $CH_2CH(CH_3)_2$ | $NH_2$ |
| H | $C(CH_3)_3$ | $NH_2$ |
| H | Cyclopropyl | $NH_2$ |
| H | $CH_2CH{=}CH_2$ | $NH_2$ |
| H | $CH_2C*CH$ | $NH_2$ *=Dreifachbindung |
| H | $CH_2CH{=}CHCH_3$ | $NH_2$ |
| H | $CH_2C*CCH_3$ | $NH_2$ *=Dreifachbindung |
| H | $CH_2Cl$ | $NH_2$ |
| H | $CH_2CH_2Cl$ | $NH_2$ |
| H | $CH_2CH_2CH_2Cl$ | $NH_2$ |
| H | $CH_2CH{=}CHCl$ | $NH_2$ |
| H | $CH_2OH$ | $NH_2$ |
| H | $CH_2CH_2OH$ | $NH_2$ |
| H | $CH_2CH_2CH_2OH$ | $NH_2$ |
| H | $CH_2OCH_3$ | $NH_2$ |
| H | $CH_2CH_2OCH_3$ | $NH_2$ |
| H | $CH_2CH_2CH_2OCH_3$ | $NH_2$ |
| H | $CH_2CO_2CH_2CH_3$ | $NH_2$ |
| H | $CH_2CH_2CO_2CH_2CH_3$ | $NH_2$ |
| H | $CH_2CH_2CH_2CO_2CH_2CH_3$ | $NH_2$ |
| H | $CH_2NH_2$ | $NH_2$ |
| H | $CH_2CH_2NH_2$ | $NH_2$ |
| H | $CH_2CH_2CH_2NH_2$ | $NH_2$ |
| H | $CH_2N(CH_3)_2$ | $NH_2$ |
| H | $CH_2CH_2N(CH_3)_2$ | $NH_2$ |
| H | $CH_2CH_2CH_2N(CH_3)_2$ | $NH_2$ |
| H | $CH_2NHCOCH_3$ | $NH_2$ |
| H | $CH_2CH_2NHCOCH_3$ | $NH_2$ |
| H | $CH_2CH_2CH_2NHCOCH_3$ | $NH_2$ |
| H | $COCH_3$ | $NH_2$ |
| H | $COCF_3$ | $NH_2$ |
| H | $COC_6H_5$ | $NH_2$ |

| $R^3$ | $R^4$ | $R^5$ |
|---|---|---|
| H | CO-(4-$CH_3$-$C_6H_4$) | $NH_2$ |
| H | $CO_2C_6H_5$ | $NH_2$ |
| H | $CO_2CH_2C_6H_5$ | $NH_2$ |
| H | F | $NH_2$ |
| H | Cl | $NH_2$ |
| H | Br | $NH_2$ |
| H | $OCH_3$ | $NH_2$ |
| H | $CF_3$ | $NH_2$ |
| H | $NO_2$ | $NH_2$ |
| H | CN | $NH_2$ |
| H | $SO_2CH_3$ | $NH_2$ |
| H | $SO_2C_6H_5$ | $NH_2$ |
| H | $SO_2$-(4-$CH_3$-$C_6H_4$) | $NH_2$ |
| H | $CH_2OCH_2C_6H_5$ | $NH_2$ |
| H | $COCH_2CH_2CH_3$ | $NH_2$ |
| H | $CO_2H$ | $NH_2$ |
| H | $CO_2CH_3$ | $NH_2$ |
| H | $CO_2CH_2CH_3$ | $NH_2$ |
| H | $CONH_2$ | $NH_2$ |
| H | $CONHCH_3$ | $NH_2$ |
| H | $CON(CH_3)_2$ | $NH_2$ |
| H | $CONHC_6H_5$ | $NH_2$ |
| H | 3-Pyridyl | $NH_2$ |
| H | 2-Pyridyl | $NH_2$ |
| H | 4-$SCH_3$-$C_6H_4$ | $NH_2$ |
| H | 2,6-$Cl_2$-$C_6H_3$ | $NH_2$ |
| H | 2,6-$OCH_3$-$C_6H_3$ | $NH_2$ |
| H | 2,4-$CH_3$-$C_6H_3$ | $NH_2$ |
| H | 2-$CO_2CH_3$-$C_6H_4$ | $NH_2$ |
| H | 3-$CF_3$-$C_6H_4$ | $NH_2$ |
| H | 4-OH-$C_6H_4$ | $NH_2$ |
| H | 3-Cl-4-$OCH_3$-$C_6H_3$ | $NH_2$ |
| H | $C_6H_5$ | NHCHO |
| H | $C_6H_5$ | $NHCOCH_3$ |
| H | $C_6H_5$ | $NHCOCH_2CH_3$ |
| H | $C_6H_5$ | $NHCOCH(CH_3)_2$ |

| $R^3$ | $R^4$ | $R^5$ |
|---|---|---|
| H | $C_6H_5$ | NHCO-Cyclopropyl |
| H | $C_6H_5$ | $NHCOCH_2C_6H_5$ |
| H | $C_6H_5$ | $NHCOC_6H_5$ |
| H | $C_6H_5$ | $NHCO_2CH_3$ |
| H | $C_6H_5$ | $NHCO_2CH_2CH_3$ |
| H | $C_6H_5$ | $NHCO_2CH(CH_3)_2$ |
| H | $C_6H_5$ | $NHCO_2$-Cyclopropyl |
| H | $C_6H_5$ | $NHCO_2CH_2C_6H_5$ |
| H | $C_6H_5$ | $NHCO_2C_6H_5$ |
| H | $C_6H_5$ | $NHCONH_2$ |
| H | $C_6H_5$ | $NHCONHCH_3$ |
| H | $C_6H_5$ | $NHCON(CH_3)_2$ |
| H | $C_6H_5$ | $NHCONHCH_2CH_3$ |
| H | $C_6H_5$ | $NHCONHCH(CH_3)_2$ |
| H | $C_6H_5$ | NHCONH-Cyclopropyl |
| H | $C_6H_5$ | $NHCONHCH_2C_6H_5$ |
| H | $C_6H_5$ | $NHCONHC_6H_5$ |
| H | $C_6H_5$ | $OCOCH_3$ |
| H | $C_6H_5$ | $OCOCH_2CH_3$ |
| H | $C_6H_5$ | $OCOCH(CH_3)_2$ |
| H | $C_6H_5$ | OCO-Cyclopropyl |
| H | $C_6H_5$ | $OCOCH_2C_6H_5$ |
| H | $C_6H_5$ | $OCOC_6H_5$ |
| H | $C_6H_5$ | $OCH_2C_6H_5$ |
| H | $C_6H_5$ | $CO_2CH_3$ |
| H | $C_6H_5$ | $CO_2CH_2CH_3$ |
| H | $C_6H_5$ | $CO_2CH(CH_3)_2$ |
| H | $C_6H_5$ | $CO_2$-Cyclopropyl |
| H | $C_6H_5$ | $CO_2CH_2C_6H_5$ |
| H | $C_6H_5$ | $CO_2C_6H_5$ |
| H | $C_6H_5$ | $SO_2CH_3$ |
| H | $C_6H_5$ | $SO_2CF_3$ |
| H | $C_6H_5$ | $SO_2CH_2C_6H_5$ |
| H | $C_6H_5$ | $SO_2C_6H_5$ |
| H | $C_6H_5$ | $NHSO_2CH_3$ |
| H | $C_6H_5$ | $NHSO_2CF_3$ |

| $R^3$ | $R^4$ | $R^5$ |
|---|---|---|
| H | $C_6H_5$ | $NHSO_2CH_2C_6H_5$ |
| H | $C_6H_5$ | $NHSO_2C_6H_5$ |
| H | CN | NHCHO |
| H | CN | $NHCOCH_3$ |
| H | CN | $NHCOCH_2CH_3$ |
| H | CN | $NHCOCH(CH_3)_2$ |
| H | CN | NHCO-Cyclopropyl |
| H | CN | $NHCOCH_2C_6H_5$ |
| H | CN | $NHCOC_6H_5$ |
| H | CN | $NHCO_2CH_3$ |
| H | CN | $NHCO_2CH_2CH_3$ |
| H | CN | $NHCO_2CH(CH_3)_2$ |
| H | CN | $NHCO_2$-Cyclopropyl |
| H | CN | $NHCO_2CH_2C_6H_5$ |
| H | CN | $NHCO_2C_6H_5$ |
| H | CN | $NHCONH_2$ |
| H | CN | $NHCONHCH_3$ |
| H | CN | $NHCON(CH_3)_2$ |
| H | CN | $NHCONHCH_2CH_3$ |
| H | CN | $NHCONHCH(CH_3)_2$ |
| H | CN | NHCONH-Cyclopropyl |
| H | CN | $NHCONHCH_2C_6H_5$ |
| H | CN | $NHCONHC_6H_5$ |
| H | CN | $OCOCH_3$ |
| H | CN | $OCOCH_2CH_3$ |
| H | CN | $OCOCH(CH_3)_2$ |
| H | CN | OCO-Cyclopropyl |
| H | CN | $OCOCH_2C_6H_5$ |
| H | CN | $OCOC_6H_5$ |
| H | CN | $OCH_2C_6H_5$ |
| H | CN | $CO_2CH_3$ |
| H | CN | $CO_2CH_2CH_3$ |
| H | CN | $CO_2CH(CH_3)_2$ |
| H | CN | $CO_2$-Cyclopropyl |
| H | CN | $CO_2CH_2C_6H_5$ |
| H | CN | $CO_2C_6H_5$ |

| $R^3$ | $R^4$ | $R^5$ |
|---|---|---|
| H | CN | $SO_2CH_3$ |
| H | CN | $SO_2CF_3$ |
| H | CN | $SO_2CH_2C_6H_5$ |
| H | CN | $SO_2C_6H_5$ |
| H | CN | $NHSO_2CH_3$ |
| H | CN | $NHSO_2CF_3$ |
| H | CN | $NHSO_2CH_2C_6H_5$ |
| H | CN | $NHSO_2C_6H_5$ |
| H | $2,6-Cl_2-C_6H_3$ | NHCHO |
| H | $2,6-Cl_2-C_6H_3$ | $NHCOCH_3$ |
| H | $2,6-Cl_2-C_6H_3$ | $NHCOCH_2CH_3$ |
| H | $2,6-Cl_2-C_6H_3$ | $NHCOCH(CH_3)_2$ |
| H | $2,6-Cl_2-C_6H_3$ | NHCO-Cyclopropyl |
| H | $2,6-Cl_2-C_6H_3$ | $NHCOCH_2C_6H_5$ |
| H | $2,6-Cl_2-C_6H_3$ | $NHCOC_6H_5$ |
| H | $2,6-Cl_2-C_6H_3$ | $NHCO_2CH_3$ |
| H | $2,6-Cl_2-C_6H_3$ | $NHCO_2CH_2CH_3$ |
| H | $2,6-Cl_2-C_6H_3$ | $NHCO_2CH(CH_3)_2$ |
| H | $2,6-Cl_2-C_6H_3$ | $NHCO_2$-Cyclopropyl |
| H | $2,6-Cl_2-C_6H_3$ | $NHCO_2CH_2C_6H_5$ |
| H | $2,6-Cl_2-C_6H_3$ | $NHCO_2C_6H_5$ |
| H | $2,6-Cl_2-C_6H_3$ | $NHCONH_2$ |
| H | $2,6-Cl_2-C_6H_3$ | $NHCONHCH_3$ |
| H | $2,6-Cl_2-C_6H_3$ | $NHCON(CH_3)_2$ |
| H | $2,6-Cl_2-C_6H_3$ | $NHCONHCH_2CH_3$ |
| H | $2,6-Cl_2-C_6H_3$ | $NHCONHCH(CH_3)_2$ |
| H | $2,6-Cl_2-C_6H_3$ | NHCONH-Cyclopropyl |
| H | $2,6-Cl_2-C_6H_3$ | $NHCONHCH_2C_6H_5$ |
| H | $2,6-Cl_2-C_6H_3$ | $NHCONHC_6H_5$ |
| H | $2,6-Cl_2-C_6H_3$ | $OCOCH_3$ |
| H | $2,6-Cl_2-C_6H_3$ | $OCOCH_2CH_3$ |
| H | $2,6-Cl_2-C_6H_3$ | $OCOCH(CH_3)_2$ |
| H | $2,6-Cl_2-C_6H_3$ | OCO-Cyclopropyl |
| H | $2,6-Cl_2-C_6H_3$ | $OCOCH_2C_6H_5$ |
| H | $2,6-Cl_2-C_6H_3$ | $OCOC_6H_5$ |
| H | $2,6-Cl_2-C_6H_3$ | $CO_2CH_3$ |

| $R^3$ | $R^4$ | $R^5$ |
|---|---|---|
| H | 2,6-$Cl_2$-$C_6H_3$ | $CO_2CH_2CH_3$ |
| H | 2,6-$Cl_2$-$C_6H_3$ | $CO_2CH(CH_3)_2$ |
| H | 2,6-$Cl_2$-$C_6H_3$ | $CO_2$-Cyclopropyl |
| H | 2,6-$Cl_2$-$C_6H_3$ | $CO_2CH_2C_6H_5$ |
| H | 2,6-$Cl_2$-$C_6H_3$ | $CO_2C_6H_5$ |
| H | 2,6-$Cl_2$-$C_6H_3$ | $SO_2CH_3$ |
| H | 2,6-$Cl_2$-$C_6H_3$ | $SO_2CF_3$ |
| H | 2,6-$Cl_2$-$C_6H_3$ | $SO_2CH_2C_6H_5$ |
| H | 2,6-$Cl_2$-$C_6H_3$ | $SO_2C_6H_5$ |
| H | 2,6-$Cl_2$-$C_6H_3$ | $NHSO_2CH_3$ |
| H | 2,6-$Cl_2$-$C_6H_3$ | $NHSO_2CF_3$ |
| H | 2,6-$Cl_2$-$C_6H_3$ | $NHSO_2CH_2C_6H_5$ |
| H | 2,6-$Cl_2$-$C_6H_3$ | $NHSO_2C_6H_5$ |

Tabelle B

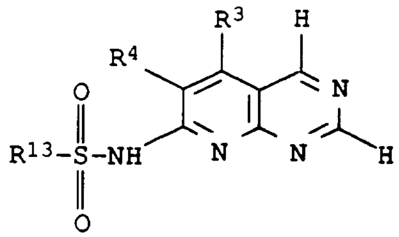

I.9

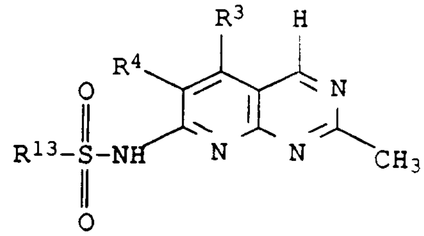

I.10

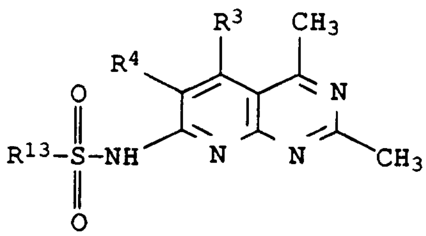

I.11

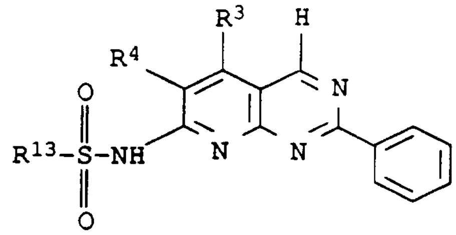

I.12

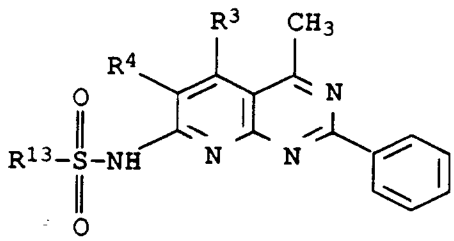

I.13

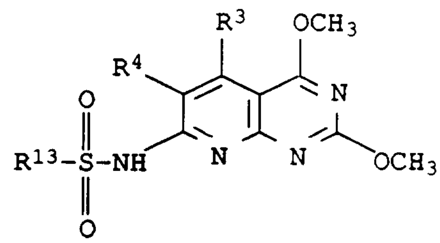

I.14

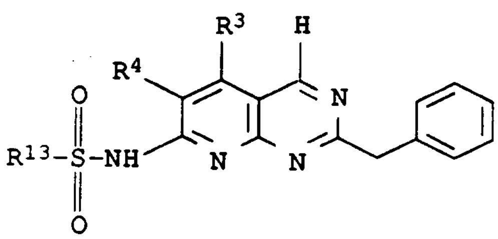

I.15

| $R^3$ | $R^4$ | $R^{13}$ |
|---|---|---|
| H | H | $C_6H_5$ |
| H | H | $2-CH_3-C_6H_4$ |
| H | H | $3-CH_3-C_6H_4$ |
| H | H | $4-CH_3-C_6H_4$ |
| H | H | $2-F-C_6H_4$ |
| H | H | $3-F-C_6H_4$ |
| H | H | $4-F-C_6H_4$ |
| H | H | $2-Cl-C_6H_4$ |
| H | H | $3-Cl-C_6H_4$ |
| H | H | $4-Cl-C_6H_4$ |
| H | H | $2-Br-C_6H_4$ |
| H | H | $3-Br-C_6H_4$ |
| H | H | $4-Br-C_6H_4$ |
| H | H | $2-OH-C_6H_4$ |
| H | H | $3-OH-C_6H_4$ |
| H | H | $4-OH-C_6H_4$ |
| H | H | $2-OCH_3-C_6H_4$ |
| H | H | $3-OCH_3-C_6H_4$ |
| H | H | $4-OCH_3-C_6H_4$ |
| H | H | $4-C_6H_5-C_6H_4$ |
| H | H | $3-C(CH_3)_3-C_6H_4$ |
| H | H | $4-C(CH_3)_3-C_6H_4$ |
| H | H | $2-CF_3-C_6H_4$ |
| H | H | $3-CF_3-C_6H_4$ |
| H | H | $4-CF_3-C_6H_4$ |
| H | H | $2-NO_2-C_6H_4$ |
| H | H | $3-NO_2-C_6H_4$ |
| H | H | $4-NO_2-C_6H_4$ |
| H | H | $2-CN-C_6H_4$ |
| H | H | $3-CN-C_6H_4$ |
| H | H | $4-CN-C_6H_4$ |
| H | H | $2-(CO_2C_2H_5)-C_6H_4$ |
| H | H | $3-(CO_2C_2H_5)-C_6H_4$ |
| H | H | $4-(CO_2C_2H_5)-C_6H_4$ |
| H | H | $2-(CO_2CH_3)-C_6H_4$ |
| H | H | $3-(CO_2CH_3)-C_6H_4$ |

| $R^3$ | $R^4$ | $R^{13}$ |
|---|---|---|
| H | H | 4-$(CO_2CH_3)$-$C_6H_4$ |
| H | H | 2-$CONH_2$-$C_6H_4$ |
| H | H | 3-$CONH_2$-$C_6H_4$ |
| H | H | 4-$CONH_2$-$C_6H_4$ |
| H | H | 2-$NH_2$-$C_6H_4$ |
| H | H | 3-$NH_2$-$C_6H_4$ |
| H | H | 4-$NH_2$-$C_6H_4$ |
| H | H | 2-$SCH_3$-$C_6H_4$ |
| H | H | 3-$SCH_3$-$C_6H_4$ |
| H | H | 4-$SCH_3$-$C_6H_4$ |
| H | H | 2,4-$(CH_3)_2$-$C_6H_3$ |
| H | H | 3,4-$(CH_3)_2$-$C_6H_3$ |
| H | H | 2,6-$(CH_3)_2$-$C_6H_3$. |
| H | H | 2,4-$(OCH_3)_2$-$C_6H_3$ |
| H | H | 3,4-$(OCH_3)_2$-$C_6H_3$ |
| H | H | 2,6-$(OCH_3)_2$-$C_6H_3$ |
| H | H | 2,4-$F_2$-$C_6H_3$ |
| H | H | 3,4-$F_2$-$C_6H_3$ |
| H | H | 2,6-$F_2$-$C_6H_3$ |
| H | H | 2,4-$Cl_2$-$C_6H_3$ |
| H | H | 3,4-$Cl_2$-$C_6H_3$ |
| H | H | 2,6-$Cl_2$-$C_6H_3$ |
| H | H | 2,4-$(OH)_2$-$C_6H_3$ |
| H | H | 3,4-$(OH)_2$-$C_6H_3$ |
| H | H | 2,6-$(OH)_2$-$C_6H_3$ |
| H | H | 2-Cl-6-$CH_3$-$C_6H_3$ |
| H | H | 2-$CO_2CH_3$-6-$CH_3$-$C_6H_3$ |
| H | H | 3-$NO_2$-4-$CH_3$-$C_6H_3$ |
| H | H | 3-$NO_2$-4-F-$C_6H_3$ |
| H | H | 3-$NO_2$-4-Cl-$C_6H_3$ |
| H | H | 3-$NO_2$-4-$OCH_3$-$C_6H_3$ |
| H | H | 2-Naphthyl |
| H | H | Thien-2-yl |
| H | H | Thien-3-yl |
| H | H | 5-$CH_3$-thien-2-yl |
| H | H | 5-Cl-thien-2-yl |

| $R^3$ | $R^4$ | $R^{13}$ |
|---|---|---|
| H | H | 5-Br-thien-2-yl |
| H | H | 2,5-$(CH_3)_2$-thien-3-yl |
| H | H | Thiazol-2-yl |
| H | H | Thiazol-4-yl |
| H | H | 5-$CH_3$-thiazol-2-yl |
| H | H | 5-Cl-thiazol-2-yl |
| H | H | 5-Br-thiazol-2-yl |
| H | H | 2,5-$(CH_3)_2$-thiazol-4-yl |
| H | H | Furan-2-yl |
| H | H | Furan-3-yl |
| H | H | 5-$CH_3$-furan-2-yl |
| H | H | 5-Cl-furan-2-yl |
| H | H | Pyrrol-2-yl |
| H | H | Pyrrol-3-yl |
| H | H | 5-$CH_3$-pyrrol-2-yl |
| H | H | 5-Br-pyrrol-2-yl |
| H | H | Oxazol-4-yl |
| H | H | Imidazol-2-yl |
| H | H | Pyridin-2-yl |
| H | H | Pyridin-3-yl |
| H | H | Pyridin-4-yl |
| H | H | Pyrazin-3-yl |
| H | H | Pyrazin-4-yl |
| H | H | Pyrrol-2-yl |
| H | H | Pyrimidin-2-yl |
| H | H | Pyrimidin-4-yl |
| H | H | Pyrimidin-5-yl |
| H | CN | $C_6H_5$ |
| H | CN | 2-$CH_3$-$C_6H_4$ |
| H | CN | 3-$CH_3$-$C_6H_4$ |
| H | CN | 4-$CH_3$-$C_6H_4$ |
| H | CN | 2-F-$C_6H_4$ |
| H | CN | 3-F-$C_6H_4$ |
| H | CN | 4-F-$C_6H_4$ |
| H | CN | 2-Cl-$C_6H_4$ |
| H | CN | 3-Cl-$C_6H_4$ |

| $R^3$ | $R^4$ | $R^{13}$ |
|---|---|---|
| H | CN | $4-Cl-C_6H_4$ |
| H | CN | $2-Br-C_6H_4$ |
| H | CN | $3-Br-C_6H_4$ |
| H | CN | $4-Br-C_6H_4$ |
| H | CN | $2-OH-C_6H_4$ |
| H | CN | $3-OH-C_6H_4$ |
| H | CN | $4-OH-C_6H_4$ |
| H | CN | $2-OCH_3-C_6H_4$ |
| H | CN | $3-OCH_3-C_6H_4$ |
| H | CN | $4-OCH_3-C_6H_4$ |
| H | CN | $4-C_6H_5-C_6H_4$ |
| H | CN | $2-Cl-6-CH_3-C_6H_3$ |
| H | CN | $2-CO_2CH_3-6-CH_3-C_6H_3$ |
| H | CN | $2,5-(OCH_3)_2-C_6H_3$ |
| H | CN | $2,5-Cl_2-C_6H_3$ |
| H | CN | $2,4,5-Cl_3-C_6H_2$ |
| H | CN | $2-CO_2CH_3-C_6H_3$ |
| H | CN | $5-Cl-2-OCH_3-C_6H_3$ |
| H | CN | $5-NO_2-2-Cl-C_6H_3$ |
| H | CN | $2-Cl-6-cyclopentenyl-C_6H_3$ |
| H | CN | 3-Cl-thien-2-yl |
| H | CN | $3-C(CH_3)_3-C_6H_4$ |
| H | CN | $4-C(CH_3)_3-C_6H_4$ |
| H | CN | $2-CF_3-C_6H_4$ |
| H | CN | $3-CF_3-C_6H_4$ |
| H | CN | $4-CF_3-C_6H_4$ |
| H | CN | $2-NO_2-C_6H_4$ |
| H | CN | $3-NO_2-C_6H_4$ |
| H | CN | $4-NO_2-C_6H_4$ |
| H | CN | $2-CN-C_6H_4$ |
| H | CN | $3-CN-C_6H_4$ |
| H | CN | $4-CN-C_6H_4$ |
| H | CN | $2-(CO_2CH_3)-C_6H_4$ |
| H | CN | $3-(CO_2CH_3)-C_6H_4$ |
| H | CN | $4-(CO_2CH_3)-C_6H_4$ |
| H | CN | $2-(CO_2C_2H_5)-C_6H_4$ |

| $R^3$ | $R^4$ | $R^{13}$ |
|---|---|---|
| H | CN | $3-(CO_2C_2H_5)-C_6H_4$ |
| H | CN | $4-(CO_2C_2H_5)-C_6H_4$ |
| H | CN | $2-CONH_2-C_6H_4$ |
| H | CN | $3-CONH_2-C_6H_4$ |
| H | CN | $4-CONH_2-C_6H_4$ |
| H | CN | $2-NH_2-C_6H_4$ |
| H | CN | $3-NH_2-C_6H_4$ |
| H | CN | $4-NH_2-C_6H_4$ |
| H | CN | $2-SCH_3-C_6H_4$ |
| H | CN | $3-SCH_3-C_6H_4$ |
| H | CN | $4-SCH_3-C_6H_4$ |
| H | CN | $2,4-(CH_3)_2-C_6H_3$ |
| H | CN | $3,4-(CH_3)_2-C_6H_3$ |
| H | CN | $2,6-(CH_3)_2-C_6H_3$ |
| H | CN | $2,4-(OCH_3)_2-C_6H_3$ |
| H | CN | $3,4-(OCH_3)_2-C_6H_3$ |
| H | CN | $2,6-(OCH_3)_2-C_6H_3$ |
| H | CN | $2,4-F_2-C_6H_3$ |
| H | CN | $3,4-F_2-C_6H_3$ |
| H | CN | $2,6-F_2-C_6H_3$ |
| H | CN | $2,4-Cl_2-C_6H_3$ |
| H | CN | $3,4-Cl_2-C_6H_3$ |
| H | CN | $2,6-Cl_2-C_6H_3$ |
| H | CN | $2,4-(OH)_2-C_6H_3$ |
| H | CN | $3,4-(OH)_2-C_6H_3$ |
| H | CN | $2,6-(OH)_2-C_6H_3$ |
| H | CN | $3-NO_2-4-CH_3-C_6H_3$ |
| H | CN | $3-NO_2-4-F-C_6H_3$ |
| H | CN | $3-NO_2-4-Cl-C_6H_3$ |
| H | CN | $3-NO_2-4-OCH_3-C_6H_3$ |
| H | CN | 2-Naphthyl |
| H | CN | Thien-2-yl |
| H | CN | Thien-3-yl |
| H | CN | $5-CH_3$-thien-2-yl |
| H | CN | 5-Cl-thien-2-yl |
| H | CN | 5-Br-thien-2-yl |

| $R^3$ | $R^4$ | $R^{13}$ |
|---|---|---|
| H | CN | 2,5-$(CH_3)_2$-thien-3-yl |
| H | CN | Thiazol-2-yl |
| H | CN | Thiazol-4-yl |
| H | CN | 5-$CH_3$-thiazol-2-yl |
| H | CN | 5-Cl-thiazol-2-yl |
| H | CN | 5-Br-thiazol-2-yl |
| H | CN | 2,5-$(CH_3)_2$-thiazol-4-yl |
| H | CN | Furan-2-yl |
| H | CN | Furan-3-yl |
| H | CN | 5-$CH_3$-furan-2-yl |
| H | CN | 5-Cl-furan-2-yl |
| H | CN | Pyrrol-2-yl |
| H | CN | Pyrrol-3-yl |
| H | CN | 5-$CH_3$-pyrrol-2-yl |
| H | CN | 5-Br-pyrrol-2-yl |
| H | CN | Oxazol-4-yl |
| H | CN | Imidazol-2-yl |
| H | CN | Pyridin-2-yl |
| H | CN | Pyridin-3-yl |
| H | CN | Pyridin-4-yl |
| H | CN | Pyrazin-3-yl |
| H | CN | Pyrazin-4-yl |
| H | CN | Pyrrol-2-yl |
| H | CN | Pyrimidin-2-yl |
| H | CN | Pyrimidin-4-yl |
| H | CN | Pyrimidin-5-yl |
| H | $C_6H_5$ | $C_6H_5$ |
| H | $C_6H_5$ | 2-$CH_3$-$C_6H_4$ |
| H | $C_6H_5$ | 3-$CH_3$-$C_6H_4$ |
| H | $C_6H_5$ | 4-$CH_3$-$C_6H_4$ |
| H | $C_6H_5$ | 2-F-$C_6H_4$ |
| H | $C_6H_5$ | 3-F-$C_6H_4$ |
| H | $C_6H_5$ | 4-F-$C_6H_4$ |
| H | $C_6H_5$ | 2-Cl-$C_6H_4$ |
| H | $C_6H_5$ | 3-Cl-$C_6H_4$ |
| H | $C_6H_5$ | 4-Cl-$C_6H_4$ |

| $R^3$ | $R^4$ | $R^{13}$ |
|---|---|---|
| H | $C_6H_5$ | $2-Br-C_6H_4$ |
| H | $C_6H_5$ | $3-Br-C_6H_4$ |
| H | $C_6H_5$ | $4-Br-C_6H_4$ |
| H | $C_6H_5$ | $2-OH-C_6H_4$ |
| H | $C_6H_5$ | $3-OH-C_6H_4$ |
| H | $C_6H_5$ | $4-OH-C_6H_4$ |
| H | $C_6H_5$ | $2-OCH_3-C_6H_4$ |
| H | $C_6H_5$ | $3-OCH_3-C_6H_4$ |
| H | $C_6H_5$ | $4-OCH_3-C_6H_4$ |
| H | $C_6H_5$ | $2-Cl-6-CH_3-C_6H_3$ |
| H | $C_6H_5$ | $2-CO_2CH_3-6-CH_3-C_6H_3$ |
| H | $C_6H_5$ | $4-C_6H_5-C_6H_4$ |
| H | $C_6H_5$ | $3-C(CH_3)_3-C_6H_4$ |
| H | $C_6H_5$ | $4-C(CH_3)_3-C_6H_4$ |
| H | $C_6H_5$ | $2-CF_3-C_6H_4$ |
| H | $C_6H_5$ | $3-CF_3-C_6H_4$ |
| H | $C_6H_5$ | $4-CF_3-C_6H_4$ |
| H | $C_6H_5$ | $2-NO_2-C_6H_4$ |
| H | $C_6H_5$ | $3-NO_2-C_6H_4$ |
| H | $C_6H_5$ | $4-NO_2-C_6H_4$ |
| H | $C_6H_5$ | $2-CN-C_6H_4$ |
| H | $C_6H_5$ | $3-CN-C_6H_4$ |
| H | $C_6H_5$ | $4-CN-C_6H_4$ |
| H | $C_6H_5$ | $2,5-(OCH_3)_2-C_6H_3$ |
| H | $C_6H_5$ | $2,5-Cl_2-C_6H_3$ |
| H | $C_6H_5$ | $2,4,5-Cl_3-C_6H_2$ |
| H | $C_6H_5$ | $2-CO_2CH_3-C_6H_3$ |
| H | $C_6H_5$ | $5-Cl-2-OCH_3-C_6H_3$ |
| H | $C_6H_5$ | $5-NO_2-2-Cl-C_6H_3$ |
| H | $C_6H_5$ | 2-Cl-6-cyclopentenyl-$C_6H_3$ |
| H | $C_6H_5$ | 3-Cl-thien-2-yl |
| H | $C_6H_5$ | $2-(CO_2CH_3)-C_6H_4$ |
| H | $C_6H_5$ | $3-(CO_2CH_3)-C_6H_4$ |
| H | $C_6H_5$ | $4-(CO_2CH_3)-C_6H_4$ |
| H | $C_6H_5$ | $2-(CO_2C_2H_5)-C_6H_4$ |
| H | $C_6H_5$ | $3-(CO_2C_2H_5)-C_6H_4$ |

| $R^3$ | $R^4$ | $R^{13}$ |
|---|---|---|
| H | $C_6H_5$ | 4-$(CO_2C_2H_5)$-$C_6H_4$ |
| H | $C_6H_5$ | 2-$CONH_2$-$C_6H_4$ |
| H | $C_6H_5$ | 3-$CONH_2$-$C_6H_4$ |
| H | $C_6H_5$ | 4-$CONH_2$-$C_6H_4$ |
| H | $C_6H_5$ | 2-$NH_2$-$C_6H_4$ |
| H | $C_6H_5$ | 3-$NH_2$-$C_6H_4$ |
| H | $C_6H_5$ | 4-$NH_2$-$C_6H_4$ |
| H | $C_6H_5$ | 2-$SCH_3$-$C_6H_4$ |
| H | $C_6H_5$ | 3-$SCH_3$-$C_6H_4$ |
| H | $C_6H_5$ | 4-$SCH_3$-$C_6H_4$ |
| H | $C_6H_5$ | 2,4-$(CH_3)_2$-$C_6H_3$ |
| H | $C_6H_5$ | 3,4-$(CH_3)_2$-$C_6H_3$ |
| H | $C_6H_5$ | 2,6-$(CH_3)_2$-$C_6H_3$ |
| H | $C_6H_5$ | 2,4-$(OCH_3)_2$-$C_6H_3$ |
| H | $C_6H_5$ | 3,4-$(OCH_3)_2$-$C_6H_3$ |
| H | $C_6H_5$ | 2,6-$(OCH_3)_2$-$C_6H_3$ |
| H | $C_6H_5$ | 2,4-$F_2$-$C_6H_3$ |
| H | $C_6H_5$ | 3,4-$F_2$-$C_6H_3$ |
| H | $C_6H_5$ | 2,6-$F_2$-$C_6H_3$ |
| H | $C_6H_5$ | 2,4-$Cl_2$-$C_6H_3$ |
| H | $C_6H_5$ | 3,4-$Cl_2$-$C_6H_3$ |
| H | $C_6H_5$ | 2,6-$Cl_2$-$C_6H_3$ |
| H | $C_6H_5$ | 2,4-$(OH)_2$-$C_6H_3$ |
| H | $C_6H_5$ | 3,4-$(OH)_2$-$C_6H_3$ |
| H | $C_6H_5$ | 2,6-$(OH)_2$-$C_6H_3$ |
| H | $C_6H_5$ | 3-$NO_2$-4-$CH_3$-$C_6H_3$ |
| H | $C_6H_5$ | 3-$NO_2$-4-F-$C_6H_3$ |
| H | $C_6H_5$ | 3-$NO_2$-4-Cl-$C_6H_3$ |
| H | $C_6H_5$ | 3-$NO_2$-4-$OCH_3$-$C_6H_3$ |
| H | $C_6H_5$ | 2-Naphthyl |
| H | $C_6H_5$ | Thien-2-yl |
| H | $C_6H_5$ | Thien-3-yl |
| H | $C_6H_5$ | 5-$CH_3$-thien-2-yl |
| H | $C_6H_5$ | 5-Cl-thien-2-yl |
| H | $C_6H_5$ | 5-Br-thien-2-yl |
| H | $C_6H_5$ | 2,5-$(CH_3)_2$-thien-3-yl |

| $R^3$ | $R^4$ | $R^{13}$ |
|---|---|---|
| H | $C_6H_5$ | Thiazol-2-yl |
| H | $C_6H_5$ | Thiazol-4-yl |
| H | $C_6H_5$ | 5-$CH_3$-thiazol-2-yl |
| H | $C_6H_5$ | 5-Cl-thiazol-2-yl |
| H | $C_6H_5$ | 5-Br-thiazol-2-yl |
| H | $C_6H_5$ | 2,5-$(CH_3)_2$-thiazol-4-yl |
| H | $C_6H_5$ | Furan-2-yl |
| H | $C_6H_5$ | Furan-3-yl |
| H | $C_6H_5$ | 5-$CH_3$-furan-2-yl |
| H | $C_6H_5$ | 5-Cl-furan-2-yl |
| H | $C_6H_5$ | Pyrrol-2-yl |
| H | $C_6H_5$ | Pyrrol-3-yl |
| H | $C_6H_5$ | 5-$CH_3$-pyrrol-2-yl |
| H | $C_6H_5$ | 5-Br-pyrrol-2-yl |
| H | $C_6H_5$ | Oxazol-4-yl |
| H | $C_6H_5$ | Imidazol-2-yl |
| H | $C_6H_5$ | Pyridin-2-yl |
| H | $C_6H_5$ | Pyridin-3-yl |
| H | $C_6H_5$ | Pyridin-4-yl |
| H | $C_6H_5$ | Pyrazin-3-yl |
| H | $C_6H_5$ | Pyrazin-4-yl |
| H | $C_6H_5$ | Pyrrol-2-yl |
| H | $C_6H_5$ | Pyrimidin-2-yl |
| H | $C_6H_5$ | Pyrimidin-4-yl |
| H | $C_6H_5$ | Pyrimidin-5-yl |
| H | $SO_2CH_3$ | $C_6H_5$ |
| H | $SO_2CH_3$ | 2-$CH_3$-$C_6H_4$ |
| H | $SO_2CH_3$ | 3-$CH_3$-$C_6H_4$ |
| H | $SO_2CH_3$ | 4-$CH_3$-$C_6H_4$ |
| H | $SO_2CH_3$ | 2-F-$C_6H_4$ |
| H | $SO_2CH_3$ | 3-F-$C_6H_4$ |
| H | $SO_2CH_3$ | 4-F-$C_6H_4$ |
| H | $SO_2CH_3$ | 2-Cl-$C_6H_4$ |
| H | $SO_2CH_3$ | 3-Cl-$C_6H_4$ |
| H | $SO_2CH_3$ | 4-Cl-$C_6H_4$ |
| H | $SO_2CH_3$ | 2-Br-$C_6H_4$ |

| $R^3$ | $R^4$ | $R^{13}$ |
|---|---|---|
| H | $SO_2CH_3$ | $3-Br-C_6H_4$ |
| H | $SO_2CH_3$ | $4-Br-C_6H_4$ |
| H | $SO_2CH_3$ | $2-OH-C_6H_4$ |
| H | $SO_2CH_3$ | $3-OH-C_6H_4$ |
| H | $SO_2CH_3$ | $4-OH-C_6H_4$ |
| H | $SO_2CH_3$ | $2-OCH_3-C_6H_4$ |
| H | $SO_2CH_3$ | $3-OCH_3-C_6H_4$ |
| H | $SO_2CH_3$ | $4-OCH_3-C_6H_4$ |
| H | $SO_2CH_3$ | $4-C_6H_5-C_6H_4$ |
| H | $SO_2CH_3$ | $3-C(CH_3)_3-C_6H_4$ |
| H | $SO_2CH_3$ | $4-C(CH_3)_3-C_6H_4$ |
| H | $SO_2CH_3$ | $2-CF_3-C_6H_4$ |
| H | $SO_2CH_3$ | $3-CF_3-C_6H_4$ |
| H | $SO_2CH_3$ | $4-CF_3-C_6H_4$ |
| H | $SO_2CH_3$ | $2-NO_2-C_6H_4$ |
| H | $SO_2CH_3$ | $3-NO_2-C_6H_4$ |
| H | $SO_2CH_3$ | $4-NO_2-C_6H_4$ |
| H | $SO_2CH_3$ | $2-CN-C_6H_4$ |
| H | $SO_2CH_3$ | $3-CN-C_6H_4$ |
| H | $SO_2CH_3$ | $4-CN-C_6H_4$ |
| H | $SO_2CH_3$ | $2-Cl-6-CH_3-C_6H_3$ |
| H | $SO_2CH_3$ | $2-CO_2CH_3-6-CH_3-C_6H_3$ |
| H | $SO_2CH_3$ | $2,5-(OCH_3)_2-C_6H_3$ |
| H | $SO_2CH_3$ | $2,5-Cl_2-C_6H_3$ |
| H | $SO_2CH_3$ | $2,4,5-Cl_3-C_6H_2$ |
| H | $SO_2CH_3$ | $2-CO_2CH_3-C_6H_3$ |
| H | $SO_2CH_3$ | $5-Cl-2-OCH_3-C_6H_3$ |
| H | $SO_2CH_3$ | $5-NO_2-2-Cl-C_6H_3$ |
| H | $SO_2CH_3$ | 2-Cl-6-cyclopentenyl-$C_6H_3$ |
| H | $SO_2CH_3$ | 3-Cl-thien-2-yl |
| H | $SO_2CH_3$ | $2-(CO_2CH_3)-C_6H_4$ |
| H | $SO_2CH_3$ | $3-(CO_2CH_3)-C_6H_4$ |
| H | $SO_2CH_3$ | $4-(CO_2CH_3)-C_6H_4$ |
| H | $SO_2CH_3$ | $2-(CO_2C_2H_5)-C_6H_4$ |
| H | $SO_2CH_3$ | $3-(CO_2C_2H_5)-C_6H_4$ |
| H | $SO_2CH_3$ | $4-(CO_2C_2H_5)-C_6H_4$ |

| $R^3$ | $R^4$ | $R^{13}$ |
|---|---|---|
| H | $SO_2CH_3$ | 2-$CONH_2$-$C_6H_4$ |
| H | $SO_2CH_3$ | 3-$CONH_2$-$C_6H_4$ |
| H | $SO_2CH_3$ | 4-$CONH_2$-$C_6H_4$ |
| H | $SO_2CH_3$ | 2-$NH_2$-$C_6H_4$ |
| H | $SO_2CH_3$ | 3-$NH_2$-$C_6H_4$ |
| H | $SO_2CH_3$ | 4-$NH_2$-$C_6H_4$ |
| H | $SO_2CH_3$ | 2-$SCH_3$-$C_6H_4$ |
| H | $SO_2CH_3$ | 3-$SCH_3$-$C_6H_4$ |
| H | $SO_2CH_3$ | 4-$SCH_3$-$C_6H_4$ |
| H | $SO_2CH_3$ | 2,4-$(CH_3)_2$-$C_6H_3$ |
| H | $SO_2CH_3$ | 3,4-$(CH_3)_2$-$C_6H_3$ |
| H | $SO_2CH_3$ | 2,6-$(CH_3)_2$-$C_6H_3$ |
| H | $SO_2CH_3$ | 2,4-$(OCH_3)_2$-$C_6H_3$ |
| H | $SO_2CH_3$ | 3,4-$(OCH_3)_2$-$C_6H_3$ |
| H | $SO_2CH_3$ | 2,6-$(OCH_3)_2$-$C_6H_3$ |
| H | $SO_2CH_3$ | 2,4-$F_2$-$C_6H_3$ |
| H | $SO_2CH_3$ | 3,4-$F_2$-$C_6H_3$ |
| H | $SO_2CH_3$ | 2,6-$F_2$-$C_6H_3$ |
| H | $SO_2CH_3$ | 2,4-$Cl_2$-$C_6H_3$ |
| H | $SO_2CH_3$ | 3,4-$Cl_2$-$C_6H_3$ |
| H | $SO_2CH_3$ | 2,6-$Cl_2$-$C_6H_3$ |
| H | $SO_2CH_3$ | 2,4-$(OH)_2$-$C_6H_3$ |
| H | $SO_2CH_3$ | 3,4-$(OH)_2$-$C_6H_3$ |
| H | $SO_2CH_3$ | 2,6-$(OH)_2$-$C_6H_3$ |
| H | $SO_2CH_3$ | 3-$NO_2$-4-$CH_3$-$C_6H_3$ |
| H | $SO_2CH_3$ | 3-$NO_2$-4-F-$C_6H_3$ |
| H | $SO_2CH_3$ | 3-$NO_2$-4-Cl-$C_6H_3$ |
| H | $SO_2CH_3$ | 3-$NO_2$-4-$OCH_3$-$C_6H_3$ |
| H | $SO_2CH_3$ | 2-Naphthyl |
| H | $SO_2CH_3$ | Thien-2-yl |
| H | $SO_2CH_3$ | Thien-3-yl |
| H | $SO_2CH_3$ | 5-$CH_3$-thien-2-yl |
| H | $SO_2CH_3$ | 5-Cl-thien-2-yl |
| H | $SO_2CH_3$ | 5-Br-thien-2-yl |
| H | $SO_2CH_3$ | 2,5-$(CH_3)_2$-thien-3-yl |
| H | $SO_2CH_3$ | Thiazol-2-yl |

| $R^3$ | $R^4$ | $R^{13}$ |
|---|---|---|
| H | $SO_2CH_3$ | Thiazol-4-yl |
| H | $SO_2CH_3$ | 5-$CH_3$-thiazol-2-yl |
| H | $SO_2CH_3$ | 5-Cl-thiazol-2-yl |
| H | $SO_2CH_3$ | 5-Br-thiazol-2-yl |
| H | $SO_2CH_3$ | 2,5-$(CH_3)_2$-thiazol-4-yl |
| H | $SO_2CH_3$ | Furan-2-yl |
| H | $SO_2CH_3$ | Furan-3-yl |
| H | $SO_2CH_3$ | 5-$CH_3$-furan-2-yl |
| H | $SO_2CH_3$ | 5-Cl-furan-2-yl |
| H | $SO_2CH_3$ | Pyrrol-2-yl |
| H | $SO_2CH_3$ | Pyrrol-3-yl |
| H | $SO_2CH_3$ | 5-$CH_3$-pyrrol-2-yl |
| H | $SO_2CH_3$ | 5-Br-pyrrol-2-yl |
| H | $SO_2CH_3$ | Oxazol-4-yl |
| H | $SO_2CH_3$ | Imidazol-2-yl |
| H | $SO_2CH_3$ | Pyridin-2-yl |
| H | $SO_2CH_3$ | Pyridin-3-yl |
| H | $SO_2CH_3$ | Pyridin-4-yl |
| H | $SO_2CH_3$ | Pyrazin-3-yl |
| H | $SO_2CH_3$ | Pyrazin-4-yl |
| H | $SO_2CH_3$ | Pyrrol-2-yl |
| H | $SO_2CH_3$ | Pyrimidin-2-yl |
| H | $SO_2CH_3$ | Pyrimidin-4-yl |
| H | $SO_2CH_3$ | Pyrimidin-5-yl |
| H | $SO_2C_6H_5$ | $C_6H_5$ |
| H | $SO_2C_6H_5$ | 2-$CH_3$-$C_6H_4$ |
| H | $SO_2C_6H_5$ | 3-$CH_3$-$C_6H_4$ |
| H | $SO_2C_6H_5$ | 4-$CH_3$-$C_6H_4$ |
| H | $SO_2C_6H_5$ | 2-F-$C_6H_4$ |
| H | $SO_2C_6H_5$ | 3-F-$C_6H_4$ |
| H | $SO_2C_6H_5$ | 4-F-$C_6H_4$ |
| H | $SO_2C_6H_5$ | 2-Cl-$C_6H_4$ |
| H | $SO_2C_6H_5$ | 3-Cl-$C_6H_4$ |
| H | $SO_2C_6H_5$ | 4-Cl-$C_6H_4$ |
| H | $SO_2C_6H_5$ | 2-Br-$C_6H_4$ |
| H | $SO_2C_6H_5$ | 3-Br-$C_6H_4$ |

| $R^3$ | $R^4$ | $R^{13}$ |
|---|---|---|
| H | $SO_2C_6H_5$ | $4\text{-}Br\text{-}C_6H_4$ |
| H | $SO_2C_6H_5$ | $2\text{-}OH\text{-}C_6H_4$ |
| H | $SO_2C_6H_5$ | $3\text{-}OH\text{-}C_6H_4$ |
| H | $SO_2C_6H_5$ | $4\text{-}OH\text{-}C_6H_4$ |
| H | $SO_2C_6H_5$ | $2\text{-}OCH_3\text{-}C_6H_4$ |
| H | $SO_2C_6H_5$ | $3\text{-}OCH_3\text{-}C_6H_4$ |
| H | $SO_2C_6H_5$ | $4\text{-}OCH_3\text{-}C_6H_4$ |
| H | $SO_2C_6H_5$ | $4\text{-}C_6H_5\text{-}C_6H_4$ |
| H | $SO_2C_6H_5$ | $3\text{-}C(CH_3)_3\text{-}C_6H_4$ |
| H | $SO_2C_6H_5$ | $4\text{-}C(CH_3)_3\text{-}C_6H_4$ |
| H | $SO_2C_6H_5$ | $2\text{-}CF_3\text{-}C_6H_4$ |
| H | $SO_2C_6H_5$ | $3\text{-}CF_3\text{-}C_6H_4$ |
| H | $SO_2C_6H_5$ | $4\text{-}CF_3\text{-}C_6H_4$ |
| H | $SO_2C_6H_5$ | $2\text{-}NO_2\text{-}C_6H_4$ |
| H | $SO_2C_6H_5$ | $3\text{-}NO_2\text{-}C_6H_4$ |
| H | $SO_2C_6H_5$ | $4\text{-}NO_2\text{-}C_6H_4$ |
| H | $SO_2C_6H_5$ | $2\text{-}CN\text{-}C_6H_4$ |
| H | $SO_2C_6H_5$ | $3\text{-}CN\text{-}C_6H_4$ |
| H | $SO_2C_6H_5$ | $4\text{-}CN\text{-}C_6H_4$ |
| H | $SO_2C_6H_5$ | $2\text{-}Cl\text{-}6\text{-}CH_3\text{-}C_6H_3$ |
| H | $SO_2C_6H_5$ | $2\text{-}CO_2CH_3\text{-}6\text{-}CH_3\text{-}C_6H_3$ |
| H | $SO_2C_6H_5$ | $2,5\text{-}(OCH_3)_2\text{-}C_6H_3$ |
| H | $SO_2C_6H_5$ | $2,5\text{-}Cl_2\text{-}C_6H_3$ |
| H | $SO_2C_6H_5$ | $2,4,5\text{-}Cl_3\text{-}C_6H_2$ |
| H | $SO_2C_6H_5$ | $2\text{-}CO_2CH_3\text{-}C_6H_3$ |
| H | $SO_2C_6H_5$ | $5\text{-}Cl\text{-}2\text{-}OCH_3\text{-}C_6H_3$ |
| H | $SO_2C_6H_5$ | $5\text{-}NO_2\text{-}2\text{-}Cl\text{-}C_6H_3$ |
| H | $SO_2C_6H_5$ | $2\text{-}Cl\text{-}6\text{-}cyclopentenyl\text{-}C_6H_3$ |
| H | $SO_2C_6H_5$ | 3-Cl-thien-2-yl |
| H | $SO_2C_6H_5$ | $2\text{-}(CO_2C_2H_5)\text{-}C_6H_4$ |
| H | $SO_2C_6H_5$ | $3\text{-}(CO_2C_2H_5)\text{-}C_6H_4$ |
| H | $SO_2C_6H_5$ | $4\text{-}(CO_2C_2H_5)\text{-}C_6H_4$ |
| H | $SO_2C_6H_5$ | $2\text{-}(CO_2CH_3)\text{-}C_6H_4$ |
| H | $SO_2C_6H_5$ | $3\text{-}(CO_2CH_3)\text{-}C_6H_4$ |
| H | $SO_2C_6H_5$ | $4\text{-}(CO_2CH_3)\text{-}C_6H_4$ |
| H | $SO_2C_6H_5$ | $2\text{-}CONH_2\text{-}C_6H_4$ |

| $R^3$ | $R^4$ | $R^{13}$ |
|---|---|---|
| H | $SO_2C_6H_5$ | $3-CONH_2-C_6H_4$ |
| H | $SO_2C_6H_5$ | $4-CONH_2-C_6H_4$ |
| H | $SO_2C_6H_5$ | $2-NH_2-C_6H_4$ |
| H | $SO_2C_6H_5$ | $3-NH_2-C_6H_4$ |
| H | $SO_2C_6H_5$ | $4-NH_2-C_6H_4$ |
| H | $SO_2C_6H_5$ | $2-SCH_3-C_6H_4$ |
| H | $SO_2C_6H_5$ | $3-SCH_3-C_6H_4$ |
| H | $SO_2C_6H_5$ | $4-SCH_3-C_6H_4$ |
| H | $SO_2C_6H_5$ | $2,4-(CH_3)_2-C_6H_3$ |
| H | $SO_2C_6H_5$ | $3,4-(CH_3)_2-C_6H_3$ |
| H | $SO_2C_6H_5$ | $2,6-(CH_3)_2-C_6H_3$ |
| H | $SO_2C_6H_5$ | $2,4-(OCH_3)_2-C_6H_3$ |
| H | $SO_2C_6H_5$ | $3,4-(OCH_3)_2-C_6H_3$ |
| H | $SO_2C_6H_5$ | $2,6-(OCH_3)_2-C_6H_3$ |
| H | $SO_2C_6H_5$ | $2,4-F_2-C_6H_3$ |
| H | $SO_2C_6H_5$ | $3,4-F_2-C_6H_3$ |
| H | $SO_2C_6H_5$ | $2,6-F_2-C_6H_3$ |
| H | $SO_2C_6H_5$ | $2,4-Cl_2-C_6H_3$ |
| H | $SO_2C_6H_5$ | $3,4-Cl_2-C_6H_3$ |
| H | $SO_2C_6H_5$ | $2,6-Cl_2-C_6H_3$ |
| H | $SO_2C_6H_5$ | $2,4-(OH)_2-C_6H_3$ |
| H | $SO_2C_6H_5$ | $3,4-(OH)_2-C_6H_3$ |
| H | $SO_2C_6H_5$ | $2,6-(OH)_2-C_6H_3$ |
| H | $SO_2C_6H_5$ | $3-NO_2-4-CH_3-C_6H_3$ |
| H | $SO_2C_6H_5$ | $3-NO_2-4-F-C_6H_3$ |
| H | $SO_2C_6H_5$ | $3-NO_2-4-Cl-C_6H_3$ |
| H | $SO_2C_6H_5$ | $3-NO_2-4-OCH_3-C_6H_3$ |
| H | $SO_2C_6H_5$ | 2-Naphthyl |
| H | $SO_2C_6H_5$ | Thien-2-yl |
| H | $SO_2C_6H_5$ | Thien-3-yl |
| H | $SO_2C_6H_5$ | $5-CH_3$-thien-2-yl |
| H | $SO_2C_6H_5$ | 5-Cl-thien-2-yl |
| H | $SO_2C_6H_5$ | 5-Br-thien-2-yl |
| H | $SO_2C_6H_5$ | $2,5-(CH_3)_2$-thien-3-yl |
| H | $SO_2C_6H_5$ | Thiazol-2-yl |
| H | $SO_2C_6H_5$ | Thiazol-4-yl |

| $R^3$ | $R^4$ | $R^{13}$ |
|---|---|---|
| H | $SO_2C_6H_5$ | 5-$CH_3$-thiazol-2-yl |
| H | $SO_2C_6H_5$ | 5-Cl-thiazol-2-yl |
| H | $SO_2C_6H_5$ | 5-Br-thiazol-2-yl |
| H | $SO_2C_6H_5$ | 2,5-$(CH_3)_2$-thiazol-4-yl |
| H | $SO_2C_6H_5$ | Furan-2-yl |
| H | $SO_2C_6H_5$ | Furan-3-yl |
| H | $SO_2C_6H_5$ | 5-$CH_3$-furan-2-yl |
| H | $SO_2C_6H_5$ | 5-Cl-furan-2-yl |
| H | $SO_2C_6H_5$ | Pyrrol-2-yl |
| H | $SO_2C_6H_5$ | Pyrrol-3-yl |
| H | $SO_2C_6H_5$ | 5-$CH_3$-pyrrol-2-yl |
| H | $SO_2C_6H_5$ | 5-Br-pyrrol-2-yl |
| H | $SO_2C_6H_5$ | Oxazol-4-yl |
| H | $SO_2C_6H_5$ | Imidazol-2-yl |
| H | $SO_2C_6H_5$ | Pyridin-2-yl |
| H | $SO_2C_6H_5$ | Pyridin-3-yl |
| H | $SO_2C_6H_5$ | Pyridin-4-yl |
| H | $SO_2C_6H_5$ | Pyrazin-3-yl |
| H | $SO_2C_6H_5$ | Pyrazin-4-yl |
| H | $SO_2C_6H_5$ | Pyrrol-2-yl |
| H | $SO_2C_6H_5$ | Pyrimidin-2-yl |
| H | $SO_2C_6H_5$ | Pyrimidin-4-yl |
| H | $SO_2C_6H_5$ | Pyrimidin-5-yl |

Die substituierten Pyido[2,3-d]pyrimidine I eignen sich als Antidots, um herbizide Wirkstoffe für Kulturpflanzen wie Kulturhirse, Reis, Mais, Getreidearten (Weizen, Roggen, Gerste, Hafer), Baumwolle, Zuckerrüben, Zuckerrohr und Soja verträglicher zu machen. Sie wirken antagonistisch auf Herbizide verschiedenster Stoffklassen wie Triazine, Phenylharnstoffderivate, Carbamate, Thiolcarbamate, Halogenacetanilide, Benzoesäurederivate sowie insbesondere Halogenphenoxyessigsäureester, substituierte Phenoxyphenoxyessigsäureester, Phenoxyphenoxypropionsäureester und Cyclohexenonderivate.

Herbizid wirksame Cyclohexenon-Derivate II sind beispielsweise aus EP-A 228 598, EP-A 230 235, EP-A 238 021, EP-A 368 227, US-A 4 432 786, 39 104 und DE-A 38 38 309 bekannt. Sie dienen vorwiegend zur Bekämpfung unerwünschter Gräser in dicotylen Kulturen und in Gräsern, die nicht zur Familie der Gramineen zählen. In Abhängigkeit der Substituenten und der Dosierung der Verbindungen des Typs II bei ihrer Anwendung können diese Cyclohexenone auch zur selektiven Bekämpfung von unerwünschten Gräsern in Gramineen-Kulturen wie Weizen und Reis eingesetzt werden.

Weitere Cyclohexenon-Derivate II lassen sich in an sich bekannter Weise nach literaturbekannten Syntheseverfahren (vgl. z.B. EP-A 169 521) darstellen, beispielsweise durch Umsetzung von Triketonen IX (bekannt z.B. aus EP-A 80 301, EP-A 125 094, EP-A 142 741, US-A 4 249 937, EP-A 137 174 und EP-A 177 913) mit Hydroxylaminen X (bekannt z.B. aus Houben-Weyl, Methoden der Organischen Chemie, Band 10/1, Seite 1181 ff):

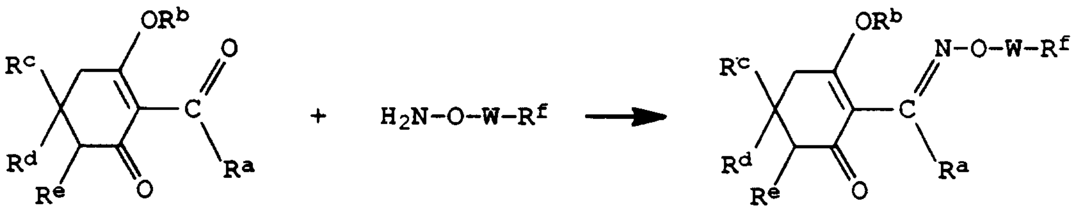

IX X II

Zweckmäßig führt man die Umsetzung in heterogener Phase in einem Lösungsmittel, bevorzugt in Gegenwart einer Base, durch, wobei das Hydroxylamin vorzugsweise als Ammoniumsalz eingesetzt wird.

Als Basen eigenen sich beispielsweise die Carbonate, Hydrogencarbonate, Acetate, Alkoholate und Oxide von Alkalimetallen und Erdalkalimetallen wie Natriumhydroxid, Kaliumhydroxid, Magnesiumoxid und Calciumoxid, des weiteren organische Basen wie Pyridin und tertiäre Amine wie Triethylamin.

Triketon und Hydroxylamin werden vorzugsweise in etwa stöchiometrischen Mengen eingesetzt. Die Menge an Base ist nicht kritisch, beträgt normalerweise aber ca. 0,5 bis 2 mol-Äquivalent, bezogen auf die Menge an IX.

Im allgemeinen liegt die Reaktionstemperatur zwischen 0 und 80 °C.

Als Lösungsmittel eignen sich beispielsweise Dimethylsulfoxid, Alkohole wie Methanol, Ethanol und Isopropanol, aromatische Kohlenwasserstoffe wie Benzol und Toluol, chlorierte Kohlenwaserstoffe wie Hexan und Cyclohexan, Ester wie Essigsäureethylester und Ether wie Diethylether, Dioxan und Tetrahydrofuran. Vorzugsweise führt man die Umsetzung in Methanol mit Natriumhydrogencarbonat als Base durch.

Die Reaktion ist nach wenigen Stunden beendet. Das Produkt II kann z.B. durch Einengen der Mischung, Verteilen des Rückstandes in Methylenchlorid/Wasser und Abdestillieren des Lösungsmittels unter vermindertem Druck isoliert werden.

Man kann für diese Umsetzung aber auch unmittelbar die freie Hydroxylaminbase, z.B. in Form einer wäßrigen Lösung, verwenden; je nach verwendetem Lösungsmittel für das Hydroxylamin X erhält man ein ein- oder zweiphasiges Reaktionsgemisch.

Geeignete Lösungsmittel für diese Variante sind beispielsweise Alkohole wie Methanol, Ethanol, Isopropanol und Cyclohexanol, aliphatische und aromatische, gegebenenfalls chlorierte Kohlenwasserstoffe wie Hexan, Cyclohexan, Methylenchlorid, Toluol und Dichlorethan, Ester wie Essigsäureethylester, Nitrile wie Acetonitril und cyclische Ether wie Dioxan und Tetrahydrofuran.

Besondere Bedingungen bezüglich des Druckes sind nicht erforderlich; normalerweise nimmt man die Umsetzung daher bei Atmosphärendruck vor.

Alkalimetallsalze der Verbindungen II können durch Behandeln der 3-Hydroxyverbindungen mit Natrium- oder Kaliumhydroxid bzw. -alkoholat in wäßriger Lösung oder in einem organischen Lösungsmittel wie Methanol, Ethanol, Aceton und Toluol erhalten werden.

Andere Metallsalze wie Mangan-, Kupfer-, Zink-, Eisen-, Calcium-, Magnesium- und Bariumsalze können aus den Natriumsalzen in üblicher Weise hergestellt werden, ebenso Ammonium- und Phosphoniumsalze mittels Ammoniak, Phosphonium-, Sulfonium- oder Sufoxoniumhydroxiden.

Die Verbindung des Typs IX können z.B. aus den entsprechenden Cyclohexan-1,3-dionen der Formel XI

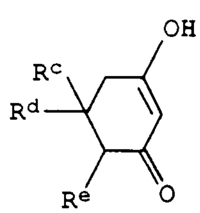

XI

nach bekannten Methoden (Tetrahedron Lett., 2491 (1975)) hergestellt werden.

Es ist auch möglich, die Verbindungen der Formel IX über die Zwischenstufe der Enolester herzustellen, die bei der Umsetzung von Verbindungen der Formel XI mit Säurechloriden in Gegenwart von Basen anfallen und anschließend mit bestimmten Imidazol- oder Pyridinderivaten umgelager werden (JP-OS

79/063 052).

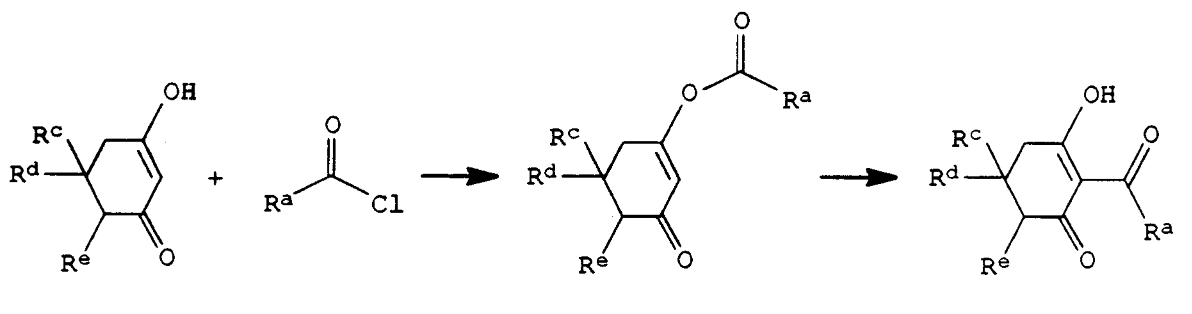

XI IX

Zu den Hydroxylaminen der Formel X gelangt man in der Regel über eine Reihe bekannter Verfahrensschritte ausgehend von bekannten Vorprodukten:

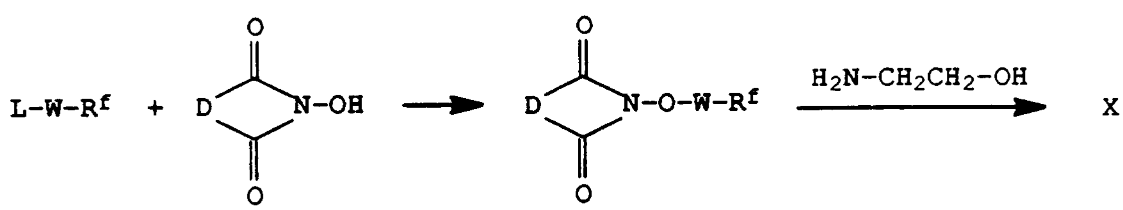


XII XIII XIV

L = die Hydroxylgruppe oder eine Abgangsgruppe, z.B. Halogen wie Chlor, Brom und Jod oder $CH_3SO_2$-O-.

Die zur Synthese der Hydroxylamine X benötigten Alkylierungsmittel sind literaturbekannt oder lassen sich nach bekannten Methoden darstellen.

Synthesen von Derivaten in denen W eine aliphatische oder olefinische Kette bedeutet, die gegebenenfalls durch Heteroatome unterbrochen sein kann, sind den folgenden Druckschriften zu entnehmen: DE-A 3 437 919; Tetrahetron Lett. 28, 2639 (1979); Org. Synth. Coll. Vol. 1, 436 (1944); DB-A 2 654 646; DE-A 2 714 561; J. Org. Chem. 52, 3587 (1987); DE-A 948 871; DE-A 948 872; J. Med. Chem. 26, 1570 (1983); Synthesis 675 (1983); J. Org. Chem. 48, 4970 (1983); Org. Synth. Coll. Vol. V, 249; EP 48 911; EP 143 952; US 4 686 735.

Zur Herstellung von Verbindungen II, in denen W eine aliphatische oder olefinische Kette und $R^f$ einen Heterocyclus bedeutet, sei auf folgende Literatur verwiesen:
J. Heterocycl. Chem. 14, 525 (1976); JP 55 051 004; JP 55 047 601; Houben Weyl: Methoden der organischen Chemie Band 4/3, S. 424 ff; DE-A-2 821 409; Chem. Ber. 114, 3667, 3674 (1981).

Herstellungsmethoden, die von geeigneten Carbinolen XII (L = OH) ausgehen, sind beispielsweise bekannt aus:
Tetrahedron 35, 329 (1979); Chem. Lett. 423, (1977); Houben/Weyl: Methoden der organischen Chemie, Band 13/9B, S. 964 ff; dto. Band 5/3, S. 862 und 899 ff; dto. Band 5/4, S. 361 ff.

Die Darstellung von Alkylierungsmitteln in denen W eine substituierte oder unsubstituierte $C_3$-$C_6$-Alkinylgruppe bedeutet, kann nach klassischen Methoden [vgl. J. Med Chem. 29, 1389 (1986); dto. 24, 678 (1981); EP-A 131 302; J. Chem. Ecol. 10, 1201 (1982)] oder durch Kupplung von 1-Alkinylderivaten mit Aryl- oder Hetarylhalogeniden in Gegenwart von Palladiumkatalysatoren [vgl. z.B. Tetrahedron Sett. 50, 4467 (1975)] erfolgen.

XII wird mit einem cyclischen Hydroxylimid XIII gekoppelt und das erhaltene geschützte Hydroxylaminderivat XIV zum freien Hydroxylamln X gespalten, bevorzugt mit 2-Aminoethanol.

Bei der Verwendung von HO-W-$R^f$ empfiehlt sich das Arbeiten nach der Mitsunobu-Variante [vgl. Synthesis, 1 (1981) und J. Med. Chem. 33, 187 (1990)].

In den cyclischen Hydroxyimiden X steht D z.B. für $C_2$-$C_3$-Alkylen, $C_2$-Alkenylen oder einen mit bis zu drei Doppelbindungen und gegebenenfalls ein Stickstoffatom enthaltenden 5- oder 6-Ring, z.B. für Phenylen, Pyridinylen, Cyclopentylen, Cyclohexylen oder Cyclohexenylen. Beispielsweise kommen folgende

Substanzen in Betracht:

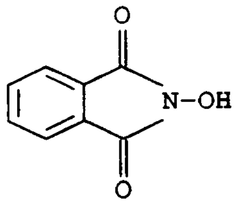 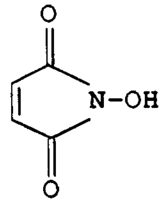 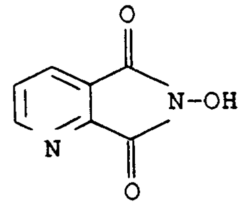

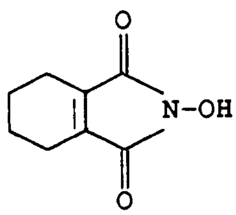 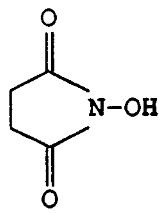 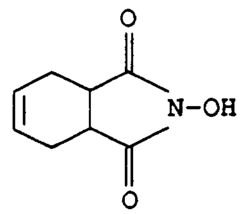

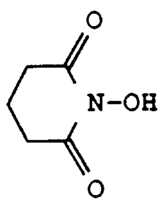

Die Umsetzung der Verbindungen IX mit den Hydroxyimiden XIII wird zweckmäßigerweise in Gegenwart einer Base ausgeführt. Geeignet sind prinzipiell alle Basen, die in der Lage sind, die Hydroxyimide XIII zu deprotonieren ohne das Imidsystem anzugreifen. Dies sind insbesondere die sogenannten nicht nucleophilen Basen. Beispielsweise genannt seien Mineralbase wie Alkalimetall- und Erdalkalimetallcarbonate, Alkalimetall- und Erdalkalimetallhydrogencarbonate, organische Basen wie aliphatische, cycloaliphatische und aromatische tertiäre Amine. Es können auch Gemische dieser Basen verwendet werden.

Als Einzelverbindungen seien folgende Basen beispielhaft aufgeführt: Natriumcarbonat, Kaliumcarbonat, Magnesiumcarbonat, Calciumcarbonat, Bariumcarbonat, die Hydrogencarbonate dieser Metalle, Trimethylamin, Triethylamin, Tributylamin, Ethyldiisopropylamin, N,N-Dimethylanilin, 4-N,N-Dimethylaminopyridin, Diazabicyclooctan, Diazabicycloundecan, N-Methyl-piperidin, 1,4-Dimethylpiperazin, Pyridin, Chinolin, Bipyridin, Phenanthrolin. Bevorzugt sind die preiswerten Basen Natrium- und Kaliumcarbonat.

Die Base wird im allgemeinen in äquivalenten Mengen bis zu einem Überschuß von 5 Äquivalenten, bezogen auf das Hydroxyimid, zugegeben. Ein höherer Überschuß ist möglich, entbehrt aber zusätzliche Vorteile. Die Verwendung einer geringen Basenmenge ist ebenfalls möglich. Bevorzugt wird jedoch eine Basenmenge von 1 bis 3, insbesondere von 1 bis 2 Äquivalenten, bezogen auf das Hydroxyimid XIII eingesetzt.

Die Verwendung von nucleophilen Basen, z.B. Alkalimetall- und Erdalkalimetallhydroxiden, insbesondere Natrium- und Kaliumhydroxid, ist ebenfalls möglich. In diesem Falle ist es vorteilhaft, die Base in äquivalenten Mengen bezüglich des Hydroxyimids XIII einzusetzen, um einem nucleophile Angriff der Hydroxylionen auf die Carbonylfunktion der Imdigruppierung vorzugbeugen.

Zweckmäßigerweise setzt man die Ausgangsverbindungen XII mit den Hydroxyimiden XIII in einem Lösungsmittel um, das sich unter den Reaktionsbedingungen inert verhält. Vorteilhafte Lösungsmittel sind z.B. polare, aprotische Lösungsmittel wie Dimethylformamid, N-Methylpyrrolidon, Dimethylsulfoxid, Sulfolan und cyclische harstoffe. Die Lösungsmittelmenge ist im allgemeinen nicht kritisch.

Die Umsetzung der Ausgangsverbindungen XII mit den Hydroxyimiden XIII kann auch unter Anwendung der Phasentransfer-Katalyse ausgeführt werden. In diesem Falle werden mit Wasser zwei Phase bildende Lösungsmittel, bevorzugt Chlorkohlenwasserstoffe, eingesetzt. Als Phasentransferkatalysatoren eignen sich die üblicherweise zu solchen Zwecken vezwendeten quartären Ammonium- und Phosphoniumsalze, Polyethylenglykole, Polyethylenglykolether und Kronenether, wie sie z.B. in Dehmlow et al.; Phase Transfer

Catalysis, S. 37-45 und S. 86-93, Verlag Chemie, Weinheim 1980, beschrieben sind. Die Phasentransferkatalystoren werden zweckmäßigerweise in Mengen von 1 bis 10 Vol%, bevorzugt in Mengen von 3 bis 5 Vol%, bezogen auf das Volumen der Reaktionsmischung, eingesetzt.

Die Umsetzung der Ausgangsverbindungen XII mit den Hydroxyimiden XIII wird im allgemeinen im Temperaturbereich zwischen 0 und 140 ° C, bevorzugt zwischen 20 und 100 ° C, insbesondere zwischen 40 und 80 ° C, durchgeführt. Zweckmäßigerweise wird dabei so vorgegangen, daß man das Hydroxyimid XIII zusammen mit der Base im Lösungsmittel vorlegt und das Ausgangsmaterial XII zu dieser Lösung dosiert. Dabei kann es sich als günstig erweisen, wenn das Hydroxyimid bei einer tieferen Temperatur, beispielsweise bei 0 bis 50 ° C, zugegeben und die Reaktionsmischung erst nach dieser Zugabe auf die eigentliche Reaktionstemperatur erhitzt wird.

In der Regel arbeitet man bei Normaldruck oder unter dem Eigendruck des Lösungsmittels.

Nach beendeter Reaktion wird die abgekühlte Reaktionsmischung zwecmäßigerweise mit Wasser versetzt, wobei sich die gebildeten Hydroxylaminderivate XIV als kristalline Festkörper oder als Öle abscheiden. Die auf diese Weise erhaltenen Hydroxylaminderivate können, falls gewünscht, durch Umkristallisation oder durch Extraktion weiter gereinigt werden.

Die Hydroxylaminderivate XIV können zwischengelagert werden oder sogleich in die Hydroxylaminderivate X mit freier Aminogruppe umgewandelt werden. Diese Umwandlung kann nach an sich bekannten Verfahren durchgeführt werden, wie sie beispielsweise in DE-A 36 15 973 und den darin zitierten Schriften beschrieben sind. Bevorzugt wird das Verfahren gemäß DE-A 36 15 973 angewandt, nach dem die Hydroxylaminderivate X mittel Etanolamin freigesetzt werden. Die Feisetzung der Hydroxylaminderivate X mit Hilfe anderer Basen wie wäßrigen Mineralbasen, mit Aminen, Hydrazinen, Hydroxylaminen oder mittels wäßriger Säuren ist ebenfalls möglich.

Auf den nach diesen Verfahren erhaltenen Reaktionsgemischen können die Hydroxylaminderivate X mittels üblicher Aufarbeitungsmethoden isoliert werden, beispielsweise durch Extration oder durch Kristallisation. Zur Erhöhung der Kristallisationstendenz dieser Hydroxylaminderivate kann es oftmals förderlich sein, diese in ihre Salze mit Mineralsäuren oder organischen Säuren überzuführen. Dazu werden im allgemeinen verdünnte Lösungen dieser Säuren mit den Hydroxylaminderivaten umgesetzt, und zwar zweckmäßigerweise in äquivalenten Mengen. Die erhaltenen Hydroxylammoniumsalze könne wie die Hydroxylaminderivate mit freier Aminogruppe direkt zu den Herbiziden der Formel II weiterverarbeitet werden oder auch, falls gewünscht, gelagert werden.

Die Cyclohexenon-Derivate II können bei der Herstellung als Isomerengemische anfallen, wobei sowohl E-/Z-Isomerengemische als auch Enantiomeren- oder Diastereoisomerengemische möglich sind. Die Isomerengemische können gewünschtenfalls nach den hierfür üblichen Methoden, z.B. durch Chromatographie oder durch Kristallisation, getrennt werden.

Als herbizide Wirkstoffe (A) kommen sowohl die reinen Enantiomeren II als auch Racemate oder Diastereoisomerengemische von Cyclohexanon-Derivaten II in Betracht.

Die Cyclohexenon-Derivate II können in mehreren tautomeren Formen geschrieben werden, die alle von der Erfindung umfaßt werden.

Herstellungsbeispiele (Cyclohexenon-Derivate)

Beispiel 1

2[1-(3-(4-Bromphenyl)-prop-2-enyloximino)-propyl]-3-hydroxy-5-(3-tetrahydrothiopyranyl)-cyclohex-2-en-1-on

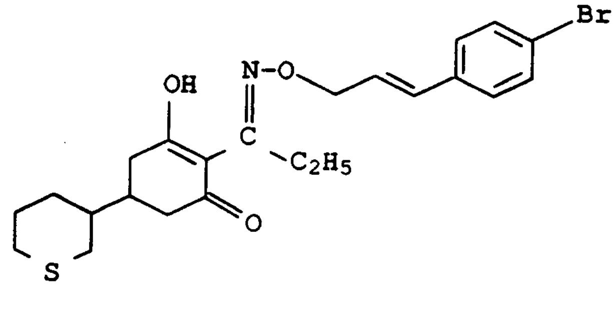

3,0 g (0,011 mol) 2-Propionyl-5-(3-tetrahydrothiopyranyl)-cyclohexan-1,3-dion und 3,0 g (0,013 mol) 3-(4-Bromphenyl)-prop-2-enyloxiamin wurden in 100 ml Methanol bei 20 ° C 16 Stunden gerührt. Das dabei

ausgefallene Reaktionsprodukt wurde bei 0 ° C abgetrennt und mit eiskaltem Methanol und Petrolether nachgewaschen und getrocknet. Ausbeute: 68,4 %; Fp.: 97-99 ° C.

Vorstufe 1.1

N-[3-(4-Bromphenyl)-prop-2-enyloxy]-phthalimid

In 350 ml trockenes N-Methylpyrrolidon gab man nacheinander 18,5 g (0,11 mol) N-Hydroxyphthalimid und 31,4 g (0,11 mol) 1-Brom-$3-(4-Bromphenyl)%-prop-2-en und tropfte anschließend bei Raumtemperatur 12,1 g (0,12 mol) Triethylamin zu. Nach viertägigem Rühren bei 20 ° C wurde die Reaktionsmischung auf 1,5 l Einswasser gegossen, abfiltriert und mit Wasser und Isopropanol nachgewaschen. Ausbeute: 86,8 %; Fp.: 161-162 ° C

Vorstufe 1.2

3-(4-Bromphenyl)-prop-2-enyloxyamin

33,4 g (0,093 mol) N-[3-(4-Bromphenyl)-prop-2-enyloxy]-phthalimid wurden portionsweise in 50 ml Ethanolamin eingetragen; die Temperatur stieg dabei bis auf 30 ° C an. Nach zweistündigem Rühren bei 60 ° C ließ man abkühlen und versetzte die Mischung mit 200 ml Dichlormethan. Es wurde mit Eiswaser ausgeschüttelt. Die organische Phase getrocknet und eingeengt und aus Petrolether kristallisiert. Ausbeute: 95,3 %; Fp.: 35-38 ° C.

Beispiel 2

2-[1-(4-(4-Fluorphenyl)-but-3-inyloximino)-butyl]-3-hydroxy-5-tetrahydropyran-4-yl-cyclohex-2-enon

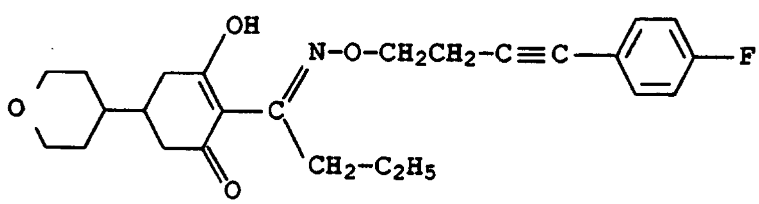

Zu einer Lösung von 4 g (15 mMol) 2-butyryl-3-hydroxy-5-tetrahydropyran-4-yl-cyclohex-2-enon in 60 ml trockenem Methanol wurden 2,7 g (15 mMol) 4-(4-Fluorphenyl)-but-3-inoxymin gegeben. Nach 16 h Rühren bei Raumtemperatur wurde das Methanol im Wasserstrahlvakuum entfernt. Das Rohprodukt reinigte man mittels Chromatographie an Kieselgel (Laufmittel: Methylenchlorid). Ausbeute: 81,2 %.

Vorstufe 2.1

4-(4-Fluorphenyl)-3-butinol

Eine Lösung von 100 g 4-Bromfluorbenzol in 350 ml Triethylamin wurde nacheinander mit 1 g Bis-(triphenylphosphin)-palladium-(II)-chlorid, 3,8 g Kupfer-(I)-jodid und 8,7 g Triphenylphosphin versetzt. Diese Mischung wurde auf Rückflußtemperatur erwärmt, wonach man bei dieser Temperatur (ca. 100 ° C) 43,4 g 3-Butinol innerhalb 20 min zutropfte. Es wurde noch 5 h bei dieser Temperatur gerührt. Nach dem Abkühlen wurde das Triethylamin abdestilliert. Der Rückstand wurde in Methyl-tert.-butylether und Wasser aufgenommen. Die wäßrige Phase wurde noch zweimal mit Methyl-tert.-butylether extrahiert, die vereinigten organischen Extrakte wurden nacheinander mit 1 N Salzsäure und mit 10 %iger Natriumbicarbonatlösung gewaschen und über Natriumsulfat getrocknet. Nach Entfernen des Lösungsmittels wurde das Rohprodukt im Hochvakuum destilliert. Ausbeute: 86 %.

Vorstufe 2.2

N-(5-(4-Fluorphenyl)-4-pentinyloxy)phthalimid

Zu einer Lösung von 33,1 g (0,186 mol) 5-Hydroxy-1-(4-fluorphenyl)-1-pentin in 430 ml trockenem Tetrahydrofuran wurden 33,4 g (0,205 mol) N-Hydroxyphthalimid und 53,8 g (0,205 mol) Triphenylphosphin gegeben. Innerhalb von 2,5 h tropfte man dann unter Temperaturkontrolle (max. 40 ° C) 35,7 g (0,205 mol) Diethylazodicarboxylat zu. Man rührte über Nacht bei Raumtemperatur, engte die Mischung im Vakuum ein und nahm mit 300 ml Dichlormethan auf. Es wurde zweimal mit Natriumcarbonatlösung und einmal mit gesättigter Kochsalzlösung gewaschen. Nach Trocknen und Einengen wurde das Rohprodukt an Kieselgel chromatographisch gereinigt. Als Eluens wurde zunächst Dichlormethan/n-Hexan benutzt, später dann reines Dichlormethan. Ausbeute: 82 %; Fp.: 85-88 ° C.

250-MHz-$^1$H-NMR (in DMSO-$d_6$):

$\delta$ [ppm] = 1,9 - 2,1 (m, 2H); 2,68 (t, 2H); 4,342 (t, 2H); 7,18 (t, 2H); 7,4 - 76 (m, 2H); 7,85 (s, 4H).

Vorstufe 2.3

5-Aminooxy-1-(4-Fluorphenyl)-1-pentin

Zu einer Mischung aus 68 ml Ethanolamin und 40 ml Dichlormethan wurden portionsweise 47,7 g (0,148 mol) des oben dargestellten Phthalimidethers gegeben. Nach 2 h Rühren bei Raumtemperatur war eine klare Lösung entstanden. Diese wurde in 300 ml eiskalte, gesättigte Kochsalzlösung gegeben. Man extrahierte die Mischung dreimal mit 100 ml Dichlormethan, wusch die vereinigten organischen Phasen einmal mit Kochsalzlösung gegen, trocknete und engte ein. Ausbeute: 95 % (Öl).

250-MHz-$^1$H-NMR (in $CDCl_3$):

$\delta$ [ppm] = 1,8-2,0 (m, 2H); 2,47 (6, 2H); 3,8 (t, 2H); 5,4 (breites s, 2H); 6,9-7,1 (m, 2H); 7,3-7,45 (m, 2H).

Beispiel 3

2-[1-[[(E)-4-(2-Thienyl)-3-butenyloxy]imino]-butyl]-3-hydroxy-5-(2H-tetrahydropyran-4-yl)-cyclohex-2-en-1-on

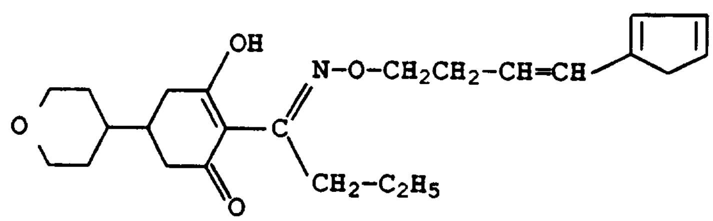

Eine Mischung aus 35 g (0,13 mol) 2-Butyryl-3-hydroxy-5-(2H-tetrahydropyran-4-yl)-2-cyclohexen-1-on und 24 g (0,14 mol) O-[(E)-4-(2-Thienyl)-3-butenyl]hydroxylamin in 300 ml Methanol wurde 16 h gerührt. Man engte im Vakuum ein und nahm in 1000 ml 10 %iger Natronlauge auf. Man extrahierte dreimal mit je 200 ml Methylenchlorid und stellte die wäßrige Phase unter Eiskühlung mit konz. Salzsäure auf pH 1 ein. Die wäßrige Phase wurde anschließend dreimal mit je 200 ml Ether extrahiet, über Magensiumsulfat getrocknet und im Vakuum eingeengt. Das Rohprodukt wurde chromatographisch an 100 g Kieselgel/Säule 30 x 15 cm, (Laufmittel: Essigester) gereinigt. Ausbeute: 85 %.

200 MHz-$^1$H-NMR (in $CDCl_3$): $\delta$ [ppm] = 0,95 (t, 3H), 1,17-1,96 (m, 9H), 2,13 (m, 1H), 2,36 (m, 1H), 2,43-2,70 (m, 3H), 2,88 (m, 2H), 3,36 (t, 2H), 4,02 (d, 2H), 4,15 (t, 2H), 6,00 (dt, 1H), 6,60 (d, 1H), 6,80-7,20 (m, 3H) 14,75 (s, 1H).

Vorstufe 3.1

(E)-4-Brom-1-(2-thienyl)-1-buten

Bei 5 bis 10 ° C tropfte man innerhalb 1 h 225 g (1,46 mol) Cyclopropyl-2-thienylcarbinol zu 972 ml 48 %iger Bromwasserstoffsäure. Nach 2 h bei Raumtemperatur wurde die organische Phase abgetrennt und

die wäßrige Lösung dreimal mit je 300 ml Dichlormethan extrahiert. Die vereinigten organischen Phasen wurden mit verd. Natronlauge und Wasser neutral gewaschen, über Magnesiumsulfat getrocknet und im Vakuum eingeengt. 322 g (94 % korrigert) rohes Bromid (GC: 92 %).

250 MHz-$^1$H-NMR (in $CDCl_3$): δ [ppm] = 2,65-2,80 (m, 2h), 3,46 (t, 2H), 5,90-6,10 (m, 1H), 6,61 (d, 1H), 6,80-7,00 (m, 2H), 7,14 (d, 1H).

Vorstufe 3.2

N-[(E)-4-(2-Thienyl)-3-butenyloxy]phthalimid

Bei 20 bis 25 °C tropfte man innerhalb 2,5 h 190 ml (1,37 mol) Triethylamin zu einer Mischung aus 283 g, (1,30 mol) des oben hergestellten Bromids, 1300 ml N-Methyl-2-pyrrolidinon, 10 g Kaliumiodid und 212 g (1,30 mol) N-Hydroxyphthalimid. Nach 4 h bei 20 bis 25 °C goß man in 4000 ml Eiswasser und ergänzte portionsweise 5000 ml 10 %ige Natronlauge. Man extrahierte darauf viermal mit je 500 ml Essigester. Die vereinigten Essigester-Phasen wurden mit verd. Natronlauge und Waser neutral gewaschen, über Magnesiumsulfat getrocknet und im Vakuum eingeengt. Das Rohprodukt wurde chromatographisch an 1000 g Kieselgel/Säule 30 x 15 cm, (Laufmittel: n-Hexan/Dichlormethan 7:3) gereinigt. Ausbeute: 29 %; Fp.: 69-71 °C (Isopropanol).

250 MHz-$^1$H-NMR (in $d_6$-DMSO): δ [ppm] = 2,55-2,70 (m, 2H), 4,28 (t, 2H), 6,00-6,20 (m, 1H), 6,77 (d, 1H), 7,00 (m, 2H), 7,35 (m, 1H), 7,87 (s, 4H).

Vorstufe 3.3

O-[(E)-4-(2-Thienyl)-3-butenyl]hydroxylamin

Eine Mischung aus 90,2 g (0,30 mol) des oben hergestellten Phthalimidethers und 136 ml Ethanolamin wurden 3 h bei 60 °C gerührt. Die kalte Reaktionsmischung goß man in 200 ml Eiswasser. Man ergänzte 200 ml ges. Kochsalz-Lösung und extrahierte das Hydrolysat dreimal mit je 300 ml Dichlormethan. Die vereinigten organischen Phasen wurden darauf dreimal mit je 100 ml ges. Kochsalz-Lösung gewaschen, über Magnesiumsulfat getrocknet und im Vakuum eingeengt. Ausbeute: 89 %.

250 MHz-$^1$H-NMR (in $CDCl_3$): δ [ppm] = 2,40-2,55 (m, 2H), 3,78 (t, 2H), 5,40 (bs, 2H), 5,95-6,20 8m, 1H), 6,57 (d, 1H), 6,80-7,15 (m, 3H).

Beispiel 4

2-[1-[[2-(2-fluorbenzyloxy)ethoxy]imino]butyl]-2-hydroxy-5-(2H-tetrahydropyran-4-yl)-2-cyclohexen-1-on

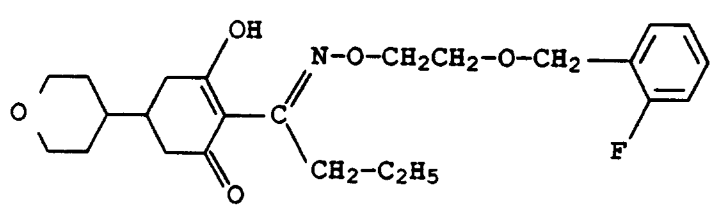

Eine Mischung aus 4,0 g (10 mmol) 2-Butyryl-3-hydroxy-5-(2H-tetrahydropyran-4-yl)-2-cyclohexen-1-on und 2,6 g (14 mmol) O-[2-(2-Fluorbenzyloxy)-ethyl]hydroxylamin in 100 ml Methanol wurde 24 h gerührt. Man engte das Reaktionsgemisch unter reduziertem Druck ein und chromtographierte das Rohprodukt an 100 g Kieselgel (Säule 30 x 4 cm; Laufmittel: Ether).

Ausbeute: 54 %

300 MHz-$^1$H-NMR (in $CDCl_3$): δ [ppm] = 0,93 (t, 3H), 1,20-1,77 (m, 7H), 1,90 (m, 1H), 2,23 (m, 2H), 2,58 (m, 2H), 2,92 (m, 2H), 3,38 (t, 2H), 3,80 (m, 2H), 4,03 (m, 2H), 4,25 (m, 2H), 4,68 8s, 2h), 6,93-7,50 (m, 4H, 14,30 (s, 1H).

Vorstufe 4.1

N-[2-(2-Fluorbenzyloxy)ethoxy]phthalimid

Zu einer Mischung aus 165 g (0,71 mol9 1-Brom-2-(2-fluorbenzyloxy)ethan, 116 g (0,7 mol) N-Hydroxyphthalimid und 710 ml n-Methyl-2-pyrrolidinon tropfte man bei 20 bis 25 °C innerhalb 1 h 108 ml Triethylamin. Nach 5 g bei 60 °C goß man die kalte Reaktionsmischung in 200 ml Eiswasser, saugte den Niederschlag ab, wusch mit Wasser und Iopropanol und trocknete i.V. über Phosphorpentoxid. Ausbeute: 82 %.

Fp.: 62-64 °C.

250 MHz-$^1$H-NMR (in $d_6$-DMS): $\delta$ [ppm] = 3,85 (m, 2H), 4,35 (m, 1H), 4,54 (s 2H), 7,10-7,40 (m, 4H), 7,88 (s, 4H).

Vorstufe 4.2

O-[2-(2-Fluorbenzyloxy)ethyl]hydroxylamin

184 g (0,58 mol) des oben hergestellten Phtalimidethers wurden portionsweise in 270 ml Ethanolamin eingetragen. Nach 3 h bei 60 °C goß man die kalte Reaktionsmischung in 1000 ml Eiswasser. Das Hydrolysat wurde dreimal mit je 800 ml Dichlormethan extrahiert. Die vereinigten organischen Phasen wurden mit 200 ml ges. Kochsalz-Lösung gewaschen, über Magnesiumsulfat getrocknet und im Vak. eingeengt. Ausbeute: 91 %.

$^1$H-NMR (250 MHz, $CDCl_3$): $\delta$ [ppm] = 3,70 (dd, 2H9, 3,85 (dd, 2H), 4,54 ( 2H), 5,50 (bs, 2H), 7,00-7,50 (m, 4H).

Die gewünschte antidotisierende Wirkung der Verbindungen I tritt insbesondere bei der Anwendung mit Herbiziden aus der Gruppe der Cyclohexenon-Derivate der allgemeinen Formel II auf, wenn deren Substituenten die folgende Bedeutung haben:

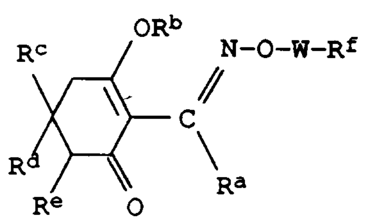

II

$R^a$

$C_1$-$C_6$-Alkyl wie vorstehend genannt, vorzugsweise $C_1$-$C_4$-Alkyl, insbesondere $C_1$-$C_2$-Alkyl;

$R^b$

Wasserstoff;

das Äquivalent eines landwirtschaftlich brauchbaren Kations;

$C_1$-$C_8$-Alkylcarbonyl, besonders $C_1$-$C_6$-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl, vorzugsweise $C_1$-$C_4$-Alkylcarbonyl, insbesondere $C_1$-$C_2$-Alkylcarbonyl;

$C_1$-$C_{10}$-Alkylsulfonyl, besonders $C_1$-$C_6$-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl, 1,1-Dimethylethylsulfonyl, Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, Hexylsulfonyl, 1,1-Dimethylpropylsulfonyl, 1,2-Dimethylpropylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl,

1,2,2-Trimethylpropylsulfonyl, 1-Ethyl-1-methylpropylsulfonyl und 1-Ethyl-2-methylpropylsulfonyl, vorzugsweise $C_1$-$C_4$-Alkylsulfonyl, insbesondere $C_1$-$C_2$-Alkylsulfonyl;

$C_1$-$C_{10}$-Alkylphosphonyl, besonders $C_1$-$C_6$-Alkylphosphonyl wie Methylphosphonyl, Ethylphosphonyl, Propylphosphonyl, 1-Methylethylphosphonyl, Butylphosphonyl, 1-Methylpropylphosphonyl, 2-Methylpropylphosphonyl, 1,1-Dimethylethylphosphonyl, Pentylphosphonyl, 1-Methylbutylphosphonyl, 2-Methylbutylphosphonyl, 3-Methylbutylphosphonyl, 2,2-Dimethylpropylphosphonyl,1-Ethylpropylphosphonyl, Hexylphosphonyl, 1,1-Dimethylpropylphosphonyl, 1,2-Dimethylpropylphosphonyl, 1-Methylpentylphosphonyl, 2-Methylpentylphosphonyl, 3-Methylpentylphosphonyl, 4-Methylpentylphosphonyl, 1,1-Dimethylbutylphosphonyl, 1,2-Dimethylbutylphosphonyl, 1,3-Dimethylbutylphosphonyl, 2,2-Dimethylbutylphosphonyl, 2,3-Dimethylbutylphosphonyl, 3,3-Dimethylbutylphosphonyl, 1-Ethylbutylphosphonyl, 2-Ethylbutylphosphonyl, 1,1,2-Trimethylpropylphosphonyl, 1,2,2-Trimethylpropylphosphonyl, 1-Ethyl-1-methylpropylphosphonyl und 1-Ethyl-2-methylpropyl-phosphonyl,vorzugsweise $C_1$-$C_4$-Alkylphosphonyl, insbesondere $C_1$-$C_2$-Alkylphosphonyl;

Benzoyl, Benzolsulfonyl oder Benzolphosphonyl, wobei die aromatischen Ringe ein bis fünf Halogenatome wie vorstehend genannt, vorzugsweise Fluor und Chlor, tragen können;

$R^c$

Wasserstoff; CN; CHO;

$C_1$-$C_6$-Alkyl wie vorstehend genannt, vorzugsweise $C_1$-$C_4$-Alkyl, insbesondere $C_1$-$C_2$-Alkyl, welches einen der folgenden Reste tragen kann:

- $C_1$-$C_4$-Alkoxy wie vorstehend im allgemeinen und im besonderen genannt;
- $C_1$-$C_4$-Alkylthio wievorstehend im allgemeinen und im besonderen genannt;
- Phenyloxy, Phenylthio, Pyridyloxy oder Pyridylthio, wobei die aromatischen Reste ihrerseits ein bis drei der folgenden Gruppen tragen können: Nitro, Cyano,
- Halogen wie vorstehend im allgemeinen und im besonderen genannt;
- $C_1$-$C_4$-Alkyl wie vorstehend im allgemeinen und im besonderen genannt;
- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;
- $C_1$-$C_4$-Halogenalkoxy, besonders $C_1$-$C_2$-Halogenalkoxy wie vorstehend im allgemeinen und im besonderen genannt;
- $C_1$-$C_4$-Alkylthio wie vorstehend im allgemeinen und im besonderen genannt;
- $C_3$-$C_6$-Alkenyl wie vorstehend im allgemeinen und im besonderen genannt;
- $C_3$-$C_6$-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy,1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Di-methyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 3,3-Dimethyl-2-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy und 1-Ethyl-2-methyl-2-propenyloxy, vorzugsweise 2-Propenyloxy;
- $C_3$-$C_6$-Alkinyl wie vorstehend im allgemeinen und im besonderen genannt;
- $C_3$-$C_6$-Alkinyloxy wie 2-Propinyloxy, 2-Butinyloxy, 3-Butinyloxy, 1-Methyl-2-propinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 1,1-Dimethyl-2-propinyloxy, 1-Ethyl-2-propinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, vorzugsweise 2-Propinyloxy;
- oder $NR^gR^h$;

$R^g$ Wasserstoff;

$C_1$-$C_4$-Alkyl wie vorstehend im allgemeinen und im besonderen genannt;

$C_3$-$C_6$-Alkenyl wie vorstehend im allgemeinen und im besonderen genannt;

$C_3$-$C_6$-Alkinyl wie vorstehend im allgemeinen und im besonderen genannt;

$C_1$-$C_6$-Alkylcarbonyl wie vorstehend genannt;
Benzoyl, welches ein bis drei der folgenden Reste tragen kann: Nitro, Cyano,

- Halogen wie vorstehend im allgemeinen und im besonderen genannt;
- $C_1$-$C_4$-Alkyl wie vorstehend im allgemeinen und im besonderen genannt;
- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;
- $C_1$-$C_4$-Alkylthio wie vorstehend im allgemeinen und im besonderen genannt;

$R^h$ Wasserstoff;
$C_1$-$C_4$-Alkyl wie vorstehend im allgemeinen und im besonderen genannt;
$C_3$-$C_6$-Alkenyl wie vorstehend im allgemeinen und im besonderen genannt;

$R^c$ bedeutet desweiteren:

$C_3$-$C_7$-Cycloalkyl wie vorstehend genannt oder $C_5$-$C_7$-Cycloalkenyl wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl und Cyclohept-4-enyl, wobei diese Ringe ein bis drei der folgenden Reste tragen können:

- Hydroxy,
- Halogen wie vorstehend im allgemeinen und im besonderen genannt;
- $C_1$-$C_4$-Alkyl wie vorstehend im allgemeinen und im besonderen genannt;
- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;
- $C_1$-$C_4$-Alkylthio wie vorstehend im allgemeinen und im besonderen genannt;
- Benzylthio,
- $C_1$-$C_4$-Alkylsulfonyl wie Methylsulfonyl, Ethylsulfonyl, Propylsulfonyl, 1-Methylethylsulfonyl, Butylsulfonyl, 1-Methyl-propylsulfonyl, 2-Methylpropylsulfonyl und 1,1-Dimethylethylsulfonyl, vorzugsweise $C_1$-$C_2$-Alkylsulfonyl;
- $C_1$-$C_4$-Alkylsulfenyl wie Methylsulfenyl, Ethylsulfenyl, Propylsulfenyl, 1-Methylethylsulfenyl, Butylsulfenyl, 1-Methyl-propylsulfenyl, 2-Methylpropylsulfenyl und 1,1-Dimethylethylsulfenyl, vorzugsweise $C_1$-$C_2$-Alkylsulfenyl;
- und $C_1$-$C_4$-Alkylsulfinyl wie Methylsulfinyl, Ethylsulfinyl, Propylsulfinyl, 1-Methylethylsulfinyl, Butylsulfinyl, 1-Methyl-propylsulfinyl, 2-Methylpropylsulfinyl und 1,1-Dimethylethylsulfinyl, vorzugsweise $C_1$-$C_2$-Alkylsulfinyl;

5-gliedrige gesättigte Ringe, welche neben Kohlenstoffringgliedern ein oder zwei Sauerstoff- oder Schwefelatome oder ein Sauerstoff- und ein Schwefelatom enthalten, wobei diese Ringe ein bis drei der folgenden Reste tragen können:

- $C_1$-$C_4$-Alkyl wie vorstehend im allgemeinen und im besonderen genannt;
- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;
- $C_1$-$C_4$-Alkylthio wie vorstehend im allgemeinen und im besonderen genannt;

6- oder 7-gliedrige gesättigte oder ein- oder zweifach ungesättigte Ringe, welche neben Kohlenstoffringgliedern ein oder zwei Sauerstoff- oder Schwefelatome oder ein bis drei Stickstoffatome oder ein oder zwei Sauerstoff- oder Schwefelatome enthalten, wobei diese Ringe ein bis drei der folgenden Reste tragen können:

- Hydroxy,
- Halogen wie vorstehend im allgemeinen und im besonderen genannt;
- $C_1$-$C_4$-Alkyl wie vorstehend im allgemeinen und im besonderen genannt;
- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;
- $C_1$-$C_4$-Alkylthio wie vorstehend im allgemeinen und im besonderen genannt;

5-gliedrige aromatische Ringe, welche neben Kohlenstoffringgliedern ein oder zwei Stickstoffatome und ein Sauerstoff- oder Schwefelatom oder zwei Stickstoffatome oder ein Sauerstoff- oder ein Schwefelatom enthalten, wobei diese Ringe ein bis drei der folgenden Reste tragen können:

- Cyano,
- Halogen wie vorstehend im allgemeinen und im besonderen genannt;
- $C_1$-$C_4$-Alkyl wie vorstehend im allgemeinen und im besonderen genannt;

- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;
- $C_1$-$C_4$-Halogenalkoxy, besonders $C_1$-$C_2$-Halogenalkoxy wie vorstehend im allgemeinen und im besonderen genannt;
- $C_1$-$C_4$-Alkylthio wie vorstehend im allgemeinen und im besonderen genannt;
- $C_2$-$C_6$-Alkenyl wie vorstehend im allgemeinen und im besonderen genannt;
- $C_2$-$C_6$-Alkenyloxy wie Ethenyloxy, 1-Propenyloxy, 2-Propenyloxy, 1-Methylethenyloxy, 1-Butenyloxy, 2-Butenyloxy, 3-Butenyloxy, 1-Methyl-1-propenyloxy, 2-Methyl-1-propenyloxy, 1-Methyl-2-propenyloxy, 2-Methyl-2-propenyloxy, 1-Pentenyloxy, 2-Pentenyloxy, 3-Pentenyloxy, 4-Pentenyloxy, 1-Methyl-1- butenyloxy, 2-Methyl-1-butenyloxy, 3-Methyl-1-butenyloxy,1-Methyl-2-butenyloxy, 2-Methyl-2-butenyloxy,3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-1-butenyloxy, 1,2-Dimethyl-2-butenyloxy, 1,2-Dimethyl-3-butenyloxy, 1,3-Dimethyl-1- butenyloxy, 1,3-Dimethyl-2-butenyloxy, 1,3-Dimethyl-3-butenyloxy, 2,2-Dimethyl-3-butenyloxy, 2,3-Dimethyl-1-butenyloxy, 2,3-Dimethyl-2-butenyloxy, 2,3-Dimethyl-3-butenyloxy, 3,3-Dimethyl-1-butenyloxy, 3,3-Dimethyl-2-butenyloxy, 1-Ethyl-1-butenyloxy, 1-Ethyl-2-butenyloxy, 1-Ethyl-3-butenyloxy, 2-Ethyl-1-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-1-propenyloxy und 1-Ethyl-2-methyl-2-propenyloxy, vorzugsweise $C_2$-$C_4$-Alkenyloxy;
- $C_2$-$C_6$-Alkinyl wie vorstehend im allgemeinen und im besonderen genannt;
- $C_2$-$C_6$-Alkinyloxy wie Ethinyloxy, 1-Propinyloxy, 2-Propinyloxy, 1-Butinyloxy, 2-Butinyloxy, 3-Butinyloxy, 1-Methyl-2-propinyloxy, 1-Pentinyloxy, 2-Pentinyloxy, 3-Pentinyloxy, 4-Pentinyloxy, 1-Methyl-2-butinyloxy, 1-Methyl-3-butinyloxy, 2-Methyl-3-butinyloxy, 3-Methyl-1-butinyloxy, 1,1-Dimethyl-2-propinyloxy, 1-Ethyl-2-propinyloxy, 1-Hexinyloxy, 2-Hexinyloxy, 3-Hexinyloxy, 4-Hexinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-1-pentinyloxy, 3-Methyl-4-pentinyloxy, 4-Methyl-1-pentinyloxy, 4-Methyl-2-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 3,3-Dimethyl-1-butinyloxy, 1-Ethyl-2-butinyloxy, 1-Ethyl-3-butinyloxy, 2-Ethyl-3-butinyloxy und 1-Ethyl-1-methyl-2-propinyloxy, vorzugsweise $C_2$-$C_4$-Alkinyloxy;
- und $C_1$-$C_4$-Alkoxy-$C_1$-$C_4$-alkyl steht für durch $C_1$-$C_4$-Alkoxy wie vorstehend genannt substituiertes $C_1$-$C_4$-Alkyl wie vorstehend genannt;

Phenyl oder Pyridyl, wobei diese Ringe ein bis drei der folgenden Reste tragen können: Nitro, Formyl, Cyano,

- Halogen wie vorstehend im allgemeinen und im besonderen genannt;
- $C_1$-$C_4$-Alkyl wie vorstehend im allgemeinen und im besonderen genannt;
- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;
- $C_1$-$C_4$-Halogenalkoxy, besonders $C_1$-$C_2$-Halogenalkoxy wie vorstehend im allgemeinen und im besonderen genannt;
- $C_1$-$C_4$-Alkylthio wie vorstehend im allgemeinen und im besonderen genannt;
- $C_2$-$C_6$-Alkenyl wie vorstehend im allgemeinen und im besonderen genannt;
- $C_2$-$C_6$-Alkenyloxy wie vorstehend im allgemeinen und im besonderen genannt;
- $C_3$-$C_6$-Alkinyl wie vorstehend im allgemeinen und im besonderen genannt;
- $C_3$-$C_6$-Alkinyloxy wie vorstehend im allgemeinen und im besonderen genannt;
- und $NR^kR^l$;

$R^k$ Wasserstoff;
$C_1$-$C_4$-Alkyl wie vorstehend im allgemeinen und im besonderen genannt;
$C_3$-$C_6$-Alkenyl wie vorstehend im allgemeinen und im besonderen genannt;
$C_3$-$C_6$-Alkinyl wie vorstehend im allgemeinen und im besonderen genannt;

$R^l$ Wasserstoff;
$C_1$-$C_4$-Alkyl wie vorstehend im allgemeinen und im besonderen genannt;

$C_3$-$C_6$-Alkenyl wie vorstehend im allgemeinen und im besonderen genannt;
$C_3$-$C_6$-Alkinyl wie vorstehend im allgemeinen und im besonderen genannt;
$C_1$-$C_6$-Alkylcarbonyl wie vorstehend genannt, vorzugsweise $C_1$-$C_4$-Alkylcarbonyl, insbesondere $C_1$-$C_2$-Alkylcarbonyl;
Benzoyl, welches ein bis drei der folgenden Reste tragen kann: Nitro, Cyano,

- Halogen wie vorstehend im allgemeinen und im besonderen genannt;
- $C_1$-$C_4$-Alkyl wie vorstehend im allgemeinen und im besonderen genannt;
- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;
- $C_1$-$C_4$-Alkylthio wie vorstehend im allgemeinen und im besonderen genannt;

$R^d$
Wasserstoff; Hydroxy;
oder, sofern $R^c$ für $C_1$-$C_6$-Alkyl wie vorstehend genannt steht, ebenfalls $C_1$-$C_6$-Alkyl;
$R^e$
Wasserstoff; Cyano;
Halogen wie vorstehend im allgemeinen und im besonderen genannt;
$C_1$-$C_4$-Alkoxycarbonyl wie vorstehend im allgemeinen und im besonderen genannt;
$C_1$-$C_4$-Alkylketoxim wie Methylketoxim, Ethylketoxim, Propylketoxim, 1-Methylethylketoxim, Butylketoxim, 1-Methylpropylketoxim, 2-Methylpropylketoxim und 1,1-Dimethylethylketoxim;
W
$C_1$-$C_6$-Alkylen [-$(CH_2)$a-; a = 1, 2, 3, 4, 5 oder 6], $C_3$-$C_6$-Alkenylen [-$(CH_2)_b$-CH=CH-$(CH)_c$-; b = 1, 2 oder 3, c = 0, 1, 2 oder 3, wobei die Summe von b + c = 1, 2, 3 oder 4 ist], oder $C_3$-$C_6$-Alkinylen [-$CH_2)_b$-C*C-$(CH)_c$-, wobei b und c die vorstehend gegebene Bedeutung haben und * für eine Dreifachbindung steht], wobei diese Gruppen $X^1$ eine Methylengruppe (= $CH_2$) und/oder ein bis drei der folgenden Reste tragen können:

- Halogen wie vorstehend im allgemeinen und im besonderen genannt;
- und $C_1$-$C_3$-Alkyl wie Methyl, Ethyl, Propyl und 1-Methylethyl, vorzugsweise Methyl;

$C_3$-$C_6$-Alkylen oder $C_3$-$C_6$-Alkenylen, wie vorstehend genannt, wobei in diesen Resten jeweils eine Methylengruppe durch Sauerstoff, Schwefel, SO, $SO_2$ oder $NR^i$ ersetzt ist [-$(CH_2)_f$-W'-$(CH_2)_g$-; f = 1, 2, 3, 4 oder 5; g = 0, 1, 2, 3 oder 4, wobei die Summe von f + g = 2, 3, 4 oder 5 ist;
W' = O, S, SO, $SO_2$ oder $NR^i$, oder
-$(CH_2)_h$-$(CH=CH)_i$-$(CH_2)_k$-W'-$(CH_2)_l$-$(CH=CH)_m$-$(CH_2)_n$-mit i,
m = 0 oder 1, wobei die Summe von i + m = 1 ist; h = 0, 1, 2 oder 3, wobei die Summe von h, i und k 1, 2, 3, 4 und 5 betragen kann; k, l, n = 0, 1, 2 oder 3, wobei die Summe von h, k, l, n = 1, 2 oder 3 ist] und wobei diese Gruppen anstelle von Wasserstoffatomen ein bis drei $C_1$-$C_3$-Alkyleste wie vorstehend genannt tragen können;

$R^i$ Wasserstoff;
$C_1$-$C_4$-Alkyl wie vorstehend im allgemeinen und im besonderen genannt;
$C_3$-$C_6$-Alkenyl wie vorstehend im allgemeinen und im besonderen genannt;
$C_3$-$C_6$-Alkinyl wie vorstehend im allgemeinen und im besonderen genannt;

$R^f$
Wasserstoff; CH=CH-$Z^1$, worin

$Z^1$ Wasserstoff; Cyano; Carboxyl; Halogen wie vorstehend im allgemeinen und im besonderen genannt;
$C_1$-$C_4$-Alkyl wie vorstehend im allgemeinen und im besonderen genannt;
$C_1$-$C_4$-Alkoxy wie vorstehend im allgemeinen und im besonderen genannt;
$C_1$-$C_8$-Alkoxycarbonyl wie vorstehend genannt, vorzugsweise $C_1$-$C_4$-Alkoxycarbonyl, insbesondere $C_1$-$C_2$-Alkoxycarbonyl;
Benzyloxycarbonyl;
Phenyl, Thienyl oder Pyridyl, wobei dieser Reste ein bis drei der folgenden Gruppen tragen können: Nitro, Cyano,

- Halogen wie vorstehend im allgemeinen und im besonderen genannt;
- $C_1$-$C_4$-Alkyl wie vorstehend im allgemeinen und im besonderen genannt;
- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;

- $C_1$-$C_4$-Halogenalkoxy, besonders $C_1$-$C_2$-Halogenalkoxy wie vorstehend im allgemeinen und im besonderen genannt;
- $C_1$-$C_4$-Alkylthio wie vorstehend im allgemeinen und im besonderen genannt;
- oder $C_3$-$C_6$-Cyckloalkyl, wie vorstehend genannt, wobei der cyclische Rest seinerseits noch ein bis drei der folgenden Gruppen tragen kann:
  - Halogen wie vorstehend im allgemeinen und im besonderen genannt;
  - $C_1$-$C_4$-Alkyl wie vorstehend im allgemeinen und im besonderen genannt;
  - $C_1$-$C_4$-Halogenalkyl, besonders $C_1$-$C_2$-Halogenalkyl wie vorstehend im allgemeinen und im besonderen genannt;
  - $C_1$-$C_4$-Alkoxy wie vorstehend im allgemeinen und im besonderen genannt;

bedeutet;

$R^f$ bedeutet ferner

Ethinyl, welches einen der folgenden Reste tragen kann:
- $C_1$-$C_4$-Alkyl wie vorstehend im allgemeinen und im besonderen genannt;
- oder $C_3$-$C_6$-Cyckloalkyl wie vorstehend genannt, wobei diese Gruppen desweiteren ein bis drei der folgenden Reste tragen können: Hydroxy,
  - Halogen wie vorstehend im allgemeinen und im besonderen genannt;
  - $C_1$-$C_4$-Alkyl wie vorstehend im allgemeinen und im besonderen genannt;
  - $C_1$-$C_4$-Halogenalkyl, besonders $C_1$-$C_2$-Halogenalkyl wie vorstehend im allgemeinen und im besonderen genannt;
  - $C_1$-$C_4$-Alkoxy wie vorstehend im allgemeinen und im besonderen genannt;

Ethinyl, welches einen der folgenden Reste trägt: Phenyl, Thienyl oder Pyridyl, wobei die aromatischen Reste ein bis drei der folgenden Gruppen tragen können: Nitro, Cyano,
- Halogen wie vorstehend im allgemeinen und im besonderen genannt;
- $C_1$-$C_4$-Alkyl wie vorstehend im allgemeinen und im besonderen genannt;
- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;
- $C_1$-$C_4$-Halogenalkoxy, besonders $C_1$-$C_2$-Halogenalkoxy wie vorstehend im allgemeinen und im besonderen genannt;
- $C_1$-$C_4$-Alkylthio wie vorstehend im allgemeinen und im besonderen genannt;

Phenyl, 5-gliedrige aromatische Ringe, welche neben Kohlenstoffringgliedern ein oder zwei Stickstoffatome und ein Sauerstoff- oder Schwefelatom oder zwei Stickstoffatome oder ein Sauerstoff- oder ein Schwefelatom enthalten, oder 6-gliedrige aromatische Ringe, welche neben Kohlenstoffringgliedern ein bis vier Stickstoffatome enthalten, wobei diese aromatischen und heteroaromatischen Gruppen partiell oder vollständig halogeniert sein können und außerdem ein bis drei der folgenden Reste tragen können:
- Nitro,
- $C_1$-$C_4$-Halogenalkoxy, besonders $C_1$-$C_2$-Halogenalkoxy wie vorstehend im allgemeinen und im besonderen genannt;
- $C_1$-$C_4$-Alkylthio wie vorstehend im allgemeinen und im besonderen genannt;
- $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2, 2-difluorethylthio, 2,2-Dichlor-2-fluorethylthio, 2,2,2-Trichlorethylthio und Pentafluorethylthio, vorzugsweise Trichlormethylthio;
- die bei $Z^1$ genannten Reste
- und $NR^kR^l$, wobei $R^k$ und $R^l$ die vorstehend gegebene Bedeutung haben.

In der Bedeutung $R^c$ sind unter 5-gliedrigen gesättigten Ringen, welche neben Kohlenstoffringgliedern ein oder zwei Sauerstoff- oder Schwefelatome oder ein Sauerstoff- und ein Schwefelatom enthalten, die folgenden Gruppen zu verstehen: 2-Tetrahydrofuranyl, 3-Tetrahydrofuranyl, 2-Tetrahydrothienyl, 3-Tetrahydrothienyl, 1,3-Dioxolan-2-yl, 1,3-Dioxolan-4-yl, 1,3-Dithiolan-2-yl, 1,3-Dithiolan-4-yl, 1,3-Thioxolan-2-yl, 1,3-Dioxolan-4-yl und 1,3-Dioxolan-5-yl.

In der Bedeutung $R^c$ sind unter 6- oder 7-gliedrigen gesättigten oder ein- oder zweifach ungesättigten Ringen, welche neben Kohlenstoffringgliedern ein oder zwei Sauerstoff- oder Schwefel- atome oder ein Sauerstoff- und ein Schwefelatom enthalten, die folgenden Gruppen zu verstehen: Oxan-2-yl, Oxan-3-yl, Oxan-4- yl, Oxepan-2-yl, Oxepan-3-yl, Oxepan-4-yl, Oxepan-5-yl, Thioxan-2-yl, Thioxan-3-yl, Thioxan-4-yl, Thioxepan-2-yl, Thioxepan-3-yl, Thioxepan-4-yl, Thioxepan-5-yl, 1,3-Dioxan-2-yl, 1,3-Dioxan-4-yl, 1,3-Dioxepan-2-yl, 1,3-Dioxepan-4-yl, 1,3-Dioxepan-5-yl, 1,3-Thioxan-2-yl, 1,3-Thioxan-4-yl, 1,3-Thioxan-5-yl,

1,3-Thioxepan-2-yl, 1,3-Thioxepan-4-yl, 1,3-Thioxepan-5-yl, 1,3-Thioxepan-6-yl, 1,3-Thioxepan-7-yl, 1,3-Dithioxan-2-yl, 1,3-Dithioxan-4-yl, 1,3-Dithioxepan-2-yl, 1,3-Dithioxepan-4-yl, 1,3-Dithioxepan-5-yl, 1,4-Dioxan-2-yl, 1,4-Dioxepan-2-yl, 1,4-Dioxepan-5-yl, 1,4-Dioxepan-6-yl, 1,4-Dithioxan-2-yl, 1,4-Dithioxepan-2-yl, 1,4-Dithioxepan-5-yl, 1,4-Dithioxepan-6-yl, 1,4-Thioxan-2-yl, 1,4-Thioxan-3-yl, 1,4-Thioxan-5-yl, 1,4-Thioxan-6-yl, 1,4-Thioxepan-2-yl, 1,4-Thioxepan-3-yl, 1,4-Thioxepan-5-yl, 1,4-Thioxepan-6-yl, 1,4-Thioxepan-7-yl, Oxin-2-yl, Oxin-3-yl, Oxin-4-yl, Oxepin-2-yl, Oxepin-3-yl, Oxepin-4-yl, Oxepin-5-yl, Thioxin-2-yl, Thioxin-3-yl, Thioxin-4-yl, Thioxepin-2-yl, Thioxepin-3-yl, Thioxepin-4-yl, Thioxepin-5-yl, 1,3-Dioxin-2-yl, 1,3-Dioxin-4-yl, 1,3-Dioxepin-2-yl, 1,3-Dioxepin-4-yl, 1,3-Dioxepin-5-yl, 1,3-Thioxin-2-yl, 1,3-Thioxin-4-yl, 1,3-Thioxin-5-yl, 1,3-Thioxepin-2-yl, 1,3-Thioxepin-4-yl, 1,3-Thioxepin-5-yl, 1,3-Thioxepin-6-yl, 1,3-Thioxepin-7-yl, 1,3-Dithioxin-2-yl, 1,3-Dithioxin-4-yl, 1,3-Dithioxepin-2-yl, 1,3-Dithioxepin-4-yl, 1,3-Dithioxepin-5-yl, 1,4-Dioxin-2-yl, 1,4-Dioxepin-2-yl, 1,4-Dioxepin-5-yl, 1,4-Dioxepin-6-yl, 1,4-Dithioxin-2-yl, 1,4-Dithioxepin-2-yl, 1,4-Dithioxepin-5-yl, 1,4-Dithioxepin-6-yl, 1,4-Thioxin-2-yl, 1,4-Thioxin-3-yl, 1,4-Thioxin-5-yl, 1,4-Thioxin-6-yl, 1,4-Thioxepin-2-yl, 1,4-Thioxepin-3-yl, 1,4-Thioxepin-5-yl, 1,4-Thioxepin-6-yl und 1,4-Thioxepin-7-yl.

In der Bedeutung $R^c$ und $R^f$ sind unter 5-gliedrigen aromatischen Ringen, welche neben Kohlenstoffatomen ein bis drei Stickstoffatome und ein Sauerstoff- oder ein Schwefelatom als Heteroatome enthalten können, oder welche neben Kohlenstoffatomen ein bis drei Stickstoffatome oder ein Sauerstoff- oder ein Schwefelatom als Heteroatome enthalten können, sind die folgenden Gruppenzu verstehen: 2-Furyl, 3-Furyl, 2-Thienyl, 3-Thienyl, 2-Pyrrolyl, 3-Pyrrolyl, 3-Isoxazolyl, 4-Isoxazolyl, 5-Isoxaiolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl.

In der Bedeutung $R^f$ sind unter 6-gliedrigen aromatischen Ringen, welche neben Kohlenstoffatomen ein bis vier Stickstoffatome als Heteroatome enthalten können, sind vorzugsweise die folgenden Gruppen zu verstehen: 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl, insbesondere 2-Pyridinyl, 3-Pyridinyl und 4-Pyridinyl.

Ganz besonders bevorzugte Cyclohexenon-Derivate der Formel II, deren Kulturpflanzenverträglichkeit durch substituierte Pyrido[2,3-d]pyrimidine I und I' verbessert werden kann, sind den folgenden Tabellen II.1 bis II.8 zu entnehmen:

Tabelle II.1

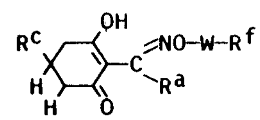

II (R^b, R^d, R^e = H)

| Nr. | $R^a$ | $R^c$ | W | $R^f$ | Literatur |
|---|---|---|---|---|---|
| A.001 | $n-C_3H_7$ | 2-(Ethylthio)propyl | $-CH_2CH_2-$ | H | DE-A 2 822 304 |
| A.002 | $C_2H_5$ | 2-(Ethylthio)propyl | $-CH_2CH=CCl-$ | H | US-A 4 440 566 |
| A.003 | $n-C_3H_7$ | 2-(Ethylthio)propyl | $-CH_2CH=CCl-$ | H | US-A 4 440 566 |
| A.004 | $n-C_3H_7$ | Tetrahydrothiopyran-3-yl | $-CH_2CH_2-$ | H | EP-A 71 707 |
| A.005 | $C_2H_5$ | Tetrahydrothiopyran-3-yl | $-CH_2CH_2-$ | H | EP-A 71 707 |
| A.006 | $CH_3$ | Tetrahydrothiopyran-3-yl | $-CH_2CH=CCH_3-$ | H | EP-A 71 707 |
| A.007 | $n-C_3H_7$ | Tetrahydropyran-3-yl | $-CH_2CH_2-$ | H | EP-A 71 707 |
| A.008 | $C_2H_5$ | Tetrahydropyran-4-yl | $-CH_2CH=CCl-$ | H | EP-A 142 741 |
| A.009 | $n-C_3H_7$ | Pyridin-3-yl | $-CH_2CH_2-$ | H | EP-A 66 195 |
| A.010 | $C_2H_5$ | $4-CH_3$-phenyl | $-CH_2CH_2-$ | H | DE-A 24 39 104 |
| A.011 | $C_2H_5$ | $4-C_2H_5$-phenyl | $-CH_2CH=CCH_3-$ | H | DE-A 38 08 072 |
| A.012 | $C_2H_5$ | $2,4,6-(CH_3)_3$-phenyl | $-CH_2CH_2-$ | H | EP-A 88 301 |
| A.013 | $n-C_3H_7$ | $4-CH_3$-cyclohexyl | $-CH_2CH=CCl-$ | H | EP-A 88 299 |
| A.014 | $n-C_3H_7$ | $4-CH_3$-cyclohexyl | $-CH_2CH=CCH_3-$ | H | EP-A 88 299 |
| A.015 | $C_2H_5$ | 3-Isopropyl-isoxazol-5-yl | $-CH_2CH=CCH_3-$ | H | EP-A 238 021 |
| A.016 | $n-C_3H_7$ | 3-Isopropyl-isoxazol-5-yl | $-CH_2CH=CCH_3-$ | H | EP-A 238 021 |
| A.017 | $C_2H_5$ | $4-(HC\equiv C-CH_2O)$-phenyl | $-CH_2CH=CCl-$ | H | EP-A 137 174 |

Tabelle II.1 (Fortsetzung)

| Nr. | $R^a$ | $R^c$ | W | $R^f$ | Literatur |
|---|---|---|---|---|---|
| A.018 | $n-C_3H_7$ | $4-C_2H_5OCH_2$-phenyl | $-CH_2CH_2-$ | H | EP-A 2 137 200 |
| A.019 | $n-C_3H_7$ | $3,4-Br_2$-tetrahydropyran-3-yl | $-CH_2CH_2-$ | H | EP-A 230 235 |
| A.020 | $n-C_3H_7$ | $3,4-Br_2$-tetrahydropyran-3-yl | $-CH_2CH=CCl-$ | H | EP-A 230 235 |
| A.021 | $n-C_3H_7$ | $2,6,6-(CH_3)_3$-cyclohex-1-enyl | $-CH_2CH=CCl-$ | H | EP-A 46 860 |
| A.022 | $n-C_3H_7$ | Cyclohexyl | $-CH_2CH_2-$ | H | JP-A 540 191 945 |
| A.023 | $n-C_3H_7$ | Cyclohex-1-enyl | $-CH_2CH_2-$ | H | EP-A 46 860 |
| A.024 | $CH_3$ | $4-CH_3$-cyclohexyl | $-CH_2CH=CCl-$ | H | EP-A 88 299 |
| A.025 | $n-C_3H_7$ | $4-CF_3$-phenyl | $-CH_2CH_2-$ | H | EP-A 137 174 |
| A.026 | $C_2H_5$ | $2,6,6-(CH_3)_3$-cyclohex-1-enyl | $-CH_2CH=CCl-$ | H | EP-A 46 860 |
| A.027 | $n-C_3H_7$ | $2-CH_3$-thiazol-4-yl | $-CH_2CH=CCH_3-$ | H | EP-A 125 094 |
| A.028 | $n-C_3H_7$ | $2-CH_3$-thiazol-4-yl | $-CH_2CH=CCl-$ | H | EP-A 125 094 |
| A.029 | $n-C_3H_7$ | $2,4,6-(CH_3)_3$-cyclohexyl | $-CH_2CH_2-$ | H | EP-A 88 299 |
| A.030 | $n-C_3H_7$ | $3-C_2H_5S-4-OH-4-CH_3$-cyclohexyl | $-CH_2CH=CH-$ | H | EP-A 228 598 |
| A.031 | $C_2H_5$ | $3,4-(OH)_2$-cyclohexyl | $-CH_2CH_2-$ | H | EP-A 228 598 |
| A.032 | $n-C_3H_7$ | $1-CH_3$-pyrazol-3-yl | $-CH_2CH_2-$ | H | EP-A 66 195 |
| A.033 | $n-C_3H_7$ | $1-CH_3$-pyrrol-3-yl | $-CH_2CH=CCl-$ | H | EP-A 66 195 |
| A.034 | $n-C_3H_7$ | $2-CH_3$-thiazol-4-yl | $-CH_2CH=CH-$ | H | EP-A 125 094 |
| A.035 | $n-C_3H_7$ | $(CH_3CH_2S)_2$-methyl | $-CH_2CH_2CH_2-$ | H | EP-A 230 260 |
| A.036 | $n-C_3H_7$ | 1-Oxo-tetrahydrothiopyran-3-yl | $-CH_2CH_2-$ | H | EP-A 115 808 |

Tabelle II.1 (Fortsetzung)

| Nr. | $R^a$ | $R^c$ | W | $R^f$ | Literatur |
|---|---|---|---|---|---|
| A.037 | $n-C_3H_7$ | 1,1-Dioxo-tetrahydrothiopyran-3-yl | $CH_2CH_2-$ | H | EP-A 115 808 |
| A.038 | $n-C_3H_7$ | 1,1-Dioxo-tetrahydrothiopyran-3-yl | $-CH_2CH=CH-$ | H | Proceedings Brit. Crop-Protection Conference -weeds 1985 Vol.1 S. 93-98 |
| A.039 | $CH_3$ | 4-F-phenyl-thioethyl | $-CH_2CH_2-$ | H | EP 254 514 |
| A.040 | $C_2H_5$ | 4-F-phenyl-thioethyl | $-CH_2CH_2-$ | H | EP 254 514 |
| A.041 | $C_2H_5$ | 4-F-phenyl-thioethyl | $-CH_2CH=CH-$ | H | EP 254 514 |
| A.042 | $C_2H_5$ | 4-F-phenyl-thioethyl | $-CH_2CH=CHCH_2-$ | H | EP 254 514 |
| A.043 | $n-C_3H_7$ | 4-F-phenyl-thioethyl | $-CH_2CH=CH-$ | H | EP 254 514 |
| A.044 | $n-C_3H_7$ | Formyl | $-CH_2CH_2-$ | H | EP 319 835 |
| A.045 | $n-C_3H_7$ | $1-CH_3S$-cyclopropyl | $-CH_2CH_2-$ | H | EP 243 313 |
| A.046 | $n-C_3H_7$ | $1-CH_3S$-cyclopropyl | H Cl<br>\| \|<br>$-CH_2C=C-$ | H | EP 243 313 |
| A.047 | $C_2H_5$ | $1-CH_3S$-cyclopropyl | H Cl<br>\| \|<br>$-CH_2C=C-$ | H | EP 243 313 |
| A.048 | $C_2H_5$ | $1-CH_3S$-cyclopropyl | H<br>\|<br>$-CH_2C=C-$<br>\|<br>Cl | H | EP 243 313 |
| A.049 | $C_2H_5$ | $1-C_2H_5S$-cyclopropyl | H Cl<br>\| \|<br>$-CH_2C=C-$ | H | EP 243 313 |

Tabelle II.1 (Fortsetzung)

| Nr. | $R^a$ | $R^c$ | W | $R^f$ | Literatur |
|---|---|---|---|---|---|
| A.050 | $n-C_3H_7$ | $1-C_2H_5S$-cyclopropyl | $-CH_2\overset{H}{C}=\overset{Cl}{C}-$ | H | EP 243 313 |
| A.051 | $C_2H_5$ | Tetrahydrothiopyran-3-yl | $-CH_2CH=CHCH_2-$ | 4-Cl-phenyl | EP-A 89 120 558 |
| A.052 | $C_2H_5$ | Tetrahydrothiopyran-3-yl | $-CH_2CH_2CH=CH-$ | 4-Cl-phenyl | EP-A 89 120 558 |
| A.053 | $C_2H_5$ | Tetrahydrothiopyran-3-yl | $-CH_2CH_2CH=CH-$ | 4-F-phenyl | EP-A 89 120 558 |
| A.054 | $n-C_3H_7$ | Tetrahydrothiopyran-3-yl | $-CH_2CH_2CH=CH-$ | 4-F-phenyl | EP-A 89 120 558 |
| A.055 | $C_2H_5$ | Tetrahydrothiopyran-3-yl | $-CH_2CH=CHCH_2-$ | Phenyl | EP-A 89 120 558 |
| A.056 | $n-C_3H_7$ | Tetrahydrothiopyran-3-yl | $-CH_2-$ | 5-Cl-thien-2-yl | EP-A 177 913 |
| A.057 | $C_2H_5$ | Tetrahydrothiopyran-3-yl | $-CH_2-$ | 5-Cl-thien-2-yl | EP-A 177 913 |
| A.058 | $C_2H_5$ | Tetrahydropyran-3-yl | $-CH_2-$ | 5-Cl-thien-2-yl | EP-A 177 913 |
| A.059 | $n-C_3H_7$ | Tetrahydropyran-4-yl | $-CH_2-$ | 5-Cl-thien-2-yl | EP-A 177 913 |
| A.060 | $n-C_3H_7$ | Tetrahydrothiopyran-3-yl | $-CH_2-$ | Thien-2-yl | EP-A 177 913 |
| A.061 | $CH_3$ | Tetrahydropyran-3-yl | $-CH_2-$ | Thien-2-yl | EP-A 177 913 |
| A.062 | $C_2H_5$ | Tetrahydropyran-4-yl | $-CH_2-$ | Thien-2-yl | EP-A 177 913 |
| A.063 | $C_2H_5$ | Tetrahydropyran-3-yl | $-(CH_2)_4-$ | 4-F-phenyl | DE-A 38 38 309 |
| A.064 | $n-C_3H_7$ | Tetrahydropyran-3-yl | $-(CH_2)_4-$ | 4-F-phenyl | DE-A 38 38 309 |
| A.065 | $C_2H_5$ | Tetrahydrothiopyran-3-yl | $-(CH_2)_4-$ | 4-F-phenyl | DE-A 38 38 309 |
| A.066 | $n-C_3H_7$ | Tetrahydrothiopyran-3-yl | $-(CH_2)_4-$ | 4-F-phenyl | DE-A 38 38 309 |
| A.067 | $C_2H_5$ | Tetrahydropyran-3-yl | $-(CH_2)_4-$ | 4-F-phenyl | DE-A 38 38 309 |

Tabelle II.1 (Fortsetzung)

| Nr. | $R^a$ | $R^c$ | W | $R^f$ | Literatur |
|---|---|---|---|---|---|
| A.068 | n-$C_3H_7$ | Tetrahydropyran-3-yl | $-(CH_2)_4-$ | 4-F-phenyl | DE-A 38 38 309 |
| A.069 | $C_2H_5$ | Tetrahydropyran-3-yl | $-(CH_2)_4-$ | 4-Cl-phenyl | DE-A 38 38 309 |
| A.070 | n-$C_3H_7$ | Tetrahydropyran-3-yl | $-(CH_2)_4-$ | 4-Cl-phenyl | DE-A 38 38 309 |
| A.071 | $C_2H_5$ | Tetrahydropyran-4-yl | $-(CH_2)_4-$ | 4-Cl-phenyl | DE-A 38 38 309 |
| A.072 | n-$C_3H_7$ | Tetrahydropyran-4-yl | $-(CH_2)_4-$ | 4-Cl-phenyl | DE-A 38 38 309 |
| A.073 | $C_2H_5$ | Tetrahydrothiopyran-3-yl | $-(CH_2)_4-$ | 4-Cl-phenyl | DE-A 38 38 309 |
| A.074 | n-$C_3H_7$ | Tetrahydrothiopyran-3-yl | $-(CH_2)_4-$ | 4-Cl-phenyl | DE-A 38 38 309 |

Tabelle II.2

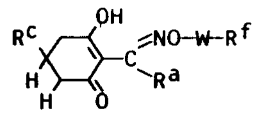

($R^b, R^d, R^e$ = H)

($R^c$ = Tetrahydrothiopyran-3-yl)

| Bsp. | $R^a$ | W | $R^f$ | phys. Daten (NMR-Daten in ppm) (Fp. in °C) |
|---|---|---|---|---|
| A.075 | $C_2H_5$ | $-CH_2-CH=CH-$ | Phenyl | 103-104 |
| A.076 | $n-C_3H_7$ | $-CH_2-CH=CH-$ | Phenyl | 4,7 (d,2H), 6,3 (dt,1H), 6,7 (d,1H), 7,2-7,5 (2m,5H) |
| A.077 | $C_2H_5$ | $-CH_2-CH=CH-$ | 4-Cl-phenyl | 106-107 |
| A.078 | $n-C_3H_7$ | $-CH_2-CH=CH-$ | 4-Cl-phenyl | 4,7 (d,2H), 6,3 (dt,1H), 6,65 (d,1H), 7,2-7,5 (m,4H) |
| A.079 | $C_2H_5$ | $-CH_2-CH=CH-$ | 4-F-phenyl | 90- 91 |
| A.080 | $n-C_3H_7$ | $-CH_2-CH=CH-$ | 4-F-phenyl | 4,6 (d,2H), 6,2 (dt,1H), 6,6 (d,1H), 7,0 (m,2H), 7,4 (m,2H) |
| A.081 | $C_2H_5$ | $-CH_2-CH=CH-$ | 2,4-$Cl_2$-phenyl | 123-124 |
| A.082 | $n-C_3H_7$ | $-CH_2-CH=CH-$ | 2,4-$Cl_2$-phenyl | 80- 82 |
| A.083 | $C_2H_5$ | $-(CH_2)_3CH=CH-$ | Phenyl | 80- 82 |
| A.084 | $n-C_3H_7$ | $-(CH_2)_3CH=CH-$ | Phenyl | 4,1 (t,2H), 6,2 (dt,1H), 6,4 (d,1H), 7,2-7,4 (m,5H) |
| A.085 | $C_2H_5$ | $-(CH_2)_3CH=CH-$ | 4-Cl-phenyl | 108-110 |
| A.086 | $n-C_3H_7$ | $-(CH_2)_3CH=CH-$ | 4-Cl-phenyl | 4,1 (t,2H), 6,2 (dt,1H), 6,4 (d,1H), 7,3 (s,4H) |
| A.087 | $C_2H_5$ | $-(CH_2)_3-$ | Phenyl | 4,0 (t,2H), 7,0-7,4 (m,5H) |
| A.088 | $n-C_3H_7$ | $-(CH_2)_3-$ | Phenyl | 4,0 (t,2H), 7,0-7,4 (m,5H) |
| A.089 | $C_2H_5$ | $-CH_2C(=CH_2)-CH_2-$ | Phenyl | 3,3 (s,2H), 4,4 (s,2H), 5,1 und 5,2 (2s,2H), 7,1-7,4 (m,5H) |
| A.090 | $n-C_3H_7$ | $-CH_2C(=CH_2)-CH_2-$ | Phenyl | 3,35 (s,2H), 4,4 (s,2H), 5,0 und 5,1 (2s,2H), 7,0-7,4 (m,5H) |

Tabelle II.2 (Fortsetzung)

| Bsp. | $R^a$ | W | $R^f$ | phys. Daten (Fp. in °C) (NMR-Daten in ppm) |
|---|---|---|---|---|
| A.091 | $C_2H_5$ | $-CH_2CH=CH-$ | 4-Br-phenyl | 89- 91 |
| A.092 | $n-C_3H_7$ | $-CH_2CH=CH-$ | 4-Br-phenyl | 97- 99 |
| A.093 | $C_2H_5$ | $-CH_2CH=CH-$ | 4-$CH_3$-phenyl | 103-105 |
| A.094 | $n-C_3H_7$ | $-CH_2CH=CH-$ | 4-$CH_3$-phenyl | 88- 90 |
| A.095 | $C_2H_5$ | $-CH_2CH=CH-$ | 4-$CF_3$-phenyl | 97- 98 |
| A.096 | $n-C_3H_7$ | $-CH_2CH=CH-$ | 4-$CF_3$-phenyl | 4,75 (d,2H), 6,45 (dt,1H), 6,75 (d,1H), 7,4-7,8 (m,4H) |
| A.097 | $C_2H_5$ | $-CH_2CH=CH-$ | 4-$C_6H_5O$-phenyl | 4,65 (d,2H), 6,25 (dt,1H), 6,65 (d,1H), 6,9-7,6 (3m,9H) |
| A.098 | $n-C_3H_7$ | $-CH_2CH=CH-$ | 4-$C_6H_5O$-phenyl | 4,65 (d,2H), 6,25 (dt,1H), 6,65 (d,1H), 6,9-7,5 (3m, 9H) |
| A.099 | $C_2H_5$ | $-CH_2CH=C(CH_3)-$ | Phenyl | 77- 78 |
| A.100 | $n-C_3H_7$ | $-CH_2CH=C(CH_3)-$ | Phenyl | 2,15 (s,3H), 4,75 (d,2H), 5,95 (t,1H), 7,2-7,6 (m,5H) |
| A.101 | $C_2H_5$ | $-CH_2CH=CH-$ | 2-Cl-phenyl | 97- 98 |
| A.102 | $n-C_3H_7$ | $-CH_2CH=CH-$ | 2-Cl-phenyl | 87- 89 |
| A.103 | $C_2H_5$ | $-(CH_2)_3-$ | 4-F-phenyl | 4,05 (t,2H), 6,9-7,2 (2m,4H) |
| A.104 | $n-C_3H_7$ | $-(CH_2)_3-$ | 4-F-phenyl | 4,05 (t,2H), 6,9-7,2 (2m,4H) |
| A.105 | $C_2H_5$ | $-(CH_2)_3-$ | 2,4-$Cl_2$-phenyl | 63- 65 |
| A.106 | $n-C_3H_7$ | $-(CH_2)_3-$ | 2,4-$Cl_2$-phenyl | 4,05 (t,2H), 7,05-7,4 (2m,3H) |
| A.107 | $C_2H_5$ | $-(CH_2)_3-$ | 4-Br-phenyl | 4,05 (t,2H), 7,05 und 7,45 (2m,4H) |
| A.108 | $n-C_3H_7$ | $-(CH_2)_3-$ | 4-Br-phenyl | 4,05 (t,2H), 7,05 und 7,45 (2m,4H) |
| A.109 | $C_2H_5$ | $-(CH_2)_3-$ | 2-Cl-phenyl | 4,1 (t,2H), 7,05-7,4 (m,4H) |
| A.110 | $n-C_3H_7$ | $-(CH_2)_3-$ | 2-Cl-phenyl | 4,1 (t,2H), 7,05-7,4 (m,4H) |

Tabelle II.2 (Fortsetzung)

| Bsp. | $R^a$ | W | $R^f$ | phys. Daten (NMR-Daten in ppm) (Fp. in °C) |
|---|---|---|---|---|
| A.111 | $C_2H_5$ | $-(CH_2)_3-$ | 4-Cl-phenyl | 4,05 (t, 2H), 7,0-7,4 (m, 4H) |
| A.112 | $n-C_3H_7$ | $-(CH_2)_3-$ | 4-Cl-phenyl | 4,05 (t, 2H), 7,0-7,4 (m, 4H) |
| A.113 | $C_2H_5$ | $-CH_2CH=CH-$ | 3,5-$Cl_2$-phenyl | 75- 77 |
| A.114 | $n-C_3H_7$ | $-CH_2CH=CH-$ | 3,5-$Cl_2$-phenyl | 70- 73 |
| A.115 | $C_2H_5$ | $-CH_2CH_2CH(CH_3)-$ | Phenyl | 1,25 (d, 3H), 3,95 (m, 2H), 7,05-7,4 (m, 5H) |
| A.116 | $n-C_3H_7$ | $-CH_2CH_2CH(CH_3)-$ | Phenyl | 1,25 (d, 3H), 3,95 (m, 2H), 7,05-7,4 (m, 5H) |
| A.117 | $C_2H_5$ | $-(CH_2)_3-$ | 3,5-$Cl_2$-phenyl | 82- 84 |
| A.118 | $n-C_3H_7$ | $-(CH_2)_3-$ | 3,5-$Cl_2$-phenyl | 4,05 (t, 2H), 7,0-7,25 (m, 3H) |
| A.119 | $C_2H_5$ | $-CH_2CH_2C(=CH_2)-$ | Phenyl | 4,15 (t, 2H), 5,15 (s, 1H), 5,25 (s, 1H), 7,2-7,6 (m, 5H) |
| A.120 | $n-C_3H_7$ | $-CH_2CH_2C(=CH_2)-$ | Phenyl | 4,15 (t, 2H), 5,15 (s, 1H), 5,25 (s, 1H), 7,2-7,6 (m, 5H) |
| A.121 | $CH_3$ | $-CH_2CH=CH-$ | 2,4-$Cl_2$-phenyl | 107-108 |
| A.122 | $CH_3$ | $-CH_2CH=CH-$ | 4-Cl-phenyl | 104-106 |
| A.123 | $C_2H_5$ | $-(CH_2)_5-$ | 4-Cl-phenyl | 4,05 (t, 2H), 7,0-7,4 (2m, 4H) |
| A.124 | $n-C_3H_7$ | $-(CH_2)_5-$ | 4-Cl-phenyl | 64- 66 |
| A.125 | $C_2H_5$ | $-CH_2CH=CH-$ | 3,4-$Cl_2$-phenyl | 4,7 (d, 2H), 6,3 (dt, 1H), 6,55 (d, 1H), 7,2-7,6 (m, 3H) |
| A.126 | $n-C_3H_7$ | $-CH_2CH=CH-$ | 3,4-$Cl_2$-phenyl | 4,7 (d, 2H), 6,3 (dt, 1H), 6,55 (d, 1H), 7,2-7,6 (m, 3H) |
| A.127 | $C_2H_5$ | $-CH_2CH(CH_3)CH_2-$ | Phenyl | 0,95 (d, 3H), 3,9 (m, 2H), 7,0-7,5 (m, 5H) |
| A.128 | $n-C_3H_7$ | $-CH_2CH(CH_3)CH_2-$ | Phenyl | 0,95 (d, 3H), 3,9 (m, 2H), 7,0-7,5 (m, 5H) |
| A.129 | $C_2H_5$ | $-(CH_2)_3-$ | 3,4-$Cl_2$-phenyl | 4,05 (t, 2H), 7,0-7,1 und 7,2-7,4 (2m, 3H) |
| A.130 | $n-C_3H_7$ | $-(CH_2)_3-$ | 3,4-$Cl_2$-phenyl | 4,05 (t, 2H), 6,95-7,1 und 7,2-7,45 (2m, 3H) |

Tabelle II.2 (Fortsetzung)

| Bsp. | $R^a$ | W | $R^f$ | phys. Daten (NMR-Daten in ppm) (Fp. in °C) |
|---|---|---|---|---|
| A.131 | $C_2H_5$ | $-CH_2CH(CH_3)CH_2-$ | 4-F-phenyl | 0,95 (d,3H), 3,9 (dd,2H), 6,8-7,2 (m,4H) |
| A.132 | n-$C_3H_7$ | $-CH_2CH(CH_3)CH_2-$ | 4-F-phenyl | 0,95 (d,3H), 3,9 (dd,2H), 6,8-7,2 (m,4H) |
| A.133 | $C_2H_5$ | $-CH_2CH(CH_3)CH_2-$ | 4-Cl-phenyl | 0,9 (d,3H), 3,9 (m,2H), 7,0-7,4 (2m,4H) |
| A.134 | n-$C_3H_7$ | $-CH_2CH(CH_3)CH_2-$ | 4-Cl-phenyl | 0,9 (d,3H), 3,9 (m,2H), 7,0-7,4 (2m,4H) |
| A.135 | $C_2H_5$ | $-CH_2CH_2C(CH_3)_2-$ | 4-F-phenyl | 1,35 (s,6H), 3,85 (t,2H), 7,0 und 7,3 (2m,4H) |
| A.136 | n-$C_3H_7$ | $-CH_2CH_2C(CH_3)_2-$ | 4-F-phenyl | 1,35 (s,6H), 3,85 (t,2H), 7,0 und 7,3 (2m,4H) |
| A.137 | $C_2H_5$ | $-CH_2CH_2C(CH_3)_2-$ | 4-Cl-phenyl | 1,35 (s,6H), 3,85 (t,2H), 7,25 (s,4H) |
| A.138 | n-$C_3H_7$ | $-CH_2CH_2C(CH_3)_2-$ | 4-Cl-phenyl | 1,35 (s,6H), 3,85 (t,2H), 7,25 (s,4H) |
| A.139 | $C_2H_5$ | $-(CH_2)_6-$ | 4-Cl-phenyl | 1,15 (t,3H), 4,05 (t,2H), 7,1 (d,2H), 7,25 (d,2H) |
| A.140 | n-$C_3H_7$ | $-(CH_2)_6-$ | 4-Cl-phenyl | 0,95 (t,3H), 4,05 (t,2H), 7,1 (d,2H), 7,25 (d,2H) |
| A.141 | $C_2H_5$ | $-(CH_2)_6-$ | 4-F-phenyl | 1,1 (t,3H), 4,0 (t,2H) |
| A.142 | n-$C_3H_7$ | $-(CH_2)_6-$ | 4-F-phenyl | 0,95 (t,3H), 4,0 (t,2H) |
| A.143 | $C_2H_5$ | $-(CH_2)_5-$ | 4-F-phenyl | 1,1 (t,3H), 4,05 (t,2H), 6,95 und 7,1 (2m,4H) |
| A.144 | n-$C_3H_7$ | $-(CH_2)_5-$ | 4-F-phenyl | 0,9 (t,3H), 4,05 (t,2H), 6,95 und 7,1 (2m,4H) |
| A.145 | $C_2H_5$ | $-CH_2CH(CH_3)-(CH_2)_3-$ | 2-$CH_3$-phenyl | 2,3 (s,3H), 3,95 (t,1H), 7,1 (m,4H) |
| A.146 | n-$C_3H_7$ | $-CH_2CH(CH_3)-(CH_2)_3-$ | 2-$CH_3$-phenyl | 2,3 (s,3H), 3,9 (t,1H), 7,05 (m,4H) |
| A.147 | $C_2H_5$ | $-CH_2CH=CH-$ | 3-Br-phenyl | 96- 98 |

Tabelle II.2 (Fortsetzung)

| Bsp. | $R^a$ | W | $R^f$ | phys. Daten (NMR-Daten in ppm) (Fp. in °C) |
|---|---|---|---|---|
| A.148 | $n-C_3H_7$ | $-CH_2CH=CH-$ | 3-Br-phenyl | 0,95(t,3H), 4,65(d,2H), 6,3(dt,1H), 6,6(d,1H), 7,1-7,6(m,4H) |
| A.149 | $C_2H_5$ | $-CH_2CH=CH-$ | 3-Cl-phenyl | 98-100 |
| A.150 | $n-C_3H_7$ | $-CH_2CH=CH-$ | 3-Cl-phenyl | 1,0(t,3H), 4,7(d,2H), 6,35(dt,1H), 6,65(d,1H), 7,2-7,5(m,4H) |
| A.151 | $C_2H_5$ | $-CH_2CH=CH-$ | 3-F-phenyl | 77- 78 |
| A.152 | $n-C_3H_7$ | $-CH_2CH=CH-$ | 3-F-phenyl | 0,95(t,3H), 4,65(d,2H), 6,3(dt,1H), 6,6(d,1H), 6,9-7,3(m,4H) |

Tabelle II.3

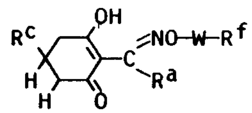

($R^b, R^d, R^e$ = H)

($R^c$ = Tetrahydropyran-3-yl)

| Bsp. | $R^a$ | W | $R^f$ | phys. Daten (NMR-Daten in ppm) (Fp. in °C) |
|---|---|---|---|---|
| A.153 | $C_2H_5$ | $-CH_2-CH=CH-$ | Phenyl | |
| A.154 | $n-C_3H_7$ | $-CH_2-CH=CH-$ | Phenyl | 4,7 (d,2H), 6,3 (dt,1H), 6,7 (d,1H), 7,2-7,5 (2m,5H) |
| A.155 | $C_2H_5$ | $-CH_2-CH=CH-$ | 4-Cl-phenyl | 106-108 |
| A.156 | $n-C_3H_7$ | $-CH_2-CH=CH-$ | 4-Cl-phenyl | 4,7 (d,2H), 6,3 (dt,1H), 6,65 (d,1H), 7,2-7,5 (m,4H) |
| A.157 | $C_2H_5$ | $-CH_2-CH=CH-$ | 4-F-phenyl | |
| A.158 | $n-C_3H_7$ | $-CH_2-CH=CH-$ | 4-F-phenyl | 4,65 (d,2H), 6,2 (dt,1H), 6,7 (d,1H), 7,0 (m,2H), 7,4 (m,2H) |
| A.159 | $C_2H_5$ | $-CH_2-CH=CH-$ | 2,4-$Cl_2$-phenyl | 135-137 |
| A.160 | $n-C_3H_7$ | $-CH_2-CH=CH-$ | 2,4-$Cl_2$-phenyl | 4,75 (d,2H), 6,3 (dt,1H), 7,0 (d,1H), 7,05-7,5 (2m,3H) |
| A.161 | $C_2H_5$ | $-(CH_2)_3CH=CH-$ | Phenyl | 4,1 (t,2H), 6,2 (dt,1H), 6,4 (d,1H), 7,2-7,4 (m,5H) |
| A.162 | $n-C_3H_7$ | $-(CH_2)_3CH=CH-$ | Phenyl | 4,1 (t,2H), 6,2 (dt,1H), 6,4 (d,1H), 7,1-7,4 (m,5H) |
| A.163 | $C_2H_5$ | $-(CH_2)_3CH=CH-$ | 4-Cl-phenyl | 92- 95 |
| A.164 | $n-C_3H_7$ | $-(CH_2)_3CH=CH-$ | 4-Cl-phenyl | 4,1 (t,2H), 6,2 (dt,1H), 6,35 (d,1H), 7,3 (s,4H) |
| A.165 | $C_2H_5$ | $-(CH_2)_3-$ | Phenyl | 4,05 (t,2H), 7,1-7,4 (m,5H) |
| A.166 | $n-C_3H_7$ | $-(CH_2)_3-$ | Phenyl | 4,05 (t,2H), 7,1-7,4 (m,5H) |
| A.167 | $C_2H_5$ | $-CH_2C(=CH_2)-CH_2-$ | Phenyl | 3,35 (s,2H), 4,4 (s,2H), 5,0 und 5,2 (2s,2H), 7,1-7,4 (m,5H) |
| A.168 | $n-C_3H_7$ | $-CH_2C(=CH_2)-CH_2-$ | Phenyl | 3,35 (s,2H), 4,4 (s,2H), 5,0 und 5,2 (2s,2H), 7,1-7,4 (m,5H) |

Tabelle II.3 (Fortsetzung)

| Bsp. | $R^a$ | W | $R^f$ | phys. Daten (NMR-Daten in ppm) (Fp. in °C) |
|---|---|---|---|---|
| A.169 | $C_2H_5$ | $-CH_2CH=CH-$ | 4-Br-phenyl | 114-116°C |
| A.170 | $n-C_3H_7$ | $-CH_2CH=CH-$ | 4-Br-phenyl | 99-100°C |
| A.171 | $C_2H_5$ | $-CH_2CH=CH-$ | $4-CH_3$-phenyl | 123-125 |
| A.172 | $n-C_3H_7$ | $-CH_2CH=CH-$ | $4-CH_3$-phenyl | 70- 72 |
| A.173 | $C_2H_5$ | $-CH_2CH=CH-$ | $4-CF_3$-phenyl | 104-106 |
| A.174 | $n-C_3H_7$ | $-CH_2CH=CH-$ | $4-CF_3$-phenyl | 4,75 (d,2H), 6,4 (dt,1H), 6,75 (d,1H), 7,4-7,8 (m,4H) |
| A.175 | $C_2H_5$ | $-CH_2CH=CH-$ | $4-C_6H_5O$-phenyl | 89- 91 |
| A.176 | $n-C_3H_7$ | $-CH_2CH=CH-$ | $4-C_6H_5O$-phenyl | 4,65 (d,2H), 6,25 (dt,1H), 6,65 (d,1H), 6,9-7,5 (3m,9H) |
| A.177 | $C_2H_5$ | $-CH_2CH=C(CH_3)-$ | Phenyl | 2,15 (s,3H), 4,75 (d,2H), 5,95 (t,1H), 7,2-7,6 (m,5H) |
| A.178 | $n-C_3H_7$ | $-CH_2CHC(CH_3)-$ | Phenyl | 2,15 (s,3H), 4,75 (d,2H), 5,95 (t,1H), 7,2-7,6 (m,5H) |
| A.179 | $C_2H_5$ | $-CH_2CH=CH-$ | 2-Cl-phenyl | 113-118 |
| A.180 | $n-C_3H_7$ | $-CH_2CH=CH-$ | 2-Cl-phenyl | 4,75 (d,2H), 6,3 (dt,1H), 7,05 (d,1H), 7,05-7,6 (m,4H) |
| A.181 | $C_2H_5$ | $-(CH_2)_3-$ | 4-F-phenyl | 4,1 (t,2H), 6,9-7,2 (2m,4H) |
| A.182 | $n-C_3H_7$ | $-(CH_2)_3-$ | 4-F-phenyl | 4,1 (t,2H), 6,8-7,15 (2m,4H) |
| A.183 | $C_2H_5$ | $-(CH_2)_3-$ | $2,4-Cl_2$-phenyl | 75- 77 |
| A.184 | $n-C_3H_7$ | $-(CH_2)_3-$ | $2,4-Cl_2$-phenyl | 4,05 (t,2H), 7,05-7,5 (2m,3H) |
| A.185 | $C_2H_5$ | $-(CH_2)_3-$ | 2-Cl-phenyl | 4,1 (t,2H), 7,0-7,4 (m,4H) |
| A.186 | $n-C_3H_7$ | $-(CH_2)_3-$ | 2-Cl-phenyl | 4,1 (t,2H), 7,0-7,4 (m,4H) |
| A.187 | $C_2H_5$ | $-(CH_2)_3-$ | 4-Cl-phenyl | 62- 64 |
| A.188 | $n-C_3H_7$ | $-(CH_2)_3-$ | 4-Cl-phenyl | 4,05 (t,2H), 7,05-7,3 (2m,4H) |

Tabelle II.3 (Fortsetzung)

| Bsp. | $R^a$ | W | $R^f$ | phys. Daten (NMR-Daten in ppm) (Fp. in °C) |
|---|---|---|---|---|
| A.189 | $C_2H_5$ | $-CH_2CH=CH_2-$ | 3,5-$Cl_2$-phenyl | 126-127 |
| A.190 | n-$C_3H_7$ | $-CH_2CH=CH_2-$ | 3,5-$Cl_2$-phenyl | 4,7 (d,2H), 6,3 (dt,1H), 6,55 (d,1H), 7,1 (m,3H) |
| A.191 | $C_2H_5$ | $-(CH_2)_3-$ | 3,5-$Cl_2$-phenyl | 79- 80 |
| A.192 | n-$C_3H_7$ | $-(CH_2)_3-$ | 3,5-$Cl_2$-phenyl | 4,05 (t,2H), 7,0-7,25 (m,3H) |
| A.193 | $C_2H_5$ | $-CH_2CH_2C(=CH_2)-$ | Phenyl | 4,15 (t,2H), 5,15 (s,1H), 5,3 (s,1H), 7,2-7,5 (m,5H) |
| A.194 | n-$C_3H_7$ | $-CH_2CH_2C(=CH_2)-$ | Phenyl | 4,15 (t,2H), 5,15 (s,1H), 5,3 (s,1H), 7,2-7,5 (m,5H) |
| A.195 | $CH_3$ | $-CH_2CH=CH_2-$ | 4-Br-phenyl | 135-137 |
| A.196 | $C_2H_5$ | $-(CH_2)_5-$ | 4-Cl-phenyl | 66- 67 |
| A.197 | n-$C_3H_7$ | $-(CH_2)_5-$ | 4-Cl-phenyl | 60- 62 |
| A.198 | $C_2H_5$ | $-CH_2C(CH_3)-CH_2-$ | Phenyl | 0,95 (d,3H), 3,9 (m,2H), 7,0-7,4 (m,5H) |
| A.199 | n-$C_3H_7$ | $-CH_2C(CH_3)-CH_2-$ | Phenyl | 0,95 (d,3H), 3,9 (m,2H), 7,0-7,4 (m,5H) |
| A.200 | $C_2H_5$ | $-CH_2CH=CH_2-$ | 3,4-$Cl_2$-phenyl | 4,65 (d,2H), 6,3 (dt,1H), 6,55 (d,1H), 7,2-7,6 (m,3H) |
| A.201 | n-$C_3H_7$ | $-CH_2CH=CH_2-$ | 3,4-$Cl_2$-phenyl | 4,65 (d,2H), 6,3 (dt,1H), 6,55 (d,1H), 7,2-7,6 (m,3H) |
| A.202 | $C_2H_5$ | $-CH_2C(CH_3)-CH_2-$ | 4-F-phenyl | 0,95 (d,3H), 3,9 (dd,2H), 6,8-7,2 (m,4H) |
| A.203 | n-$C_3H_7$ | $-CH_2C(CH_3)-CH_2-$ | 4-F-phenyl | 0,95 (d,3H), 3,9 (dd,2H), 6,8-7,2 (m,4H) |
| A.204 | $C_2H_5$ | $-CH_2C(CH_3)-CH_2-$ | 4-Cl-phenyl | 0,95 (d,3H), 3,9 (m mit dd, 4H), 7,0-7,4 (2m,4H) |
| A.205 | n-$C_3H_7$ | $-CH_2C(CH_3)-CH_2-$ | 4-Cl-phenyl | 0,95 (d,3H), 3,9 (m mit dd, 4H), 7,0-7,4 (2m,4H) |
| A.206 | $C_2H_5$ | $-CH_2CH_2C(CH_3)_2-$ | 4-F-phenyl | 1,3 (s,6H), 3,85 (m mit t, 4H), 6,9 und 7,3 (2m,4H) |
| A.207 | n-$C_3H_7$ | $-CH_2CH_2C(CH_3)_2-$ | 4-F-phenyl | 1,3 (s,6H), 3,85 (m mit t, 4H), 6,9 und 7,3 (2m,4H) |
| A.208 | $C_2H_5$ | $-CH_2CH_2C(CH_3)_2-$ | 4-Cl-phenyl | 1,35 (s,6H), 3,9 (m mit t, 4H), 7,25 (s,4H) |

Tabelle II.3 (Fortsetzung)

| Bsp. | $R^a$ | W | $R^f$ | phys. Daten (NMR-Daten in ppm) (Fp. in °C) |
|---|---|---|---|---|
| A.209 | $n-C_3H_7$ | $-CH_2CH_2C(CH_3)_2-$ | 4-Cl-phenyl | 1,35(s,6H), 3,9(m mit t, 4H), 7,25(s,4H) |
| A.210 | $C_2H_5$ | $-(CH_2)_5-$ | 4-F-phenyl | 1,1(t,3H), 4,05(t,2H), 6,95 und 7,1(2m,4H) |
| A.211 | $n-C_3H_7$ | $-(CH_2)_5-$ | 4-F-phenyl | 0,95(t,3H), 4,05(t,2H), 6,95 und 7,1(2m,4H) |
| A.212 | $C_2H_5$ | $-CH_2CH=CH-$ | 3-Br-phenyl | 1,1(t,3H), 4,65(d,2H), 6,35(dt,1H), 6,6(d,1H), 7,2-7,6(m,4H) |
| A.213 | $n-C_3H_7$ | $-CH_2CH=CH-$ | 3-Br-phenyl | 1,0(t,3H), 4,65(d,2H), 6,35(dt,1H), 6,6(d,1H), 7,1-7,5(m,4H) |
| A.214 | $C_2H_5$ | $-CH_2CH=CH-$ | 3-Cl-phenyl | 1,1(t,3H), 4,7(d,2H), 6,35(dt,1H), 6,6(d,1H), 7,2-7,5(m,4H) |
| A.215 | $n-C_3H_7$ | $-CH_2CH=CH-$ | 3-Cl-phenyl | 1,0(t,3H), 4,7(d,2H), 6,3(dt,1H), 6,6(d,1H), 7,2-7,5(m,4H) |
| A.216 | $C_2H_5$ | $-CH_2CH=CH-$ | 3-F-phenyl | 66- 68 |
| A.217 | $n-C_3H_7$ | $-CH_2CH=CH-$ | 3-F-phenyl | 1,0(t,3H), 4,7(d,2H), 6,3(dt,1H), 6,6(d,1H), 6,8-7,4(m,4H) |

Tabelle II.4

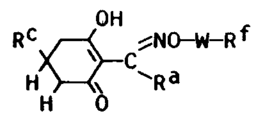

($R^b$, $R^d$, $R^e$ = H)

($R^c$ = Tetrahydropyran-4-yl)

| Bsp. | $R^a$ | W | $R^f$ | phys. Daten (NMR-Daten in ppm) (Fp. in °C) |
|---|---|---|---|---|
| A.218 | $C_2H_5$ | $-CH_2-CH=CH-$ | Phenyl | 129-130 |
| A.219 | $n-C_3H_7$ | $-CH_2-CH=CH-$ | Phenyl | 85- 87 |
| A.220 | $C_2H_5$ | $-CH_2-CH=CH-$ | 4-Cl-phenyl | 130-131 |
| A.221 | $n-C_3H_7$ | $-CH_2-CH=CH-$ | 4-Cl-phenyl | 108-110 |
| A.222 | $C_2H_5$ | $-CH_2-CH=CH-$ | 4-F-phenyl | 118-120 |
| A.223 | $n-C_3H_7$ | $-CH_2-CH=CH-$ | 4-F-phenyl | 87- 89 |
| A.224 | $C_2H_5$ | $-CH_2-CH=CH-$ | 2,4-$Cl_2$-phenyl | 95- 97 |
| A.225 | $n-C_3H_7$ | $-CH_2-CH=CH-$ | 2,4-$Cl_2$-phenyl | 93- 95 |
| A.226 | $C_2H_5$ | $-(CH_2)_3CH=CH-$ | Phenyl | 77- 78 |
| A.227 | $n-C_3H_7$ | $-(CH_2)_3CH=CH-$ | Phenyl | 67- 68 |
| A.228 | $C_2H_5$ | $-(CH_2)_3CH=CH-$ | 4-Cl-phenyl | 99-100 |
| A.229 | $n-C_3H_7$ | $-(CH_2)_3CH=CH-$ | 4-Cl-phenyl | 4,05 (t, 2H), 6,2 (dt, 1H), 6,4 (d, 1H), 7,3 (s, 4H) |
| A.230 | $C_2H_5$ | $-(CH_2)_3-$ | Phenyl | 4,1 (t, 2H), 7,0-7,4 (m, 5H) |
| A.231 | $n-C_3H_7$ | $-(CH_2)_3-$ | Phenyl | 4,1 (t, 2H), 7,0-7,4 (m, 5H) |
| A.232 | $C_2H_5$ | $-CH_2C(=CH_2)-CH_2-$ | Phenyl | 3,4 (s, 2H), 4,4 (s, 2H), 5,0 und 5,2 (2s, 2H), 7,1-7,4 (m, 5H) |
| A.233 | $n-C_3H_7$ | $-CH_2C(=CH_2)-CH_2-$ | Phenyl | 3,35 (s, 2H), 4,4 (s, 2H), 5,0 und 5,1 (2s, 2H), 7,1-7,4 (m, 5H) |

Tabelle II.4 (Fortsetzung)

| Bsp. | $R^a$ | W | $R^f$ | phys. Daten (NMR-Daten in ppm) (Fp. in °C) |
|---|---|---|---|---|
| A.234 | $C_2H_5$ | $-CH_2CH=CH$ | 4-Br-phenyl | 140-142 |
| A.235 | $n-C_3H_7$ | $-CH_2CH=CH$ | 4-Br-phenyl | 117-119 |
| A.236 | $C_2H_5$ | $-CH_2CH=CH$ | 4-$CH_3$-phenyl | 135-137 |
| A.237 | $n-C_3H_7$ | $-CH_2CH=CH$ | 4-$CH_3$-phenyl | 97- 98 |
| A.238 | $C_2H_5$ | $-CH_2CH=CH$ | 4-$CF_3$-phenyl | 103-104 |
| A.239 | $n-C_3H_7$ | $-CH_2CH=CH$ | 4-$CF_3$-phenyl | 114-116 |
| A.240 | $C_2H_5$ | $-CH_2CH=CH$ | 4-$C_6H_5O$-phenyl | 64- 66 |
| A.241 | $n-C_3H_7$ | $-CH_2CH=CH$ | 4-$C_6H_5O$-phenyl | 4,65 (d,2H), 6,2 (dt,1H), 6,65 (d,1H), 6,9-7,5 (3m,9H) |
| A.242 | $C_2H_5$ | $-CH_2CH=C(CH_3)-$ | Phenyl | 70- 72 |
| A.243 | $n-C_3H_7$ | $-CH_2CH=C(CH_3)-$ | Phenyl | 2,15 (s,3H), 4,75 (d,2H), 5,95 (t,1H), 7,2-7,6 (m,5H) |
| A.244 | $C_2H_5$ | $-CH_2CH=CH-$ | 2-Cl-phenyl | 85- 87 |
| A.245 | $n-C_3H_7$ | $-CH_2CH=CH-$ | 2-Cl-phenyl | 90- 92 |
| A.246 | $C_2H_5$ | $-(CH_2)_3-$ | 4-F-phenyl | 65- 67 |
| A.247 | $n-C_3H_7$ | $-(CH_2)_3-$ | 4-F-phenyl | 64- 66 |
| A.248 | $C_2H_5$ | $-(CH_2)_3-$ | 2,4-$Cl_2$-phenyl | 4,05 (t,2H), 7,05-7,4 (2m,3H) |
| A.249 | $n-C_3H_7$ | $-(CH_2)_3-$ | 2,4-$Cl_2$-phenyl | 65- 67 |
| A.250 | $C_2H_5$ | $-(CH_2)_3-$ | 4-Br-phenyl | 111-112 |
| A.251 | $C_2H_5$ | $-(CH_2)_3-$ | 2-Cl-phenyl | 4,1 (t,2H), 7,0-7,4 (m,4H) |
| A.252 | $n-C_3H_7$ | $-(CH_2)_3-$ | 2-Cl-phenyl | 4,1 (t,2H), 7,05-7,45 (m,4H) |
| A.253 | $C_2H_5$ | $-(CH_2)_3-$ | 4-Cl-phenyl | 97- 99 |
| A.254 | $n-C_3H_7$ | $-(CH_2)_3-$ | 4-Cl-phenyl | 84- 86 |

Tabelle II.4 (Fortsetzung)

| Bsp. | $R^a$ | W | $R^f$ | phys. Daten (NMR-Daten in ppm) (Fp. in °C) |
|---|---|---|---|---|
| A.255 | $C_2H_5$ | $-CH_2CH=CH-$ | 3,5-$Cl_2$-phenyl | 127-128 |
| A.256 | n-$C_3H_7$ | $-CH_2CH=CH-$ | 3,5-$Cl_2$-phenyl | 80- 81 |
| A.257 | $C_2H_5$ | $-CH_2CH_2CH(CH_3)-$ | Phenyl | 1,25 (d,3H), 4,0 (m,2H), 7,05-7,4 (m,5H) |
| A.258 | n-$C_3H_7$ | $-CH_2CH_2CH(CH_3)-$ | Phenyl | 1,25 (d,3H), 4,0 (m,2H), 7,0-7,4 (m,5H) |
| A.259 | $C_2H_5$ | $-(CH_2)_3-$ | 3,5-$Cl_2$-phenyl | 105-107 |
| A.260 | n-$C_3H_7$ | $-(CH_2)_3-$ | 3,5-$Cl_2$-phenyl | 73- 75 |
| A.261 | $C_2H_5$ | $-CH_2CH_2C(=CH_2)-$ | Phenyl | 4,15 (t,2H), 5,15 (s,1H), 5,3 (s,1H), 7,2-7,6 (m,5H) |
| A.262 | n-$C_3H_7$ | $-CH_2CH_2C(=CH_2)-$ | Phenyl | 4,15 (t,2H), 5,15 (s,1H), 5,3 (s,1H), 7,2-7,6 (m,5H) |
| A.263 | $C_2H_5$ | $-(CH_2)_5-$ | 4-Cl-phenyl | 66- 67 |
| A.264 | n-$C_3H_7$ | $-(CH_2)_5-$ | 4-Cl-phenyl | 61- 63 |
| A.265 | $C_2H_5$ | $-CH_2C(CH_3)-CH_2-$ | Phenyl | 0,9 (d,3H), 3,9 (m,2H), 7,0-7,4 (m,5H) |
| A.266 | n-$C_3H_7$ | $-CH_2C(CH_3)-CH_2-$ | Phenyl | 0,9 (d,3H), 3,9 (m,2H), 7,0-7,4 (m,5H) |
| A.267 | $C_2H_5$ | $-CH_2CH=CH-$ | 3,4-$Cl_2$-phenyl | 103-105 |
| A.268 | n-$C_3H_7$ | $-CH_2CH=CH-$ | 3,4-$Cl_2$-phenyl | 4,65 (d,2H), 6,3 (dt,1H), 6,55 (d,1H), 7,2-7,6 (m,3H) |
| A.269 | $C_2H_5$ | $-(CH_2)_3-$ | 3,4-$Cl_2$-phenyl | 3,95-4,1 (m,4H), 7,0-7,1 und 7,2-7,45 (2m,3H) |
| A.270 | $C_2H_5$ | $-CH_2C(CH_3)-CH_2-$ | 4-F-phenyl | 0,90 (d,3H), 3,85 (dd,2H), 6,8-7,2 (m,4H) |
| A.271 | n-$C_3H_7$ | $-CH_2C(CH_3)-CH_2-$ | 4-F-phenyl | 0,90 (d,3H), 3,85 (dd,2H), 6,8-7,2 (m,4H) |
| A.272 | $C_2H_5$ | $-CH_2C(CH_3)-CH_2-$ | 4-Cl-phenyl | 0,90 (d,3H), 3,85 (dd,2H), 7,0-7,4 (2m,4H) |
| A.273 | n-$C_3H_7$ | $-CH_2C(CH_3)-CH_2-$ | 4-Cl-phenyl | 0,90 (d,3H), 3,85 (dd,2H), 7,0-7,4 (2m,4H) |

Tabelle II.4 (Fortsetzung)

| Bsp. | $R^a$ | W | $R^f$ | phys. Daten (NMR-Daten in ppm) (Fp. in °C) |
|---|---|---|---|---|
| A.274 | $C_2H_5$ | $-CH_2CH_2C(CH_3)_2-$ | 4-F-phenyl | 1,35 (s,6H), 3,85 (t,2H), 7,0 und 7,3 (2m,4H) |
| A.275 | $n-C_3H_7$ | $-CH_2CH_2C(CH_3)_2-$ | 4-F-phenyl | 1,35 (s,6H), 3,85 (t,2H), 7,0 und 7,3 (2m,4H) |
| A.276 | $C_2H_5$ | $-CH_2CH_2C(CH_3)_2-$ | 4-Cl-phenyl | 1,35 (s,6H), 3,85 (t,2H), 7,25 (s,4H) |
| A.277 | $n-C_3H_7$ | $-CH_2CH_2C(CH_3)_2-$ | 4-Cl-phenyl | 1,35 (s,6H), 3,85 (t,2H), 7,25 (s,4H) |
| A.278 | $C_2H_5$ | $-(CH_2)_6-$ | 4-Cl-phenyl | 1,15 (t,3H), 3,35 (t,2H), 7,1 (d,2H), 7,25 (d,2H) |
| A.279 | $n-C_3H_7$ | $-(CH_2)_6-$ | 4-Cl-phenyl | 0,95 (t,3H), 3,35 (t,2H), 7,1 (d,2H), 7,25 (d,2H) |
| A.280 | $C_2H_5$ | $-(CH_2)_6-$ | 4-F-phenyl | 1,1 (t,3H), 3,35 (t,2H) |
| A.281 | $n-C_3H_7$ | $-(CH_2)_6-$ | 4-F-phenyl | 0,95 (t,3H), 3,35 (t,2H) |
| A.282 | $C_2H_5$ | $-(CH_2)_5-$ | 4-F-phenyl | 1,15 (t,3H), 3,35 (t,2H), 6,95 und 7,1 (2m,4H) |
| A.283 | $n-C_3H_7$ | $-(CH_2)_5-$ | 4-F-phenyl | 0,95 (t,3H), 3,35 (t,2H), 6,95 und 7,1 (2m,4H) |
| A.284 | $C_2H_5$ | $-CH_2CH(CH_3)-CH_2CH_2CH_2-$ | $2-CH_3$-phenyl | 2,3 (s,3H), 7,05 (m,4H) |
| A.285 | $n-C_3H_7$ | $-CH_2CH(CH_3)-CH_2CH_2CH_2-$ | $2-CH_3$-phenyl | 2,3 (s,3H), 7,1 (m,4H) |
| A.286 | $n-C_3H_7$ | $-CH_2CH=CH-$ | 3-F-phenyl | 61- 62 |
| A.287 | $C_2H_5$ | $-CH_2CH=CH-$ | 3-Br-phenyl | 103-105 |
| A.288 | $n-C_3H_7$ | $-CH_2CH=CH-$ | 3-Br-phenyl | 80- 82 |
| A.289 | $C_2H_5$ | $-CH_2CH=CH-$ | 3-Cl-phenyl | 109-111 |
| A.290 | $n-C_3H_7$ | $-CH_2CH=CH-$ | 3-Cl-phenyl | 89- 91 |
| A.291 | $C_2H_5$ | $-CH_2CH=CH-$ | 3-F-phenyl | 122-123 |

Tabelle II.5

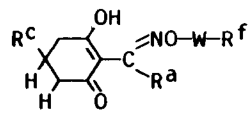

($R^b$, $R^d$, $R^e$ = H)

| Bsp. | $R^c$ | $R^a$ | W | $R^f$ | phys. Daten (NMR-Daten in ppm) (Fp. in °C) |
|---|---|---|---|---|---|
| A.292 | 2-Ethylthiopropyl | $n-C_3H_7$ | $-(CH_2)_3-$ | Phenyl | 4,05 (t,2H), 7,15-7,4 (m,5H) |
| A.293 | 2,4,6-Trimethyl-phenyl | $C_2H_5$ | $-CH_2CH=CH-$ | $2,4-Cl_2$-phenyl | 106-107 |
| A.294 | 2,4,6-Trimethyl-phenyl | $C_2H_5$ | $-CH_2CH=CH-$ | 4-F-phenyl | 2,2 (s,3H), 2,35 (s,6H), 4,7 (d,2H), 6,3 (dt,1H), 6,65 (d,1H), 7,0 (m,2H), 7,4 (m,2H) |
| A.295 | Phenyl | $n-C_3H_7$ | $-CH_2CH=CH-$ | 4-F-phenyl | 55- 57 |
| A.296 | 4-(Benzoylamino)-phenyl | $n-C_3H_7$ | $-CH_2CH=CH-$ | 4-F-phenyl | 80- 82 |
| A.297 | 5,6-Dihydrothio-pyran-3-yl | $C_2H_5$ | $-CH_2CH=CH-$ | 4-F-phenyl | 94- 96 |
| A.298 | Cyclohexyl | $n-C_3H_7$ | $-CH_2CH=CH-$ | 4-F-phenyl | 67- 69 |
| A.299 | 3-Isopropyl-isoxazol-5-yl | $n-C_3H_7$ | $-CH_2CH=CH-$ | 4-F-phenyl | 103-104 |
| A.300 | 5,6-Dihydrothio-pyran-3-yl | $C_2H_5$ | $-CH_2CH=CH-$ | $2,4-Cl_2$-phenyl | 88- 89 |
| A.301 | Cyclohex-3-enyl | $n-C_3H_7$ | $-CH_2CH=CH-$ | $2,4-Cl_2$-phenyl | 75- 77 |
| A.302 | 3-Isopropyl-isoxazol-5-yl | $n-C_3H_7$ | $-CH_2CH=CH-$ | $2,4-Cl_2$-phenyl | 113-115 |
| A.303 | 3-Isopropyl-isothiazol-5-yl | $C_2H_5$ | $-CH_2CH=CH-$ | $2,4-Cl_2$-phenyl | 82- 83 |

Tabelle II.5 (Fortsetzung)

| Bsp. | $R^c$ | $R^a$ | W | $R^f$ | phys. Daten (NMR-Daten in ppm) (Fp. in °C) |
|---|---|---|---|---|---|
| A.304 | 4-Ethylphenyl | $C_2H_5$ | $-CH_2CH{=}CH-$ | 2,4-$Cl_2$-phenyl | 81- 82 |
| A.305 | 3-Isopropyl-isothiazol-5-yl | $C_2H_5$ | $-CH_2CH{=}CH-$ | 4-F-phenyl | 98-101 |
| A.306 | N-Isopropyl-pyrrol-3-yl | n-$C_3H_7$ | $-CH_2CH{=}CH-$ | 4-F-phenyl | 54- 56 |
| A.307 | 3-Nitro-4-fluor-phenyl | n-$C_3H_7$ | $-CH_2CH{=}CH-$ | 4-Br-phenyl | 124-126 |
| A.308 | Cyclohex-3-enyl | n-$C_3H_7$ | $-CH_2CH{=}CH-$ | 4-Br-phenyl | 68- 71 |
| A.309 | Thien-3-yl | n-$C_3H_7$ | $-CH_2CH{=}CH-$ | 4-Br-phenyl | 85- 87 |
| A.310 | 4-(Prop-2-inoxy)-phenyl | $C_2H_5$ | $-CH_2CH{=}CH-$ | 4-Br-phenyl | 126-129 |
| A.311 | 2-Ethylthiopropyl | $C_2H_5$ | $-CH_2CH{=}CH-$ | 2,4-$Cl_2$-phenyl | 4,7(d,2H), 6,3(dt,1H), 7,0(d,1H), 7,2-7,6(m,3H) |
| A.312 | 3-Isopropyl-isoxazol-5-yl | $CH_3$ | $-CH_2CH{=}CH-$ | 4-Cl-phenyl | 113-115 |
| A.313 | Ethoxycarbonyl | n-$C_3H_7$ | $-CH_2CH{=}CH-$ | 4-Cl-phenyl | 44- 45 |
| A.314 | 4-Ethylphenyl | $C_2H_5$ | $-CH_2CH{=}CH-$ | 4-Cl-phenyl | 104-106 |
| A.315 | 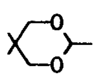 | $C_2H_5$ | $-CH_2CH{=}CH-$ | 4-Cl-phenyl | 68- 70 |
| A.316 | Cyclohex-1-enyl | n-$C_3H_7$ | $-CH_2CH{=}CH-$ | 4-Cl-phenyl | 63- 64 |
| A.317 | 4-(Benzoylamino)-phenyl | n-$C_3H_7$ | $-CH_2CH{=}CH-$ | 4-Cl-phenyl | 132-134 |

Tabelle II.5 (Fortsetzung)

| Bsp. | $R^c$ | $R^a$ | W | $R^f$ | phys. Daten (NMR-Daten in ppm) (Fp. in °C) |
|---|---|---|---|---|---|
| A.318 | 4-(Prop-2-inoxy)-phenyl | $C_2H_5$ | $-CH_2CH=CH-$ | 4-Cl-phenyl | 122-124 |
| A.319 | 2-Ethylthiophenyl | n-$C_3H_7$ | $-(CH_2)_6-$ | 4-Cl-phenyl | 0,95 (t,3H), 4,0 (t,2H), 7,1 (d,2H), 7,25 (d,2H) |
| A.320 | 2,4,6-Trimethyl-phenyl | $C_2H_5$ | $-(CH_2)_6-$ | 4-Cl-phenyl | 1,15 (t,3H), 2,25 (s,3H), 6,85 (s,2H) |
| A.321 | 2,4,6-Trimethyl-phenyl | $C_2H_5$ | $-(CH_2)_6-$ | 4-F-phenyl | 1,2 (t,3H), 2,25 (s,3H), 4,05 (t,2H) |
| A.322 | 2-Ethylthiopropyl | n-$C_3H_7$ | $-(CH_2)_6-$ | 4-F-phenyl | 0,95 (t,3H), 4,0 (t, 2H) |

Tabelle II.6

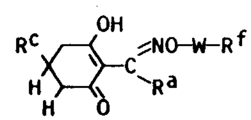

($R^b$, $R^d$, $R^e$ = H)

| Verb. Nr. | $R^c$ | $R^a$ | W | $R^f$ | $^1H$-NMR*) [δ ppm] | Fp. [°C] |
|---|---|---|---|---|---|---|
| A.323 | Tetrahydrothiopyran-3-yl | n-$C_3H_7$ | $-CH_2-C\equiv C-$ | Phenyl | 4,9(s,2H); 7,2-7,6(2m,5H) | |
| A.324 | Tetrahydrothiopyran-3-yl | n-$C_3H_7$ | $-CH_2-C\equiv C-CH_2-$ | Phenyl | 3,6(s,2H); 4,7(s,2H), 7,2-7,5(m,5H) | |
| A.325 | Tetrahydrothiopyran-3-yl | $C_2H_5$ | $-CH_2-C\equiv C-CH_2-$ | Phenyl | 3,65(s,2H); 4,7(s,2H); 7,2-7,5(m,5H) | |
| A.326 | Tetrahydropyran-3-yl | n-$C_3H_7$ | $-CH_2-C\equiv C-CH_2-$ | Phenyl | 3,65(s,2H); 4,7(s,2H); 7,2-7,5(m,5H) | |
| A.327 | 2-Ethylthiopropyl | n-$C_3H_7$ | $-CH_2-C\equiv C-$ | Phenyl | 4,9(s,2H); 7,3-7,6(m,5H) | |
| A.328 | 2-Ethylthiopropyl | n-$C_3H_7$ | $-CH_2-C\equiv C-CH_2-$ | Phenyl | 3,65(s,2H); 4,7(s,2H); 7,2-7,5(m,5H) | |
| A.329 | Tetrahydropyran-4-yl | $C_2H_5$ | $-CH_2-C\equiv C-CH_2-$ | Phenyl | 3,65(s,2H); 4,7(s,2H); 7,2-7,5(m,5H) | |
| A.330 | Tetrahydropyran-4-yl | n-$C_3H_7$ | $-CH_2-C\equiv C-CH_2-$ | Phenyl | 3,6(s,2H); 4,65(s,2H); 7,1-7,6(m,5H) | |
| A.331 | Tetrahydropyran-4-yl | n-$C_3H_7$ | $-(CH_2)_3-C\equiv C-$ | 4-F-phenyl | 4,25(t,2H); 6,8-7,5(2m,4H) | |

*) ausgewählte Signale

Tabelle II.6 (Fortsetzung)

| Verb. Nr. | $R^c$ | $R^a$ | W | $R^f$ | $^1H$-NMR*) [δ ppm] | Fp. [°C] |
|---|---|---|---|---|---|---|
| A.332 | Tetrahydropyran-4-yl | $C_2H_5$ | $-(CH_2)_3-C{\equiv}C-$ | 4-F-phenyl | 4,25(t,2H); 6,8-7,5(2m,4H) | |
| A.333 | Tetrahydrothiopyran-3-yl | $C_2H_5$ | $-(CH_2)_3-C{\equiv}C-$ | 4-F-phenyl | 4,25(t,2H); 6,8-7,5(2m,4H) | |
| A.334 | Tetrahydrothiopyran-3-yl | $n-C_3H_7$ | $-(CH_2)_3-C{\equiv}C-$ | 4-F-phenyl | 4,25(t,2H); 6,8-7,5(2m,4H) | |
| A.335 | Tetrahydropyran-3-yl | $C_2H_5$ | $-(CH_2)_3-C{\equiv}C-$ | 4-F-phenyl | 4,25(t,2H); 6,8-7,5(2m,4H) | |
| A.336 | Tetrahydropyran-3-yl | $n-C_3H_7$ | $-(CH_2)_3-C{\equiv}C-$ | 4-F-phenyl | 4,25(t,2H); 6,8-7,5(2m,4H) | |
| A.337 | Tetrahydrothiopyran-3-yl | $C_2H_5$ | $-CH_2CH_2-C{\equiv}C-$ | 4-F-phenyl | 4.25(t); 6.98(dd); 7.35(dd) | 74- 90 |
| A.338 | Tetrahydrothiopyran-3-yl | $n-C_3H_7$ | $-CH_2CH_2-C{\equiv}C-$ | 4-F-phenyl | 4.25(t); 6.98(dd); 7.35(dd) | - |
| A.339 | Tetrahydropyran-3-yl | $C_2H_5$ | $-CH_2CH_2-C{\equiv}C-$ | 4-F-phenyl | 4.25(t); 6.98(dd); 7.35(dd) | 55- 61 |
| A.340 | Tetrahydropyran-3-yl | $n-C_3H_7$ | $-CH_2CH_2-C{\equiv}C-$ | 4-F-phenyl | 4.25(t); 6.98(dd); 7.35(dd) | - |
| A.341 | Tetrahydropyran-4-yl | $C_2H_5$ | $-CH_2CH_2-C{\equiv}C-$ | 4-F-phenyl | 4.25(t); 6.98(dd); 7.35(dd) | 83- 87 |
| A.342 | Tetrahydropyran-4-yl | $n-C_3H_7$ | $-CH_2CH_2-C{\equiv}C-$ | 4-F-phenyl | 4.25(t); 6.98(dd); 7.35(dd) | 98-102 |

*) ausgewählte Signale

Tabelle II.6 (Fortsetzung)

| Verb. Nr. | $R^c$ | $R^a$ | W | $R^f$ | $^1H$-NMR*) [δ ppm] | Fp. [°C] |
|---|---|---|---|---|---|---|
| A.343 | 3-Isopropylisoxazol-5-yl | $n-C_3H_7$ | $-CH_2CH_2-C{\equiv}C-$ | 4-F-phenyl | 4.25(t); 5.94(s); 7.0(dd); 7.37(dd) | - |
| A.344 | 4-Methylphenyl | $n-C_3H_7$ | $-CH_2CH_2-C{\equiv}C-$ | 4-F-phenyl | 4.25(t); 6.98(dd); 7.15(m); 7.35(dd) | 65-69 |
| A.345 | 3,4-Dibromtetrahydropyran-3-yl | $n-C_3H_7$ | $-CH_2CH_2-C{\equiv}C-$ | 4-F-phenyl | 4.25(t); 6.98(dd); 7.35(dd) | - |
| A.346 | Tetrahydrothiopyran-3-yl | $n-C_3H_7$ | $-CH_2CH_2-C{\equiv}C-$ | 4-Cl-phenyl | 4.25(t); 7.25(d); 7.35(d) | - |
| A.347 | Tetrahydrothiopyran-3-yl | $C_2H_5$ | $-CH_2CH_2-C{\equiv}C-$ | 4-Cl-phenyl | 4.25(t); 7.25(d); 7.35(d) | 82- 86 |
| A.348 | Tetrahydropyran-3-yl | $C_2H_5$ | $-CH_2CH_2-C{\equiv}C-$ | 4-Cl-phenyl | 4.25(t); 7.25(d); 7.35(d) | 99-101 |
| A.349 | Tetrahydropyran-3-yl | $n-C_3H_7$ | $-CH_2CH_2-C{\equiv}C-$ | 4-Cl-phenyl | 4.25(t); 7.25(d); 7.35(d) | |
| A.350 | Tetrahydropyran-4-yl | $C_2H_5$ | $-CH_2CH_2-C{\equiv}C-$ | 4-Cl-phenyl | 4.25(t); 7.25(d); 7.35(d) | 98-101 |
| A.351 | Tetrahydropyran-4-yl | $n-C_3H_7$ | $-CH_2CH_2-C{\equiv}C-$ | 4-Cl-phenyl | 4.25(t); 7.25(d); 7.35(d) | 115-118 |

*) ausgewählte Signale

Tabelle II.6 (Fortsetzung)

| Verb. Nr. | $R^c$ | $R^a$ | W | $R^f$ | $^1H$-NMR*) [δ ppm] | Fp. [°C] |
|---|---|---|---|---|---|---|
| A.352 | 3-Isopropylisoxazol-5-yl | $n-C_3H_7$ | $-CH_2CH_2-C\equiv C-$ | 4-Cl-phenyl | 4.25(t); 5.9(s); 7.25(d); 7.35(d) | 71- 74 |
| A.353 | 4-Methylphenyl | $n-C_3H_7$ | $-CH_2CH_2-C\equiv C-$ | 4-Cl-phenyl | 4.25(t); 7.45(m); 7.28(M) | 93- 6 |
| A.354 | 3,4-Dibromtetrahydro-pyran-3-yl | $n-C_3H_7$ | $-CH_2CH_2-C\equiv C-$ | 4-Cl-phenyl | 4.25(t); 7.25(d); 7.25(d) | - |
| A.355 | Tetrahydrothiopyran-3-yl | $C_2H_5$ | $-(CH_2)_3-C\equiv C-$ | 4-Cl-phenyl | 1.15(t); 4.2(t); 7.25(d); 7.35(d) | |
| A.356 | Tetrahydrothiopyran-3-yl | $n-C_3H_7$ | $-(CH_2)_3-C\equiv C-$ | 4-Cl-phenyl | 0.98(t); 4.2(t); 7.25(d); 7.35(d) | |
| A.357 | Tetrahydropyran-3-yl | $C_2H_5$ | $-(CH_2)_3-C\equiv C-$ | 4-Cl-phenyl | 1.15(t); 4.2(t); 7.25(d); 7.35(d) | |
| A.358 | Tetrahydropyran-3-yl | $n-C_3H_7$ | $-(CH_2)_3-C\equiv C-$ | 4-Cl-phenyl | 0.95(t); 4.2(t); 7.25(d); 7.35(d) | |

*) ausgewählte Signale

Tabelle II.6 (Fortsetzung)

| Verb. Nr. | $R^c$ | $R^a$ | W | $R^f$ | $^1H$-NMR*) [δ ppm] | Fp. [°C] |
|---|---|---|---|---|---|---|
| A.359 | Tetrahydropyran-4-yl | $C_2H_5$ | $-(CH_2)_3-C{\equiv}C-$ | 4-Cl-phenyl | 1.15(t); 4.2(t); 7.25(d); 7.35(d) | |
| A.360 | Tetrahydropyran-4-yl | $n-C_3H_7$ | $-(CH_2)_3-C{\equiv}C-$ | 4-Cl-phenyl | 0.98(t); 4.2(t); 7.25(d); 7.35(d) | |
| A.361 | Tetrahydrothiopyran-3-yl | $C_2H_5$ | $-CH_2-CH_2-C{\equiv}C-$ | 2-Thienyl | 1.15(t); 2.75(t); 4.25(t); 7.05(m); 7.2(m) | |
| A.362 | Tetrahydrothiopyran-3-yl | $n-C_3H_7$ | $-CH_2-CH_2-C{\equiv}C-$ | 2-Thienyl | 0.95(t); 2.75(t); 4.25(t); 7.05(m); 7.2(m) | |
| A.363 | Tetrahydropyran-3-yl | $C_2H_5$ | $-CH_2-CH_2-C{\equiv}C-$ | 2-Thienyl | 1.15(t); 2.75(t); 4.25(t); 7.05(m); 7.2(m) | |
| A.364 | Tetrahydropyran-3-yl | $n-C_3H_7$ | $-CH_2-CH_2-C{\equiv}C-$ | 2-Thienyl | 0.95(t); 2.75(t); 4.25(t); 7.05(m); 7.2(m) | |
| A.365 | Tetrahydropyran-4-yl | $C_2H_5$ | $-CH_2-CH_2-C{\equiv}C-$ | 2-Thienyl | 1.15(t); 2.75(t); 4.25(t); 7.05(m); 7.2(m) | |
| A.366 | Tetrahydropyran-4-yl | $n-C_3H_7$ | $-CH_2-CH_2-C{\equiv}C-$ | 2-Thienyl | 0.95(t); 2.75(t); 4.25(t); 7.05(m); 7.2(m) | |

*) ausgewählte Signale

Tabelle II.6 (Fortsetzung)

| Verb. Nr. | $R^c$ | $R^a$ | W | $R^f$ | $^1H$-NMR*) [δ ppm] | Fp. [°C] |
|---|---|---|---|---|---|---|
| 3.45 | Tetrahydrothiopyran-3-yl | $C_2H_5$ | $-CH_2CH=C(CH_3)-C{\equiv}C-$ **) | 4-Cl-phenyl | 1.15(t); 2.0(s); 4.8(d); 5.9(t) | |
| 3.46 | Tetrahydrothiopyran-3-yl | $n-C_3H_7$ | $-CH_2CH=C(CH_3)-C{\equiv}C-$ **) | 4-Cl-phenyl | 0.95(t); 2.0(s); 4.8(d); 5.9(t) | |
| 3.47 | Tetrahydropyran-4-yl | $C_2H_5$ | $-CH_2CH=C(CH_3)-C{\equiv}C-$ **) | 4-Cl-phenyl | 1.15(t); 2.0(s); 4.8(d); 5.9(t) | |
| 3.48 | Tetrahydropyran-4-yl | $n-C_3H_7$ | $-CH_2CH=C(CH_3)-C{\equiv}C-$ **) | 4-Cl-phenyl | 0.95(t); 2.0(s); 4.8(d); 5.9(t) | |
| 3.49 | 2-Ethylthiopropyl | $n-C_3H_7$ | $-CH_2CH=C(CH_3)-C{\equiv}C-$ **) | 4-Cl-phenyl | 0.95(t); 2.0(s); 4.8(d); 5.9(t) | |
| 3.50 | 2,4,6-Trimethylphenyl | $C_2H_5$ | $-CH_2CH=C(CH_3)-C{\equiv}C-$ **) | 4-Cl-phenyl | 1.2(t); 2.0(s); 4.8(d); 5.95(t) | |
| 3.51 | Tetrahydrothiopropan-3-yl | $C_2H_5$ | $-CH_2CH=C(CH_3)-C{\equiv}C-$ **) | 4-F-phenyl | 1.1(t); 2.0(s); 4.8(d); 5.9(t) | |
| 3.52 | Tetrahydrothiopropan-3-yl | $n-C_3H_7$ | $-CH_2CH=C(CH_3)-C{\equiv}C-$ **) | 4-F-phenyl | 0.95(t); 2.0(s); 4.8(d); 5.9(t) | |

*) ausgewählte Signale

**) Z-Konfiguration an der Doppelbindung

Tabelle II.6 (Fortsetzung)

| Verb. Nr. | $R^c$ | $R^a$ | W | $R^f$ | $^1$H-NMR*) [δ ppm] |
|---|---|---|---|---|---|
| A.375 | Tetrahydropyran-4-yl | $n-C_3H_7$ | $-CH_2-CH=C(CH_3)-C\equiv C-$ **) | 4-F-phenyl | 0.95(t); 2.0(s); 4.8(d); 5.9(t) |
| A.376 | Tetrahydropyran-4-yl | $C_2H_5$ | $-CH_2-CH=C(CH_3)-C\equiv C-$ **) | 4-F-phenyl | 1.15(t); 2.0(s); 4.8(d); 5.9(t) |
| A.377 | 2-Ethylthiopropyl | $n-C_3H_7$ | $-CH_2-CH=C(CH_3)-C\equiv C-$ **) | 4-F-phenyl | 0.95(t); 2.0(s); 4.8(d); 5.9(t) |

*) ausgewählte Signale

**) Z-Konfiguration an der Doppelbindung

Tabelle II.7

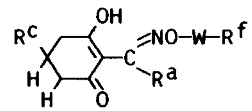

($R^b$, $R^d$, $R^e$ = H)

| Nr. | $R^a$ | $R^c$ | W | $R^f$ | phys. Daten NMR-Daten in ppm Fp in °C |
|---|---|---|---|---|---|
| A.378 | $C_2H_5$ | Tetrahydropyran-3-yl | $-(CH_2)_2-$ | Furan-2-yl | |
| A.379 | $n-C_3H_7$ | Tetrahydropyran-3-yl | $-(CH_2)_2-$ | Furan-2-yl | |
| A.380 | $C_2H_5$ | Tetrahydropyran-4-yl | $-(CH_2)_2-$ | Furan-2-yl | |
| A.381 | $n-C_3H_7$ | Tetrahydropyran-4-yl | $-(CH_2)_2-$ | Furan-2-yl | |
| A.382 | $C_2H_5$ | Tetrahydrothiopyran-3-yl | $-(CH_2)_2-$ | Furan-2-yl | |
| A.383 | $n-C_3H_7$ | Tetrahydrothiopyran-3-yl | $-(CH_2)_2-$ | Furan-2-yl | |
| A.384 | $C_2H_5$ | Tetrahydropyran-3-yl | $-(CH_2)_2-$ | Thien-2-yl | 3.92 (m, 2H), 4.33 (t, 2H), 6.82 (m, 1H), 6.93 (m, 1H), 7.13 (m, 1H) |
| A.385 | $n-C_3H_7$ | Tetrahydropyran-3-yl | $-(CH_2)_2-$ | Thien-2-yl | 3.92 (m, 2H), 4.33 (t, 2H), 6.82 (m, 1H), 6.93 (m, 1H), 7.13 (m, 1H) |
| A.386 | $C_2H_5$ | Tetrahydropyran-4-yl | $-(CH_2)_2-$ | Thien-2-yl | 4.00 (m, 2H), 4.33 (t, 2H), 6.82 (m, 1H), 6.93 (m, 1H), 7.13 (m, 1H) |
| A.387 | $n-C_3H_7$ | Tetrahydropyran-4-yl | $-(CH_2)_2-$ | Thien-2-yl | 4.00 (m, 2H), 4.33 (t, 2H), 6.82 (m, 1H), 6.93 (m, 1H), 7.13 (m, 1H) |
| A.388 | $C_2H_5$ | Tetrahydrothiopyran-3-yl | $-(CH_2)_2-$ | Thien-2-yl | 4.30 (t, 2H), 6.82 (m, 1H), 6.93 (m, 1H), 7.13 (m, 1H) |
| A.389 | $n-C_3H7$ | Tetrahydrothiopyran-3-yl | $-(CH_2)_2-$ | Thien-2-yl | 4.30 (t, 2H), 6.82 (m, 1H), 6.93 (m, 1H), 7.13 (m, 1H) |
| A.390 | $C_2H_5$ | Tetrahydropyran-3-yl | $-(CH_2)_2-$ | Pyrid-2-yl | 3.90 (m, 2H), 4.46 (t, 2H), 7.20 (m, 2H), 7.67 (m, 1H), 8.50 (m, 1H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.391 | $n-C_3H_7$ | Tetrahydropyran-3-yl | $-(CH_2)_2-$ | Pyrid-2-yl | |
| A.392 | $C_2H_5$ | Tetrahydropyran-4-yl | $-(CH_2)_2-$ | Pyrid-2-yl | 4.00 (m, 2H), 4.46 (t, 2H), 7.20 (m, 2H), 7.67 (m, 1H), 8.50 (m, 1H) |
| A.393 | $n-C_3H_7$ | Tetrahydropyran-4-yl | $-(CH_2)_2-$ | Pyrid-2-yl | |
| A.394 | $C_2H_5$ | Tetrahydrothiopyran-3-yl | $-(CH_2)_2-$ | Pyrid-2-yl | 4.46 (t, 2H), 7.20 (m, 2H), 7.67 (m, 1H), 8.50 (m, 1H) |
| A.395 | $n-C_3H_7$ | Tetrahydrothiopyran-3-yl | $-(CH_2)_2-$ | Pyrid-2-yl | |
| A.396 | $C_2H_5$ | Tetrahydropyran-3-yl | $-(CH_2)_3-$ | Furan-2-yl | 3.93 (m, 2H), 4.10 (t, 2H), 6.00 (m, 1H), 6.26 (m, 1H), 7.33 (m, 1H) |
| A.397 | $n-C_3H_7$ | Tetrahydropyran-3-yl | $-(CH_2)_3-$ | Furan-2-yl | 3.93 (m, 2H), 4.10 (t, 2H), 6.00 (m, 1H), 6.26 (m, 1H), 7.33 (m, 1H) |
| A.398 | $C_2H_5$ | Tetrahydropyran-4-yl | $-(CH_2)_3-$ | Furan-2-yl | 78 - 82 |
| A.399 | $n-C_3H_7$ | Tetrahydropyran-4-yl | $-(CH_2)_3-$ | Furan-2-yl | 48 - 52 |
| A.400 | $C_2H_5$ | Tetrahydrothiopyran-3-yl | $-(CH_2)_3-$ | Furan-2-yl | 54 - 58 |
| A.401 | $n-C_3H_7$ | Tetrahydrothiopyran-3-yl | $-(CH_2)_3-$ | Furan-2-yl | 4.10 (t, 2H), 6.00 (m, 1H), 6.26 (m, 1H), 7.33 (m, 1H) |
| A.402 | $C_2H_5$ | Tetrahydropyran-3-yl | $-(CH_2)_3-$ | Thien-2-yl | 72 - 74 |
| A.403 | $n-C_3H_7$ | Tetrahydropyran-3-yl | $-(CH_2)_3-$ | Thien-2-yl | 3.93 (m, 2H), 4.10 (t, 2H), 6.82 (m, 1H), 6.93 (m, 1H), 7.33 (m, 1H) |
| A.404 | $C_2H_5$ | Tetrahydropyran-4-yl | $-(CH_2)_3-$ | Thien-2-yl | 86 - 90 |
| A.405 | $n-C_3H_7$ | Tetrahydropyran-4-yl | $-(CH_2)_3-$ | Thien-2-yl | 55 - 58 |
| A.406 | $C_2H_5$ | Tetrahydrothiopyran-3-yl | $-(CH_2)_3-$ | Thien-2-yl | 4.12 (t, 2H), 6.82 (m, 1H), 6.93 (m, 1H), 7.13 (m, 1H), |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.407 | $n-C_3H_7$ | Tetrahydrothiopyran-3-yl | $-(CH_2)_3-$ | Thien-2-yl | 4.12 (t, 2H), 6.82 (m, 1H), 6.93 (m, 1H), 7.13 (m, 1H), |
| A.408 | $C_2H_5$ | Tetrahydropyran-3-yl | $-(CH_2)_3-$ | Thien-3-yl | 73 - 74 |
| A.409 | $n-C_3H_7$ | Tetrahydropyran-3-yl | $-(CH_2)_3-$ | Thien-3-yl | 4.05 (t, 2H), 6.95 (m, 2H), 7.25 (m, 1H) |
| A.410 | $C_2H_5$ | Tetrahydropyran-4-yl | $-(CH_2)_3-$ | Thien-3-yl | 105 - 107 |
| A.411 | $n-C_3H_7$ | Tetrahydropyran-4-yl | $-(CH_2)_3-$ | Thien-3-yl | 68 - 70 |
| A.412 | $C_2H_5$ | Tetrahydrothiopyran-3-yl | $-(CH_2)_3-$ | Thien-3-yl | 57 - 59 |
| A.413 | $n-C_3H_7$ | Tetrahydrothiopyran-3-yl | $-(CH_2)_3-$ | Thien-3-yl | 4.05 (t, 2H), 6.95 (m, 2H), 7.25 (m, 1H) |
| A.414 | $C_2H_5$ | Tetrahydropyran-3-yl | $-(CH_2)_3-$ | $1-CH_3$-pyrrol-2-yl | 3.90 (m, 2H), 4.12 (t, 2H), 5.90 (m, 1H), 6.06 (m, 1H), 6,53 (m, 1H) |
| A.415 | $n-C_3H_7$ | Tetrahydropyran-3-yl | $-(CH_2)_3-$ | $1-CH_3$-pyrrol-2-yl | 3.90 (m, 2H), 4.12 (t, 2H), 5.90 (m, 1H), 6.06 (m, 1H), 6,53 (m, 1H) |
| A.416 | $C_2H_5$ | Tetrahydropyran-4-yl | $-(CH_2)_3-$ | $1-CH_3$-pyrrol-2-yl | 4.00 (m, 2H), 4.12 (t, 2H), 5.90 (m, 1H), 6.06 (m, 1H), 6,53 (m, 1H) |
| A.417 | $n-C_3H_7$ | Tetrahydropyran-4-yl | $-(CH_2)_3-$ | $1-CH_3$-pyrrol-2-yl | 4.00 (m, 2H), 4.12 (t, 2H), 5.90 (m, 1H), 6.06 (m, 1H), 6,53 (m, 1H) |
| A.418 | $C_2H_5$ | Tetrahydropyran-3-yl | $-(CH_2)_3-$ | $1-CH_3$-pyrrol-2-yl | 4.12 (t, 2H), 5.90 (m, 1H), 6.06 (m, 1H), 6,53 (m, 1H) |
| A.419 | $n-C_3H_7$ | Tetrahydropyran-3-yl | $-(CH_2)_3-$ | $1-CH_3$-pyrrol-2-yl | 4.12 (t, 2H), 5.90 (m, 1H), 6.06 (m, 1H), 6,53 (m, 1H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.420 | $C_2H_5$ | Tetrahydropyran-3-yl | $-CH_2CH(CH_3)-CH_2-$ | Thien-2-yl | 35 |
| A.421 | $n-C_3H_7$ | Tetrahydropyran-3-yl | $-CH_2CH(CH_3)-CH_2-$ | Thien-2-yl | 6.85-7.20 (m, 3H) |
| A.422 | $C_2H_5$ | Tetrahydropyran-4-yl | $-CH_2CH(CH_3)-CH_2-$ | Thien-2-yl | 59- 61 |
| A.423 | $n-C_3H_7$ | Tetrahydropyran-4-yl | $-CH_2CH(CH_3)-CH_2-$ | Thien-2-yl | 6.70-7.20 (m, 3H) |
| A.424 | $C_2H_5$ | Tetrahydrothiopyran-3-yl | $-CH_2CH(CH_3)-CH_2-$ | Thien-2-yl | 6.70-7.20 (m, 3H) |
| A.425 | $n-C_3H_7$ | Tetrahydrothiopyran-3-yl | $-CH_2CH(CH_3)-CH_2-$ | Thien-2-yl | 6.70-7,20 (m, 3H) |
| A.426 | $C_2H_5$ | Tetrahydropyran-3-yl | $-CH_2CH(CH_3)-CH_2-$ | Thien-3-yl | 38- 40 |
| A.427 | $n-C_3H_7$ | Tetrahydropyran-3-yl | $-CH_2CH(CH_3)-CH_2-$ | Thien-3-yl | 6.80-7.30 (m, 3H) |
| A.428 | $C_2H_5$ | Tetrahydropyran-4-yl | $-CH_2CH(CH_3)-CH_2-$ | Thien-3-yl | 58 - 60 |
| A.429 | $n-C_3H_7$ | Tetrahydropyran-4-yl | $-CH_2CH(CH_3)-CH_2-$ | Thien-3-yl | 6.80-7.40 (m, 3H) |
| A.430 | $C_2H_5$ | Tetrahydrothiopyran-3-yl | $-CH_2CH(CH_3)-CH_2-$ | Thien-3-yl | 6.90 (m, 2H), 7.25 (m, 1H) |
| A.431 | $n-C_3H_7$ | Tetrahydrothiopyran-3-yl | $-CH_2CH(CH_3)-CH_2-$ | Thien-3-yl | 6.90 (m, 2H), 7.30 (m, 1H) |
| A.432 | $C_2H_5$ | Tetrahydropyran-3-yl | $-CH_2CH(CH_3)-CH_2-$ | $5-CH_3$-thien-2-yl | 48 - 50 |
| A.433 | $n-C_3H_7$ | Tetrahydropyran-3-yl | $-CH_2CH(CH_3)-CH_2-$ | $5-CH_3$-thien-2-yl | 2.40 (s, 3H), 6.55 (s, 2H) |
| A.434 | $C_2H_5$ | Tetrahydropyran-4-yl | $-CH_2CH(CH_3)-CH_2-$ | $5-CH_3$-thien-2-yl | 2.40 (s, 3H), 6.55 (s, 2H) |
| A.435 | $n-C_3H_7$ | Tetrahydropyran-4-yl | $-CH_2CH(CH_3)-CH_2-$ | $5-CH_3$-thien-2-yl | 2.40 (s, 3H), 6.55 (s, 2H) |
| A.436 | $C_2H_5$ | Tetrahydrothiopyran-3-yl | $-CH_2CH(CH_3)-CH_2-$ | $5-CH_3$-thien-2-yl | 2.45 (s, 3H), 6.75 (s, 2H) |
| A.437 | $n-C_3H_7$ | Tetrahydrothiopyran-3-yl | $-CH_2CH(CH_3)-CH_2-$ | $5-CH_3$-thien-2-yl | 56 - 58 |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.438 | $C_2H_5$ | Tetrahydropyran-3-yl | $-CH_2CH=CH-$ | Furan-2-yl | 4.70 (d, 2H), 6.10-6.60 (m, 4H), 7.40 (s, 1H) |
| A.439 | $n-C_3H_7$ | Tetrahydropyran-3-yl | $-CH_2CH=CH-$ | Furan-2-yl | 4.70 (d, 2H), 6.00-6.60 (m, 4H), 7.40 (s, 1H) |
| A.440 | $C_2H_5$ | Tetrahydropyran-4-yl | $-CH_2CH=CH-$ | Furan-2-yl | 99-100 |
| A.441 | $n-C_3H_7$ | Tetrahydropyran-4-yl | $-CH_2CH=CH-$ | Furan-2-yl | 4.70 (d, 2H), 6.10-6.60 (m, 4H), 7.40 (s, 1H) |
| A.442 | $C_2H_5$ | Tetrahydrothiopyran-3-yl | $-CH_2CH=CH-$ | Furan-2-yl | 4.65 (d, 2H), 6.10-6.60 (m, 4H), 7.40 (s, 1H) |
| A.443 | $n-C_3H_7$ | Tetrahydrothiopyran-3-yl | $-CH_2CH=CH-$ | Furan-2-yl | 4.70 (d, 2H), 6.10-6.60 (m, 4H), 7.40 (s, 1H) |
| A.444 | $C_2H_5$ | Tetrahydrothiopyran-3-yl | $-CH_2CH=CH-$ | 5-Cl-thien-2-yl | 4.60 (d, 2H), 6.00 (dt, 1H), 6.70 (d, 1H), 6.80 (m, 2H) |
| A.445 | $n-C_3H_7$ | Tetrahydrothiopyran-3-yl | $-CH_2CH=CH-$ | 5-Cl-thien-2-yl | 4.60 (d, 2H), 6.00 (dt, 1H), 6.70 (d, 1H), 6.80 (m, 2H) |
| A.446 | $C_2H_5$ | Tetrahydropyran-3-yl | $-CH_2CH=CH-$ | Thien-2-yl | 112-114 |
| A.447 | $n-C_3H_7$ | Tetrahydropyran-3-yl | $-CH_2CH=CH-$ | Thien-2-yl | 67-68 |
| A.448 | $C_2H_5$ | Tetrahydropyran-4-yl | $-CH_2CH=CH-$ | Thien-2-yl | 123-125 |
| A.449 | $n-C_3H_7$ | Tetrahydropyran-4-yl | $-CH_2CH=CH-$ | Thien-2-yl | 70-72 |
| A.450 | $C_2H_5$ | Tetrahydrothiopyran-3-yl | $-CH_2CH=CH-$ | Thien-2-yl | 104-106 |
| A.451 | $n-C_3H_7$ | Tetrahydrothiopyran-3-yl | $-CH_2CH=CH-$ | Thien-2-yl | 85-88 |
| A.452 | $C_2H_5$ | 2,4,6-Trimethylphenyl | $-CH_2CH=CH-$ | Thien-2-yl | 4.65 (d, 2H), 6.10-6.30 (m, 1H), 6.70-7.20 (m, 6H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.453 | $C_2H_5$ | Tetrahydropyran-3-yl | $-CH_2CH=CH-$ | Thien-3-yl | 87-90 |
| A.454 | $n-C_3H_7$ | Tetrahydropyran-3-yl | $-CH_2CH=CH-$ | Thien-3-yl | 3.90(m,2H), 4.67(d,2H), 6.12 (dt,1H), 6.63(d,1H), 7.20(m,3H) |
| A.455 | $C_2H_5$ | Tetrahydropyran-4-yl | $-CH_2CH=CH-$ | Thien-3-yl | 128-135 |
| A.456 | $n-C_3H_7$ | Tetrahydropyran-4-yl | $-CH_2CH=CH-$ | Thien-3-yl | 92-95 |
| A.457 | $C_2H_5$ | Tetrahydrothiopyran-3-yl | $-CH_2CH=CH-$ | Thien-3-yl | 79-81 |
| A.458 | $n-C_3H_7$ | Tetrahydrothiopyran-3-yl | $-CH_2CH=CH-$ | Thien-3-yl | 86-92 |
| A.459 | $C_2H_5$ | Tetrahydropyran-3-yl | $-CH_2CH=CH-$ | $5-CH_3$-thien-2-yl | 88-89 |
| A.460 | $n-C_3H_7$ | Tetrahydropyran-3-yl | $-CH_2CH=CH-$ | $5-CH_3$-thien-2-yl | 70-71 |
| A.461 | $C_2H_5$ | Tetrahydropyran-4-yl | $-CH_2CH=CH-$ | $5-CH_3$-thien-2-yl | 108-110 |
| A.462 | $n-C_3H_7$ | Tetrahydropyran-4-yl | $-CH_2CH=CH-$ | $5-CH_3$-thien-2-yl | 104-105 |
| A.463 | $C_2H_5$ | Tetrahydrothiopyran-3-yl | $-CH_2CH=CH-$ | $5-CH_3$-thien-2-yl | 111-112 |
| A.464 | $n-C_3H_7$ | Tetrahydrothiopyran-3-yl | $-CH_2CH=CH-$ | $5-CH_3$-thien-2-yl | 75-77 |
| A.465 | $C_2H_5$ | Tetrahydropyran-3-yl | $-CH_2CH=CH-$ | 4-Br-thien-2-yl | 78-80 |
| A.466 | $n-C_3H_7$ | Tetrahydropyran-3-yl | $-CH_2CH=CH-$ | 4-Br-thien-2-yl | 6.70(d,1H), 6.95(s,1H), 7.05 (s,1H) |
| A.467 | $C_2H_5$ | Tetrahydropyran-4-yl | $-CH_2CH=CH-$ | 4-Br-thien-2-yl | 122-124 |
| A.468 | $n-C_3H_7$ | Tetrahydropyran-4-yl | $-CH_2CH=CH-$ | 4-Br-thien-2-yl | 88-90 |
| A.469 | $C_2H_5$ | Tetrahydrothiopyran-3-yl | $-CH_2CH=CH-$ | 4-Br-thien-2-yl | 72-74 |
| A.470 | $n-C_3H_7$ | Tetrahydrothiopyran-3-yl | $-CH_2CH=CH-$ | 4-Br-thien-2-yl | 6.70(d,1H), 6.90(s,1H), 7.05 (s,1H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.471 | $C_2H_5$ | Tetrahydropyran-3-yl | $-CH_2CH=CH-$ | Pyrid-3-yl | 146-148 |
| A.472 | n-$C_3H_7$ | Tetrahydropyran-3-yl | $-CH_2CH=CH-$ | Pyrid-3-yl | 6.40(dt, 1H), 7.30, 7.75, 8.40-8.70(3m, 4H) |
| A.473 | $C_2H_5$ | Tetrahydropyran-4-yl | $-CH_2CH=CH-$ | Pyrid-3-yl | 164-165 |
| A.474 | n-$C_3H_7$ | Tetrahydropyran-4-yl | $-CH_2CH=CH-$ | Pyrid-3-yl | 73-78 |
| A.475 | $C_2H_5$ | Tetrahydrothiopyran-3-yl | $-CH_2CH=CH-$ | Pyrid-3-yl | 6.40(dt, 1H), 7.30, 7.75, 8.40-8.70(3m, 4H) |
| A.476 | n-$C_3H_7$ | Tetrahydrothiopyran-3-yl | $-CH_2CH=CH-$ | Pyrid-3-yl | 6.40(dt, 1H), 7.30, 7.75, 8.40-8.70(3m, 4H) |
| A.477 | $C_2H_5$ | Tetrahydropyran-3-yl | $-CH_2C(CH_3)=CH-$ | Thien-2-yl | |
| A.478 | n-$C_3H_7$ | Tetrahydropyran-3-yl | $-CH_2C(CH_3)=CH-$ | Thien-2-yl | |
| A.479 | $C_2H_5$ | Tetrahydropyran-4-yl | $-CH_2C(CH_3)=CH-$ | Thien-2-yl | 97-98 |
| A.480 | n-$C_3H_7$ | Tetrahydropyran-4-yl | $-CH_2C(CH_3)=CH-$ | Thien-2-yl | 6.65 (s, 1H), 6.90-7.30 (2m, 3H), |
| A.481 | $C_2H_5$ | Tetrahydrothiopyran-3-yl | $-CH_2C(CH_3)=CH-$ | Thien-2-yl | 88-90 |
| A.482 | n-$C_3H_7$ | Tetrahydrothiopyran-3-yl | $-CH_2C(CH_3)=CH-$ | Thien-2-yl | 6.65 (s, 1H), 6.90-7.80 (2m, 3H), |
| A.483 | $C_2H_5$ | Tetrahydropyran-3-yl | $-CH_2C(CH_3)=CH-$ | Thien-3-yl | 6.50 (s, 1H), 7.00-7.40 (m, 3H) |
| A.484 | n-$C_3H_7$ | Tetrahydropyran-3-yl | $-CH_2C(CH_3)=CH-$ | Thien-3-yl | 6.50 (s, 1H), 7.00-7.40 (m, 3H) |
| A.485 | $C_2H_5$ | Tetrahydropyran-4-yl | $-CH_2C(CH_3)=CH-$ | Thien-3-yl | 88-90 |
| A.486 | n-$C_3H_7$ | Tetrahydropyran-4-yl | $-CH_2C(CH_3)=CH-$ | Thien-3-yl | 6.55 (s, 1H), 7.00-7.40 (m, 3H), |
| A.487 | $C_2H_5$ | Tetrahydrothiopyran-3-yl | $-CH_2C(CH_3)=CH-$ | Thien-3-yl | 6.55 (s, 1H), 7.00-7.40 (m, 3H) |
| A.488 | n-$C_3H_7$ | Tetrahydrothiopyran-3-yl | $-CH_2C(CH_3)=CH-$ | Thien-3-yl | 6.50 (s, 1H), 7.00-7.40 (m, 3H), |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.489 | $C_2H_5$ | Tetrahydropyran-3-yl | $-CH_2C(CH_3)=CH-$ | 5-$CH_3$-thien-2-yl | 108-110 |
| A.490 | n-$C_3H_7$ | Tetrahydropyran-3-yl | $-CH_2C(CH_3)=CH-$ | 5-$CH_3$-thien-2-yl | 6.60 (s,1H), 6.65-7.00 (m,2H) |
| A.491 | $C_2H_5$ | Tetrahydropyran-4-yl | $-CH_2C(CH_3)=CH-$ | 5-$CH_3$-thien-2-yl | 111-112 |
| A.492 | n-$C_3H_7$ | Tetrahydropyran-4-yl | $-CH_2C(CH_3)=CH-$ | 5-$CH_3$-thien-2-yl | 6.60 (s,1H), 6.65-7.00 (m,2H), |
| A.493 | $C_2H_5$ | Tetrahydrothiopyran-3-yl | $-CH_2C(CH_3)=CH-$ | 5-$CH_3$-thien-2-yl | 119-120 |
| A.494 | n-$C_3H_7$ | Tetrahydrothiopyran-3-yl | $-CH_2C(CH_3)=CH-$ | 5-$CH_3$-thien-2-yl | 6.55 (s,1H), 6.60-7.00 (m,2H), |
| A.495 | $C_2H_5$ | Tetrahydropyran-3-yl | $-CH_2C(CH_3)=CH-$ | 5-Cl-thien-2-yl | 82-85 |
| A.496 | n-$C_3H_7$ | Tetrahydropyran-3-yl | $-CH_2C(CH_3)=CH-$ | 5-Cl-thien-2-yl | 6.70 (s,1H), 6.90 (m,2H) |
| A.497 | $C_2H_5$ | Tetrahydropyran-4-yl | $-CH_2C(CH_3)=CH-$ | 5-Cl-thien-2-yl | 124-126 |
| A.498 | n-$C_3H_7$ | Tetrahydropyran-4-yl | $-CH_2C(CH_3)=CH-$ | 5-Cl-thien-2-yl | 97-98 |
| A.499 | $C_2H_5$ | Tetrahydrothiopyran-3-yl | $-CH_2C(CH_3)=CH-$ | 5-Cl-thien-2-yl | 103-105 |
| A.500 | n-$C_3H_7$ | Tetrahydrothiopyran-3-yl | $-CH_2C(CH_3)=CH-$ | 5-Cl-thien-2-yl | 6.65 (s,1H), 6.90 (m,2H), |
| A.501 | $C_2H_5$ | Tetrahydropyran-3-yl | $-(CH_2)_4-$ | Furan-2-yl | 3.93 (m,2H), 4.07 (m,2H), 6.00 (m,1H), 6.26 (m,1H), 7.30 (m,1H) |
| A.502 | n-$C_3H_7$ | Tetrahydropyran-3-yl | $-(CH_2)_4-$ | Furan-2-yl | 3.93 (m,2H), 4.07 (m,2H), 6.00 (m,1H), 6.26 (m,1H), 7.30 (m,1H) |
| A.503 | $C_2H_5$ | Tetrahydropyran-4-yl | $-(CH_2)_4-$ | Furan-2-yl | 3.90-4.13 (m,4H), 6.00 (m,1H), 6.26 (m,1H), 7.30 (m,1H) |
| A.504 | n-$C_3H_7$ | Tetrahydropyran-4-yl | $-(CH_2)_4-$ | Furan-2-yl | 3.90-4.13 (m,4H), 6.00 (m,1H), 6.26 (m,1H), 7.30 (m,1H) |
| A.505 | $C_2H_5$ | Tetrahydrothiopyran-3-yl | $-(CH_2)_4-$ | Furan-2-yl | 4.05 (m,2H), 6.00 (m,1H) 6.26 (m,1H), 7.30 (m,1H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.506 | $n-C_3H_7$ | Tetrahydrothiopyran-3-yl | $-(CH_2)_4-$ | Furan-2-yl | 4.05 (m,2H), 6.00 (m,1H), 6.26 (m,1H), 7.30 (m,1H) |
| A.507 | $C_2H_5$ | Tetrahydropyran-3-yl | $-(CH_2)_4-$ | $5-CH_3$-furan-2-yl | 62-64 |
| A.508 | $n-C_3H_7$ | Tetrahydropyran-3-yl | $-(CH_2)_4-$ | $5-CH_3$-furan-2-yl | 3.93 (m,2H), 4.07 (m,2H), 5.87 (m, 2H) |
| A.509 | $C_2H_5$ | Tetrahydropyran-4-yl | $-(CH_2)_4-$ | $5-CH_3$-furan-2-yl | 76-78 |
| A.510 | $n-C_3H_7$ | Tetrahydropyran-4-yl | $-(CH_2)_4-$ | $5-CH_3$-furan-2-yl | 3.90-4.15 (m,4H), 5.87 (m,2H) |
| A.511 | $C_2H_5$ | Tetrahydrothiopyran-3-yl | $-(CH_2)_4-$ | $5-CH_3$-furan-2-yl | 4.07 (m,2H), 5.87 (m,2H) |
| A.512 | $n-C_3H_7$ | Tetrahydrothiopyran-3-yl | $-(CH_2)_4-$ | $5-CH_3$-furan-2-yl | 4.07 (m,2H), 5.87 (m,2H) |
| A.513 | $C_2H_5$ | Tetrahydropyran-3-yl | $-(CH_2)_4-$ | Thien-2-yl | 3.80-4.15 (m,4H), 6.80 (dd,1H), 6.93 (dd,1H), 7.13 (dd,1H) |
| A.514 | $n-C_3H_7$ | Tetrahydropyran-3-yl | $-(CH_2)_4-$ | Thien-2-yl | 3.80-4.15 (m,4H), 6.80 (dd,1H), 6.93 (dd,1H), 7.13 (dd,1H) |
| A.515 | $C_2H_5$ | Tetrahydropyran-4-yl | $-(CH_2)_4-$ | Thien-2-yl | 3.90-4.23 (m,4H), 6.80 (dd,1H), 6.93 (dd,1H), 7.13 (dd,1H) |
| A.516 | $n-C_3H_7$ | Tetrahydropyran-4-yl | $-(CH_2)_4-$ | Thien-2-yl | 3.90-4.23 (m,4H), 6.80 (dd,1H), 6.93 (dd,1H), 7.13 (dd,1H) |
| A.517 | $C_2H_5$ | Tetrahydrothiopyran-3-yl | $-(CH_2)_4-$ | Thien-2-yl | 4.06 (m,2H), 6.80 (dd,1H), 6.93 (dd,1H), 7.13 (dd,1H) |
| A.518 | $n-C_3H_7$ | Tetrahydrothiopyran-3-yl | $-(CH_2)_4-$ | Thien-2-yl | 4.06 (m,2H), 6.80 (dd,1H), 6.93 (dd,1H), 7.13 (dd,1H) |
| A.519 | $C_2H_5$ | Tetrahydropyran-3-yl | $-(CH_2)_4-$ | $5-CH_3$-thien-2-yl | 3.85-4.13 (m,4H), 6.53 (s,2H) |
| A.520 | $n-C_3H_7$ | Tetrahydropyran-3-yl | $-(CH_2)_4-$ | $5-CH_3$-thien-2-yl | 3.80-4.13 (m,4H), 6.53 (s,2H) |
| A.521 | $C_2H_5$ | Tetrahydropyran-4-yl | $-(CH_2)_4-$ | $5-CH_3$-thien-2-yl | 3.90-4.15 (m,4H), 6.50 (s,2H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.522 | $n\text{-}C_3H_7$ | Tetrahydropyran-4-yl | $-(CH_2)_4-$ | 5-$CH_3$-thien-2-yl | 3.94-4.15 (m,4H), 6.53 (s,2H) |
| A.523 | $C_2H_5$ | Tetrahydrothiopyran-3-yl | $-(CH_2)_4-$ | 5-$CH_3$-thien-2-yl | 4.08 (m,2H), 6.55 (s,2H) |
| A.524 | $n\text{-}C_3H_7$ | Tetrahydrothiopyran-3-yl | $-(CH_2)_4-$ | 5-$CH_3$-thien-2-yl | 4.08 (m,2H), 6.56 (s,2H) |
| A.525 | $C_2H_5$ | Tetrahydropyran-3-yl | $-(CH_2)_4-$ | 5-Cl-thien-2-yl | 3.93 (m,2H), 4.10 (m,2H), 6.53 (d,1H), 6.70 (d,1H) |
| A.526 | $n\text{-}C_3H_7$ | Tetrahydropyran-3-yl | $-(CH_2)_4-$ | 5-Cl-thien-2-yl | 3.93 (m,2H), 4.10 (m,2H), 6.53 (d,1H), 6.70 (d,1H) |
| A.527 | $C_2H_5$ | Tetrahydropyran-4-yl | $-(CH_2)_4-$ | 5-Cl-thien-2-yl | 3.90-4.10 (m,4H), 6.53 (d,1H) 6.70 (d,1H) |
| A.528 | $n\text{-}C_3H_7$ | Tetrahydropyran-4-yl | $-(CH_2)_4-$ | 5-Cl-thien-2-yl | 3.90-4.10 (m,4H), 6.53 (d,1H) 6.70 (d,1H) |
| A.529 | $C_2H_5$ | Tetrahydrothiopyran-3-yl | $-(CH_2)_4-$ | 5-Cl-thien-2-yl | 4.10 (m,2H), 6.53 (d,1H), 6.70 (d,1H) |
| A.530 | $n\text{-}C_3H_7$ | Tetrahydrothiopyran-3-yl | $-(CH_2)_4-$ | 5-Cl-thien-2-yl | 4.10 (m,2H), 6.53 (d,1H), 6.70 (d,1H) |
| A.531 | $C_2H_5$ | Tetrahydropyran-3-yl | $-(CH_2)_4-$ | 5-$C_2H_5$-thien-2-yl | 3.80-4.09 (m,4H), 6.60 (s,2H) |
| A.532 | $n\text{-}C_3H_7$ | Tetrahydropyran-3-yl | $-(CH_2)_4-$ | 5-$C_2H_5$-thien-2-yl | 3.80-4.09 (m,4H), 6.60 (s,2H) |
| A.533 | $C_2H_5$ | Tetrahydropyran-4-yl | $-(CH_2)_4-$ | 5-$C_2H_5$-thien-2-yl | 3.93-4.09 (m,4H), 6.60 (s,2H) |
| A.534 | $n\text{-}C_3H_7$ | Tetrahydropyran-4-yl | $-(CH_2)_4-$ | 5-$C_2H_5$-thien-2-yl | 3.93-4.09 (m,4H), 6.60 (s,2H) |
| A.535 | $C_2H_5$ | Tetrahydrothiopyran-3-yl | $-(CH_2)_4-$ | 5-$C_2H_5$-thien-2-yl | 4.03 (m,2H), 6.60 (s,2H) |
| A.536 | $n\text{-}C_3H_7$ | Tetrahydrothiopyran-3-yl | $-(CH_2)_4-$ | 5-$C_2H_5$-thien-2-yl | 4.03 (m,2H), 6.60 (s,2H) |
| A.537 | $C_2H_5$ | Tetrahydropyran-3-yl | $-(CH_2)_4-$ | 1-$CH_3$-pyrrol-2-yl | 64-66 |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.538 | $n-C_3H_7$ | Tetrahydropyran-3-yl | $-(CH_2)_4-$ | $1-CH_3$-pyrrol-2-yl | 3.90 (m,2H), 4.09 (t,2H), 5.87 (m,1H), 6.03 (m,1H), 6.53 (m,1H) |
| A.539 | $C_2H_5$ | Tetrahydropyran-4-yl | $-(CH_2)_4-$ | $1-CH_3$-pyrrol-2-yl | 82-84 |
| A.540 | $n-C_3H_7$ | Tetrahydropyran-4-yl | $-(CH_2)_4-$ | $1-CH_3$-pyrrol-2-yl | 4.00 (m,22), 4.09 (t,2H), 5.87 (m,1H), 6.03 (m,1H), 6.53 (m,1H) |
| A.541 | $C_2H_5$ | Tetrahydrothiopyran-3-yl | $-(CH_2)_4-$ | $1-CH_3$-pyrrol-2-yl | 4.09 (t,2H), 5.87 (m,1H), 6.03 (m,1H), 6.53 (m,1H) |
| A.542 | $n-C_3H_7$ | Tetrahydrothiopyran-3-yl | $-(CH_2)_4-$ | $1-CH_3$-pyrrol-2-yl | 4.09 (t,2H), 5.87 (m,1H), 6.03 (m,1H), 6.53 (m,1H) |
| A.543 | $C_2H_5$ | Tetrahydropyran-4-yl | $-CH_2CH_2CH=CH-$ | Furan-2-yl | 4.13 (t,2H), 6.00-6.42 (m,4H), 7.33 (bs,1H) |
| A.544 | $C_2H_5$ | Tetrahydropyran-3-yl | $-CH_2CH_2CH=CH-$ | Furan-3-yl | 4.13 (t,2H), 5.92 (m,1H), 6.33 (d,1H), 6.55 (bs,1H), 7.40 (d,2H) |
| A.545 | $C_2H_5$ | Tetrahydropyran-4-yl | $-CH_2CH_2CH=CH-$ | Furan-3-yl | 4.13 (m,2H), 5.92 (m,1H), 6.33 (d,1H), 6.55 (s,1H), 7.40 (d,2H) |
| A.546 | $C_2H_5$ | Tetrahydrothiopyran-3-yl | $-CH_2CH_2CH=CH-$ | Furan-3-yl | 4.13 (t,2H), 5.92 (m,1H), 6.33 (d,1H), 6.55 (bs,1H), 7.40 (d,2H) |
| A.547 | $C_2H_5$ | Tetrahydropyran-3-yl | $-CH_2CH_2CH=CH-$ | Thien-2-yl | 4.15 (t, 2H), 6.00 (dt, 1H), 6.60 (d, 1H), 6.90 (m, 2H), 7.10 (d, 1H) |
| A.548 | $n-C_3H_7$ | Tetrahydropyran-3-yl | $-CH_2CH_2CH=CH-$ | Thien-2-yl | 4.10 (t, 2H), 6.00 (dt, 1H), 6.60 (d, 1H), 6.80-7.20 (m, 3H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.549 | $C_2H_5$ | Tetrahydropyran-4-yl | $-CH_2CH_2-CH=CH-$ | Thien-2-yl | 4.15 (t, 2H), 6.00 (dt, 1H), 6.60 (d, 1H), 6.80-7.20 (m, 3H), |
| A.550 | $n-C_3H_7$ | Tetrahydropyran-4-yl | $-CH_2CH_2-CH=CH-$ | Thien-2-yl | 4.15 (t, 2H), 6.00 (dt, 1H), 6.60 (d, 1H), 6.80-7.20 (m, 3H), |
| A.551 | $C_2H_5$ | Tetrahydrothiopyran-3-yl | $-CH_2CH_2-CH=CH-$ | Thien-2-yl | 4.15 (t, 2H), 6.00 (dt, 1H), 6.60 (d, 1H), 6.80-7.20 (m, 3H), |
| A.552 | $n-C_3H_7$ | Tetrahydrothiopyran-3-yl | $-CH_2CH_2-CH=CH-$ | Thien-2-yl | 4.15 (t, 2H), 6.00 (dt, 1H), 6.60 (d, 1H), 6.80-7.30 (m, 3H) |
| A.553 | $C_2H_5$ | 2,4,6-Trimethylphenyl | $-CH_2CH_2-CH=CH-$ | Thien-2-yl | 4.20 (t, 2H), 6.10 (dt, 1H), 6.60 (d, 1H), 6.80-7.20 (m, 5H) |
| A.554 | $C_2H_5$ | Tetrahydropyran-3-yl | $-CH_2CH_2-CH=CH-$ | $5-CH_3$-thien-2-yl | 4.13 (t, 2H), 5.87 (dt, 1H) 6.37-6.73 (m, 3H) |
| A.555 | $n-C_3H_7$ | Tetrahydropyran-3-yl | $-CH_2CH_2-CH=CH-$ | $5-CH_3$-thien-2-yl | 4.13 (t, 2H), 5.87 (dt, 1H) 6.37-6.73 (m, 3H) |
| A.556 | $C_2H_5$ | Tetrahydropyran-4-yl | $-CH_2CH_2-CH=CH-$ | $5-CH_3$-thien-2-yl | 4.13 (t, 2H), 5.87 (dt, 1H) 6.37-6.73 (m, 3H) |
| A.557 | $n-C_3H_7$ | Tetrahydropyran-4-yl | $-CH_2CH_2-CH=CH-$ | $5-CH_3$-thien-2-yl | 4.13 (t, 2H), 5.87 (dt, 1H) 6.37-6.73 (m, 3H) |
| A.558 | $C_2H_5$ | Tetrahydrothiopyran-3-yl | $-CH_2CH_2-CH=CH-$ | $5-CH_3$-thien-2-yl | 4.13 (t, 2H), 5.88 (dt, 1H) 6.37-6.73 (m, 3H) |
| A.559 | $n-C_3H_7$ | Tetrahydrothiopyran-3-yl | $-CH_2CH_2-CH=CH-$ | $5-CH_3$-thien-2-yl | 4.13 (t, 2H), 5.88 (dt, 1H) 6.37-6.73 (m, 3H) |
| A.560 | $C_2H_5$ | Tetrahydropyran-3-yl | $-CH_2CH_2-CH=CH-$ | 5-Cl-thien-2-yl | 4.15 (t, 2H), 5.93 (dt, 1H), 6.46 (d, 1H), 6.63 (d, 1H), 6.75 (d, 1H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.561 | $n-C_3H_7$ | Tetrahydropyran-3-yl | $-CH_2CH_2-CH=CH-$ | 5-Cl-thien-2-yl | 4.15 (t, 2H), 5.93 (dt, 1H), 6.46 (d, 1H), 6.63 (d, 1H), 6.75 (d, 1H) |
| A.562 | $C_2H_5$ | Tetrahydropyran-4-yl | $-CH_2CH_2-CH=CH-$ | 5-Cl-thien-2-yl | 4.15 (t, 2H), 5.93 (dt, 1H), 6.46 (d, 1H), 6.63 (d, 1H), 6.75 (d, 1H) |
| A.563 | $n-C_3H_7$ | Tetrahydropyran-4-yl | $-CH_2CH_2-CH=CH-$ | 5-Cl-thien-2-yl | 4.15 (t, 2H), 5.93 (dt, 1H), 6.46 (d, 1H), 6.63 (d, 1H), 6.75 (d, 1H) |
| A.564 | $C_2H_5$ | Tetrahydrothiopyran-3-yl | $-CH_2CH_2-CH=CH-$ | 5-Cl-thien-2-yl | 4.15 (t, 2H), 5.93 (dt, 1H), 6.46 (d, 1H), 6.63 (d, 1H), 6.75 (d, 1H) |
| A.565 | $n-C_3H_7$ | Tetrahydrothiopyran-3-yl | $-CH_2CH_2-CH=CH-$ | 5-Cl-thien-2-yl | 4.15 (t, 2H), 5.93 (dt, 1H), 6.46 (d, 1H), 6.63 (d, 1H), 6.75 (d, 1H) |
| A.566 | $C_2H_5$ | Tetrahydropyran-3-yl | $-CH_2CH_2-CH=CH-$ | Thien-3-yl | 4.15 (t, 2H), 6.07 (dt, 1H), 6.50 (d, 1H), 7.00-7.32 (m, 3H) |
| A.567 | $n-C_3H_7$ | Tetrahydropyran-3-yl | $-CH_2CH_2-CH=CH-$ | Thien-3-yl | 4.15 (t, 2H), 6.07 (dt, 1H), 6.50 (d, 1H), 7.00-7.32 (m, 3H) |
| A.568 | $C_2H_5$ | Tetrahydropyran-4-yl | $-CH_2CH_2-CH=CH-$ | Thien-3-yl | 4.20 (t, 2H), 6.07 (dt, 1H), 6.50 (d, 1H), 7.03-7.32 (m, 3H) |
| A.569 | $n-C_3H_7$ | Tetrahydropyran-4-yl | $-CH_2CH_2-CH=CH-$ | Thien-3-yl | 4.20 (t, 2H), 6.07 (dt, 1H), 6.50 (d, 1H), 7.03-7.32 (m, 3H) |
| A.570 | $C_2H_5$ | Tetrahydrothiopyran-3-yl | $-CH_2CH_2-CH=CH-$ | Thien-3-yl | 4.17 (t, 2H), 6.07 (dt, 1H), 6.50 (d, 1H), 7.00-7.36 (m, 3H) |
| A.571 | $n-C_3H_7$ | Tetrahydrothiopyran-3-yl | $-CH_2CH_2-CH=CH-$ | Thien-3-yl | 4.17 (t, 2H), 6.07 (dt, 1H), 6.50 (d, 1H), 7.00-7.36 (m, 3H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.572 | $C_2H_5$ | Tetrahydropyran-3-yl | $-CH_2CH_2-CH=CH-$ | 2-Cl-thien-3-yl | 4.20 (t, 2H), 6.10 (dt, 1H), 6.52 (d, 1H), 7.05 (s, 2H), |
| A.573 | $n-C_3H_7$ | Tetrahydropyran-3-yl | $-CH_2CH_2-CH=CH-$ | 2-Cl-thien-3-yl | 4.20 (t, 2H), 6.10 (dt, 1H), 6.52 (d, 1H), 7.05 (s, 2H), |
| A.574 | $C_2H_5$ | Tetrahydropyran-4-yl | $-CH_2CH_2-CH=CH-$ | 2-Cl-thien-3-yl | 4.20 (t, 2H), 6.13 (dt, 1H), 6.52 (d, 1H), 7.07 (s, 2H), |
| A.575 | $n-C_3H_7$ | Tetrahydropyran-4-yl | $-CH_2CH_2-CH=CH-$ | 2-Cl-thien-3-yl | 4.20 (t, 2H), 6.13 (dt, 1H), 6.52 (d, 1H), 7.07 (s, 2H), |
| A.576 | $C_2H_5$ | Tetrahydrothiopyran-3-yl | $-CH_2CH_2-CH=CH-$ | 2-Cl-thien-3-yl | 4.20 (t, 2H), 6.12 (dt, 1H), 6.53 (d, 1H), 7.10 (s, 2H), |
| A.577 | $n-C_3H_7$ | Tetrahydrothiopyran-3-yl | $-CH_2CH_2-CH=CH-$ | 2-Cl-thien-3-yl | 4.20 (t, 2H), 6.12 (dt, 1H), 6.53 (d, 1H), 7.10 (s, 2H), |
| A.578 | $C_2H_5$ | Tetrahydropyran-3-yl | $-CH_2CH_2-CH=CH-$ | 5-Cl-thien-3-yl | 4.17 (t, 2H), 6.00 (dt, 1H), 6.33 (d, 1H), 6.83 (s, 1H), 7.03 (s, 1H) |
| A.579 | $n-C_3H_7$ | Tetrahydropyran-3-yl | $-CH_2CH_2-CH=CH-$ | 5-Cl-thien-3-yl | 4.17 (t, 2H), 6.00 (dt, 1H), 6.33 (d, 1H), 6.83 (s, 1H), 7.03 (s, 1H) |
| A.580 | $C_2H_5$ | Tetrahydropyran-4-yl | $-CH_2CH_2-CH=CH-$ | 5-Cl-thien-3-yl | 4.17 (t, 2H), 6.00 (dt, 1H), 6.33 (d, 1H), 6.83 (s, 1H), 7.03 (s, 1H) |
| A.581 | $n-C_3H_7$ | Tetrahydropyran-4-yl | $-CH_2CH_2-CH=CH-$ | 5-Cl-thien-3-yl | 4.17 (t, 2H), 6.00 (dt, 1H), 6.33 (d, 1H), 6.83 (s, 1H), 7.03 (s, 1H) |
| A.582 | $C_2H_5$ | Tetrahydrothiopyran-3-yl | $-CH_2CH_2-CH=CH-$ | 5-Cl-thien-3-yl | 4.20 (t, 2H), 6.00 (dt, 1H), 6.33 (d, 1H), 6.83 (s, 1H), 7.03 (s, 1H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.583 | $n-C_3H_7$ | Tetrahydrothiopyran-3-yl | $-CH_2CH_2-CH=CH-$ | 5-Cl-thien-3-yl | 4.20 (t, 2H), 6.00 (dt, 1H), 6.33 (d, 1H), 6.83 (s, 1H), 7.03 (s, 1H) |
| A.584 | $C_2H_5$ | Tetrahydropyran-3-yl | $-CH_2CH_2CH=CHCH_3-$ | Thien-2-yl | 3.90 (m, 2H), 6.90 (m, 2H), 7.10 (d, 1H) |
| A.585 | $n-C_3H_7$ | Tetrahydropyran-3-yl | $-CH_2CH_2CH=CHCH_3-$ | Thien-2-yl | 3.90 (m, 2H), 6.90 (m, 2H), 7.10 (d, 1H) |
| A.586 | $C_2H_5$ | Tetrahydropyran-4-yl | $-CH_2CH_2CH=CHCH_3-$ | Thien-2-yl | |
| A.587 | $n-C_3H_7$ | Tetrahydropyran-4-yl | $-CH_2CH_2CH=CHCH_3-$ | Thien-2-yl | |
| A.588 | $C_2H_5$ | Tetrahydrothiopyran-3-yl | $-CH_2CH_2CH=CHCH_3-$ | Thien-3-yl | 6.90 (m, 2H), 7.10 (d, 1H) |
| A.589 | $n-C_3H_7$ | Tetrahydrothiopyran-3-yl | $-CH_2CH_2CH=CHCH_3-$ | Thien-3-yl | 6.90 (m, 2H), 7.10 (d, 1H) |
| A.590 | $C_2H_5$ | Tetrahydropyran-3-yl | $-(CH_2)_5-$ | Furan-2-yl | 5.93 (m, 1H), 6.27 (m, 1H), 7.27 (m, 1H) |
| A.591 | $n-C_3H_7$ | Tetrahydropyran-3-yl | $-(CH_2)_5-$ | Furan-2-yl | 5.93 (m, 1H), 6.27 (m, 1H), 7.27 (m, 1H) |
| A.592 | $C_2H_5$ | Tetrahydropyran-4-yl | $-(CH_2)_5-$ | Furan-2-yl | 5.90 (m, 1H), 6.24 (m, 1H), 7.24 (m, 1H) |
| A.593 | $n-C_3H_7$ | Tetrahydropyran-4-yl | $-(CH_2)_5-$ | Furan-2-yl | 50-53 |
| A.594 | $C_2H_5$ | Tetrahydrothiopyran-3-yl | $-(CH_2)_5-$ | Furan-2-yl | 5.93 (m, 1H), 6.27 (m, 1H), 7.27 (m, 1H) |
| A.595 | $n-C_3H_7$ | Tetrahydrothiopyran-3-yl | $-(CH_2)_5-$ | Furan-2-yl | 5.93 (m, 1H), 6.27 (m, 1H), 7.27 (m, 1H) |
| A.596 | $C_2H_5$ | Tetrahydropyran-3-yl | $-(CH_2)_5-$ | Thien-2-yl | 43-45 |
| A.597 | $n-C_3H_7$ | Tetrahydropyran-3-yl | $-(CH_2)_5-$ | Thien-2-yl | 73-75 |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.598 | $C_2H_5$ | Tetrahydropyran-4-yl | $-(CH_2)_5-$ | Thien-2-yl | 91-93 |
| A.599 | $n-C_3H_7$ | Tetrahydropyran-4-yl | $-(CH_2)_5-$ | Thien-2-yl | 74-75 |
| A.600 | $C_2H_5$ | Tetrahydrothiopyran-3-yl | $-(CH_2)_5-$ | Thien-2-yl | 4.07 (t, 2H), 6.80 (m, 1H), 6.90 (m, 1H), 7.10 (m, 1H) |
| A.601 | $n-C_3H_7$ | Tetrahydrothiopyran-3-yl | $-(CH_2)_5-$ | Thien-2-yl | 4.07 (t, 2H), 6.80 (m, 1H), 6.90 (m, 1H), 7.10 (m, 1H) |
| A.602 | $C_2H_5$ | Tetrahydropyran-3-yl | $-(CH_2)_5-$ | $1-CH_3$-pyrrol-2-yl | 3.90 (m, 2H), 4.12 (t, 2H), 5.90 (m, 1H), 6.06 (m, 1H), 6.53 (m, 1H) |
| A.603 | $n-C_3H_7$ | Tetrahydropyran-3-yl | $-(CH_2)_5-$ | $1-CH_3$-pyrrol-2-yl | 3.90 (m, 2H), 4.12 (t, 2H), 5.90 (m, 1H), 6.06 (m, 1H), 6.53 (m, 1H) |
| A.604 | $C_2H_5$ | Tetrahydropyran-4-yl | $-(CH_2)_5-$ | $1-CH_3$-pyrrol-2-yl | 4.00 (m, 2H), 4.12 (t, 2H), 5.90 (m, 1H), 6.06 (m, 1H), 6.53 (m, 1H) |
| A.605 | $n-C_3H_7$ | Tetrahydropyran-4-yl | $-(CH_2)_5-$ | $1-CH_3$-pyrrol-2-yl | 4.00 (m, 2H), 4.12 (t, 2H), 5.90 (m, 1H), 6.06 (m, 1H), 6.53 (m, 1H) |
| A.606 | $C_2H_5$ | Tetrahydrothiopyran-3-yl | $-(CH_2)_5-$ | $1-CH_3$-pyrrol-2-yl | 4.12 (t, 2H), 5.90 (m, 1H), 6.06 (m, 1H), 6.53 (m, 1H) |
| A.607 | $n-C_3H_7$ | Tetrahydrothiopyran-3-yl | $-(CH_2)_5-$ | $1-CH_3$-pyrrol-2-yl | 4.12 (t, 2H), 5.90 (m, 1H), 6.06 (m, 1H), 6.53 (m, 1H) |
| A.608 | $C_2H_5$ | Tetrahydropyran-3-yl | $-(CH_2)_6-$ | Furan-2-yl | 5.90 (m, 1H), 6.27 (m, 1H), 7.27 (m, 1H) |
| A.609 | $n-C_3H_7$ | Tetrahydropyran-3-yl | $-(CH_2)_6-$ | Furan-2-yl | 5.90 (m, 1H), 6.27 (m, 1H), 7.27 (m, 1H) |
| A.610 | $C_2H_5$ | Tetrahydropyran-4-yl | $-(CH_2)_6-$ | Furan-2-yl | 5.93 (m, 1H), 6.27 (m, 1H), 7.27 (m, 1H) |
| A.611 | $n-C_3H_7$ | Tetrahydropyran-4-yl | $-(CH_2)_6-$ | Furan-2-yl | 5.93 (m, 1H), 6.27 (m, 1H), 7.27 (m, 1H) |

Tabelle II.7 (Fortsetzung)

| Nr. | $R^a$ | $R^c$ | W | $R^f$ | phys. Daten NMR-Daten in ppm Fp in °C |
|---|---|---|---|---|---|
| A.612 | $C_2H_5$ | Tetrahydrothiopyran-3-yl | $-(CH_2)_6-$ | Furan-2-yl | 5.93 (m, 1H), 6.27 (m, 1H), 7.27 (m, 1H) |
| A.613 | $n-C_3H_7$ | Tetrahydrothiopyran-3-yl | $-(CH_2)_6-$ | Furan-2-yl | 5.93 (m, 1H), 6.27 (m, 1H), 7.27 (m, 1H) |
| A.614 | $C_2H_5$ | Tetrahydropyran-3-yl | $-(CH_2)_6-$ | Thien-2-yl | 6.77 (m, 1H), 6.90 (m, 1H), 7.10 (m, 1H) |
| A.615 | $n-C_3H_7$ | Tetrahydropyran-3-yl | $-(CH_2)_6-$ | Thien-2-yl | 6.77 (m, 1H), 6.90 (m, 1H), 7.10 (m, 1H) |
| A.616 | $C_2H_5$ | Tetrahydropyran-4-yl | $-(CH_2)_6-$ | Thien-2-yl | 50-52 |
| A.617 | $n-C_3H_7$ | Tetrahydropyran-4-yl | $-(CH_2)_6-$ | Thien-2-yl | 6.80 (m, 1H), 6.90 (m, 1H), 7.10 (m, 1H) |
| A.618 | $C_2H_5$ | Tetrahydrothiopyran-3-yl | $-(CH_2)_6-$ | Thien-2-yl | 6.80 (m, 1H), 6.90 (m, 1H), 7.10 (m, 1H) |
| A.619 | $n-C_3H_7$ | Tetrahydrothiopyran-3-yl | $-(CH_2)_6-$ | Thien-2-yl | 6.80 (m, 1H), 6.90 (m, 1H), 7.10 (m, 1H) |
| A.620 | $C_2H_5$ | Tetrahydropyran-3-yl | $-(CH_2)_6-$ | 1-$CH_3$-pyrrol-2-yl | 5.90 (m, 1H), 6.06 (m, 1H), 6.53 (m, 1H) |
| A.621 | $n-C_3H_7$ | Tetrahydropyran-3-yl | $-(CH_2)_6-$ | 1-$CH_3$-pyrrol-2-yl | 5.90 (m, 1H), 6.06 (m, 1H), 6.53 (m, 1H) |
| A.622 | $C_2H_5$ | Tetrahydropyran-4-yl | $-(CH_2)_6-$ | 1-$CH_3$-pyrrol-2-yl | 5.87 (m, 1H), 6.06 (m, 1H), 6.53 (m, 1H) |
| A.623 | $n-C_3H_7$ | Tetrahydropyran-4-yl | $-(CH_2)_6-$ | 1-$CH_3$-pyrrol-2-yl | 5.87 (m, 1H), 6.06 (m, 1H), 6.53 (m, 1H) |
| A.624 | $C_2H_5$ | Tetrahydrothiopyran-3-yl | $-(CH_2)_6-$ | 1-$CH_3$-pyrrol-2-yl | 5.90 (m, 1H), 6.06 (m, 1H), 6.50 (m, 1H) |
| A.625 | $n-C_3H_7$ | Tetrahydrothiopyran-3-yl | $-(CH_2)_6-$ | 1-$CH_3$-pyrrol-2-yl | 5.90 (m, 1H), 6.06 (m, 1H), 6.50 (m, 1H) |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.626 | $C_2H_5$ | Tetrahydropyran-3-yl | $-CH_2CH_2-O-$ | Phenyl | 42- 45 |
| A.627 | $n-C_3H_7$ | Tetrahydropyran-3-yl | $-CH_2CH_2-O-$ | Phenyl | 3,90 (m, 2H), 4,20 (t, 2H), 4,40 (m, 2H), 6,80-7,00 (m, 3H), 7,13-7,37 (m, 2H) |
| A.628 | $C_2H_5$ | Tetrahydropyran-4-yl | $-CH_2CH_2-O-$ | Phenyl | 106-107 |
| A.629 | $n-C_3H_7$ | Tetrahydropyran-4-yl | $-CH_2CH_2-O-$ | Phenyl | 72- 73 |
| A.630 | $C_2H_5$ | Tetrahydrothiopyran-3-yl | $-CH_2CH_2-O-$ | Phenyl | 52- 55 |
| A.631 | $n-C_3H_7$ | Tetrahydrothiopyran-3-yl | $-CH_2CH_2-O-$ | Phenyl | 92 |
| A.632 | $C_2H_5$ | Tetrahydropyran-3-yl | $-CH_2CH_2-O-$ | 2-F-phenyl | 76- 78 |
| A.633 | $n-C_3H_7$ | Tetrahydropyran-3-yl | $-CH_2CH_2-O-$ | 2-F-phenyl | 72- 77 |
| A.634 | $C_2H_5$ | Tetrahydropyran-4-yl | $-CH_2CH_2-O-$ | 2-F-phenyl | 121-125 |
| A.635 | $n-C_3H_7$ | Tetrahydropyran-4-yl | $-CH_2CH_2-O-$ | 2-F-phenyl | 103-107 |
| A.636 | $C_2H_5$ | Tetrahydrothiopyran-3-yl | $-CH_2CH_2-O-$ | 2-F-phenyl | 82- 86 |
| A.637 | $n-C_3H_7$ | Tetrahydrothiopyran-3-yl | $-CH_2CH_2-O-$ | 2-F-phenyl | 81- 85 |
| A.638 | $C_2H_5$ | Tetrahydropyran-3-yl | $-CH_2CH_2-O-$ | 3-F-phenyl | 62- 68 |
| A.639 | $n-C_3H_7$ | Tetrahydropyran-3-yl | $-CH_2CH_2-O-$ | 3-F-phenyl | 3,90 (m, 2H), 4,20 (t, 2H), 4,40 (m, 2H) 6,70 (m, 3H), 7,25 (m, 1H), |
| A.640 | $C_2H_5$ | Tetrahydropyran-4-yl | $-CH_2CH_2-O-$ | 3-F-phenyl | 103-109 |
| A.641 | $n-C_3H_7$ | Tetrahydropyran-4-yl | $-CH_2CH_2-O-$ | 3-F-phenyl | 73- 79 |

Tabelle II.7 (Fortsetzung)

| Nr. | $R^a$ | $R^c$ | W | $R^f$ | phys. Daten / $^1H$-NMR [δ in ppm], Fp. [°C] |
|---|---|---|---|---|---|
| A.642 | $C_2H_5$ | Tetrahydrothiopyran-3-yl | $-CH_2CH_2-O-$ | 3-F-phenyl | 4,20 (t, 2H), 4,40 (m, 2H), 6,70 (m, 3H), 7,25 (m, 1H) |
| A.643 | $n-C_3H_7$ | Tetrahydrothiopyran-3-yl | $-CH_2CH_2-O-$ | 3-F-phenyl | |
| A.644 | $C_2H_5$ | Tetrahydropyran-3-yl | $-CH_2CH_2-O-$ | 4-F-phenyl | 64- 67 |
| A.645 | $n-C_3H_7$ | Tetrahydropyran-3-yl | $-CH_2CH_2-O-$ | 4-F-phenyl | 70- 72 |
| A.646 | $C_2H_5$ | Tetrahydropyran-4-yl | $-CH_2CH_2-O-$ | 4-F-phenyl | 101-103 |
| A.647 | $n-C_3H_7$ | Tetrahydropyran-4-yl | $-CH_2CH_2-O-$ | 4-F-phenyl | 107-109 |
| A.648 | $C_2H_5$ | Tetrahydrothiopyran-3-yl | $-CH_2CH_2-O-$ | 4-F-phenyl | 105-108 |
| A.649 | $n-C_3H_7$ | Tetrahydrothiopyran-3-yl | $-CH_2CH_2-O-$ | 4-F-phenyl | 82- 84 |
| A.650 | $C_2H_5$ | Tetrahydropyran-3-yl | $-CH_2CH_2-O-$ | 2-Cl-phenyl | 74- 80 |
| A.651 | $n-C_3H_7$ | Tetrahydropyran-3-yl | $-CH_2CH_2-O-$ | 2-Cl-phenyl | 67- 71 |
| A.652 | $C_2H_5$ | Tetrahydropyran-4-yl | $-CH_2CH_2-O-$ | 2-Cl-phenyl | 4,00 (m, 2H), 4,27 (t, 2H), 4,47 (m, 2H), 7,20 (t, 1H), 7,37 (d, 1H) |
| A.653 | $n-C_3H_7$ | Tetrahydropyran-4-yl | $-CH_2CH_2-O-$ | 2-Cl-phenyl | 68- 72 |
| A.654 | $C_2H_5$ | Tetrahydrothiopyran-3-yl | $-CH_2CH_2-O-$ | 2-Cl-phenyl | 74- 78 |
| A.655 | $n-C_3H_7$ | Tetrahydrothiopyran-3-yl | $-CH_2CH_2-O-$ | 2-Cl-phenyl | 72-78 |
| A.656 | $C_2H_5$ | Tetrahydropyran-3-yl | $-CH_2CH_2-O-$ | 3-Cl-phenyl | |
| A.657 | $n-C_3H_7$ | Tetrahydropyran-3-yl | $-CH_2CH_2-O-$ | 3-Cl-phenyl | |
| A.658 | $C_2H_5$ | Tetrahydropyran-4-yl | $-CH_2CH_2-O-$ | 3-Cl-phenyl | |
| A.659 | $n-C_3H_7$ | Tetrahydropyran-4-yl | $-CH_2CH_2-O-$ | 3-Cl-phenyl | |
| A.660 | $C_2H_5$ | Tetrahydrothiopyran-3-yl | $-CH_2CH_2-O-$ | 3-Cl-phenyl | |
| A.661 | $n-C_3H_7$ | Tetrahydrothiopyran-3-yl | $-CH_2CH_2-O-$ | 3-Cl-phenyl | |

Tabelle II.7 (Fortsetzung)

| Nr. | $R^a$ | $R^c$ | W | $R^f$ | phys. Daten / $^1H$-NMR [$\delta$ in ppm], Fp. [°C] |
|---|---|---|---|---|---|
| A.662 | $C_2H_5$ | Tetrahydropyran-3-yl | $-CH_2CH_2-O-$ | 4-Cl-phenyl | 3,93 (m, 2H), 4,20 (t, 2H), 4,43 (m, 2H), 6,90 (m, 2H), 7,25 (m, 2H) |
| A.663 | n-$C_3H_7$ | Tetrahydropyran-3-yl | $-CH_2CH_2-O-$ | 4-Cl-phenyl | 3,93 (m, 2H), 4,20 (t, 2H), 4,43 (m, 2H), 6,90 (m, 2H), 7,25 (m, 2H) |
| A.664 | $C_2H_5$ | Tetrahydropyran-4-yl | $-CH_2CH_2-O-$ | 4-Cl-phenyl | 116-118 |
| A.665 | n-$C_3H_7$ | Tetrahydropyran-4-yl | $-CH_2CH_2-O-$ | 4-Cl-phenyl | 104-106 |
| A.666 | $C_2H_5$ | Tetrahydrothiopyran-3-yl | $-CH_2CH_2-O-$ | 4-Cl-phenyl | 74- 77 |
| A.667 | n-$C_3H_7$ | Tetrahydrothiopyran-3-yl | $-CH_2CH_2-O-$ | 4-Cl-phenyl | 86- 88 |
| A.668 | $C_2H_5$ | Tetrahydropyran-3-yl | $-CH_2CH_2-O-$ | 2-$CF_3$-phenyl | |
| A.669 | n-$C_3H_7$ | Tetrahydropyran-3-yl | $-CH_2CH_2-O-$ | 2-$CF_3$-phenyl | |
| A.670 | $C_2H_5$ | Tetrahydropyran-4-yl | $-CH_2CH_2-O-$ | 2-$CF_3$-phenyl | |
| A.671 | n-$C_3H_7$ | Tetrahydropyran-4-yl | $-CH_2CH_2-O-$ | 2-$CF_3$-phenyl | |
| A.672 | $C_2H_5$ | Tetrahydrothiopyran-3-yl | $-CH_2CH_2-O-$ | 2-$CF_3$-phenyl | |
| A.673 | n-$C_3H_7$ | Tetrahydrothiopyran-3-yl | $-CH_2CH_2-O-$ | 2-$CF_3$-phenyl | |
| A.674 | $C_2H_5$ | Tetrahydropyran-3-yl | $-CH_2CH_2-O-$ | 3-$CF_3$-phenyl | |
| A.675 | n-$C_3H_7$ | Tetrahydropyran-3-yl | $-CH_2CH_2-O-$ | 3-$CF_3$-phenyl | |
| A.676 | $C_2H_5$ | Tetrahydropyran-4-yl | $-CH_2CH_2-O-$ | 3-$CF_3$-phenyl | |
| A.677 | n-$C_3H_7$ | Tetrahydropyran-4-yl | $-CH_2CH_2-O-$ | 3-$CF_3$-phenyl | |
| A.678 | $C_2H_5$ | Tetrahydrothiopyran-3-yl | $-CH_2CH_2-O-$ | 3-$CF_3$-phenyl | |
| A.679 | n-$C_3H_7$ | Tetrahydrothiopyran-3-yl | $-CH_2CH_2-O-$ | 3-$CF_3$-phenyl | |
| A.680 | $C_2H_5$ | Tetrahydropyran-3-yl | $-CH_2CH_2-O-$ | 4-$CF_3$-phenyl | 72- 77 |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.681 | $n-C_3H_7$ | Tetrahydropyran-3-yl | $-CH_2CH_2-O-$ | $4-CF_3$-phenyl | 3,90 (m,2H), 4,27 (t,2H), 4,47 (m,2H) 7,00 (d,2H), 7,55 (d,2H) |
| A.682 | $C_2H_5$ | Tetrahydropyran-4-yl | $-CH_2CH_2-O-$ | $4-CF_3$-phenyl | |
| A.683 | $n-C_3H_7$ | Tetrahydropyran-4-yl | $-CH_2CH_2-O-$ | $4-CF_3$-phenyl | 90- 94 |
| A.684 | $C_2H_5$ | Tetrahydrothiopyran-3-yl | $-CH_2CH_2-O-$ | $4-CF_3$-phenyl | 73- 79 |
| A.685 | $n-C_3H_7$ | Tetrahydrothiopyran-3-yl | $-CH_2CH_2-O-$ | $4-CF_3$-phenyl | 4,27 (t,2H), 4,47 (m,2H), 7,00 (d,2H) 7,55 (d,2H) |
| A.686 | $C_2H_5$ | Tetrahydropyran-3-yl | $-CH_2CH_2-O-$ | $2,4-Cl_2$-phenyl | 73- 75 |
| A.687 | $n-C_3H_7$ | Tetrahydropyran-3-yl | $-CH_2CH_2-O-$ | $2,4-Cl_2$-phenyl | 69- 73 |
| A.688 | $C_2H_5$ | Tetrahydropyran-4-yl | $-CH_2CH_2-O-$ | $2,4-Cl_2$-phenyl | 4,00 (m,2H), 4,25 (t,2H), 4,45 (t,2H) 6,87 (d,1H), 7,17 (d,1H), 7,37 (d,1H) |
| A.689 | $n-C_3H_7$ | Tetrahydropyran-4-yl | $-CH_2CH_2-O-$ | $2,4-Cl_2$-phenyl | 4,00 (m,2H), 4,25 (t,2H), 4,45 (t,2H) 6,87 (d,1H), 7,17 (d,1H), 7,37 (d,1H) |
| A.690 | $C_2H_5$ | Tetrahydrothiopyran-3-yl | $-CH_2CH_2-O-$ | $2,4-Cl_2$-phenyl | 4,25 (t,2H), 4,45 (t,2H), 6,87 (d,1H) 7,17 (d,1H), 7,37 (d,1H) |
| A.691 | $n-C_3H_7$ | Tetrahydrothiopyran-3-yl | $-CH_2CH_2-O-$ | $2,4-Cl_2$-phenyl | 4,25 (t,2H), 4,45 (t,2H), 6,87 (d,1H) 7,17 (d,1H), 7,37 (d,1H) |
| A.692 | $C_2H_5$ | Tetrahydropyran-3-yl | $-CH_2CH_2-O-$ | $2,4,6-Cl_3$-phenyl | 90- 93 |
| A.693 | $n-C_3H_7$ | Tetrahydropyran-3-yl | $-CH_2CH_2-O-$ | $2,4,6-Cl_3$-phenyl | 83- 87 |
| A.694 | $C_2H_5$ | Tetrahydropyran-4-yl | $-CH_2CH_2-O-$ | $2,4,6-Cl_3$-phenyl | 79- 82 |
| A.695 | $n-C_3H_7$ | Tetrahydropyran-4-yl | $-CH_2CH_2-O-$ | $2,4,6-Cl_3$-phenyl | 4,00 (m,2H), 4,27 (t,2H), 4,45 (m,2H), 7,32 (s,2H) |
| A.696 | $C_2H_5$ | Tetrahydrothiopyran-3-yl | $-CH_2CH_2-O-$ | $2,4,6-Cl_3$-phenyl | 105-108 |
| A.697 | $n-C_3H_7$ | Tetrahydrothiopyran-3-yl | $-CH_2CH_2-O-$ | $2,4,6-Cl_3$-phenyl | 4,27 (t,2H), 4,45 (m,2H), 7,82 (s,2H) |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.698 | $C_2H_5$ | Tetrahydropyran-3-yl | $-CH_2CH_2-O-$ | 4-$NO_2$-phenyl | 3,90 (m, 2H), 4,32 (m, 2H), 4,50 (m, 2H), 7,00 (d, 2H), 8,20 (d, 2H) |
| A.699 | n-$C_3H_7$ | Tetrahydropyran-3-yl | $-CH_2CH_2-O-$ | 4-$NO_2$-phenyl | 3,90 (m, 2H), 4,32 (m, 2H), 4,50 (m, 2H) 7,00 (d, 2H), 8,20 (d, 2H) |
| A.700 | $C_2H_5$ | Tetrahydropyran-4-yl | $-CH_2CH_2-O-$ | 4-$NO_2$-phenyl | 126-129 |
| A.701 | n-$C_3H_7$ | Tetrahydropyran-4-yl | $-CH_2CH_2-O-$ | 4-$NO_2$-phenyl | 138-141 |
| A.702 | $C_2H_5$ | Tetrahydrothiopyran-3-yl | $-CH_2CH_2-O-$ | 4-$NO_2$-phenyl | 4,32 (m, 2H), 4,50 (m, 2H), 7,00 (d, 2H), 8,20 (d, 2H) |
| A.703 | n-$C_3H_7$ | Tetrahydrothiopyran-3-yl | $-CH_2CH_2-O-$ | 4-$NO_2$-phenyl | 4,32 (m, 2H), 4,50 (m, 2H), 7,00 (d, 2H) 8,20 (d, 2H) |
| A.704 | $C_2H_5$ | Tetrahydropyran-3-yl | $-CH_2CH(CH_3)-O-$ | Phenyl | |
| A.705 | n-$C_3H_7$ | Tetrahydropyran-3-yl | $-CH_2CH(CH_3)-O-$ | Phenyl | |
| A.706 | $C_2H_5$ | Tetrahydropyran-4-yl | $-CH_2CH(CH_3)-O-$ | Phenyl | |
| A.707 | n-$C_3H_7$ | Tetrahydropyran-4-yl | $-CH_2CH(CH_3)-O-$ | Phenyl | |
| A.708 | $C_2H_5$ | Tetrahydrothiopyran-3-yl | $-CH_2CH(CH_3)-O-$ | Phenyl | |
| A.709 | n-$C_3H_7$ | Tetrahydrothiopyran-3-yl | $-CH_2CH(CH_3)-O-$ | Phenyl | |
| A.710 | $C_2H_5$ | Tetrahydropyran-3-yl | $-CH_2CH(CH_3)-O-$ | 4-F-phenyl | |
| A.711 | n-$C_3H_7$ | Tetrahydropyran-3-yl | $-CH_2CH(CH_3)-O-$ | 4-F-phenyl | |
| A.712 | $C_2H_5$ | Tetrahydropyran-4-yl | $-CH_2CH(CH_3)-O-$ | 4-F-phenyl | |
| A.713 | n-$C_3H_7$ | Tetrahydropyran-4-yl | $-CH_2CH(CH_3)-O-$ | 4-F-phenyl | |
| A.714 | $C_2H_5$ | Tetrahydrothiopyran-3-yl | $-CH_2CH(CH_3)-O-$ | 4-F-phenyl | |
| A.715 | n-$C_3H_7$ | Tetrahydrothiopyran-3-yl | $-CH_2CH(CH_3)-O-$ | 4-F-phenyl | |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.716 | $C_2H_5$ | Tetrahydropyran-3-yl | $-CH_2CH(CH_3)-O-$ | 4-Cl-phenyl | |
| A.717 | $n-C_3H_7$ | Tetrahydropyran-3-yl | $-CH_2CH(CH_3)-O-$ | 4-Cl-phenyl | |
| A.718 | $C_2H_5$ | Tetrahydropyran-4-yl | $-CH_2CH(CH_3)-O-$ | 4-Cl-phenyl | 1,35 (m, 3H), 4,05-4,30 (m, 2H), 4,60 (m, 1H), 6,80-7,40 (m, 4H) |
| A.719 | $n-C_3H_7$ | Tetrahydropyran-4-yl | $-CH_2CH(CH_3)-O-$ | 4-Cl-phenyl | 1,35 (m, 3H), 4,05-4,30 (m, 2H), 4,60 (m, 1H), 6,80-7,40 (m, 4H) |
| A.720 | $C_2H_5$ | Tetrahydrothiopyran-3-yl | $-CH_2CH(CH_3)-O-$ | 4-Cl-phenyl | 1,35 (m, 3H), 4,05-4,25 (m, 2H), 4,60 (m, 1H), 6,80-7,40 (m, 4H) |
| A.721 | $n-C_3H_7$ | Tetrahydrothiopyran-3-yl | $-CH_2CH(CH_3)-O-$ | 4-Cl-phenyl | 1,35 (m, 3H), 4,05-4,30 (m, 2H), 4,60 (m, 1H), 6,80-7,40 (m, 4H) |
| A.722 | $C_2H_5$ | Tetrahydropyran-3-yl | $-CH_2CH_2-S-$ | Phenyl | 3,90 (m, 2H), 4,23 (t, 2H), 7,17-7,43 (m, 5H) |
| A.723 | $n-C_3H_7$ | Tetrahydropyran-3-yl | $-CH_2CH_2-S-$ | Phenyl | 65 |
| A.724 | $C_2H_5$ | Tetrahydropyran-4-yl | $-CH_2CH_2-S-$ | Phenyl | 3,97 (m, 2H), 4,23 (t, 2H), 7,17-7,43 (m, 5H) |
| A.725 | $n-C_3H_7$ | Tetrahydropyran-4-yl | $-CH_2CH_2-S-$ | Phenyl | 3,97 (m, 2H), 4,23 (t, 2H), 7,17-7,43 (m, 5H) |
| A.726 | $C_2H_5$ | Tetrahydrothiopyran-3-yl | $-CH_2CH_2-S-$ | Phenyl | 4,23 (t, 2H), 7,17-7,43 (m, 5H) |
| A.727 | $n-C_3H_7$ | Tetrahydrothiopyran-3-yl | $-CH_2CH_2-S-$ | Phenyl | 4,23 (t, 2H), 7,17-7,43 (m, 5H) |
| A.728 | $C_2H_5$ | Tetrahydropyran-3-yl | $-CH_2CH_2-S-$ | 4-F-phenyl | 3,90 (m, 2H), 4,17 (t, 2H), 7,00 (m, 2H), 7,40 (m, 2H) |
| A.729 | $n-C_3H_7$ | Tetrahydropyran-3-yl | $-CH_2CH_2-S-$ | 4-F-phenyl | 3,90 (m, 2H), 4,17 (t, 2H), 7,00 (m, 2H), 7,40 (m, 2H) |
| A.730 | $C_2H_5$ | Tetrahydropyran-4-yl | $-CH_2CH_2-S-$ | 4-F-phenyl | 4,00 (m, 2H), 4,17 (t, 2H), 7,00 (m, 2H), 7,40 (m, 2H) |
| A.731 | $n-C_3H_7$ | Tetrahydropyran-4-yl | $-CH_2CH_2-S-$ | 4-F-phenyl | 4,00 (m, 2H), 4,17 (t, 2H), 7,00 (m, 2H), 7,40 (m, 2H) |

Tabelle II.7 (Fortsetzung)

| Nr. | $R^a$ | $R^c$ | W | $R^f$ | phys. Daten / $^1H$-NMR [$\delta$ in ppm], Fp. [°C] |
|---|---|---|---|---|---|
| A.732 | $C_2H_5$ | Tetrahydrothiopyran-3-yl | $-CH_2CH_2-S-$ | 4-F-phenyl | 4,17 (t,2H), 7,00 (m,2H), 7,40 (m,2H), |
| A.733 | n-$C_3H_7$ | Tetrahydrothiopyran-3-yl | $-CH_2CH_2-S-$ | 4-F-phenyl | 4,17 (t,2H), 7,00 (m,2H), 7,40 (m,2H), |
| A.734 | $C_2H_5$ | Tetrahydropyran-3-yl | $-CH_2CH_2-S-$ | 4-Cl-phenyl | 71- 75 |
| A.735 | n-$C_3H_7$ | Tetrahydropyran-3-yl | $-CH_2CH_2-S-$ | 4-Cl-phenyl | 63- 65 |
| A.736 | $C_2H_5$ | Tetrahydropyran-4-yl | $-CH_2CH_2-S-$ | 4-Cl-phenyl | 4,00 (m,2H), 4,20 (t,2H), 7,30 (m,4H) |
| A.737 | n-$C_3H_7$ | Tetrahydropyran-4-yl | $-CH_2CH_2-S-$ | 4-Cl-phenyl | 4,00 (m,2H), 4,20 (t,2H), 7,30 (m,4H) |
| A.738 | $C_2H_5$ | Tetrahydrothiopyran-3-yl | $-CH_2CH_2-S-$ | 4-Cl-phenyl | 4,20 (t,2H), 7,30 (m,4H) |
| A.739 | n-$C_3H_7$ | Tetrahydrothiopyran-3-yl | $-CH_2CH_2-S-$ | 4-Cl-phenyl | 4,20 (t,2H), 7,30 (m,4H) |
| A.740 | $C_2H_5$ | Tetrahydropyran-3-yl | $-CH_2CH_2-S-$ | 2-Cl-phenyl | 3,90 (m,2H), 4,25 (t,2H), 7,10-7,50 (m,4H) |
| A.741 | n-$C_3H_7$ | Tetrahydropyran-3-yl | $-CH_2CH_2-S-$ | 2-Cl-phenyl | 3,90 (m,2H), 4,25 (t,2H), 7,10-7,50 (m,4H) |
| A.742 | $C_2H_5$ | Tetrahydropyran-4-yl | $-CH_2CH_2-S-$ | 2-Cl-phenyl | 4,00 (m,2H), 4,25 (t,2H), 7,10-7,50 (m,4H) |
| A.743 | n-$C_3H_7$ | Tetrahydropyran-4-yl | $-CH_2CH_2-S-$ | 2-Cl-phenyl | 4,00 (m,2H), 4,25 (t,2H), 7,10-7,50 (m,4H) |
| A.744 | $C_2H_5$ | Tetrahydrothiopyran-3-yl | $-CH_2CH_2-S-$ | 2-Cl-phenyl | 4,25 (t,2H) 7,10-7,50 (m,4H) |
| A.745 | n-$C_3H_7$ | Tetrahydrothiopyran-3-yl | $-CH_2CH_2-S-$ | 2-Cl-phenyl | 4,25 (t,2H) 7,10-7,50 (m,4H) |
| A.746 | $C_2H_5$ | Tetrahydropyran-3-yl | $-CH_2CH_2-S-$ | 2,6-$Cl_2$-phenyl | 3,90 (m,2H) 4,20 (t,2H), 7,20 (t,1H) 7,40 (d,2H) |
| A.747 | n-$C_3H_7$ | Tetrahydropyran-3-yl | $-CH_2CH_2-S-$ | 2,6-$Cl_2$-phenyl | 3,90 (m,2H) 4,20 (t,2H), 7,20 (t,1H) 7,40 (d,2H) |
| A.748 | $C_2H_5$ | Tetrahydropyran-4-yl | $-CH_2CH_2-S-$ | 2,6-$Cl_2$-phenyl | 61- 64 |
| A.749 | n-$C_3H_7$ | Tetrahydropyran-4-yl | $-CH_2CH_2-S-$ | 2,6-$Cl_2$-phenyl | 4,00 (m,2H) 4,20 (t,2H), 7,20 (t,1H) 7,40 (d,2H) |

Tabelle II.7 (Fortsetzung)

| Nr. | $R^a$ | $R^c$ | W | $R^f$ | phys. Daten / $^1H$-NMR [δ in ppm], Fp. [°C] |
|---|---|---|---|---|---|
| A.750 | $C_2H_5$ | Tetrahydrothiopyran-3-yl | $-CH_2CH_2-S-$ | 2,6-$Cl_2$-phenyl | 4,20 (t,2H) 7,20 (t,2H), 7,40 (d,2H) |
| A.751 | n-$C_3H_7$ | Tetrahydrothiopyran-3-yl | $-CH_2CH_2-S-$ | 2,6-$Cl_2$-phenyl | 4,20 (t,2H) 7,20 (t,2H), 7,40 (d,2H) |
| A.752 | $C_2H_5$ | Tetrahydropyran-3-yl | $-(CH_2)_3-O-$ | Phenyl | 3,90 (m,2H), 4,03 (t,2H), 4,23 (t,2H), 6,90 (m,3H), 7,27 (m,2H) |
| A.753 | n-$C_3H_7$ | Tetrahydropyran-3-yl | $-(CH_2)_3-O-$ | Phenyl | 3,90 (m,2H), 4,03 (t,2H), 4,23 (t,2H), 6,90 (m,3H), 7,27 (m,2H) |
| A.754 | $C_2H_5$ | Tetrahydropyran-4-yl | $-(CH_2)_3-O-$ | Phenyl | 3,97 (m,2H), 4,03 (t,2H), 4,23 (t,2H), 6,90 (m,3H), 7,27 (m,2H) |
| A.755 | n-$C_3H_7$ | Tetrahydropyran-4-yl | $-(CH_2)_3-O-$ | Phenyl | 3,97 (m,2H), 4,03 (t,2H), 4,23 (t,2H), 6,90 (m,3H), 7,27 (m,2H) |
| A.756 | $C_2H_5$ | Tetrahydrothiopyran-3-yl | $-(CH_2)_3-O-$ | Phenyl | 4,03 (t,2H), 4,23 (t,2H), 6,90 (m,3H), 7,27 (m,2H) |
| A.757 | n-$C_3H_7$ | Tetrahydrothiopyran-3-yl | $-(CH_2)_3-O-$ | Phenyl | 4,03 (t,2H), 4,23 (t,2H), 6,90 (m,3H), 7,27 (m,2H) |
| A.758 | $C_2H_5$ | Tetrahydropyran-3-yl | $-(CH_2)_3-O-$ | 2-F-phenyl | 3,90 (m,2H), 4,10 (t,2H), 4,27 (t,2H), 6,80-7,15 (m,4H) |
| A.759 | n-$C_3H_7$ | Tetrahydropyran-3-yl | $-(CH_2)_3-O-$ | 2-F-phenyl | 3,90 (m,2H), 4,10 (t,2H), 4,27 (t,2H), 6,80-7,15 (m,4H) |
| A.760 | $C_2H_5$ | Tetrahydropyran-4-yl | $-(CH_2)_3-O-$ | 2-F-phenyl | 4,00 (m,2H), 4,10 (t,2H), 4,27 (t,2H), 6,80-7,15 (m,4H) |
| A.761 | n-$C_3H_7$ | Tetrahydropyran-4-yl | $-(CH_2)_3-O-$ | 2-F-phenyl | 76- 80 |
| A.762 | $C_2H_5$ | Tetrahydrothiopyran-3-yl | $-(CH_2)_3-O-$ | 2-F-phenyl | 4,10 (t,2H), 4,27 (t,2H), 6,80-7,15(m,4H), |
| A.763 | n-$C_3H_7$ | Tetrahydrothiopyran-3-yl | $-(CH_2)_3-O-$ | 2-F-phenyl | 4,10 (t,2H), 4,27 (t,2H), 6,80-7,15(m,4H), |
| A.764 | $C_2H_5$ | Tetrahydropyran-3-yl | $-(CH_2)_3-O-$ | 3-F-phenyl | 3,90 (m,2H), 4,05 (t,2H), 4,27 (t,2H), 6,67 (m,3H), 7,23 (m,1H) |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.765 | $n-C_3H_7$ | Tetrahydropyran-3-yl | $-(CH_2)_3-O-$ | 3-F-phenyl | 3,90 (m,2H), 4,05 (t,2H), 4,27 (t,2H), 6,67 (m,3H), 7,23 (m,1H) |
| A.766 | $C_2H_5$ | Tetrahydropyran-4-yl | $-(CH_2)_3-O-$ | 3-F-phenyl | 3,90-4,10 (m,4H), 4,27 (t,2H), 6,67 (m,3H), 7,23 (m,1H) |
| A.767 | $n-C_3H_7$ | Tetrahydropyran-4-yl | $-(CH_2)_3-O-$ | 3-F-phenyl | 3,90-4,10 (m,4H), 4,27 (t,2H), 6,67 (m,3H), 7,23 (m,1H) |
| A.768 | $C_2H_5$ | Tetrahydrothiopyran-3-yl | $-(CH_2)_3-O-$ | 3-F-phenyl | 4,05 (t,2H), 4,27 (t,2H), 6,67 (m,3H) 7,23 (m,1H) |
| A.769 | $n-C_3H_7$ | Tetrahydrothiopyran-3-yl | $-(CH_2)_3-O-$ | 3-F-phenyl | 4,05 (t,2H), 4,27 (t,2H), 6,67 (m,3H) 7,23 (m,1H) |
| A.770 | $C_2H_5$ | Tetrahydropyran-3-yl | $-(CH_2)_3-O-$ | 4-F-phenyl | 3,90 (m,2H), 4,03 (t,2H), 4,27 (t,2H), 6,90 (m,2H), 7,00 (m,2H) |
| A.771 | $n-C_3H_7$ | Tetrahydropyran-3-yl | $-(CH_2)_3-O-$ | 4-F-phenyl | 3,90 (m,2H), 4,03 (t,2H), 4,27 (t,2H), 6,90 (m,2H), 7,00 (m,2H) |
| A.772 | $C_2H_5$ | Tetrahydropyran-4-yl | $-(CH_2)_3-O-$ | 4-F-phenyl | 3,90-4,06 (m,4H), 4,23 (t,2H), 6,90 (m,2H), 7,00 (m,2H) |
| A.773 | $n-C_3H_7$ | Tetrahydropyran-4-yl | $-(CH_2)_3-O-$ | 4-F-phenyl | 3,90-4,06 (m,4H), 4,28 (t,2H), 6,90 (m,2H), 7,00 (m,2H) |
| A.774 | $C_2H_5$ | Tetrahydrothiopyran-3-yl | $-(CH_2)_3-O-$ | 4-F-phenyl | 4,03 (t,2H), 4,27 (t,2H), 6,90 (m,2H), 7,00 (m,2H) |
| A.775 | $n-C_3H_7$ | Tetrahydrothiopyran-3-yl | $-(CH_2)_3-O-$ | 4-F-phenyl | 4,03 (t,2H), 4,27 (t,2H), 6,90 (m,2H), 7,00 (m,2H) |
| A.776 | $C_2H_5$ | Tetrahydropyran-3-yl | $-(CH_2)_3-O-$ | 2-Cl-phenyl | |
| A.777 | $n-C_3H_7$ | Tetrahydropyran-3-yl | $-(CH_2)_3-O-$ | 2-Cl-phenyl | |
| A.778 | $C_2H_5$ | Tetrahydropyran-4-yl | $-(CH_2)_3-O-$ | 2-Cl-phenyl | |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.779 | $n-C_3H_7$ | Tetrahydropyran-4-yl | $-(CH_2)_3-O-$ | 2-Cl-phenyl | |
| A.780 | $C_2H_5$ | Tetrahydrothiopyran-3-yl | $-(CH_2)_3-O-$ | 2-Cl-phenyl | |
| A.781 | $n-C_3H_7$ | Tetrahydrothiopyran-3-yl | $-(CH_2)_3-O-$ | 2-Cl-phenyl | |
| A.782 | $C_2H_5$ | Tetrahydropyran-3-yl | $-(CH_2)_3-O-$ | 3-Cl-phenyl | 3,90 (m,2H), 4,06 (t,2H), 4,27 (t,2H), 6,77 (m,1H), 6,90 (m,2H), 7,17 (m,1H) |
| A.783 | $n-C_3H_7$ | Tetrahydropyran-3-yl | $-(CH_2)_3-O-$ | 3-Cl-phenyl | 3,90 (m,2H), 4,06 (t,2H), 4,27 (t,2H), 6,77 (m,1H), 6,90 (m,2H), 7,17 (m,1H) |
| A.784 | $C_2H_5$ | Tetrahydropyran-4-yl | $-(CH_2)_3-O-$ | 3-Cl-phenyl | 3,90-4,10 (m,4H), 4,27 (t,2H), 6,77 (m,1H), 6,90 (m,2H), 7,17 (m,1H) |
| A.785 | $n-C_3H_7$ | Tetrahydropyran-4-yl | $-(CH_2)_3-O-$ | 3-Cl-phenyl | 3,90-4,10 (m,4H), 4,27 (t,2H), 6,77 (m,1H), 6,90 (m,2H), 7,17 (m,1H) |
| A.786 | $C_2H_5$ | Tetrahydrothiopyran-3-yl | $-(CH_2)_3-O-$ | 3-Cl-phenyl | 4,06 (t,2H), 4,27 (t,2H), 6,77 (m,1H), 6,90 (m,2H), 7,17 (m,1H) |
| A.787 | $n-C_3H_7$ | Tetrahydrothiopyran-3-yl | $-(CH_2)_3-O-$ | 3-Cl-phenyl | 4,06 (t,2H), 4,27 (t,2H), 6,77 (m,1H), 6,90 (m,2H), 7,17 (m,1H) |
| A.788 | $C_2H_5$ | Tetrahydropyran-3-yl | $-(CH_2)_3-O-$ | 4-Cl-phenyl | 3,90 (m,2H), 4,03 (t,2H), 4,23 (t,2H), 6,80 (m,2H), 7,20 (m,2H) |
| A.789 | $n-C_3H_7$ | Tetrahydropyran-3-yl | $-(CH_2)_3-O-$ | 4-Cl-phenyl | 3,90 (m,2H), 4,03 (t,2H), 4,23 (t,2H), 6,80 (m,2H), 7,20 (m,2H) |
| A.790 | $C_2H_5$ | Tetrahydropyran-4-yl | $-(CH_2)_3-O-$ | 4-Cl-phenyl | 3,90-4,09 (m,4H), 4,23 (t,2H), 6,80 (m,2H), 7,20 (m,2H) |
| A.791 | $n-C_3H_7$ | Tetrahydropyran-4-yl | $-(CH_2)_3-O-$ | 4-Cl-phenyl | 3,90-4,09 (m,4H), 4,23 (t,2H), 6,80 (m,2H), 7,20 (m,2H) |
| A.792 | $C_2H_5$ | Tetrahydrothiopyran-3-yl | $-(CH_2)_3-O-$ | 4-Cl-phenyl | 4,03 (t,2H), 4,23 (t,2H), 6,80 (m,2H), 7,20 (m,2H) |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.793 | n-$C_3H_7$ | Tetrahydrothiopyran-3-yl | $-(CH_2)_3-O-$ | 4-Cl-phenyl | 4,03 (t,2H), 4,23 (t,2H), 6,80 (m,2H), 7,20 (m,2H) |
| A.794 | $C_2H_5$ | Tetrahydropyran-3-yl | $-(CH_2)_3-O-$ | 4-$NO_2$-phenyl | 3,90 (m,2H), 4,20 (t,2H), 4,28 (t,2H), 6,93 (d,2H), 8,20 (d,H) |
| A.795 | n-$C_3H_7$ | Tetrahydropyran-3-yl | $-(CH_2)_3-O-$ | 4-$NO_2$-phenyl | 3,90 (m,2H), 4,20 (t,2H), 4,28 (t,2H), 6,93 (d,2H), 8,20 (d,2H) |
| A.796 | $C_2H_5$ | Tetrahydropyran-4-yl | $-(CH_2)_3-O-$ | 4-$NO_2$-phenyl | 4,00 (m,2H), 4,20 (t,2H), 4,28 (t,2H), 6,93 (d,2H), 8,20 (d,2H) |
| A.797 | n-$C_3H_7$ | Tetrahydropyran-4-yl | $-(CH_2)_3-O-$ | 4-$NO_2$-phenyl | 4,00 (m,2H), 4,20 (t,2H), 4,28 (t,2H), 6,93 (d,2H), 8,20 (d,2H) |
| A.798 | $C_2H_5$ | Tetrahydrothiopyran-3-yl | $-(CH_2)_3-O-$ | 4-$NO_2$-phenyl | 4,20 (t,2H), 4,28 (t,2H), 6,93 (d,2H), 8,20 (d,2H) |
| A.799 | n-$C_3H_7$ | Tetrahydrothiopyran-3-yl | $-(CH_2)_3-O-$ | 4-$NO_2$-phenyl | 4,20 (t,2H), 4,28 (t,2H), 6,93 (d,2H), 8,20 (d,2H) |
| A.800 | $C_2H_5$ | Tetrahydropyran-3-yl | $-(CH_2)_3-O-$ | 4-Br-phenyl | 3,90 (m,2H), 4,00 (t,2H), 4,27 (t,2H), 6,80 (d,2H), 7,37 (d,2H) |
| A.801 | n-$C_3H_7$ | Tetrahydropyran-3-yl | $-(CH_2)_3-O-$ | 4-Br-phenyl | 3,90 (m,2H), 4,00 (t,2H), 4,27 (t,2H), 6,80 (d,2H), 7,37 (d,2H) |
| A.802 | $C_2H_5$ | Tetrahydropyran-4-yl | $-(CH_2)_3-O-$ | 4-Br-phenyl | 3,90-4,10 (m,4H), 4,27 (t,2H), 6,80 (d,2H), 7,37 (d,2H) |
| A.803 | n-$C_3H_7$ | Tetrahydropyran-4-yl | $-(CH_2)_3-O-$ | 4-Br-phenyl | 3,90-4,10 (m,4H), 4,27 (t,2H), 6,80 (d,2H), 7,37 (d,2H) |
| A.804 | $C_2H_5$ | Tetrahydrothiopyran-4-yl | $-(CH_2)_3-O-$ | 4-Br-phenyl | 4,00 (t,2H), 4,27 (t,2H), 6,80 (d,2H), 7,37 (d,2H) |
| A.805 | n-$C_3H_7$ | Tetrahydrothiopyran-4-yl | $-(CH_2)_3-O-$ | 4-Br-phenyl | 4,00 (t,2H), 4,27 (t,2H), 6,80 (d,2H), 7,37 (d,2H) |

Tabelle II.7 (Fortsetzung)

| Nr. | $R^a$ | $R^c$ | W | $R^f$ | phys. Daten / $^1H$-NMR [δ in ppm], Fp. [°C] |
|---|---|---|---|---|---|
| A.806 | $C_2H_5$ | Tetrahydropyran-3-yl | $-(CH_2)_3-S-$ | Phenyl | 3,90 (m, 2H), 4,17 (t, 2H), 7,10-7,40 (m, 5H) |
| A.807 | n-$C_3H_7$ | Tetrahydropyran-3-yl | $-(CH_2)_3-S-$ | Phenyl | 3,90 (m, 2H), 4,17 (t, 2H), 7,10-7,40 (m, 5H) |
| A.808 | $C_2H_5$ | Tetrahydropyran-4-yl | $-(CH_2)_3-S-$ | Phenyl | 4,00 (m, 2H), 4,17 (t, 2H), 7,10-7,40 (m, 5H) |
| A.809 | n-$C_3H_7$ | Tetrahydropyran-4-yl | $-(CH_2)_3-S-$ | Phenyl | 4,00 (m, 2H), 4,17 (t, 2H), 7,10-7,40 (m, 5H) |
| A.810 | $C_2H_5$ | Tetrahydrothiopyran-3-yl | $-(CH_2)_3-S-$ | Phenyl | 4,17 (t, 2H), 7,10-7,40 (m, 5H) |
| A.811 | n-$C_3H_7$ | Tetrahydrothiopyran-3-yl | $-(CH_2)_3-S-$ | Phenyl | 4,17 (t, 2H), 7,10-7,40 (m, 5H) |
| A.812 | $C_2H_5$ | Tetrahydropyran-3-yl | $-(CH_2)_3-S-$ | 4-F-phenyl | 3,90 (m, 2H), 7,33 (m, 2H), 4,17 (t, 2H), 7,00 (t, 2H), |
| A.813 | n-$C_3H_7$ | Tetrahydropyran-3-yl | $-(CH_2)_3-S-$ | 4-F-phenyl | 3,90 (m, 2H), 7,33 (m, 2H), 4,17 (t, 2H), 7,00 (t, 2H), |
| A.814 | $C_2H_5$ | Tetrahydropyran-4-yl | $-(CH_2)_3-S-$ | 4-F-phenyl | 4,00 (m, 2H), 7,33 (m, 2H), 4,17 (t, 2H), 7,00 (t, 2H), |
| A.815 | n-$C_3H_7$ | Tetrahydropyran-4-yl | $-(CH_2)_3-S-$ | 4-F-phenyl | 4,00 (m, 2H), 7,33 (m, 2H), 4,17 (t, 2H), 7,00 (t, 2H), |
| A.816 | $C_2H_5$ | Tetrahydrothiopyran-3-yl | $-(CH_2)_3-S-$ | 4-F-phenyl | 4,17 (t, 2H), 7,00 (t, 2H), 7,33 (m, 2H) |
| A.817 | n-$C_3H_7$ | Tetrahydrothiopyran-3-yl | $-(CH_2)_3-S-$ | 4-F-phenyl | 4,17 (t, 2H), 7,00 (t, 2H), 7,33 (m, 2H) |
| A.818 | $C_2H_5$ | Tetrahydropyran-3-yl | $-(CH_2)_3-S-$ | 4-Cl-phenyl | 3,90 (m, 2H), 4,17 (t, 2H), 7,27 (s, 4H) |
| A.819 | n-$C_3H_7$ | Tetrahydropyran-3-yl | $-(CH_2)_3-S-$ | 4-Cl-phenyl | 3,90 (m, 2H), 4,17 (t, 2H), 7,27 (s, 4H) |
| A.820 | $C_2H_5$ | Tetrahydropyran-4-yl | $-(CH_2)_3-S-$ | 4-Cl-phenyl | 4,00 (m, 2H), 4,17 (t, 2H), 7,27 (s, 4H) |
| A.821 | n-$C_3H_7$ | Tetrahydropyran-4-yl | $-(CH_2)_3-S-$ | 4-Cl-phenyl | 4,00 (m, 2H), 4,17 (t, 2H), 7,27 (s, 4H) |
| A.822 | $C_2H_5$ | Tetrahydrothiopyran-3-yl | $-(CH_2)_3-S-$ | 4-Cl-phenyl | 4,17 (t, 2H), 7,27 (s, 4H) |
| A.823 | n-$C_3H_7$ | Tetrahydrothiopyran-3-yl | $-(CH_2)_3-S-$ | 4-Cl-phenyl | 4,17 (t, 2H), 7,27 (s, 4H) |

Tabelle II.7 (Fortsetzung)

| Nr. | $R^a$ | $R^c$ | W | $R^f$ | phys. Daten / $^1H$-NMR [δ in ppm], Fp. [°C] |
|---|---|---|---|---|---|
| A.824 | $C_2H_5$ | Tetrahydropyran-3-yl | $-(CH_2)_3-S-$ | 2-Cl-phenyl | 3,90 (m, 2H), 4,20 (t, 2H), 7,07-7,40 (m, 4H) |
| A.825 | n-$C_3H_7$ | Tetrahydropyran-3-yl | $-(CH_2)_3-S-$ | 2-Cl-phenyl | 3,90 (m, 2H), 4,20 (t, 2H), 7,07-7,40 (m, 4H) |
| A.826 | $C_2H_5$ | Tetrahydropyran-4-yl | $-(CH_2)_3-S-$ | 2-Cl-phenyl | 4,00 (m, 2H), 4,20 (t, 2H), 7,07-7,40 (m, 4H) |
| A.827 | n-$C_3H_7$ | Tetrahydropyran-4-yl | $-(CH_2)_3-S-$ | 2-Cl-phenyl | 4,00 (m, 2H), 4,20 (t, 2H), 7,07-7,40 (m, 4H) |
| A.828 | $C_2H_5$ | Tetrahydrothiopyran-3-yl | $-(CH_2)_3-S-$ | 2-Cl-phenyl | 4,20 (t, 2H), 7,07-7,40 (m, 4H) |
| A.829 | n-$C_3H_7$ | Tetrahydrothiopyran-3-yl | $-(CH_2)_3-S-$ | 2-Cl-phenyl | 4,20 (t, 2H), 7,07-7,40 (m, 4H) |
| A.830 | $C_2H_5$ | Tetrahydropyran-3-yl | $-(CH_2)_3-S-$ | 3-Cl-phenyl | 3,90 (m, 2H), 7,30 (m, 1H), 4,20 (t, 2H), 7,17 (m, 3H) |
| A.831 | n-$C_3H_7$ | Tetrahydropyran-3-yl | $-(CH_2)_3-S-$ | 3-Cl-phenyl | 3,90 (m, 2H), 7,30 (m, 1H), 4,20 (t, 2H), 7,17 (m, 3H) |
| A.832 | $C_2H_5$ | Tetrahydropyran-4-yl | $-(CH_2)_3-S-$ | 3-Cl-phenyl | 4,00 (m, 2H), 7,30 (m, 1H), 4,20 (t, 2H), 7,17 (m, 3H) |
| A.833 | n-$C_3H_7$ | Tetrahydropyran-4-yl | $-(CH_2)_3-S-$ | 3-Cl-phenyl | 4,00 (m, 2H), 7,30 (m, 1H), 4,20 (t, 2H), 7,17 (m, 3H) |
| A.834 | $C_2H_5$ | Tetrahydrothiopyran-3-yl | $-(CH_2)_3-S-$ | 3-Cl-phenyl | 4,20 (t, 2H), 7,17 (m, 3H), 7,30 (m, 1H) |
| A.835 | n-$C_3H_7$ | Tetrahydrothiopyran-3-yl | $-(CH_2)_3-S-$ | 3-Cl-phenyl | 4,20 (t, 2H), 7,17 (m, 3H), 7,30 (m, 1H) |
| A.836 | $C_2H_5$ | Tetrahydropyran-3-yl | $-(CH_2)_3-S-$ | 2,5-$Cl_2$-phenyl | 3,90 (m, 2H), 7,20 (d, 1H), 4,20 (t, 2H), 7,30 (d, 1H), 7,07 (dd, 1H), |
| A.837 | n-$C_3H_7$ | Tetrahydropyran-3-yl | $-(CH_2)_3-S-$ | 2,5-$Cl_2$-phenyl | 3,90 (m, 2H), 7,20 (d, 1H), 4,20 (t, 2H), 7,30 (d, 1H), 7,07 (dd, 1H), |
| A.838 | $C_2H_5$ | Tetrahydropyran-4-yl | $-(CH_2)_3-S-$ | 2,5-$Cl_2$-phenyl | 4,00 (m, 2H), 7,20 (d, 1H), 4,20 (t, 2H), 7,30 (d, 1H), 7,07 (dd, 1H), |
| A.839 | n-$C_3H_7$ | Tetrahydropyran-4-yl | $-(CH_2)_3-S-$ | 2,5-$Cl_2$-phenyl | 4,00 (m, 2H), 7,20 (d, 1H), 4,20 (t, 2H), 7,30 (d, 1H), 7,07 (dd, 1H), |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.840 | $C_2H_5$ | Tetrahydrothiopyran-3-yl | $-(CH_2)_3-S-$ | 2,5-$Cl_2$-phenyl | 4,20 (t,2H), 7,07 (dd,1H), 7,20 (d,1H) 7,30 (d,1H) |
| A.841 | n-$C_3H_7$ | Tetrahydrothiopyran-3-yl | $-(CH_2)_3-S-$ | 2,5-$Cl_2$-phenyl | 4,20 (t,2H), 7,07 (dd,1H), 7,20 (d,1H) 7,30 (d,1H) |
| A.842 | $C_2H_5$ | Tetrahydropyran-3-yl | $-(CH_2)_3-S-$ | 2,6-$Cl_2$-phenyl | 3,90 (m,2H), 4,20 (t,2H), 7,20 (t,1H) 7,40 (d,2H) |
| A.843 | n-$C_3H_7$ | Tetrahydropyran-3-yl | $-(CH_2)_3-S-$ | 2,6-$Cl_2$-phenyl | 3,90 (m,2H), 4,20 (t,2H), 7,20 (t,1H) 7,40 (d,2H) |
| A.844 | $C_2H_5$ | Tetrahydropyran-4-yl | $-(CH_2)_3-S-$ | 2,6-$Cl_2$-phenyl | 4,00 (m,2H), 4,20 (t,2H), 7,20 (t,1H) 7,40 (d,2H) |
| A.845 | n-$C_3H_7$ | Tetrahydropyran-4-yl | $-(CH_2)_3-S-$ | 2,6-$Cl_2$-phenyl | 4,00 (m,2H), 4,20 (t,2H), 7,20 (t,1H) 7,40 (d,2H) |
| A.846 | $C_2H_5$ | Tetrahydrothiopyran-3-yl | $-(CH_2)_3-S-$ | 2,6-$Cl_2$-phenyl | 4,20 (t,2H), 7,20 (t,1H), 7,40 (d,2H) |
| A.847 | n-$C_3H_7$ | Tetrahydrothiopyran-3-yl | $-(CH_2)_3-S-$ | 2,6-$Cl_2$-phenyl | 4,20 (t,2H), 7,20 (t,1H), 7,40 (d,2H) |
| A.848 | $C_2H_5$ | Tetrahydropyran-3-yl | $-CH_2CH_2OCH_2-$ | Phenyl | 3,90 (m,2H), 4,25 (t,2H), 4,58 (s,2H), 7,38 (s,5H) |
| A.849 | n-$C_3H_7$ | Tetrahydropyran-3-yl | $-CH_2CH_2OCH_2-$ | Phenyl | 3,90 (m,2H), 4,25 (t,2H), 4,58 (s,2H), 7,38 (s,5H) |
| A.850 | $C_2H_5$ | Tetrahydropyran-4-yl | $-CH_2CH_2OCH_2-$ | Phenyl | 4,03 (m,2H), 4,33 (m,2H), 4,60 (s,2H), 7,40 (s,5H) |
| A.851 | n-$C_3H_7$ | Tetrahydropyran-4-yl | $-CH_2CH_2OCH_2-$ | Phenyl | 4,03 (m,2H), 4,33 (m,2H), 4,60 (s,2H), 7,40 (s,5H) |
| A.852 | $C_2H_5$ | Tetrahydrothiopyran-3-yl | $-CH_2CH_2OCH_2-$ | Phenyl | 4,27 (m,2H), 4,57 (s,2H), 7,35 (s,5H) |
| A.853 | n-$C_3H_7$ | Tetrahydrothiopyran-3-yl | $-CH_2CH_2OCH_2-$ | Phenyl | 4,27 (m,2H), 4,57 (s,2H), 7,35 (s,5H) |
| A.854 | $C_2H_5$ | Tetrahydropyran-3-yl | $-CH_2CH_2OCH_2-$ | 2-F-phenyl | 3,93 (m,2H), 4,27 (m,2H), 4,67 (s,2H), 6,93-7,50 (m,4H) |

Tabelle II.7 (Fortsetzung)

| Nr. | $R^a$ | $R^c$ | W | $R^f$ | phys. Daten / $^1H$-NMR [δ in ppm], Fp. [°C] |
|---|---|---|---|---|---|
| A.855 | n-$C_3H_7$ | Tetrahydropyran-3-yl | $-CH_2CH_2OCH_2-$ | 2-F-phenyl | 3,93 (m,2H), 4,27 (m,2H), 4,67 (s,2H), 6,93-7,50 (m,4H) |
| A.856 | $C_2H_5$ | Tetrahydropyran-4-yl | $-CH_2CH_2OCH_2-$ | 2-F-phenyl | 4,03 (m,2H), 4,27 (m,2H), 4,63 (s,2H), 6,97-7,50 (m,4H) |
| A.857 | n-$C_3H_7$ | Tetrahydropyran-4-yl | $-CH_2CH_2OCH_2-$ | 2-F-phenyl | 4,03 (m,2H), 4,27 (m,2H), 4,63 (s,2H), 6,97-7,50 (m,4H) |
| A.858 | $C_2H_5$ | Tetrahydrothiopyran-3-yl | $-CH_2CH_2OCH_2-$ | 2-F-phenyl | 4,27 (m,2H), 4,67 (s,2H), 6,97-7,50 (m,4H) |
| A.859 | n-$C_3H_7$ | Tetrahydrothiopyran-3-yl | $-CH_2CH_2OCH_2-$ | 2-F-phenyl | 4,27 (m,2H), 4,67 (s,2H), 6,97-7,50 (m,4H) |
| A.860 | $C_2H_5$ | Tetrahydropyran-3-yl | $-CH_2CH_2OCH_2-$ | 3-F-phenyl | 3,93 (m,2H), 4,27 (m,2H), 4,57 (s,2H), 6,90-7,15 (m,3H), 7,23-7,40 (m,1H) |
| A.861 | n-$C_3H_7$ | Tetrahydropyran-3-yl | $-CH_2CH_2OCH_2-$ | 3-F-phenyl | 3,93 (m,2H), 4,27 (m,2H), 4,57 (s,2H), 6,90-7,15 (m,3H), 7,23-7,40 (m,1H) |
| A.862 | $C_2H_5$ | Tetrahydropyran-4-yl | $-CH_2CH_2OCH_2-$ | 3-F-phenyl | 4,03 (m,2H), 4,25 (m,2H), 4,60 (s,2H), 6,90-7,18 (m,3H), 7,26-7,40 (m,1H) |
| A.863 | n-$C_3H_7$ | Tetrahydropyran-4-yl | $-CH_2CH_2OCH_2-$ | 3-F-phenyl | 4,03 (m,2H), 4,25 (m,2H), 4,60 (s,2H), 6,90-7,18 (m,3H), 7,26-7,40 (m,1H) |
| A.864 | $C_2H_5$ | Tetrahydrothiopyran-3-yl | $-CH_2CH_2OCH_2-$ | 3-F-phenyl | 4,27 (m,2H), 4,60 (s,2H), 6,90-7,15 (m,3H), 7,23-7,40 (m,1H) |
| A.865 | n-$C_3H_7$ | Tetrahydrothiopyran-3-yl | $-CH_2CH_2OCH_2-$ | 3-F-phenyl | 4,27 (m,2H), 4,60 (s,2H), 6,90-7,15 (m,3H), 7,23-7,40 (m,1H) |
| A.866 | $C_2H_5$ | Tetrahydropyran-3-yl | $-CH_2CH_2OCH_2-$ | 4-F-phenyl | 3,93 (m,2H), 4,23 (m,2H), 4,53 (s,2H), 7,00 (m,2H), 7,30 (m,2H) |
| A.867 | n-$C_3H_7$ | Tetrahydropyran-3-yl | $-CH_2CH_2OCH_2-$ | 4-F-phenyl | 3,93 (m,2H), 4,23 (m,2H), 4,53 (s,2H), 7,00 (m,2H), 7,30 (m,2H) |
| A.868 | $C_2H_5$ | Tetrahydropyran-4-yl | $-CH_2CH_2OCH_2-$ | 4-F-phenyl | 92 |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.869 | $n-C_3H_7$ | Tetrahydropyran-4-yl | $-CH_2CH_2OCH_2-$ | 4-F-phenyl | 4,00 (m, 2H), 4,23 (m, 2H), 4,53 (s, 2H), 7,03 (m, 2H), 7,30 (m, 2H) |
| A.870 | $C_2H_5$ | Tetrahydrothiopyran-3-yl | $-CH_2CH_2OCH_2-$ | 4-F-phenyl | 4,27 (m, 2H), 4,53 (s, 2H), 7,03 (m, 2H), 7,30 (m, 2H) |
| A.871 | $n-C_3H_7$ | Tetrahydrothiopyran-3-yl | $-CH_2CH_2OCH_2-$ | 4-F-phenyl | 4,27 (m, 2H), 4,53 (s, 2H), 7,03 (m, 2H), 7,30 (m, 2H) |
| A.872 | $C_2H_5$ | Tetrahydropyran-3-yl | $-CH_2CH_2OCH_2-$ | 2-Cl-phenyl | |
| A.873 | $n-C_3H_7$ | Tetrahydropyran-3-yl | $-CH_2CH_2OCH_2-$ | 2-Cl-phenyl | |
| A.874 | $C_2H_5$ | Tetrahydropyran-4-yl | $-CH_2CH_2OCH_2-$ | 2-Cl-phenyl | |
| A.875 | $n-C_3H_7$ | Tetrahydropyran-4-yl | $-CH_2CH_2OCH_2-$ | 2-Cl-phenyl | |
| A.876 | $C_2H_5$ | Tetrahydrothiopyran-3-yl | $-CH_2CH_2OCH_2-$ | 2-Cl-phenyl | |
| A.877 | $n-C_3H_7$ | Tetrahydrothiopyran-3-yl | $-CH_2CH_2OCH_2-$ | 2-Cl-phenyl | |
| A.878 | $C_2H_5$ | Tetrahydropyran-3-yl | $-CH_2CH_2OCH_2-$ | 3-Cl-phenyl | |
| A.879 | $n-C_3H_7$ | Tetrahydropyran-3-yl | $-CH_2CH_2OCH_2-$ | 3-Cl-phenyl | |
| A.880 | $C_2H_5$ | Tetrahydropyran-4-yl | $-CH_2CH_2OCH_2-$ | 3-Cl-phenyl | |
| A.881 | $n-C_3H_7$ | Tetrahydropyran-4-yl | $-CH_2CH_2OCH_2-$ | 3-Cl-phenyl | |
| A.882 | $C_2H_5$ | Tetrahydrothiopyran-3-yl | $-CH_2CH_2OCH_2-$ | 3-Cl-phenyl | |
| A.883 | $n-C_3H_7$ | Tetrahydrothiopyran-3-yl | $-CH_2CH_2OCH_2-$ | 3-Cl-phenyl | |
| A.884 | $C_2H_5$ | Tetrahydropyran-3-yl | $-CH_2CH_2OCH_2-$ | 4-Cl-phenyl | 3,93 (m, 2H), 4,27 (m, 2H), 4,53 (s, 2H), 7,28 (m, 4H) |
| A.885 | $n-C_3H_7$ | Tetrahydropyran-3-yl | $-CH_2CH_2OCH_2-$ | 4-Cl-phenyl | 3,93 (m, 2H), 4,27 (m, 2H), 4,53 (s, 2H), 7,28 (m, 4H) |
| A.886 | $C_2H_5$ | Tetrahydropyran-4-yl | $-CH_2CH_2OCH_2-$ | 4-Cl-phenyl | 67- 72 |

Tabelle II.7 (Fortsetzung)

| Nr. | $R^a$ | $R^c$ | W | $R^f$ | phys. Daten / $^1H$-NMR [δ in ppm], Fp. [°C] |
|---|---|---|---|---|---|
| A.887 | $n-C_3H_7$ | Tetrahydropyran-4-yl | $-CH_2CH_2OCH_2-$ | 4-Cl-phenyl | 4,00 (m, 2H), 4,23 (m, 2H), 4,53 (s, 2H), 7,28 (m, 4H) |
| A.888 | $C_2H_5$ | Tetrahydrothiopyran-3-yl | $-CH_2CH_2OCH_2-$ | 4-Cl-phenyl | 4,27 (m, 2H), 4,53 (s, 2H), 7,28 (m, 4H) |
| A.889 | $n-C_3H_7$ | Tetrahydrothiopyran-3-yl | $-CH_2CH_2OCH_2-$ | 4-Cl-phenyl | 4,27 (m, 2H), 4,53 (s, 2H), 7,28 (m, 4H) |
| A.890 | $C_2H_5$ | Tetrahydropyran-3-yl | $-CH_2CH_2OCH_2-$ | $2-CH_3$-phenyl | 3,93 (m, 2H), 4,23 (m, 2H), 4,57 (s, 2H), 7,09-7,33 (m, 4H) |
| A.891 | $n-C_3H_7$ | Tetrahydropyran-3-yl | $-CH_2CH_2OCH_2-$ | $2-CH_3$-phenyl | 3,93 (m, 2H), 4,23 (m, 2H), 4,57 (s, 2H), 7,09-7,33 (m, 4H) |
| A.892 | $C_2H_5$ | Tetrahydropyran-4-yl | $-CH_2CH_2OCH_2-$ | $2-CH_3$-phenyl | 4,00 (m, 2H), 4,23 (m, 2H), 4,57 (s, 2H), 7,09-7,33 (m, 4H) |
| A.893 | $n-C_3H_7$ | Tetrahydropyran-4-yl | $-CH_2CH_2OCH_2-$ | $2-CH_3$-phenyl | 4,00 (m, 2H), 4,23 (m, 2H), 4,57 (s, 2H), 7,09-7,33 (m, 4H) |
| A.894 | $C_2H_5$ | Tetrahydrothiopyran-3-yl | $-CH_2CH_2OCH_2-$ | $2-CH_3$-phenyl | 4,23 (m, 2H), 4,57 (s, 2H), 7,09-7,33 (m, 4H) |
| A.895 | $n-C_3H_7$ | Tetrahydrothiopyran-3-yl | $-CH_2CH_2OCH_2-$ | $2-CH_3$-phenyl | 4,23 (m, 2H), 4,57 (s, 2H), 7,09-7,33 (m, 4H) |
| A.896 | $C_2H_5$ | Tetrahydropyran-3-yl | $-CH_2CH_2OCH_2-$ | $3-CH_3$-phenyl | 3,93 (m, 2H), 4,25 (m, 2H), 4,57 (s, 2H), 7,00-7,32 (m, 4H) |
| A.897 | $n-C_3H_7$ | Tetrahydropyran-3-yl | $-CH_2CH_2OCH_2-$ | $3-CH_3$-phenyl | 3,93 (m, 2H), 4,25 (m, 2H), 4,57 (s, 2H), 7,00-7,32 (m, 4H) |
| A.898 | $C_2H_5$ | Tetrahydropyran-4-yl | $-CH_2CH_2OCH_2-$ | $3-CH_3$-phenyl | 4,00 (m, 2H), 4,27 (m, 2H), 4,57 (s, 2H), 7,00-7,32 (m, 4H) |
| A.899 | $n-C_3H_7$ | Tetrahydropyran-4-yl | $-CH_2CH_2OCH_2-$ | $3-CH_3$-phenyl | 4,00 (m, 2H), 4,27 (m, 2H), 4,57 (s, 2H), 7,00-7,32 (m, 4H) |
| A.900 | $C_2H_5$ | Tetrahydrothiopyran-3-yl | $-CH_2CH_2OCH_2-$ | $3-CH_3$-phenyl | 4,27 (m, 2H), 4,60 (s, 2H), 7,00-7,32 (m, 4H) |
| A.901 | $n-C_3H_7$ | Tetrahydrothiopyran-3-yl | $-CH_2CH_2OCH_2-$ | $3-CH_3$-phenyl | 4,27 (m, 2H), 4,60 (s, 2H), 7,00-7,32 (m, 4H) |

Tabelle II.7 (Fortsetzung)

| Nr. | $R^a$ | $R^c$ | W | $R^f$ | phys. Daten / $^1H$-NMR [δ in ppm], Fp. [°C] |
|---|---|---|---|---|---|
| A.902 | $C_2H_5$ | Tetrahydropyran-3-yl | $-CH_2CH_2OCH_2-$ | 4-$CH_3$-phenyl | 3,93 (m, 2H), 4,20 (m, 2H), 4,53 (s, 2H), 7,07-7,30 (m, 4H) |
| A.903 | n-$C_3H_7$ | Tetrahydropyran-3-yl | $-CH_2CH_2OCH_2-$ | 4-$CH_3$-phenyl | 3,93 (m, 2H), 4,20 (m, 2H), 4,53 (s, 2H), 7,07-7,30 (m, 4H) |
| A.904 | $C_2H_5$ | Tetrahydropyran-4-yl | $-CH_2CH_2OCH_2-$ | 4-$CH_3$-phenyl | 4,00 (m, 2H), 4,23 (m, 2H), 4,57 (s, 2H), 7,03-7,27 (m, 4H) |
| A.905 | n-$C_3H_7$ | Tetrahydropyran-4-yl | $-CH_2CH_2OCH_2-$ | 4-$CH_3$-phenyl | 4,00 (m, 2H), 4,23 (m, 2H), 4,57 (s, 2H), 7,03-7,27 (m, 4H) |
| A.906 | $C_2H_5$ | Tetrahydrothiopyran-3-yl | $-CH_2CH_2OCH_2-$ | 4-$CH_3$-phenyl | 4,23 (m, 2H), 4,57 (s, 2H), 7,07-7,30 (m, 4H) |
| A.907 | n-$C_3H_7$ | Tetrahydrothiopyran-3-yl | $-CH_2CH_2OCH_2-$ | 4-$CH_3$-phenyl | 4,28 (m, 2H), 4,57 (s, 2H), 7,07-7,30 (m, 4H) |
| A.908 | $C_2H_5$ | Tetrahydropyran-3-yl | $-CH_2CH_2OCH_2-$ | 4-tert.-$C_4H_9$ | 3,93 (m, 2H), 4,23 (m, 2H), 4,53 (s, 2H), 7,20-7,40 (m, 4H) |
| A.909 | n-$C_3H_7$ | Tetrahydropyran-3-yl | $-CH_2CH_2OCH_2-$ | 4-tert.-$C_4H_9$ | 3,93 (m, 2H), 4,23 (m, 2H), 4,53 (s, 2H), 7,20-7,40 (m, 4H) |
| A.910 | $C_2H_5$ | Tetrahydropyran-4-yl | $-CH_2CH_2OCH_2-$ | 4-tert.-$C_4H_9$ | 4,00 (m, 2H), 4,23 (m, 2H), 4,53 (s, 2H), 7,20-7,40 (m, 4H) |
| A.911 | n-$C_3H_7$ | Tetrahydropyran-4-yl | $-CH_2CH_2OCH_2-$ | 4-tert.-$C_4H_9$ | 4,00 (m, 2H), 4,23 (m, 2H), 4,53 (s, 2H), 7,20-7,40 (m, 4H) |
| A.912 | $C_2H_5$ | Tetrahydrothiopyran-3-yl | $-CH_2CH_2OCH_2-$ | 4-tert.-$C_4H_9$ | 4,23 (m, 2H), 4,53 (s, 2H), 7,20-7,40 (m, 4H) |
| A.913 | n-$C_3H_7$ | Tetrahydrothiopyran-3-yl | $-CH_2CH_2OCH_2-$ | 4-tert.-$C_4H_9$ | 4,23 (m, 2H), 4,53 (s, 2H), 7,20-7,40 (m, 4H) |
| A.914 | $C_2H_5$ | Tetrahydropyran-3-yl | $-CH_2CH_2SCH_2-$ | Phenyl | 3,73 (s, 2H), 3,90 (m, 2H), 4,17 (t, 2H), 7,28 (s, 5H) |
| A.915 | n-$C_3H_7$ | Tetrahydropyran-3-yl | $-CH_2CH_2SCH_2-$ | Phenyl | 3,73 (s, 2H), 3,90 (m, 2H), 4,17 (t, 2H), 7,28 (s, 5H) |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.916 | $C_2H_5$ | Tetrahydropyran-4-yl | $-CH_2CH_2SCH_2-$ | Phenyl | 3,77 (s,2H), 4,00 (m,2H), 4,13 (t,2H), 7,28 (s,5H) |
| A.917 | $n-C_3H_7$ | Tetrahydropyran-4-yl | $-CH_2CH_2SCH_2-$ | Phenyl | 3,77 (s,2H), 4,00 (m,2H), 4,13 (t,2H), 7,28 (s,5H) |
| A.918 | $C_2H_5$ | Tetrahydrothiopyran-3-yl | $-CH_2CH_2SCH_2-$ | Phenyl | 3,80 (s,2H), 4,13 (t,2H), 7,28 (s,5H) |
| A.919 | $n-C_3H_7$ | Tetrahydrothiopyran-3-yl | $-CH_2CH_2SCH_2-$ | Phenyl | 3,80 (s,2H), 4,13 (t,2H), 7,28 (s,5H) |
| A.920 | $C_2H_5$ | Tetrahydropyran-3-yl | $-CH_2CH_2SCH_2-$ | 4-F-phenyl | 3,72 (s,2H), 3,90 (m,2H), 4,13 (t,2H), 7,00 (m,2H), 7,30 (m,2H) |
| A.921 | $n-C_3H_7$ | Tetrahydropyran-3-yl | $-CH_2CH_2SCH_2-$ | 4-F-phenyl | 3,72 (s,2H), 3,90 (m,2H), 4,13 (t,2H), 7,00 (m,2H), 7,30 (m,2H) |
| A.922 | $C_2H_5$ | Tetrahydropyran-4-yl | $-CH_2CH_2SCH_2-$ | 4-F-phenyl | 63- 65 |
| A.923 | $n-C_3H_7$ | Tetrahydropyran-4-yl | $-CH_2CH_2SCH_2-$ | 4-F-phenyl | 3,73 (s,2H), 4,00 (m,2H), 4,13 (t,2H), 7,00 (m,2H), 7,30 (m,2H) |
| A.924 | $C_2H_5$ | Tetrahydrothiopyran-3-yl | $-CH_2CH_2SCH_2-$ | 4-F-phenyl | 3,75 (s,2H), 4,13 (t,2H), 7,00 (m,2H), 7,30 (m,2H) |
| A.925 | $n-C_3H_7$ | Tetrahydrothiopyran-3-yl | $-CH_2CH_2SCH_2-$ | 4-F-phenyl | 3,75 (s,2H), 4,13 (t,2H), 7,00 (m,2H), 7,30 (m,2H) |
| A.926 | $C_2H_5$ | Tetrahydropyran-3-yl | $-CH_2CH_2SCH_2-$ | 4-Cl-phenyl | 3,77 (s,2H), 3,93 (m,2H), 4,13 (t,2H), 7,30 (s,4H) |
| A.927 | $n-C_3H_7$ | Tetrahydropyran-3-yl | $-CH_2CH_2SCH_2-$ | 4-Cl-phenyl | 3,77 (s,2H), 3,93 (m,2H), 4,13 (t,2H), 7,30 (s,4H) |
| A.928 | $C_2H_5$ | Tetrahydropyran-4-yl | $-CH_2CH_2SCH_2-$ | 4-Cl-phenyl | 3,73 (s,2H), 4,00 (m,2H), 4,17 (t,2H), 7,30 (s,4H) |
| A.929 | $n-C_3H_7$ | Tetrahydropyran-4-yl | $-CH_2CH_2SCH_2-$ | 4-Cl-phenyl | 3,73 (s,2H), 4,00 (m,2H), 4,17 (t,2H), 7,30 (s,4H) |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.930 | $C_2H_5$ | Tetrahydrothiopyran-3-yl | $-CH_2CH_2SCH_2-$ | 4-Cl-phenyl | 3,73 (s,2H), 4,13 (m,2H), 7,30 (s,4H) |
| A.931 | n-$C_3H_7$ | Tetrahydrothiopyran-3-yl | $-CH_2CH_2SCH_2-$ | 4-Cl-phenyl | 3,73 (s,2H), 4,13 (m,2H), 7,30 (s,4H) |
| A.932 | $C_2H_5$ | Tetrahydropyran-3-yl | $-(CH_2)_4-O-$ | Phenyl | 3,70-4,20 (m,6H), 6,90 (m,3H), 7,30 (m,2H) |
| A.933 | n-$C_3H_7$ | Tetrahydropyran-3-yl | $-(CH_2)_4-O-$ | Phenyl | 3,70-4,20 (m,6H), 6,90 (m,3H), 7,30 (m,2H) |
| A.934 | $C_2H_5$ | Tetrahydropyran-4-yl | $-(CH_2)_4-O-$ | Phenyl | 3,83-4,23 (m,6H), 6,90 (m,3H), 7,30 (m,2H) |
| A.935 | n-$C_3H_7$ | Tetrahydropyran-4-yl | $-(CH_2)_4-O-$ | Phenyl | 3,83-4,23 (m,6H), 6,90 (m,3H), 7,30 (m,2H) |
| A.936 | $C_2H_5$ | Tetrahydrothiopyran-3-yl | $-(CH_2)_4-O-$ | Phenyl | 4,00 (bs,2H), 4,13 (bs,2H), 6,90 (m,3H) 7,30 (m,2H) |
| A.937 | n-$C_3H_7$ | Tetrahydrothiopyran-3-yl | $-(CH_2)_4-O-$ | Phenyl | 4,00 (bs,2H), 4,13 (bs,2H), 6,90 (m,3H) 7,30 (m,2H) |
| A.938 | $C_2H_5$ | Tetrahydropyran-3-yl | $-(CH_2)_4-O-$ | 2-F-phenyl | 3,93 (m,2H), 4,00-4,20 (m,4H), 6,80-7,15 (m,4H) |
| A.939 | n-$C_3H_7$ | Tetrahydropyran-3-yl | $-(CH_2)_4-O-$ | 2-F-phenyl | 3,93 (m,2H), 4,00-4,20 (m,4H), 6,80-7,15 (m,4H) |
| A.940 | $C_2H_5$ | Tetrahydropyran-4-yl | $-(CH_2)_4-O-$ | 2-F-phenyl | 68- 72 |
| A.941 | n-$C_3H_7$ | Tetrahydropyran-4-yl | $-(CH_2)_4-O-$ | 2-F-phenyl | 3,90-4,20 (m,6H), 6,80-7,15 (m,4H) |
| A.942 | $C_2H_5$ | Tetrahydrothiopyran-3-yl | $-(CH_2)_4-O-$ | 2-F-phenyl | 4,00-4,20 (m,4H), 6,80-7,15 (m,4H) |
| A.943 | n-$C_3H_7$ | Tetrahydrothiopyran-3-yl | $-(CH_2)_4-O-$ | 2-F-phenyl | 4,00-4,20 (m,4H), 6,80-7,15 (m,4H) |
| A.944 | $C_2H_5$ | Tetrahydropyran-3-yl | $-(CH_2)_4-O-$ | 3-F-phenyl | |
| A.945 | n-$C_3H_7$ | Tetrahydropyran-3-yl | $-(CH_2)_4-O-$ | 3-F-phenyl | |
| A.946 | $C_2H_5$ | Tetrahydropyran-4-yl | $-(CH_2)_4-O-$ | 3-F-phenyl | |
| A.947 | n-$C_3H_7$ | Tetrahydropyran-4-yl | $-(CH_2)_4-O-$ | 3-F-phenyl | |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.948 | $C_2H_5$ | Tetrahydrothiopyran-3-yl | $-(CH_2)_4-O-$ | 3-F-phenyl | |
| A.949 | $n-C_3H_7$ | Tetrahydrothiopyran-3-yl | $-(CH_2)_4-O-$ | 3-F-phenyl | |
| A.950 | $C_2H_5$ | Tetrahydropyran-3-yl | $-(CH_2)_4-O-$ | 4-F-phenyl | 3,80-4,20 (m,6H), 6,75-7,05 (m,4H) |
| A.951 | $n-C_3H_7$ | Tetrahydropyran-3-yl | $-(CH_2)_4-O-$ | 4-F-phenyl | 3,80-4,20 (m,6H), 6,75-7,05 (m,4H) |
| A.952 | $C_2H_5$ | Tetrahydropyran-4-yl | $-(CH_2)_4-O-$ | 4-F-phenyl | 3,90-4,20 (m,6H), 6,75-7,05 (m,4H) |
| A.953 | $n-C_3H_7$ | Tetrahydropyran-4-yl | $-(CH_2)_4-O-$ | 4-F-phenyl | 3,90-4,20 (m,6H), 6,75-7,05 (m,4H) |
| A.954 | $C_2H_5$ | Tetrahydrothiopyran-3-yl | $-(CH_2)_4-O-$ | 4-F-phenyl | 3,90-4,20 (m,4H), 6,75-7,05 (m,4H) |
| A.955 | $n-C_3H_7$ | Tetrahydrothiopyran-3-yl | $-(CH_2)_4-O-$ | 4-F-phenyl | 3,90-4,20 (m,4H), 6,75-7,05 (m,4H) |
| A.956 | $C_2H_5$ | Tetrahydropyran-3-yl | $-(CH_2)_4-O-$ | 4-Cl-phenyl | 3,80-4,20 (m,6H), 6,80 (m,2H), 7,20 (m,2H) |
| A.957 | $n-C_3H_7$ | Tetrahydropyran-3-yl | $-(CH_2)_4-O-$ | 4-Cl-phenyl | 3,80-4,20 (m,6H), 6,80 (m,2H), 7,20 (m,2H) |
| A.958 | $C_2H_5$ | Tetrahydropyran-4-yl | $-(CH_2)_4-O-$ | 4-Cl-phenyl | 3,90-4,20 (m,6H), 6,80 (m,2H), 7,20 (m,2H) |
| A.959 | $n-C_3H_7$ | Tetrahydropyran-4-yl | $-(CH_2)_4-O-$ | 4-Cl-phenyl | 3,90-4,20 (m,6H), 6,80 (m,2H), 7,20 (m,2H) |
| A.960 | $C_2H_5$ | Tetrahydrothiopyran-3-yl | $-(CH_2)_4-O-$ | 4-Cl-phenyl | 3,90-4,20 (m,4H), 6,80 (m,2H), 7,20 (m,2H) |
| A.961 | $n-C_3H_7$ | Tetrahydrothiopyran-3-yl | $-(CH_2)_4-O-$ | 4-Cl-phenyl | 3,90-4,20 (m,4H), 6,80 (m,2H), 7,20 (m,2H) |
| A.962 | $C_2H_5$ | Tetrahydropyran-3-yl | $-(CH_2)_4-O-$ | 2,6-$Cl_2$-phenyl | 3,93 (m,2H), 4,00-4,25 (m,4H), 7,00 (t,1H), 7,30 (d,2H) |
| A.963 | $n-C_3H_7$ | Tetrahydropyran-3-yl | $-(CH_2)_4-O-$ | 2,6-$Cl_2$-phenyl | 3,93 (m,2H), 4,00-4,25 (m,4H), 7,00 (t,1H), 7,30 (d,2H) |
| A.964 | $C_2H_5$ | Tetrahydropyran-4-yl | $-(CH_2)_4-O-$ | 2,6-$Cl_2$-phenyl | 3,90-4,25 (m,6H), 7,00 (t,1H), 7,30 (d,2H) |
| A.965 | $n-C_3H_7$ | Tetrahydropyran-4-yl | $-(CH_2)_4-O-$ | 2,6-$Cl_2$-phenyl | 3,90-4,25 (m,6H), 7,00 (t,1H), 7,30 (d,2H) |
| A.966 | $C_2H_5$ | Tetrahydrothiopyran-3-yl | $-(CH_2)_4-O-$ | 2,6-$Cl_2$-phenyl | 4,00-4,20 (m,4H), 7,00 (t,1H), 7,30 (d,2H) |
| A.967 | $n-C_3H_7$ | Tetrahydrothiopyran-3-yl | $-(CH_2)_4-O-$ | 2,6-$Cl_2$-phenyl | 4,00-4,20 (m,4H), 7,00 (t,1H), 7,30 (d,2H) |

Tabelle II.7 (Fortsetzung)

| Nr. | $R^a$ | $R^c$ | W | $R^f$ | phys. Daten / $^1H$-NMR [δ in ppm], Fp. [°C] |
|---|---|---|---|---|---|
| A.968 | $C_2H_5$ | Tetrahydropyran-3-yl | $-CH_2CH_2OCH_2CH_2-$ | Phenyl | 3,90 (m, 2H), 4,20 (m, 2H), 7,25 (m, 5H) |
| A.969 | $n-C_3H_7$ | Tetrahydropyran-3-yl | $-CH_2CH_2OCH_2CH_2-$ | Phenyl | 3,90 (m, 2H), 4,20 (m, 2H), 7,25 (m, 5H) |
| A.970 | $C_2H_5$ | Tetrahydropyran-4-yl | $-CH_2CH_2OCH_2CH_2-$ | Phenyl | |
| A.971 | $n-C_3H_7$ | Tetrahydropyran-4-yl | $-CH_2CH_2OCH_2CH_2-$ | Phenyl | |
| A.972 | $C_2H_5$ | Tetrahydrothiopyran-3-yl | $-CH_2CH_2OCH_2CH_2-$ | Phenyl | 4,20 (m, 2H), 7,25 (m, 5H) |
| A.973 | $n-C_3H_7$ | Tetrahydrothiopyran-3-yl | $-CH_2CH_2OCH_2CH_2-$ | Phenyl | 4,20 (m, 2H), 7,25 (m, 5H) |
| A.974 | $C_2H_5$ | Tetrahydropyran-3-yl | $-CH_2CH_2OCH_2CH_2-$ | 4-F-phenyl | 3,90 (m, 2H), 4,17 (m, 2H), 6,93 (m, 2H), 7,13 (m, 2H) |
| A.975 | $n-C_3H_7$ | Tetrahydropyran-3-yl | $-CH_2CH_2OCH_2CH_2-$ | 4-F-phenyl | 3,90 (m, 2H), 4,17 (m, 2H), 6,93 (m, 2H), 7,13 (m, 2H) |
| A.976 | $C_2H_5$ | Tetrahydropyran-4-yl | $-CH_2CH_2OCH_2CH_2-$ | 4-F-phenyl | |
| A.977 | $n-C_3H_7$ | Tetrahydropyran-4-yl | $-CH_2CH_2OCH_2CH_2-$ | 4-F-phenyl | |
| A.978 | $C_2H_5$ | Tetrahydrothiopyran-3-yl | $-CH_2CH_2OCH_2CH_2-$ | 4-F-phenyl | 4,17 (m, 2H), 6,93 (m, 2H), 7,13 (m, 2H) |
| A.979 | $n-C_3H_7$ | Tetrahydrothiopyran-3-yl | $-CH_2CH_2OCH_2CH_2-$ | 4-F-phenyl | 4,17 (m, 2H), 6,93 (m, 2H), 7,13 (m, 2H) |
| A.980 | $C_2H_5$ | Tetrahydropyran-3-yl | $-CH_2CH_2OCH_2CH_2-$ | 4-Cl-phenyl | 3,90 (m, 2H), 4,17 (m, 2H), 7,13 (m, 4H) |
| A.981 | $n-C_3H_7$ | Tetrahydropyran-3-yl | $-CH_2CH_2OCH_2CH_2-$ | 4-Cl-phenyl | 3,90 (m, 2H), 4,17 (m, 2H), 7,13 (m, 4H) |
| A.982 | $C_2H_5$ | Tetrahydropyran-4-yl | $-CH_2CH_2OCH_2CH_2-$ | 4-Cl-phenyl | |
| A.983 | $n-C_3H_7$ | Tetrahydropyran-4-yl | $-CH_2CH_2OCH_2CH_2-$ | 4-Cl-phenyl | |
| A.984 | $C_2H_5$ | Tetrahydrothiopyran-3-yl | $-CH_2CH_2OCH_2CH_2-$ | 4-Cl-phenyl | 4,17 (m, 2H), 7,13 (m, 4H) |
| A.985 | $n-C_3H_7$ | Tetrahydrothiopyran-3-yl | $-CH_2CH_2OCH_2CH_2-$ | 4-Cl-phenyl | 4,17 (m, 2H), 7,13 (m, 4H) |

Tabelle II.7 (Fortsetzung)

| Nr. | $R^a$ | $R^c$ | W | $R^f$ | phys. Daten / $^1$H-NMR [δ in ppm], Fp. [°C] |
|---|---|---|---|---|---|
| A.986 | $C_2H_5$ | Tetrahydropyran-3-yl | $-(CH_2)_5-O-$ | Phenyl | 3,80-4,17 (m,6H), 6,90 (m,3H), 7,27 (m,2H) |
| A.987 | $n-C_3H_7$ | Tetrahydropyran-3-yl | $-(CH_2)_5-O-$ | Phenyl | 3,80-4,17 (m,6H), 6,90 (m,3H), 7,27 (m,2H) |
| A.988 | $C_2H_5$ | Tetrahydropyran-4-yl | $-(CH_2)_5-O-$ | Phenyl | 3,90-4,17 (m,6H), 6,90 (m,3H), 7,27 (m,2H) |
| A.989 | $n-C_3H_7$ | Tetrahydropyran-4-yl | $-(CH_2)_5-O-$ | Phenyl | 3,90-4,17 (m,6H), 6,90 (m,3H), 7,27 (m,2H) |
| A.990 | $C_2H_5$ | Tetrahydrothiopyran-3-yl | $-(CH_2)_5-O-$ | Phenyl | 3,97 (t,2H), 7,27 (m,2H), 4,07 (t,2H), 6,90 (m,3H), |
| A.991 | $n-C_3H_7$ | Tetrahydrothiopyran-3-yl | $-(CH_2)_5-O-$ | Phenyl | 3,97 (t,2H), 7,27 (m,2H), 4,07 (t,2H), 6,90 (m,3H), |
| A.992 | $C_2H_5$ | Tetrahydropyran-3-yl | $-(CH_2)_5-O-$ | 4-F-phenyl | 3,90 (m,4H), 4,03 (t,2H), 6,70-7,03 (m,4H) |
| A.993 | $n-C_3H_7$ | Tetrahydropyran-3-yl | $-(CH_2)_5-O-$ | 4-F-phenyl | 3,90 (m,4H), 4,03 (t,2H), 6,70-7,03 (m,4H) |
| A.994 | $C_2H_5$ | Tetrahydropyran-4-yl | $-(CH_2)_5-O-$ | 4-F-phenyl | 3,83-4,13 (m,6H), 6,70-7,03 (m,4H) |
| A.995 | $n-C_3H_7$ | Tetrahydropyran-4-yl | $-(CH_2)_5-O-$ | 4-F-phenyl | 3,83-4,13 (m,6H), 6,70-7,03 (m,4H) |
| A.996 | $C_2H_5$ | Tetrahydrothiopyran-3-yl | $-(CH_2)_5-O-$ | 4-F-phenyl | 3,90 (t,2H), 4,03 (t,2H) 6,70-7,03 (m,4H) |
| A.997 | $n-C_3H_7$ | Tetrahydrothiopyran-3-yl | $-(CH_2)_5-O-$ | 4-F-phenyl | 3,90 (t,2H), 4,03 (t,2H) 6,70-7,03 (m,4H) |
| A.998 | $C_2H_5$ | Tetrahydropyran-3-yl | $-(CH_2)_5-O-$ | 4-Cl-phenyl | 3,80-4,10 (m,6H), 6,80 (d,2H), 7,20 (d,2H) |
| A.999 | $n-C_3H_7$ | Tetrahydropyran-3-yl | $-(CH_2)_5-O-$ | 4-Cl-phenyl | 3,80-4,10 (m,6H), 6,80 (d,2H), 7,20 (d,2H) |
| A.1000 | $C_2H_5$ | Tetrahydropyran-4-yl | $-(CH_2)_5-O-$ | 4-Cl-phenyl | 3,87-4,10 (m,6H), 6,80 (d,2H), 7,20 (d,2H) |
| A.1001 | $n-C_3H_7$ | Tetrahydropyran-4-yl | $-(CH_2)_5-O-$ | 4-Cl-phenyl | 3,87-4,10 (m,6H), 6,80 (d,2H), 7,20 (d,2H) |
| A.1002 | $C_2H_5$ | Tetrahydrothiopyran-3-yl | $-(CH_2)_5-O-$ | 4-Cl-phenyl | 54- 61 |
| A.1003 | $n-C_3H_7$ | Tetrahydrothiopyran-3-yl | $-(CH_2)_5-O-$ | 4-Cl-phenyl | 3,90 (t,2H), 7,20 (d,2H), 4,07 (t,2H), 6,80 (d,2H) |

Tabelle II.8

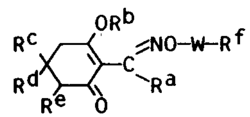

II

| Nr. | $R^a$ | $R^b$ | $R^c$ | $R^d$ | $R^e$ | W | $R^f$ | Lit./$^1$H-NMR-Daten [ppm] |
|---|---|---|---|---|---|---|---|---|
| A.1004 | n-$C_3H_7$ | Na | Methyl | $CH_3$ | $COOCH_3$ | $-CH_2CH=CH-$ | H | DE-A 2 439 104 |
| A.1005 | n-$C_3H_7$ | H | Methyl | $CH_3$ | $C(CH_3)=NOCH_3$ | $-CH_2CH_2-$ | H | EP-A 172 551 |
| A.1006 | n-$C_3H_7$ | Na | Tetrahydro-thiopyran-3-yl | H | H | $-CH_2CH=CH-$ | 4-F-phenyl | 0,8(t,3H), 4,5(d,2H), 6,35(dt,1H), 6,6(d,1H), 7,0-7,6(2m,4H) |
| A.1007 | $C_2H_5$ | Na | Tetrahydro-thiopyran-3-yl | H | H | $-CH_2CH=CH-$ | 4-F-phenyl | 0,8(t,3H), 4,5(d,2H), 6,35(dt,1H), 6,6(d,1H), 7,0-7,6(2m,4H) |
| A.1008 | n-$C_3H_7$ | O-CO— 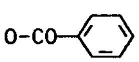 | Tetrahydro-thiopyran-3-yl | H | H | $-CH_2CH=CH-$ | 4-F-phenyl | 0,9(t,3H), 4,75(d,2H), 6,1(dt,1H), 6,4(d,1H), 6,9-8,0(5m,9H) |
| A.1009 | $C_2H_5$ | O-CO— 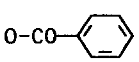 | Tetrahydro-thiopyran-3-yl | H | H | $-CH_2CH=CH-$ | 4-F-phenyl | 0,95(t,3H), 4,75(d,2H), 6,1(dt,1H), 6,4(d,1H), 6,9-8,0(5m,9H) |

Außerdem tritt die gewünschte antidotisierende Wirkung der Verbindungen I insbesondere bei der Anwendung mit Herbiziden aus der Gruppe der 2-(4-Heteroaryloxy)- oder 2-(4-Aryloxy)-phenoxycarbonsäurederivate der Formel III auf, wenn deren Substituenten die folgende Bedeutung haben:

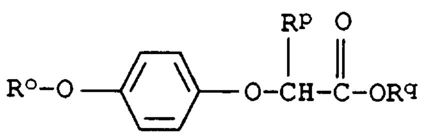

III

$R^o$

Phenyl, Pyridyl, Benzoxazolyl, Benzthiazolyl oder Benzpyrazinyl, wobei diese aromatischen und heteroaromatischen Ringsysteme ein oder zwei der folgenden Reste tragen können:

- Nitro,
- Halogen wie vorstehend im allgemeinen und im besonderen genannt;
- $C_1$-$C_4$-Alkyl wie vorstehend im allgemeinen und im besonderen genannt;
- $C_1$-$C_4$-Halogenalkyl, besonders $C_1$-$C_2$-Halogenalkyl wie vorstehend im allgemeinen und im besonderen genannt;
- $C_1$-$C_4$-Alkoxy wie vorstehend im allgemeinen und im besonderen genannt;
- $C_1$-$C_4$-Halogenalkoxy, besonders $C_1$-$C_2$-Halogenalkoxy wie vorstehend im allgemeinen und im besonderen genannt;
- $C_1$-$C_4$-Alkylthio wie vorstehend im allgemeinen und im besonderen genannt;

Wasserstoff oder Methyl;

$R^q$

Wasserstoff;

$C_1$-$C_4$-Alkyl wie vorstehend im allgemeinen und im besonderen genannt;

$C_3$-$C_4$-Alkenyl wie Allyl, 2-Butenyl und 3-Butenyl;

$C_3$-$C_4$-Alkinyl wie Propargyl, 2-Butinyl und 3-Butinyl;

$C_1$-$C_4$-Alkoxy-$C_1$-$C_4$-alkyl wie vorstehend im allgemeinen und im besonderen genannt;

$C_3$-$C_4$-Alkylideniminooxy-$C_2$-$C_3$-alkyl steht für durch Propylideniminooxy oder Butylideniminooxy substituiertes $C_2$-$C_3$-Alkyl wie Ethyl, Propyl und 1-Methylethyl; Tetrahydrofuranylmethyl; Isoxazolidinyl;

oder das Äquivalent eines landwirtschaftlich brauchbaren Kations.

Derartige Verbindungen sind aus der Literatur bekannt (vgl. z.B. DE-A 22 23 894, DE-A 24 33 067, DE-A 25 76 251, DE-A 30 04 770, DE-A 32 46 847, BE-A 868 875, BE-A 858 618, EP-A 054 715, EP-A 248 968, EP-A 323 127 und US 4,753,673).

Die 2-(4-Heteroaryloxy)- und 2-(4-Aryloxy-phenoxycarbonsäurederivate III können ein oder mehrere Asymmetriezentren enthalten. Sie wirken als Racemate, wie sie bei den meisten Herstellungsverfahren anfallen, können gewünschtenfalls aber auch nach den hierfür üblichen Methoden, als reine Isomere dargestellt oder aufgetrennt werden.

Sowohl die Racemate als auch die reinen Isomeren dienen zur Bekämpfung von unerwünschten Pflanzen aus der Familie der Gramineen. Die Verträglichkeit dieser Substanzen für Kulturpflanzen variiert jedoch zwischen kommerziell akzeptabel und unverträglich, je nach Substituenten und Aufwandmenge.

Spezielle Beispiele für herbizide 2-(4-Heteroaryloxy)- und 2-(4-Aryloxy)-phenoxycarbonsäurederivate der Formel III, deren Kulturpflanzenverträglichkeit durch substituierte 3-Pyrido[2,3-d]pyrimidine I verbessert werden kann, sind in der folgenden Tabelle III.1 aufgeführt:

Tabelle III.1

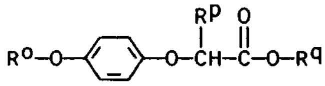

III

| Nr. | $R^o$ | $R^p$ | $R^q$ | Literatur |
|---|---|---|---|---|
| B.01 | 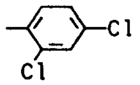 | $CH_3$ | $-CH_3$ | DE-A 22 23 894 |
| B.02 | 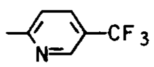 | $CH_3$ | $-n-C_4H_9$ | BE-A 868 875 |
| B.03 | 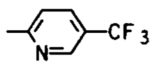 | $CH_3$ | $-CH_2CH_2OCH_2H_5$ | US-A 4 753 673 |
| B.04 | 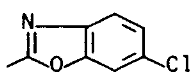 | $CH_3$ | $-C_2H_5$ | BE-A 858 618 |
| B.05 | 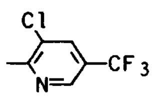 | $CH_3$ | $-CH_3$ | BE-A 868 875 |
| B.06 | 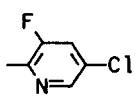 | $CH_3$ | $-CH_2-C \equiv CH$ | EP-A 248 968 |
| B.07 | 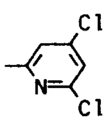 | $CH_3$ | 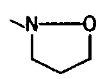 | DE-A 32 46 847 |
| B.08 | 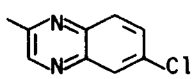 | $CH_3$ | $-C_2H_5$ | DE-A 30 04 770 |
| B.09 | 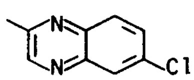 | $CH_3$ | $-CH_2CH_2-ON=C(CH_3)_2$ | EP 54 715 |
| B.10 | 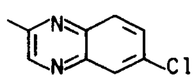 | $CH_3$ | $-CH_2$ 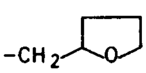 | EP-A 323 727 |

Die herbiziden Wirkstoffe und die antidotisch wirkenden Verbindungen können gemeinsam oder getrennt nach dem Auflaufen auf die Blätter und Sprossen der Kulturpflanzen und unerwünschten Gräser ausgebracht werden. Bevorzugt bringt man jedoch die herbiziden und antidotischen Wirkstoffe gleichzeitig auf das Feld. Bei getrennter Ausbringung von Antidot und herbizidem Wirkstoff wird vorzugsweise das Antidot zuerst ausgebracht.

Der antidotische und der herbizide Wirkstoff können gemeinsam oder getrennt formuliert werden und dann in suspendierbarer, emulgierbarer oder löslicher Form zur Bereitung von Spritzmitteln vorliegen.

Antidotische Effekte werden auch durch Bahandlung der Kulturpflanzensamen oder der Stecklinge mit dem Antidot vor der Aussaat bzw. vor dem Auspflanzen erzielt. Der herbizide Wirkstoff wird dann allein in der üblichen Weise appliziert.

Bei der Saatgutbehandlung werden im allgemeinen Wirkstoffmengen von 0,1 bis 10 g, vorzugsweise 1 bis 2 g, je Kilogramm Saatgut benötigt.

Bei der Applikation des Antidots durch Samenquellung oder bei der Stecklingsbehandlung werden bevorzugt Lösungen eingesetzt, die den antagonistischen Wirkstoff in einer Konzentration von 1 bis 10.000 ppm, insbesondere von 100 bis 10.000 ppm, enthalten.

In den verschiedenen Pflanzenkulturen benötigt man üblicherweise unterschiedliche Mengen an antidotisch wirksamer Verbindung I und herbizider Verbindung II oder III, wobei die Mengenverhältnisse in breiten Bereichen variabel sind. Sie sind abhängig von der Struktur der Cyclohexenon-Derivate II bzw. der Heteroaryloxy- und Aryloxyphenoxyessigsäurederivate III, der substituierten Pyrido[2,3-d]pyrimidine I und der jeweiligen Pflanzenkultur, auf die die Verbindungen ausgebracht werden. Geeignete Anteilsverhältnisse von herbizidem Wirkstoff zu antidotisch wirksamen substituierten Pyrido[2,3-d]pyrimidine I liegen zwischen 1:10 und 1:0,01, vorzugsweise zwischen 1:4 und 1:0,1.

Die erfindungsgemäßen Mittel bzw. bei getrennter Ausbringung die herbiziden Wirkstoffe oder das Antidot werden beispielsweise in Form von direkt versprühbaren Lösungen, Pulvern, Suspensionen, auch hochprozentigen wäßrigen, öligen oder sonstigen Suspensionen, Dispersionen, Emulsionen, öldisperesionen, Pasten, Stäubemitteln, Streumitteln oder Granulaten durch Versprühen, Vernebeln, Verstäuben, Verstreuen oder Gießen angewendet. Die Anwendungsform richtet sich hierbei ganz nach dem jeweiligen Verwendungszweck.

Zur Herstellung von direkt versprühbaren Lösungen, Emulsionen, Pasten und Öldispersionen kommen Mineralölfraktionen von mittlerem bis hohem Siedepunkt, wie Kerosin und Dieselöl, ferner Kohlenteeröle, sowie Öle und Fette pflanzlichen oder tierischen Ursprungs, aliphatische, cyclische oder aromatische Kohlenwasserstoffe, beispielsweise Methanol, Ethanol, Isopropanol, Butanol, Chloroform, Tetrachlorkohlenstoff, Cyclohexanol, Cyclohexanon, Chlorbenol, Toluol, Xylole, Paraffin, Tetrahydronaphthalin, alkylierte Naphthaline oder deren Derivate oder Isophoron, sowie stark polare Lösungsmittel wie Dimethylformamid, Dimethylsulfoxid, N-Methylpyrrolidon und vorzugsweise Wasser, in Betracht.

Wäßrige Anwendungsformen können aus Emulsionskonzentraten, Pasten, netzbaren Pulvern (Spritzpulvern) oder Öldispersionen durch Zusatz von Wasser bereitet werden. Zur Herstellung von Emulsionen, Pasten oder Öldispersionen können herbizider Wirkstoff und/oder Antidot als solche oder in einem Öl oder Lösungsmittel gelöst, mitteles Netz-, Haft-, Dispergier oder Emulgiermittel mit Wasser homogenisiert werden. Es können aber auch aus herbizidem Wirkstoff und/oder Antidot Netz-, Haft-, Dispergier- oder Emulgiermittel in Wasser homogenisiert werden. Es können aber auch aus herbizidem Wirkstoff und/oder Antidot Netz-, Haft-, Dispergier- oder Emulgiermittel und gewünschtenfalls Lösungsmittel oder Öl bestehende Konzentrate hergestellt werden, die zur Verdünnung mit Wasser geeignet sind.

Als oberflächenaktive Salze kommen Alkalimetall-, Erdalkalimetall-, Ammoniumsalze von Ligninsulfonsäure, Naphthalinsulfonsäure, Phenolsulfonsäure, Alkylarylsulfonate, Alkylsulfate, Alkylsulfonate, Alkalimetall- und Erdalkalimetallsalze der Dibutylnaphthalinsulfonsäure, Laurylethersulfat, Fettalkoholsulfate, fettsaure Alkalimetall- und Erdalkalimetallsalze, Salze sulfatierter Hexadecanole, Heptadecanole, Octadecanole, Salze von sulfatierten Fettalkoholglykolethern, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctyl-phenolether, ethoxylierte Isooctylphenol, Octylphenol oder Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether ethoxyliertes Polyoxypropylen, Laurylalkoholpolyglykoletheracetal, Sorbitester, Lignin-Sulfitablaugen und Methylcellulose in Betracht.

Pulver, Streu- und Stäubemittel können durch Mischen oder gemeinsames Vermahlen von herbizidem Wirkstoff und/oder Antidot mit einem festen Trägerstoff hergestellt werden.

Granulate, z.B. Umhüllungs-, Imprägnierungs- und Homogenisierungsgranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kreide, Talkum, Bolus, Lös, Ton, Dolomit, Diatomeenerde, Calcium- und Magnesiumsulfat, Magnesiumoxid, gemahlene Kunststoffe, Düngemittel wie Ammoniumsulfat, Ammoniumphosphat, Ammoniumnitrat, Harnstoffe, und pflanzliche Produkte wie Getriedemehl, Baumrinden-, Holz- und Nußschalenmehl, Cellulosepulver und andere feste Trägerstoffe.

Die Formulierungen enthalten 0,02 bis 95 Gew.%, vorzugsweise 0,5 bis 90 Gew.% an herbizidem Wirkstoff und Antidot. Die Aufwandmengen an herbizidem Wirkstoff betragen 0,05 bis 5 kg/ha.

Die herbiziden Mittel können neben den antagonistisch wirksamen substituierten Pyrido[2,3-d]-pyrimidine I und dem Herbizid aus der Gruppe der Cyclohexenone II oder der (Heteroaryloxy)- bzw. Aryloxyphenoxycarbonsäuren III weitere herbizide oder wachstumsregulierende Wirkstoffe anderer chemischer Sturktur enthalten, wobei der antagonistische Effekt der substituierten Pyrido[2,3-d]pyrimidine I erhalten bleibt.

Herstellungsbeispiele (erfindungsgemaße substituierte Pyrido[2,3-d]pyrimidine I):

Beispiel 1

7-(4-Fluorphenyl)-2-methyl-pyrido[2,3-d]pyrimidin

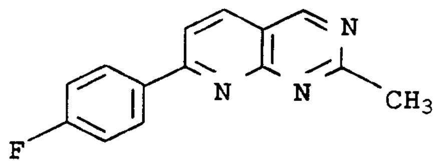

Eine Suspension von 30,1 g (0,22 mol) 4-Amino-5-formyl-2-methylpyrimidin und 31,7 g (0,23 mol) 4-Fluoracetophenon in 395 ml Methanol wurde bei 20-25`C langsam mit 15 ml 40 gew.-%iger wässriger Kaliumhydroxidlösung versetzt, wobei eine homogene Lösung entstand. Nach 20 Std. rühren bei ca. 20 ° C wurde der gebildete Feststoff abgetrennt und aus Ethanol umkristallisiert. Ausbeute: 50 %; Smp. > 200 ° C.

Beispiel 2

2-Methyl-7-(2-thienyl)-pyrido[2,3-d]pyrimidin

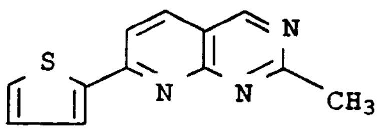

Eine Suspension von 2,0 g (14,6 mmol) 4-Amino-5-formyl-2-methylpyrimidin und 1,93 g (15,3 mmol) 2-Acetylthiophen in 25 ml Methanol wurde mit 1 ml 40 gew.-%iger wässriger Kaliumhydroxidlösung versetzt. Die Reaktionsmischung wurde anschließend 20 Std. bei 20-25 ° C gerührt, wonach man das Lösungsmittel entfernte. Nach Aufnehmen des Rückstandes in Dichlormethan wurde die organische Phase mit Wasser gewaschen, getrocknet und eingeengt. Ausbeute: 17 %; Smp.: 175-180 ° C.

Beispiel 3

7-Amino-6-(4-fluorphenyl)-2-methyl-pyrido[2,3-d]pyrimidin

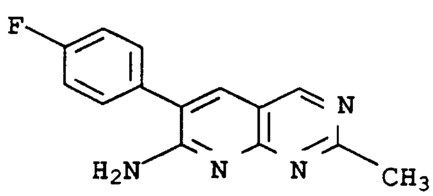

Zu einer Suspension von 90 g (0,66 mol) 4-Amino-5-formyl-2-methylpyrimidin und 88,7 g (0,66 mol) p-Fluorphenylacetonitril in 900 ml Methanol wurden bei 40 ° C 60 ml 40 gew.-%ige wässrige Kaliumhydroxidlösung gegeben, wobei eine homogene Lösung entstand. Nach Abkühlen auf ca. 20 ° C trennt man den gebildeten Niederschlag ab. Die alkoholische Phase wurde mit 1 l Wasser versetzt, wodurch weiteres Produkt auskristallisierte. Ausbeute: 78 %; Smp.: 252-254 ° C.

Beispiel 4

7-Amino-6-(3-methylphenyl)-2-methyl-pyrido[2,3-d]pyrimidin

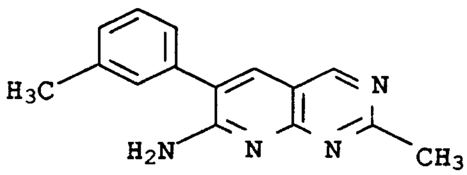

Eine Mischung von 1,52 g (11,6 mmol) m-Methylphenylacetonitril und 1,59 g (11,6 mmol) 4-Amino-5-formyl-2-methylpyridin in 15 ml Methanol wurde bei 42 ° C mit 1 ml 40 gew.-%iger wässriger Kaliumhydroxidlösung versetzt. Nach Abkühlen auf ca. 20 ° C wurde der gebildete Feststoff abgetrennt und mit Diethylether nachgewaschen. Ausbeute: 60 %; Smp.: 177-180 ° C.

Beispiel 5

6-Cyano-7-hydroxy-2-methyl-pyrido[2,3-d]pyrimidin

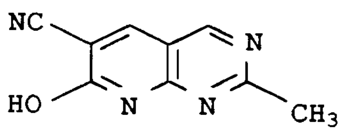

Eine Suspension aus 3 g (22 mmol) 4-Amino-5-formyl-3-methylpyrimin, 5 g (44 mmol) Ethylcyanoacetat und 850 mg (100 mmol) Piperidin in 20 ml Ethanol wurde 20 Std. bei 20-25 ° C gerührt. Der gebildete feinkörnige Feststoff wurde abgetrennt, unter reduziertem Druck von Lösungsmittelresten befreit und zweimal mit je 20 ml Methanol aufgeschlämmt. Das Rohprodukt wurde schließlich mit Diethylether gewaschen.
Ausbeute: 50 % (feines Pulver); Smp.: > 200 ° C.

Beispiel 6

7-Hydroxy-6-(4-methylphenylsulfonyl)-2-methyl-pyrido[2,3-d]-pyrimidin

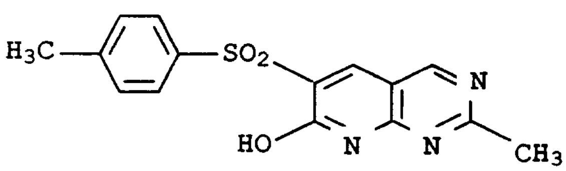

Eine Suspension von 3 g (22 mmol) 4-Amino-5-formyl-3-methylpyrimidin,10,6 g (44 mmol) Ethyl-p-tolylsulphonyl-acetat und 1,5 g (176 mmol) Piperidin in 30 ml Ethanol wurde 1 Std. bei Rückflußtemperatur gerührt. Anschließend goß man das Reaktionsgemisch in Diethylether, wonach der entstandene Feststoff abgetrennt und mit Diethylether gewaschen wurde.
Ausbeute: 45 %; Smp.: > 200 ° C.

In den folgenden Tabellen 1 bis 5 sind noch weitere Verbindungen aufgeführt, die auf die gleichen Weisen hergestellt wurden oder herstellbar sind.

Tabelle 1

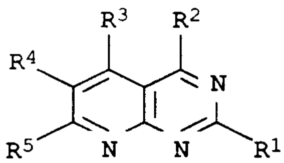

$R^4 = H$

| Beispiel-Nr. | $R^1$ | $R^2$ | $R^3$ | $R^5$ | Fp. [°C] | Lit. |
|---|---|---|---|---|---|---|
| 1.001 | H | H | H | $C_6H_5$ | 188 | a) |
| 1.002 | H | H | H | $4-CH_3-C_6H_4$ | 230 | |
| 1.003 | H | H | H | 1-Naphthyl | 272 | a) |
| 1.004 | H | H | H | Thien-2-yl | 194-195 | |
| 1.005 | H | H | H | Pyridin-2-yl | 200 | a) |
| 1.006 | H | H | OH | $C_6H_5$ | 294 | d) |
| 1.007 | $CH_3$ | H | H | $C_6H_5$ | > 200 | |
| 1.008 | $CH_3$ | H | H | $2-CH_3-C_6H_4$ | 184 | |
| 1.009 | $CH_3$ | H | H | $4-CH_3-C_6H_4$ | > 200 | |
| 1.010 | $CH_3$ | H | H | $2-F-C_6H_4$ | 200-201 | |
| 1.011 | $CH_3$ | H | H | $3-F-C_6H_4$ | 198-200 | |
| 1.012 | $CH_3$ | H | H | $4-F-C_6H_4$ | > 200 | |
| 1.013 | $CH_3$ | H | H | $2-Cl-C_6H_4$ | 174-178 | |
| 1.014 | $CH_3$ | H | H | $3-Cl-C_6H_4$ | 156-160 | |
| 1.015 | $CH_3$ | H | H | $4-Cl-C_6H_4$ | > 200 | |
| 1.016 | $CH_3$ | H | H | $3-Br-C_6H_4$ | 178-181 | |
| 1.017 | $CH_3$ | H | H | $3-CH_3O-C_6H_4$ | 150-155 | |
| 1.018 | $CH_3$ | H | H | $4-CH_3O-C_6H_4$ | > 200 | |
| 1.019 | $CH_3$ | H | H | 4-Biphenyl | > 200 | |
| 1.020 | $CH_3$ | H | H | 4-t.-Butyl-$C_6H_4$ | > 200 | |
| 1.021 | $CH_3$ | H | H | $4-NO_2-C_6H_4$ | > 200 | |
| 1.022 | $CH_3$ | H | H | $4-CN-C_6H_4$ | > 200 | |
| 1.023 | $CH_3$ | H | H | $3,4-Cl_2-C_6H_3$ | > 200 | |
| 1.024 | $CH_3$ | H | H | $2,4-(OCH_3)_2-C_6H_3$ | 165-167 | |
| 1.025 | $CH_3$ | H | H | $3,4-(OCH_3)_2-C_6H_3$ | 188-192 | |
| 1.026 | $CH_3$ | H | H | 3,4-Methylendioxy-$C_6H_3$ | | |
| 1.027 | $CH_3$ | H | H | $3-NO_2-4-Cl-C_6H_3$ | > 200 | |
| 1.028 | $CH_3$ | H | H | $3-NO_2-4-OCH_3-C_6H_3$ | > 200 | |

| Beispiel-Nr. | $R^1$ | $R^2$ | $R^3$ | $R^5$ | Fp. [°C] | Lit. |
|---|---|---|---|---|---|---|
| 1.029 | $CH_3$ | H | H | 2-Naphthyl | > 200 | |
| 1.030 | $CH_3$ | H | H | Thien-2-yl | 175-180 | |
| 1.031 | $CH_3$ | H | H | Thien-3-yl | 191-193 | |
| 1.032 | $CH_3$ | H | H | 5-Cl-thien-2-yl | > 200 | |
| 1.033 | $CH_3$ | H | H | 5-$CH_3$-isoxazol-3-yl | > 200 | |
| 1.034 | $CH_3$ | $CH_3$ | H | Pyridin-2-yl | 184 | |
| 1.035 | $CH_3$ | $CH_3$ | H | Pyridin-3-yl | 191-197 | |
| 1.036 | $CH_3$ | $CH_3$ | H | Pyridin-4-yl | 190-191 | |
| 1.037 | $CH_3$ | $CH_3$ | H | $C_6H_5$ | 135-136 | |
| 1.038 | $CH_3$ | $CH_3$ | H | 2-$CH_3$-$C_6H_4$ | 131 | |
| 1.039 | $CH_3$ | $CH_3$ | H | 4-$CH_3$-$C_6H_4$ | 162-165 | |
| 1.040 | $CH_3$ | $CH_3$ | H | Thien-2-yl | 167-168 | |
| 1.041 | $CH_3$ | $CH_3$ | H | Furan-2-yl | 157-160 | |
| 1.042 | $C_6H_5$ | H | H | $C_6H_5$ | 190 | |
| 1.043 | $C_6H_5$ | H | H | 4-$CH_3$-$C_6H_4$ | 226 | |
| 1.044 | $C_6H_5$ | H | H | Thien-2-yl | 172-176 | |
| 1.045 | $C_6H_5$ | H | H | Pyridin-2-yl | 200 | |
| 1.046 | H | $C_6H_5$ | H | $C_6H_5$ | 128-130 | b) |
| 1.047 | H | $C_6H_5$ | H | 4-Cl-$C_6H_4$ | 202-204 | b) |
| 1.048 | H | $C_6H_5$ | H | 4-F-$C_6H_4$ | 153-155 | b) |
| 1.049 | $C_6H_5$ | $CH_3$ | H | $C_6H_5$ | 210-213 | |
| 1.050 | $C_6H_5$ | $CH_3$ | H | 4-$CH_3$-$C_6H_4$ | 194-197 | |
| 1.051 | $C_6H_5$ | $CH_3$ | H | Thien-2-yl | 188-191 | |
| 1.052 | $OCH_3$ | $OCH_3$ | H | $C_6H_5$ | > 250 | |
| 1.053 | $OCH_3$ | $OCH_3$ | H | 4-$CH_3$-$C_6H_4$ | > 250 | |
| 1.054 | $OCH_3$ | $OCH_3$ | H | Thien-2-yl | 201-204 | |
| 1.055 | $CH_2C_6H_5$ | H | H | $C_6H_5$ | 135-137 | |
| 1.056 | $CH_2C_6H_5$ | H | H | 4-$CH_3$-$C_6H_4$ | 181-182 | |
| 1.057 | $CH_2C_6H_5$ | H | H | Thienyl | 190-191 | |
| 1.058 | $CH_3$ | H | H | Tetralin-2-yl | 223-226 | |
| 1.059 | $CH_3$ | H | H | 2-Cl-5-$NO_2$-$C_6H_3$ | 209-211 | |
| 1.060 | $CH_3$ | H | H | 2,5-$(CH_3)_2$-thien-3-yl | 170 | |
| 1.061 | $CH_3$ | H | H | 3-$CH_3$-thien-2-yl | 120 | |
| 1.062 | $CH_3$ | H | H | 5-$CH_3$-thien-2-yl | Öl | |
| 1.063 | $CH_3$ | H | H | 2,5-$Cl_2$-thien-3-yl | 211-212 | |

| Beispiel-Nr. | $R^1$ | $R^2$ | $R^3$ | $R^5$ | Fp. [°C] | Lit. |
|---|---|---|---|---|---|---|
| 1.064 | $CH_3$ | H | H | Benzthien-2-yl | 176-178 | |
| 1.065 | $CH_3$ | H | H | 2,5-$Cl_2$-$C_6H_3$ | 152-156 | |
| 1.066 | $CH_3$ | H | H | 3,5-$Cl_2$-$C_6H_3$ | 166 | |
| 1.067 | $CH_3$ | H | H | 2,3,4-$Cl_3$-$C_6H_2$ | > 200 | |
| 1.068 | $CH_3$ | H | H | 2-$CH_3O$-3,5-$Cl_2$-$C_6H_2$ | 196-197 | |
| 1.069 | $CH_3$ | H | H | 2-$CH_3O$-$C_6H_4$ | 160-165 | |
| 1.070 | $CH_3$ | H | H | 4-$CF_3$-$C_6H_4$ | 182 | |
| 1.071 | $CH_3$ | H | H | 4-N$(CH_2)_5$-$C_6H_4$ | > 200 | |

Tabelle 2

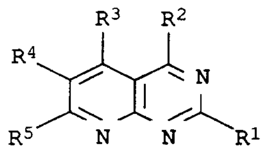

$R^3$ = H

| Bsp.Nr. | $R^4$ | $R^1$ | $R^2$ | $R^5$ | Fp.[°C] | Lit. |
|---|---|---|---|---|---|---|
| 2.001 | H | H | H | $NHSO_2-C_6H_5$ | 188 | |
| 2.002 | $CH_3$ | H | H | $C_6H_5$ | 169 | a) |
| 2.003 | $C_6H_5$ | H | H | $CH_3$ | 203 | a) |
| 2.004 | $C_6H_5$ | H | H | $C_6H_5$ | 157 | a) |
| 2.005 | $2,6-Cl_2-C_6H_3$ | H | H | $NHCOCH_3$ | 215-217 | f) |
| 2.006 | $CH_3$ | $CH_3$ | H | $C_6H_5$ | 175-178 | |
| 2.007 | $C_2H_5$ | $CH_3$ | H | $C_6H_5$ | 92- 94 | |
| 2.008 | $n-C_3H_7$ | $CH_3$ | H | $C_6H_5$ | 77- 81 | |
| 2.009 | $2,6-Cl_2-C_6H_3$ | $CH_3$ | H | NHCOH | 257-259 | f) |
| 2.010 | $2,6-Cl_2-C_6H_3$ | $CH_3$ | H | $NHCOCH_3$ | 202-203 | f) |
| 2.011 | $2,6-Cl_2-C_6H_3$ | $CH_3$ | H | $NHCOC_2H_5$ | 192-193 | f) |
| 2.012 | $2,6-Cl_2-C_6H_3$ | $CH_3$ | H | $NHCO_2CH_3$ | 136-139 | f) |
| 2.013 | H | $CH_3$ | $CH_3$ | $NHSO_2-(2-Cl-6-CH_3-C_6H_3)$ | > 215 | e) |
| 2.014 | H | $CH_3$ | $CH_3$ | $NHSO_2-(2-Carbo-methoxy-6-CH_3-C_6H_3)$ | 171-173 | e) |
| 2.015 | H | $CH_3$ | $CH_3$ | $NHSO_2-(2,6-Cl_2-C_6H_3)$ | >230 | e) |
| 2.016 | H | $CH_3$ | $CH_3$ | $NHSO_2-(2-Cl-6-CH_3-C_6H_3)$ | > 215 | e) |
| 2.017 | H | $CH_3$ | $CH_3$ | $NHSO_2-(3-OCH_3-C_6H_4)$ | 193-194 | |
| 2.018 | H | $CH_3$ | $CH_3$ | $NHSO_2-(2-Cl-C_6H_4)$ | 231-232 | |
| 2.019 | H | $CH_3$ | $CH_3$ | $NHSO_2-(2-F-C_6H_4)$ | 172-173 | |
| 2.020 | $CH_3$ | $CH_3$ | $CH_3$ | $C_6H_5$ | 120-123 | |
| 2.021 | $C_6H_5$ | $CH_3$ | $CH_3$ | $CH_3$ | 120-123 | |
| 2.022 | $SO_2CH_3$ | $CH_3$ | $CH_3$ | $NHSO_2-(2-Cl-C_6H_4)$ | >230 | e) |
| 2.023 | $SO_2CH_3$ | $CH_3$ | $CH_3$ | $NHSO_2-(2,5-Cl_2-C_6H_3)$ | >230 | e) |
| 2.024 | $SO_2CH_3$ | $CH_3$ | $CH_3$ | $NHSO_2-(2-F-C_6H_4)$ | 107-109 | e) |
| 2.025 | $SO_2CH_3$ | $CH_3$ | $CH_3$ | $NHSO_2-(2-Carbo-methoxyphenyl)$ | 220-222 | e) |

| Bsp.Nr. | $R^4$ | $R^1$ | $R^2$ | $R^5$ | Fp. [°C] | Lit. |
|---|---|---|---|---|---|---|
| 2.026 | $SO_2CH_3$ | $CH_3$ | $CH_3$ | $NHSO_2$-(2-Cl-6-cyclopentyl-$C_6H_3$) | > 215 | e) |
| 2.027 | $SO_2CH_3$ | $CH_3$ | $CH_3$ | $NHSO_2$-(2,6-$Cl_2$-$C_6H_3$) | > 230 | e) |
| 2.028 | $C_6H_5$ | $OCH_3$ | $OCH_3$ | $NHSO_2$-(2-$Cl_2$-$C_6H_4$) | 226-228 | e) |
| 2.029 | $C_6H_5$ | $OCH_3$ | $OCH_3$ | $NHSO_2$-(2-F-$C_6H_4$) | 198-199 | e) |
| 2.030 | $C_6H_5$ | $OCH_3$ | $OCH_3$ | $NHSO_2$-(2,6-$Cl_2$-$C_6H_3$) | > 230 | e) |
| 2.031 | $C_6H_5$ | $OCH_3$ | $OCH_3$ | $NHSO_2$-(2-Carbo-methoxyphenyl) | 108-111 | e) |
| 2.032 | $SO_2CH_3$ | $OCH_3$ | $OCH_3$ | $NHSO_2$-(2-Cl-$C_6H_4$) | > 215 | e) |
| 2.033 | $SO_2CH_3$ | $OCH_3$ | $OCH_3$ | $NHSO_2$-(2-F-$C_6H_4$) | 155-157 | e) |
| 2.034 | $SO_2CH_3$ | $OCH_3$ | $OCH_3$ | $NHSO_2$-(2,6-$Cl_2$-$C_6H_3$) | 213-214 | e) |
| 2.035 | $SO_2CH_3$ | $OCH_3$ | $OCH_3$ | $NHSO_2$-(2,5-$Cl_2$-$C_6H_3$) | 172-175 | e) |
| 2.036 | $SO_2CH_3$ | $OCH_3$ | $OCH_3$ | $NHSO_2$-(2-Cl-6-$CH_3$-$C_6H_3$) | 226-227 | e) |
| 2.037 | $SO_2CH_3$ | $OCH_3$ | $OCH_3$ | $NHSO_2$-(2,5-$(OCH_3)_2$-$C_6H_3$) | 128 | e) |
| 2.038 | $C_6H_5$ | $OC_2H_5$ | $OC_2H_5$ | $NHSO_2$-(2-F-$C_6H_4$) | 198 | e) |
| 2.039 | $C_6H_5$ | $OC_2H_5$ | $OC_2H_5$ | $NHSO_2$-(2-Cl-$C_6H_4$) | 169-173 | e) |
| 2.040 | $CH_3$ | $CH_3$ | H | 3-Thienyl | 172-175 | |
| 2.041 | $CH_3$ | $CH_3$ | H | 2-Thienyl | 152-154 | |

Tabelle 3

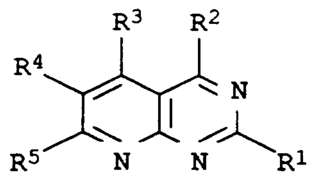

$R^5$ = OH

| Bsp.Nr. | $R^1$ | $R^2$ | $R^3$ | $R^4$ | Fp. [°C] | Lit. |
|---|---|---|---|---|---|---|
| 3.001 | H | H | H | $CO_2C_2H_5$ | 196-199 | |
| 3.002 | H | H | H | CN | > 250 | |
| 3.003 | H | H | OH | H | > 340 | d) |
| 3.004 | H | H | OH | $CH_3$ | > 360 | d) |
| 3.005 | H | H | OH | $C_2H_5$ | 317 | d) |
| 3.006 | H | H | OH | $C_6H_5$ | > 360 | d) |
| 3.007 | H | H | OH | $CO_2C_2H_5$ | 246-248 | d) |
| 3.008 | $CH_3$ | H | H | $CO_2C_2H_5$ | > 200 | c) |
| 3.009 | $CH_3$ | H | H | CN | > 200 | |
| 3.010 | $CH_3$ | H | H | $SO_2CH_3$ | > 250 | |
| 3.011 | $CH_3$ | H | H | $SO_2-(4-CH_3-C_6H_4)$ | > 200 | |
| 3.012 | $CH_3$ | H | H | $4-F-C_6H_4$ | > 260 | |
| 3.013 | $CH_3$ | $CH_3$ | H | $CO_2C_2H_5$ | 176-177 | |
| 3.014 | $CH_3$ | $CH_3$ | H | CN | > 250 | |
| 3.015 | $C_6H_5$ | H | H | $CO_2C_2H_5$ | > 250 | |
| 3.016 | $C_6H_5$ | H | H | CN | > 250 | |
| 3.017 | $C_6H_5$ | $CH_3$ | H | $CO_2C_2H_5$ | > 230 | |
| 3.018 | $C_6H_5$ | $CH_3$ | H | CN | > 250 | |
| 3.019 | $OCH_3$ | $OCH_3$ | H | $CO_2C_2H_5$ | 240-243 | |
| 3.020 | $OCH_3$ | $OCH_3$ | H | CN | > 250 | |
| 3.021 | $CH_2C_6H_5$ | H | H | $CO_2C_2H_5$ | 193 | |
| 3.022 | $CH_2C_6H_5$ | H | H | CN | 180-185 | |
| 3.023 | $CH_3$ | H | H | 2-Pyridyl | > 220 | |

Tabelle 4

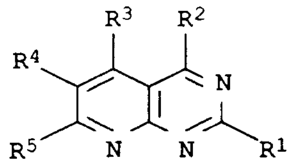

$R^5 = NH_2$

| Bsp.Nr. | $R^1$ | $R^2$ | $R^3$ | $R^4$ | Fp. [°C] | Lit. |
|---|---|---|---|---|---|---|
| 4.001 | H | H | OH | H | > 340 | d) |
| 4.002 | H | H | OH | $C_6H_5$ | > 340 | d) |
| 4.003 | H | H | H | $C_6H_5$ | 289-290 | g) |
| 4.004 | H | H | H | $2-CH_3-C_6H_4$ | 253-255 | f) |
| 4.005 | H | H | H | $2-Cl-C_6H_4$ | 269-270 | g) |
| 4.006 | H | H | H | $2-Br-C_6H_4$ | 265-267 | g) |
| 4.007 | H | H | H | $4-Br-C_6H_4$ | 265-267 | f) |
| 4.008 | H | H | H | $2,6-Cl_2-C_6H_3$ | 328-330 | f) |
| 4.009 | H | H | H | Pyridin-3-yl | 295-297 | g) |
| 4.010 | $CH_3$ | H | H | $C_6H_5$ | 229-230 | g) |
| 4.011 | $CH_3$ | H | H | $2-CH_3-C_6H_4$ | 234-235 | f) |
| 4.012 | $CH_3$ | H | H | $3-CH_3-C_6H_4$ | 180-188 | |
| 4.013 | $CH_3$ | H | H | $2-Cl-C_6H_4$ | 256-260 | f) |
| 4.014 | $CH_3$ | H | H | $3-Cl-C_6H_4$ | 208-209 | |
| 4.015 | $CH_3$ | H | H | $4-Cl-C_6H_4$ | 262-264 | g) |
| 4.016 | $CH_3$ | H | H | $2-F-C_6H_4$ | 278-279 | g) |
| 4.017 | $CH_3$ | H | H | $4-F-C_6H_4$ | 252-254 | |
| 4.018 | $CH_3$ | H | H | $2-Br-C_6H_4$ | 228-230 | f) |
| 4.019 | $CH_3$ | H | H | $3-CH_3O-C_6H_4$ | 175-180 | |
| 4.020 | $CH_3$ | H | H | $4-CH_3O-C_6H_4$ | > 200 | |
| 4.021 | $CH_3$ | H | H | $4-NO_2-C_6H_4$ | 299-301 | g) |
| 4.022 | $CH_3$ | H | H | $2,4-Cl_2-C_6H_3$ | 259-261 | f) |
| 4.023 | $CH_3$ | H | H | $2,6-Cl_2-C_6H_3$ | 288-290 | g) |
| 4.024 | $CH_3$ | H | H | Carbamoyl | 232-233 | g) |
| 4.025 | $CH_3$ | H | H | Pyridin-3-yl | 296-298 | g) |
| 4.026 | $CH_3$ | $CH_3$ | H | $2-CH_3-C_6H_4$ | 234-235 | g) |
| 4.027 | $CH_3$ | $CH_3$ | H | $2-F-C_6H_4$ | 222-223 | |
| 4.028 | $CH_3$ | $CH_3$ | H | $3-F-C_6H_4$ | > 230 | |
| 4.029 | $CH_3$ | $CH_3$ | H | $2-CH_3O-C_6H_4$ | 210-212 | |

| Bsp.Nr. | $R^1$ | $R^2$ | $R^3$ | $R^4$ | Fp. [°C] | Lit. |
|---|---|---|---|---|---|---|
| 4.030 | $CH_3$ | $CH_3$ | H | $3\text{-}CH_3O\text{-}C_6H_4$ | 211-214 | |
| 4.031 | $CH_3$ | $CH_3$ | H | $4\text{-}CH_3O\text{-}C_6H_4$ | > 250 | |
| 4.032 | $CH_3$ | $CH_3$ | H | $2,6\text{-}Cl_2\text{-}C_6H_3$ | 239-240 | f) |
| 4.033 | $CH_3$ | $CH_3$ | H | CN | > 210 | |

Tabelle 5

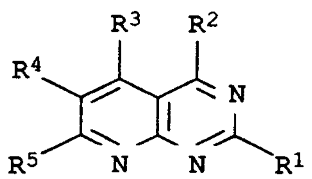

$R^4$ = CN

| Bsp.Nr. | $R^3$ | $R^1$ | $R^2$ | $R^5$ | Fp. [°C] | Lit. |
|---|---|---|---|---|---|---|
| 5.001 | H | $CH_3$ | $CH_3$ | $NHSO_2\text{-}(2\text{-}Cl\text{-}C_6H_4)$ | > 230 | e) |
| 5.002 | H | $CH_3$ | $CH_3$ | $NHSO_2\text{-}(2\text{-}F\text{-}C_6H_4)$ | 210-212 | e) |
| 5.003 | H | $CH_3$ | $CH_3$ | $NHSO_2\text{-}(2,6\text{-}Cl_2\text{-}C_6H_3)$ | > 230 | e) |
| 5.004 | H | $CH_3$ | $CH_3$ | $NHSO_2\text{-}(2,5\text{-}Cl_2\text{-}C_6H_3)$ | > 230 | e) |
| 5.005 | H | $CH_3$ | $CH_3$ | $NHSO_2\text{-}(2,5\text{-}(CH_3O)_2\text{-}C_6H_3)$ | 137 | e) |
| 5.006 | H | $CH_3$ | $CH_3$ | $NHSO_2\text{-}(2\text{-}Cl\text{-}6\text{-}CH_3\text{-}C_6H_3)$ | 233-235 | e) |
| 5.007 | H | $CH_3$ | $CH_3$ | $NHSO_2\text{-}(2\text{-carbomethoxy-}C_6H_4)$ | 220-225 | e) |
| 5.008 | H | $OCH_3$ | $OCH_3$ | $NHSO_2\text{-}C_6H_5$ | 199-201 | e) |
| 5.009 | H | $OCH_3$ | $OCH_3$ | $NHSO_2\text{-}(2\text{-}F\text{-}C_6H_4)$ | 153-156 | e) |
| 5.010 | H | $OCH_3$ | $OCH_3$ | $NHSO_2\text{-}(2,6\text{-}Cl_2\text{-}C_6H_3)$ | 218-220 | e) |
| 5.011 | H | $OCH_3$ | $OCH_3$ | $NHSO_2\text{-}(2\text{-carbomethoxy-}C_6H_4)$ | 211-215 | e) |

Literatur:


a) Evans et al., J. Org. Chem. 40, 1438 (1975)
b) Söllhuber-Kretzer et al., Arch. Pharm. 316, 346 (1983)
c) Nishino et al., Bull Chem. Soc. Jpn. 45, 1127 (1972)
d) Bredereck et al., Chem. Ber. 96, 1868 (1963)
e) EP-A 329 012 (BASF)
f) EP-A 18 151 (Warner-Lambert)
g) Bennett et al., J. Med. Chem. 24, 382 (1981)


Beispiele zur biologischen Wirkung

Der Einfluß verschiedener Vertreter der erfindungsgemäßen herbiziden Mittel bzw. Mittelkombinationen, bestehend aus Herbizid und antidotisch wirkender Verbindung, auf das Wachstum von erwünschten und unerwünschten Pflanzen im Vergleich zum herbiziden Wirkstoff allein wird durch die folgenden biologischen Beispiele aus Gewächshausversuchen belegt:
Bei Gewächshausversuchen dienten als Kulturpflanzen Plastikblumentöpfe mit rund 300 $cm^3$ Inhalt und

lehmigem Sand mit etwa 3,0 Gew.-% Humus als Substrat. Die Samen der Testpflanzen wurden nach Arten getrennt, flach eingesät und befeuchtet. Danach wurden die Gefäße mit durchsichtigen Plastikhauben abgedeckt, bis die Samen gleichmäßig gekeimt und die Pflanzen angewachsen waren.

Liste der Testpflanzen

| Lateinischer Name | Deutscher Name | Englischer Name |
|---|---|---|
| Setaria viridis | Grüne Borstenhirse | green foxtail |
| Triticum aestivum | Sommerweizen | spring wheat |
| Zea mays | Mais | corn |

Für die Nachauflaufbehandlung wurden die Testpflanzen je nach Wuchsform erst bis zu einer Wuchshöhe von 3 bis 20 cm gezüchtet und dann behandelt. Die herbiziden Mittel wurden hierbei in Wasser als Verteilungsmittel suspendiert oder emulgiert und mittels fein verteilender Düsen gespritzt.

Als Beispielherbizide der Cyclohexenon-Derivate II dienten

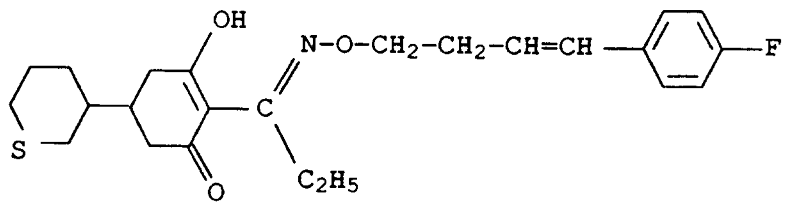

Nr. A.053

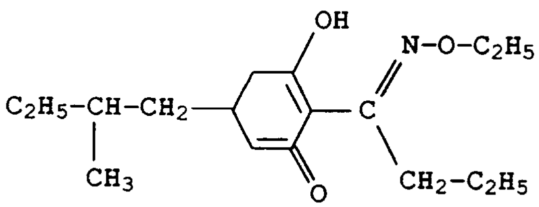

Nr. A.001

(Handelsname: Sethoxydim)

Sämtliche antidotisch wirkenden Verbindungen wurden für die Nachauflaufbehandlung in einem Gemisch, bestehend aus 80 Gew.-% Cyclohexanon als Verdünnungsmittel und 20 Gew.-% Tensid (Emulphor EL*)) mit 10 Gew.-% Wirkstoff aufbereitet.

Zum Vergleich wurde der herbizide Wirkstoff als 10 bis 20 gew.-%iger Emulsionskonzentrat formuliert und jeweils unter Zugabe von derjenigen Menge an Lösungsmittelsystem in die Spritzbrühe eingesetzt, mit welcher die antidotisch wirkende Verbindung in den Tabellen angegebenen Aufwandmengen ausgebracht wurden. Die Herstellung der Lösung erfolgte durch einmischen des Wirkstoffs in eine Lösung aus 93 Gew.-% Xylol und Gew.-% Lutensoll AP-8 **).

Nach Applikation der jeweiligen Wirkstoffmischung wurden die Testpflanzen im Gewächshaus kultiviert, und zwar wärmeliebende Arten bei etwa 18 bis 30 ° C, solche gemäßigterer Klimate bei ca. 10 bis 25 ° C.

Die Versuchsperiode erstreckte sich über 3 bis 5 Wochen. Während dieser Zeit wurden die Pflanzen gepflegt, wobei ihre Reaktionen auf die Wirkstoff-Behandlungen erfaßt wurden.

*) ethoxyliertes Rizinusöl (caster oil)

**) nichtionisches oberflächenaktives Mittel auf Bagis von Alkylphenolpolyethylenglykolether

Bewertet wurde die Schädigung durch die chemischen Mittel anhand einer Skala von 0 bis 100 % im Vergleich zu den unbehandelten Kontrollpflanzen. Dabei bedeutet 0 keine Schädigung und 100 eine völlige Zerstörung der Pflanzen.

Die Verbesserung der Verträglichkeit von herbiziden Cyclohexenon-Derivaten II für Kulturpflanzen aus der Familie der Gramineen (Gräser) wie Weizen und Mais durch die Pyrido[2,3-d]pyrimidine I ist den folgenden Tabellen X.1 bis X.5 zu entnehmen:

Tabelle X.1

Verbesserung der Verträglichkeit des Herbizids Nr. A.001 für Mais durch Zumischen einer antidotischen Beispielverbindung bei Nachauflaufanwendung; Gewächshausversuch

| Antidot Nr. | Aufwandmenge [kg/ha a.S.] | | Testpflanzen und Schädigung [%] | |
|---|---|---|---|---|
| | Antidot | Herbizid | Kulturpfanze Mais (Sorte "Lixis") | unerwünschte Pflanze Setaria viridis |
| --- | --- | 0,015 | 90 | 85 |
| 2.039 | 0,015 | 0,015 | 15 | 75 |
| 4.026 | 0,015 | 0,015 | 55 | 85 |
| 4.013 | 0,015 | 0,015 | 55 | 80 |
| 4.010 | 0,015 | 0,015 | 40 | 80 |
| 4.015 | 0,015 | 0,015 | 50 | 85 |
| 4.019 | 0,015 | 0,015 | 25 | 85 |
| 1.007 | 0,015 | 0,015 | 15 | 70 |
| 1.009 | 0,015 | 0,015 | 40 | 75 |
| 1.030 | 0,015 | 0,015 | 25 | 75 |
| 1.012 | 0,015 | 0,015 | 55 | 75 |

Tabelle X.2

Verbesserung der Verträglichkeit des Herbizids Nr. A.001 für Mais und Weizen durch Zumischen einer antidotischen Beispielverbindung bei Nachauflaufanwendung; Gewächshausversuch

| Antidot Nr. | Aufwandmenge [kg/ha a.S.] Antidot | Herbizid | Testpflanzen und Schädigung [%] Kulturpfanzen Mais*) | Weizen**) | unerwünschte Pflanze Setaria viridis |
|---|---|---|---|---|---|
| --- | --- | 0,06 | 95 | 75 | 95 |
| 2.039 | 0,06 | 0,06 | 65 | 40 | 95 |
| 4.033 | 0,06 | 0,06 | 75 | 40 | 95 |
| 4.019 | 0,06 | 0,06 | -- | 45 | 98 |
| 1.007 | 0,06 | 0,06 | -- | 0 | 95 |
| 1.009 | 0,06 | 0,06 | -- | 20 | 95 |
| 1.030 | 0,06 | 0,06 | 60 | 15 | 90 |

*) Sorte "Lixis" **) Sommerweizen, Sorte "Star" Tabelle 7

Tabelle X.3

Verbesserung der Verträglichkeit des Herbizids Nr. A.053 für Mais und Weizen durch Zumischen einer antidotischen Beispielverbindung bei Nachauflaufanwendung; Gewächshausversuch

| Antidot Nr. | Aufwandmenge [kg/ha a.S.] | | Testpflanzen und Schädigung [%] | | |
|---|---|---|---|---|---|
| | | | Kulturpfanzen | | unerwünschte Pflanze |
| | Antidot | Herbizid | Mais*) | Weizen**) | Setaria viridis |
| --- | --- | 0,03 | 90 | 70 | 98 |
| 1.015 | 0,03 | 0,03 | 50 | 30 | 98 |
| 1.009 | 0,03 | 0,03 | 20 | 20 | 90 |
| 1.030 | 0,03 | 0,03 | 10 | 20 | 70 |
| 1.021 | 0,03 | 0,03 | -- | 20 | 90 |
| 1.020 | 0,03 | 0,03 | -- | 35 | 95 |
| 1.008 | 0,03 | 0,03 | -- | 30 | 95 |
| 1.031 | 0,03 | 0,03 | -- | 10 | 85 |
| 1.019 | 0,03 | 0,03 | -- | 30 | 90 |
| 1.023 | 0,03 | 0,03 | -- | 10 | 85 |
| 1.024 | 0,03 | 0,03 | -- | 0 | 80 |
| 1.013 | 0,03 | 0,03 | -- | 20 | 75 |
| 1.032 | 0,03 | 0,03 | -- | 20 | 80 |
| 1.022 | 0,03 | 0,03 | -- | 40 | 98 |

*) Sorte "Lixis"   **) Sommerweizen, Sorte "Star"

Tabelle X.4

Verbesserung der Verträglichkeit des Herbizids Nr. A.721 für Mais und Weizen durch Zumischen einer antidotischen Beispielverbindung bei Nachauflaufanwendung; Gewächshausversuch

| Antidot Nr. | Aufwandmenge [kg/ha a.S.] | | Testpflanzen und Schädigung | | |
|---|---|---|---|---|---|
| | Antidot | Herbizid | Kulturpflanzen | | unerwünschte Pflanzee Setaria viridis |
| | | | Mais* | Weizen** | |
| --- | --- | 0,125 | 35 | 90 | 100 |
| 1.001 | 0,125 | 0,125 | - | 20 | 100 |
| 1.004 | 0,125 | 0,125 | - | 25 | 98 |
| 1.044 | 0,125 | 0,125 | 0 | 45 | 100 |
| 1.052 | 0,125 | 0,125 | 0 | - | 100 |
| 1.062 | 0,125 | 0,125 | 0 | - | 100 |
| 3.002 | 0,125 | 0,125 | 0 | 10 | 98 |
| 3.013 | 0,125 | 0,125 | 0 | 45 | 100 |
| 3.014 | 0,125 | 0,125 | 0 | 65 | 100 |
| 3.018 | 0,125 | 0,125 | 0 | 45 | 100 |

* Sorte "Merlin" ** Sommerweizen, Sorte "Star"

Tabelle X.5

Verbesserung der Verträglichkeit des Herbizids Nr. A.721 für Mais durch Zumischen einer antidotischen Beispielverbindung bei Nachauflaufanwendung; Gewächshausversuch

| Antidot Nr. | Aufwandmenge [kg/ha a.S.] | | Testpflanzen und Schädigung | |
|---|---|---|---|---|
| | Antidot | Herbizid | Kulturpflanze Mais* | unerwünschte Pflanze Setaria viridis |
| --- | --- | 0,125 | 40 | 100 |
| 2.014 | 0,125 | 0,125 | 0 | 85 |
| 2.023 | 0,125 | 0,125 | 20 | 95 |
| 2.024 | 0,125 | 0,125 | 0 | 100 |
| 2.025 | 0,125 | 0,125 | 0 | 85 |
| 2.027 | 0,125 | 0,125 | 15 | 95 |
| 2.028 | 0,125 | 0,125 | 10 | 100 |
| 2.029 | 0,125 | 0,125 | 0 | 85 |
| 2.033 | 0,125 | 0,125 | 0 | 95 |
| 2.036 | 0,125 | 0,125 | 0 | 90 |
| 2.037 | 0,125 | 0,125 | 25 | 95 |
| 5.005 | 0,125 | 0,125 | 10 | 95 |

* Sorte "Merlin"

## Patentansprüche

1. Herbizide Mittel, enthaltend mindestens ein substituiertes Pyrido[2,3-d]pyrimidin der allgemeinen Formel I

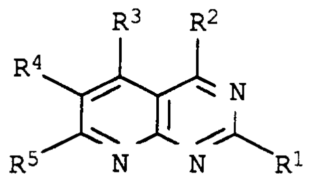

I

in der die Variablen folgende Bedeutung haben:

$R^1$, $R^2$
Wasserstoff; $C_1$-$C_8$-Alkyl; $C_1$-$C_8$-Halogenalkyl; $C_1$-$C_6$-Alkoxy; $C_1$-$C_6$-Halogenalkoxy; $C_1$-$C_4$-Alkoxy-$C_1$-$C_6$-alkyl; $C_1$-$C_8$-Alkylamino; $C_2$-$C_8$-Alkenyl; $C_2$-$C_8$-Alkinyl;

$C_3$-$C_8$-Cycloalkyl, an welches ein Benzolrest anneliert sein kann, wobei diese Gruppe noch ein bis drei der folgenden Reste tragen kann: Hydroxy, Halogen, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

Phenyl, Naphthyl, Phenyl-$C_1$-$C_6$-alkyl, 5-gliedrige aromatische Ringe, welche neben Kohlenstoffatomen ein bis drei Stickstoffatome und ein Sauerstoff- oder ein Schwefelatom als Heteroatome enthalten können, oder welche neben Kohlenstoffatomen ein bis drei Stickstoffatome oder ein Sauerstoff- oder ein Schwefelatom als Heteroatome enthalten können, 6-gliedrige aromatische Ringe, welche neben Kohlenstoffatomen ein bis drei Stickstoffatome als Heteroatome enthalten können, wobei an die vorstehend genannten 5- und 6-gliedrigen Heteroaromaten ein Benzolring anneliert sein kann, und wobei die aromatischen und heteroaromatischen Reste zusätzlich ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_3$-$C_6$-Alkenyl und $C_3$-$C_6$-Alkinyl;

$R^3$
Hydroxy; Amino; Halogen; $C_1$-$C_6$-Alkylthio; Di-($C_1$-$C_8$-alkyl)-amino; $C_3$-$C_8$-Cycloalkylamino; $C_1$-$C_8$-Alkoxycarbonyl;

oder eine der für $R^1$ genannten Gruppen;

$R^4$
eine der für $R^1$ genannten Gruppen;

CN; $NO_2$; COOH; CSOH; Di-($C_1$-$C_4$-alkyl)-amino-$C_1$-$C_4$-alkyl;

$SO_2$-$R^6$; C(=X)-$R^7$; C(=Y)-$R^8$, oder $R^7$-C($YR^9$)-$ZR^{10}$;

| | |
|---|---|
| $R^6$ | eine der für $R^1$ genannten Gruppen; |
| | Hydroxy; Amino; Di-($C_1$-$C_8$-alkyl)-amino; $C_3$-$C_8$-Cycloalkylamino; $C_1$-$C_6$-Alkylthio; |
| $R^7$ | Amino; Oxyamino (-NH-OH); $C_1$-$C_8$-Alkylamino; |
| | Di-($C_1$-$C_8$-alkyl)-amino; $C_3$-$C_8$-Cycloalkylamino; $C_1$-$C_8$-Alkoxy; $C_1$-$C_6$-Alkylthio; Phenylamino; |
| $R^8$ | eine der für $R^1$ genannten Gruppen; |
| $R^9$,$R^{10}$ | $C_1$-$C_8$-Alkyl; $C_1$-$C_6$-Halogenalkyl; |
| | $C_1$-$C_4$-Alkoxy-$C_2$-$C_6$-alkyl; $C_2$-$C_8$-Alkenyl; oder |
| $R^9$ und $R^{10}$ | gemeinsam $-CH_2CH_2-$, $-CH_2CH_2CH_2-$ oder $-CH_2CH_2CH_2CH_2-$, wobei ein oder zwei Wasserstoffatome in diesen Gruppen durch die folgenden Reste ersetzt sein können: =O, $C_1$-$C_8$-Alkyl, $C_1$-$C_6$-Halogenalkyl oder $C_1$-$C_6$-Alkoxy; |
| X | Sauerstoff, Schwefel oder $NR^{11}$, worin |
| $R^{11}$ | für eine der für $R^1$ genannten Gruppen steht, oder die folgende Bedeutung hat: |

Wasserstoff; Hydroxy; Amino; Di-($C_1$-$C_8$-alkyl)-amino; $C_3$-$C_8$-Cycloalkylamino;

Phenoxy, Naphthyloxy, Phenylamino oder Naphthylamino, wobei die aromatischen Reste ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkylthio, $C_3$-$C_6$-Alkenyl und $C_3$-$C_6$-Alkinyl;

Y Sauerstoff oder Schwefel;

$R^5$

eine der für $R^1$ genannten Gruppen;

Hydroxy; Amino; Halogen; $C_1$-$C_6$-Alkylthio; Di-($C_1$-$C_8$-alkyl)-amino; $C_3$-$C_8$-Cycloalkylamino; Pyrrolidin-1-yl; Piperidin-1-yl; Morpholin-l-yl; $C_1$-$C_8$-Alkylcarbonyloxy; $C_1$-$C_4$-Halogenalkylcarbonyloxy; $C_1$-$C_8$-Alkylsulfonyloxy; $C_1$-$C_8$-Halogenalkylsulfonyloxy;

Phenoxy, Naphthyloxy, Phenylamino, Naphthylamino, Benzyloxy, Benzylamino, Benzoyloxy, 2-Naphthoyloxy oder Phenylsulfonyloxy, wobei die aromatischen Reste ein bis drei der folgenden Gruppen tragen können: Halogen, $C_1$-$C_4$-Alkyl, $C_1$-$C_4$-Halogenalkyl und $C_1$-$C_4$-Alkoxy;

$N(R^{12})$-$SO_2$-$R^{13}$; $N(R^{12})$-CO-$R^{14}$; $N(R^{12})$-CS-$R^{14}$;

$R^{12}$ Wasserstoff; $C_1$-$C_4$-Alkyl;

Phenyl, welches ein bis drei der folgenden Reste tragen kann: Halogen, $C_1$-$C_4$-Alkyl, $C_1$-$C_4$-Halogenalkyl und $C_1$-$C_4$-Alkoxy;

$R^{13}$ eine der für $R^1$ genannten Gruppen;

Amino, Di-($C_1$-$C_8$-alkyl)-amino oder $C_3$-$C_8$-Cycloalkylamino;

$R^{14}$ eine der für $R^1$ genannten Gruppen;

Amino; Oxyamino (-NH-OH); Di-($C_1$-$C_6$-alkyl)-amino; $C_3$-$C_8$-Cycloalkylamino;

sowie die pflanzenverträglichen Salze derjenigen Verbindungen I, bei denen mindestens einer der Substituenten $R^1$ bis $R^5$ eine saure oder basische Gruppe bedeutet,

und mindestens einen herbiziden Wirkstoff aus

A) der Gruppe der Cyclohexenon-Derivate der allgemeinen Formel II,

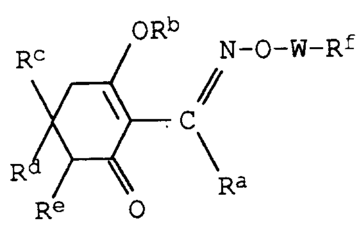

II

in der die Substituenten die folgende Bedeutung haben:

$R^a$

$C_1$-$C_6$-Alkyl;

$R^b$

Wasserstoff;

das Äquivalent eines landwirtschaftlich brauchbaren Kations;

$C_1$-$C_8$-Alkylcarbonyl; $C_1$-$C_{10}$-Alkylsulfonyl; $C_1$-$C_{10}$-Alkylphosphonyl;

Benzoyl, Benzolsulfonyl oder Benzolphosphonyl, wobei die aromatischen Ringe ein bis fünf Halogenatome tragen können;

$R^c$
Wasserstoff; CN; CHO;

$C_1$-$C_6$-Alkyl, welches einen der folgenden Reste tragen kann: $C_1$-$C_4$-Alkoxy, $C_1$-$C_4$-Alkylthio, Phenyloxy, Phenylthio, Pyridyloxy oder Pyridylthio, wobei die aromatischen Reste ihrerseits ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalk- oxy, $C_1$-$C_4$-Alkylthio, $C_3$-$C_6$-Alkenyl, $C_3$-$C_6$-Alkenyloxy, $C_3$-$C_6$-Alkinyl, $C_3$-$C_6$-Alkinyloxy oder $NR^gR^h$;

$R^g$ Wasserstoff; $C_1$-$C_4$-Alkyl; $C_3$-$C_6$-Alkenyl; $C_3$-$C_6$-Alkinyl; $C_1$-$C_6$-Alkylcarbonyl;

Benzoyl, welches ein bis drei der folgenden Reste tragen kann: Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio;

$R^h$ Wasserstoff; $C_1$-$C_4$-Alkyl; $C_3$-$C_6$-Alkenyl; $C_3$-$C_6$-Alkinyl;

$R^c$ bedeutet desweiteren:

$C_3$-$C_7$-Cycloalkyl oder $C_5$-$C_7$-Cycloalkenyl, wobei diese Ringe ein bis drei der folgenden Reste tragen können: Hydroxy, Halogen, $C_1$-$C_4$-Alkyl, $C_1$-$C_4$-Halogenalkyl, $C_1$-$C_4$-Alkoxy, $C_1$-$C_4$-Alkylthio, Benzylthio, $C_1$-$C_4$-Alkylsulfonyl, $C_1$-$C_4$-Alkylsulfenyl und $C_1$-$C_4$-Alkylsulfinyl;

5-gliedrige gesättigte Ringe, welche neben Kohlenstoffringgliedern ein oder zwei Sauerstoff- oder Schwefelatome oder ein Sauerstoff- und ein Schwefelatom enthalten, wobei diese Ringe ein bis drei der folgenden Reste tragen können: $C_1$-$C_4$-Alkyl, $C_1$-$C_4$-Halogenalkyl, $C_1$-$C_4$-Alkoxy und $C_1$-$C_4$-Alkylthio;

6- oder 7-gliedrige gesättigte oder ein- oder zweifach ungesättigte Ringe, welche neben Kohlenstoffringgliedern ein oder zwei Sauerstoff- oder Schwefelatome oder oder ein Sauerstoff- und ein Schwefelatom enthalten, wobei diese Ringe ein bis drei der folgenden Reste tragen können: Hydroxy, Halogen, $C_1$-$C_4$-Alkyl, $C_1$-$C_4$-Halogenalkyl, $C_1$-$C_4$-Alkoxy und $C_1$-$C_4$-Alkylthio;

5-gliedrige aromatische Ringe, welche neben Kohlenstoffringgliedern ein oder zwei Stickstoffatome und ein Sauerstoff- oder Schwefelatom oder ein bis drei Stickstoffatome oder ein Sauerstoff- oder ein Schwefelatom enthalten, wobei diese Ringe ein bis drei der folgenden Reste tragen können: Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio, $C_2$-$C_6$-Alkenyl, $C_2$-$C_6$-Alkenyloxy, $C_2$-$C_6$-Alkinyl, $C_2$-$C_6$-Alkinyloxy und $C_1$-$C_4$-Alkoxy-$C_1$-$C_4$-alkyl;

Phenyl oder Pyridyl, wobei diese Ringe ein bis drei der folgenden Reste tragen können: Nitro, Formyl, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$- Alkylthio, $C_2$-$C_6$-Alkenyl, $C_2$-$C_6$-Alkenyloxy, $C_3$-$C_6$-Alkinyl, $C_3$-$C_6$-Alkinyloxy und $NR^kR^l$;

$R^k$ Wasserstoff; $C_1$-$C_4$-Alkyl; $C_3$-$C_6$-Alkenyl; $C_3$-$C_6$-Alkinyl;

$R^l$ Wasserstoff; $C_1$-$C_4$-Alkyl; $C_3$-$C_6$-Alkenyl; $C_3$-$C_6$-Alkinyl; $C_1$-$C_6$-Alkylcarbonyl;

Benzoyl, welches ein bis drei der folgenden Reste tragen kann: Nitro, Cyano, Halogen, $C_1$-$C_4$-Alkyl, $C_1$-$C_4$-Halogenalkyl, $C_1$-$C_4$-Alkoxy und $C_1$-$C_4$-Alkylthio;

$R^d$
Wasserstoff; Hydroxy;

oder, sofern $R^c$ für $C_1$-$C_6$-Alkyl steht, ebenfalls $C_1$-$C_6$-Alkyl;

$R^e$
Wasserstoff; Cyano; Halogen; $C_1$-$C_4$-Alkoxycarbonyl; $C_1$-$C_4$-Alkylketoxim;

W
$C_1$-$C_6$-Alkylen $C_3$-$C_6$-Alkenylen oder $C_3$-$C_6$-Alkinylen, wobei diese Gruppen eine Methylengruppe (= $CH_2$) und/oder ein bis drei der folgenden Reste tragen können: Halogen und $C_1$-$C_3$-Alkyl;

$C_3$-$C_6$-Alkylen oder $C_3$-$C_6$-Alkenylen, wobei in diesen Resten jeweils eine Methylengruppe durch Sauerstoff, Schwefel, SO, $SO_2$ oder $NR^i$ ersetzt ist, und wobei in diesen Gruppen ein bis drei Wasserstoffatome durch $C_1$-$C_3$-Alkylreste ersetzt sein können;

$R^i$ Wasserstoff; $C_1$-$C_4$-Alkyl; $C_3$-$C_6$-Alkenyl; $C_3$-$C_6$-Alkinyl;

$R^f$

Wasserstoff; CH = CH-$Z^1$, worin

$Z^1$ Wasserstoff; Cyano; Carboxyl; Halogen; $C_1$-$C_4$-Alkyl; $C_1$-$C_4$-Halogenalkyl; $C_1$-$C_4$-Alkoxy; $C_1$-$C_8$-Alkoxycarbonyl; Benzyloxycarbonyl;

$C_3$-$C_6$-Cycloalkyl, welches seinerseits ein bis drei der folgenden Reste tragen kann: Hydroxy, Halogen, $C_1$-$C_4$-Alkyl, $C_1$-$C_4$-Halogenalkyl und $C_1$-$C_4$-Alkoxy;

Phenyl, Halogenphenyl, Dihalogenphenyl, Thienyl oder Pyridyl, wobei dieser Reste ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_3$-$C_6$-Cyckloalkyl, wobei der cyclische Rest seinerseits noch ein bis drei der folgenden Gruppen tragen kann: Halogen, $C_1$-$C_4$-Alkyl, $C_1$-$C_4$-Halogenalkyl und $C_1$-$C_4$-Alkoxy, bedeutet;

$R^f$ bedeutet ferner

Ethinyl, welches einen der folgenden Reste tragen kann: $C_1$-$C_4$-Alkyl oder $C_3$-$C_6$-Cyckloalkyl, wobei diese Gruppen desweiteren ein bis drei der folgenden Reste tragen können: Hydroxy, Halogen, $C_1$-$C_4$-Alkyl, $C_1$-$C_4$-Halogenalkyl und $C_1$-$C_4$-Alkoxy;

Ethinyl, welches einen der folgenden Reste trägt: Phenyl, Thienyl oder Pyridyl, wobei die aromatischen Reste ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

Phenyl, 5-gliedrige aromatische Ringe, welche neben Kohlenstoffringgliedern ein oder zwei Stickstoffatome und ein Sauerstoff- oder Schwefelatom oder ein bis drei Stickstoffatome oder ein Sauerstoff- oder ein Schwefelatom enthalten, oder 6-gliedrige aromatische Ringe, welche neben Kohlenstoffringgliedern ein bis vier Stickstoffatome enthalten, wobei diese aromatischen und heteroaromatischen Gruppen ein bis drei der folgenden Reste tragen können: Nitro, $C_1$-$C_4$-Alkoxy, $C_1$-$C_4$-Halogenalkoxy, $C_1$-$C_4$-Alkylthio, $C_1$-$C_4$-Halogenalkylthio, die bei $Z^l$ genannten Reste und $NR^kR^l$, wobei $R^k$ und $R^l$ die vorstehend gegebene Bedeutung haben;

oder

B) der Gruppe der 2-(4-Heteroaryloxy)- oder 2-(4-Aryloxy)-phenoxycarbonsäurederivate der Formel III

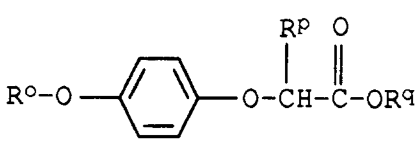

III

in der die Substituenten die folgende Bedeutung haben:

$R^o$

Phenyl, Pyridyl, Benzoxazolyl, Benzthiazolyl oder Benzpyrazinyl, wobei diese aromatischen und heteroaromatischen Ringsysteme ein oder zwei der folgenden Reste tragen können: Nitro, Halogen, $C_1$-$C_4$-Alkyl, $C_1$-$C_4$-Halogenalkyl, $C_1$-$C_4$-Alkoxy, $C_1$-$C_4$-Halogenalkoxy und $C_1$-$C_4$-Alkylthio;

$R^p$

Wasserstoff oder Methyl;

$R^q$

Wasserstoff; $C_1$-$C_4$-Alkyl; $C_3$-$C_4$-Alkenyl; $C_3$-$C_4$-Alkinyl; $C_1$-$C_4$-Alkoxy-$C_1$-$C_4$-alkyl; $C_3$-$C_4$-Alkylideniminooxy-$C_2$-$C_3$-alkyl; Tetrahydrofuranylmethyl; Isoxazolidinyl;

oder das Äquivalent eines landwirtschaftlich brauchbaren Kations.

**2.** Herbizide Mittel nach Anspruch 1, wobei $R^5$ einen der für $R^1$ genannten Reste, Hydroxyl, $-N(R^{12})-SO_2-R^{13}$ oder eine Gruppe $-N(R^{12})-C(X)R^{14}$ bedeutet.

**3.** Herbizide Mittel nach Anspruch 1, wobei $R^1$ und $R^2$ die folgende Bedeutung haben:

Wasserstoff; $C_1$-$C_6$-Alkyl; $C_1$-$C_4$-Halogenalkyl; $C_1$-$C_4$-Alkoxy-$C_1$-$C_6$-alkyl; $C_2$-$C_8$-Alkenyl; $C_2$-$C_8$-Alkinyl;

$C_3$-$C_8$-Cycloalkyl, an welches ein Benzolrest anneliert sein kann, wobei diese Gruppe noch ein bis drei der folgenden Reste tragen kann: Hydroxy, Halogen, $C_1$-$C_4$-Alkyl, $C_1$-$C_4$-Halogenalkyl, $C_1$-$C_4$-Alkoxy und $C_1$-$C_4$-Alkylthio;

Phenyl, Naphthyl, Phenyl-$C_1$-$C_6$-alkyl, 5-gliedrige aromatische Ringe, welche neben Kohlenstoffatomen ein bis drei Stickstoffatome und ein Sauerstoff- oder ein Schwefelatom als Heteroatome enthalten können, oder welche neben Kohlenstoffatomen ein bis drei Stickstoffatome oder ein Sauerstoff- oder ein Schwefelatom als Heteroatome enthalten können, 6-gliedrige aromatische Ringe, welche neben Kohlenstoffatomen ein bis drei Stickstoffatome als Heteroatome enthalten können, wobei an die vorstehend genannten 5- und 6-gliedrigen Heteroaromaten ein Benzolring anneliert sein kann, und wobei die aromatischen und heteroaromatischen Reste zusätzlich ein bis drei der folgenden Gruppen tragen können: Nitro, Cyano, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkoxycarbonyl, $C_1$-$C_4$-Alkyl- thio, $C_3$-$C_6$-Alkenyl und $C_3$-$C_6$-Alkinyl.

**4.** Herbizide Mittel nach den Ansprüchen 1 bis 3, enthaltend mindestens ein substituiertes Pyrido[2,3-d]-pyrimidin I und mindestens ein Herbizid II oder ein Herbizid III im Gewichtsverhältnis 0,01:1 bis 10:1.

**5.** Substituierte Pyrido[2,3-d]pyrimidine der allgemeinen Formel I'

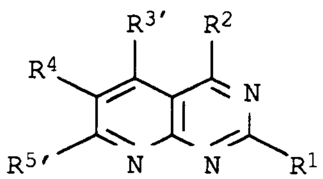

I'

in der die Reste $R^1$, $R^2$ und $R^4$ die in Anspruch 1 gegebene Bedeutung haben und $R^{3'}$ und $R^{5'}$ wie folgt definiert sind:

$R^{3'}$
Halogen; $C_1$-$C_6$-Alkylthio; oder eine der für $R^1$ genannten Gruppen;

$R^{5'}$
eine der für $R^1$ genannten Gruppen;

Hydroxy; Halogen; $C_1$-$C_6$-Alkylthio; $C_1$-$C_8$-Alkylcarbonyloxy; $C_1$-$C_8$-Alkylsulfonyloxy; Phenoxy; Benzoyloxy;

Phenylsulfonyloxy, wobei der aromatische Rest ein bis drei der folgenden Gruppen tragen kann: Halogen, $C_1$-$C_4$-Alkyl, $C_1$-$C_4$-Halogenalkyl und $C_1$-$C_4$-Alkoxy;

mit der Maßgabe, daß $R^1$ und $R^{3'}$ nicht gleichzeitig Wasserstoff bedeuten, wenn $R^2$ für Wasserstoff oder Phenyl und $R^4$ für Phenyl oder $R^{5'}$ für Phenyl, Halogenphenyl, Naphthyl oder Pyridyl steht, und mit der Maßgabe, daß die Reste $R^2$, $R^{3'}$, $R^4$ und $R^{5'}$ nicht gleichzeitig Wasserstoff bedeuten, wenn $R^1$ für Wasserstoff oder Pyridyl steht,

sowie die pflanzenverträglichen Salze derjenigen Verbindungen I', bei denen mindestens einer der Substituenten $R^1$, $R^2$, $R^{3'}$, $R^4$ und $R^{5'}$ eine saure oder basische Gruppe bedeutet.

6. Verfahren zur Herstellung der substituierten Pyrido[2,3-d]pyrimidine I' gemäß Anspruch 5, dadurch gekennzeichnet, daß man ein 4-Aminopyrimidin der Formel IV

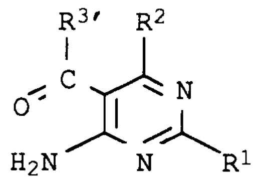

IV

mit einer Methylencarbonylverbindung der Formel V

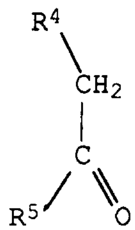

V

oder mit einem Acetonitril der Formel VI

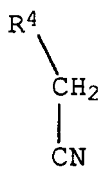

VI

umsetzt und das Verfahrensprodukt gewünschtenfalls in ein anderes Derivat I überführt.

7. Verfahren zur Bekämpfung unerwünschten Pflanzenwuchses, dadurch gekennzeichnet, daß man mindestens ein substituiertes Pyrido[2,3-d]pyrimidin I und mindestens
   A) ein Cyclohexenon-Derivat der Formel II oder
   B) ein 2-(4-Heteroaryloxy)- oder 2-(4-Aryloxy)-phenoxycarbonsäurederivat der Formel III
   gemäß Anspruch 1 vor, bei oder nach der Aussaat der Kulturpflanzen, vor oder während des Auflaufens der Kulturpflanzen gleichzeitig oder nacheinander ausbringt.

8. Verfahren zur selektiven Bekämpfung von unerwünschtem Pflanzenwuchs, dadurch gekennzeichnet, daß man die Blätter der Kulturpflanzen und der unerwünschten Pflanzen im Nachauflaufverfahren mit mindestens einem substituierten Pyrido[2,3-d]pyrimidin I und mindestens
   A) einem Cyclohexenon-Derivat der Formel II oder
   B) einem 2-(4-Heteroaryloxy)- oder 2-(4-Aryloxy)-phenoxycarbonsäurederivatder Formel III
   gemäß Anspruch 1 gleichzeitig oder nacheinander behandelt.

9. Verfahren zur Verhinderung der Schädigung von Kulturpflanzen durch
   A) herbizide Cyclohexenon-Derivate der Formel II oder
   B) herbizide 2-(4-Heteroaryloxy)- oder 2-(4-Aryloxy)-phenoxycarbonsäurederivat der Formel III
   gemäß Anspruch 1, dadurch gekennzeichnet, daß man das Saatgut der Kulturpflanzen mit einer antagonistisch wirksamen Menge eines substituierten Pyrido[2,3-d]pyrimidins der Formel I behandelt.

10. Verfahren gemäß den Ansprüchen 7 bis 9, dadurch gekennzeichnet, daß die Kulturpflanzen Gerste, Weizen, Mais, Kultursorghum und Reis sind.